# EUROPEAN PATENT SPECIFICATION

(11) **EP 2 550 972 B1**
(45) Date of publication and mention of the grant of the patent: **21.02.2018**
(21) Application number: 12179244.4
(22) Date of filing: 01.04.2008
(51) Int. Cl.: A61K 38/17, G01N 33/74, A61P 3/04, A61P 3/06, A61P 3/10, A61P 9/00, A61P 9/12, A61P 11/00, A61P 35/00, A61P 1/16

(54) **A Klotho-beta agonist antibody for use in the treatment of diabetes mellitus or insulin resistance**
Klotho-Beta Antikörper zur Behandlung von Diabetes Mellitus und Insulinresistenz
Un anticorps agoniste de Klotho-béta à utiliser dans le traitement du diabète et de la résistance à l'insuline

(30) Priority: 02.04.2007 US 909699 P; 04.05.2007 US 916187 P
(43) Date of publication of application: 30.01.2013
(62) Divisional of application: 08826247.2
(73) Proprietor: Genentech, Inc., South San Francisco, CA 94080 (US)
(72) Inventor: Desnoyers, Luc, San Francisco, CA California 94133 (US)
(74) Representative: Hayes, Emily Anne Luxford

(56) References cited:
- DATABASE WPI Week 200645 22 June 2006 (2006-06-22) Thomson Scientific, London, GB; AN 2006-437636 XP002689429, -& JP 2006 158339 A (UNIV KYOTO) 22 June 2006 (2006-06-22)
- DATABASE WPI Week 200132 21 March 2001 (2001-03-21) Thomson Scientific, London, GB; AN 2001-304533 XP002689430, -& JP 2001 072607 A (KYOWA HAKKO KOGYO KK) 21 March 2001 (2001-03-21)
- ITO SHINJI ET AL: "Impaired negative feedback suppression of bile acid synthesis in mice lacking beta Klotho", JOURNAL OF CLINICAL INVESTIGATION, vol. 115, no. 8, August 2005 (2005-08), pages 2202-2208, XP002542526, ISSN: 0021-9738
- DATABASE WPI Week 200402 25 November 2003 (2003-11-25) Thomson Scientific, London, GB; AN 2004-015995 XP002689431, -& JP 2003 334088 A (FARUMA DESIGN KK) 25 November 2003 (2003-11-25)
- MOSCHETTA ANTONIO ET AL: "Weaving beta Klotho into bile acid metabolism", JOURNAL OF CLINICAL INVESTIGATION, AMERICAN SOCIETY FOR CLINICAL INVESTIGATION, US, vol. 115, no. 8, 1 August 2005 (2005-08-01), pages 2075-2077, XP002542528, ISSN: 0021-9738, DOI: 10.1172/JCI26046
- YUICHIRO SAITO ET AL: "In Vivo klotho Gene Delivery Protects against Endothelial Dysfunction in Multiple Risk Factor Syndrome", BIOCHEMICAL AND BIOPHYSICAL RESEARCH COMMUNICATIONS, vol. 276, no. 2, 1 September 2000 (2000-09-01), pages 767-772, XP055047794, ISSN: 0006-291X, DOI: 10.1006/bbrc.2000.3470
- ARRESE M ET AL: "Beta.Klotho: a new kid on the bile acid biosynthesis block", HEPATOLOGY, WILEY, USA, vol. 43, no. 1, 1 January 2006 (2006-01-01), pages 191-193, XP002542529, ISSN: 0270-9139, DOI: 10.1002/HEP.21022

### Sequence Listing

<110> GENENTECH, INC.
<120> KLOTHO BETA
<130> EAH/FP6840573
<140> EP
   <141> 2008-04-01
<150> EP 08826247.2
   <151> 2008-04-01
<150> PCT/US2008/059032
   <151> 2008-04-01
<150> US 60/909,699
   <151> 2007-04-02
<150> US 60/916,187
   <151> 2007-05-04
<160> 58
<210> 1
   <211> 3132
   <212> DNA
   <213> Homo sapiens
<400> 1
   atgaagccag gctgtgcggc aggatctcca gggaatgaat ggattttctt 50
   cagcactgat gaaataacca cacgctatag gaatacaatg tccaacgggg 100
   gattgcaaag atctgtcatc ctgtcagcac ttattctgct acgagctgtt 150
   actggattct ctggagatgg aagagctata tggtctaaaa atcctaattt 200
   tactccggta aatgaaagtc agctgtttct ctatgacact ttccctaaaa 250
   actttttctg gggtattggg actggagcat tgcaagtgga agggagttgg 300
   aagaaggatg gaaaaggacc ttctatatgg gatcatttca tccacacaca 350
   ccttaaaaat gtcagcagca cgaatggttc cagtgacagt tatatttttc 400
   tggaaaaaga cttatcagcc ctggatttta taggagtttc tttttatcaa 450
   ttttcaattt cctggccaag gcttttcccc gatggaatag taacagttgc 500
   caacgcaaaa ggtctgcagt actacagtac tcttctggac gctctagtgc 550
   ttagaaacat tgaacctata gttactttat accactggga tttgcctttg 600
   gcactacaag aaaaatatgg ggggtggaaa aatgatacca taatagatat 650
   cttcaatgac tatgccacat actgtttcca gatgtttggg gaccgtgtca 700
   aatattggat tacaattcac aacccatatc tagtggcttg gcatgggtat 750
   gggacaggta tgcatgcccc tggagagaag ggaaatttag cagctgtcta 800
   cactgtggga cacaacttga tcaaggctca ctcgaaagtt tggcataact 850
   acaacacaca tttccgccca catcagaagg gttggttatc gatcacgttg 900
   ggatctcatt ggatcgagcc aaaccggtcg gaaaacacga tggatatatt 950
   caaatgtcaa caatccatgg tttctgtgct tggatggttt gccaacccta 1000
   tccatgggga tggcgactat ccagagggga tgagaaagaa gttgttctcc 1050
   gttctaccca ttttctctga agcagagaag catgagatga gaggcacagc 1100
   tgatttcttt gccttttctt ttggacccaa caacttcaag cccctaaaca 1150
   ccatggctaa aatgggacaa aatgtttcac ttaatttaag agaagcgctg 1200
   aactggatta aactggaata caacaaccct cgaatcttga ttgctgcgaa 1250
   tggctggttc acagacagtc gtgtgaaaac agaagacacc acggccatct 1300
   acatgatgaa gaatttcctc agccaggtgc ttcaagcaat aaggttagat 1350
   gaaatacgag tgtttggtta tactgcctgg tctctcctgg atggctttga 1400
   atggcaggat gcttacacca tccgccgagg attattttat gtggatttta 1450
   acagtaaaca gaaagagcgg aaacctaagt cttcagcaca ctactacaaa 1500
   cagatcatac gagaaaatgg tttttcttta aaagagtcca cgccagatgt 1550
   gcagggccag tttccctgtg acttctcctg gggtgtcact gaatctgttc 1600
   ttaagcccga gtctgtggct tcgtccccac agttcagcga tcctcatctg 1650
   tacgtgtgga acgccactgg caacagactg ttgcaccgag tggaaggggt 1700
   gaggctgaaa acacgacccg ctcaatgcac agattttgta aacatcaaaa 1750
   aacaacttga gatgttggca agaatgaaag tcacccacta ccggtttgct 1800
   ctggattggg cctcggtcct tcccactggc aacctgtccg cggtgaaccg 1850
   acaggccctg aggtactaca ggtgcgtggt cagtgagggg ctgaagcttg 1900
   gcatctccgc gatggtcacc ctgtattatc cgacccacgc ccacctaggc 1950
   ctccccgagc ctctgttgca tgccgacggg tggctgaacc catcgacggc 2000
   cgaggccttc caggcctacg ctgggctgtg cttccaggag ctgggggacc 2050
   tggtgaagct ctggatcacc atcaacgcgc ctaaccggct aagtgacatc 2100
   tacaaccgct ctggcaacga cacctacggg gcggcgcaca acctgctggt 2150
   ggcccacgcc ctggcctggc gcctctacga ccggcagttc aggccctcac 2200
   agcgcggggc cgtgtcgctg tcgctgcacg cggactgggc ggaacccgcc 2250
   aacccctatg ctgactcgca ctggagggcg gccgagcgct tcctgcagtt 2300
   cgagatcgcc tggttcgccg agccgctctt caagaccggg gactaccccg 2350
   cggccatgag ggaatacatt gcctccaagc accgacgggg gctttccagc 2400
   tcggccctgc cgcgcctcac cgaggccgaa aggaggctgc tcaagggcac 2450
   ggtcgacttc tgcgcgctca accacttcac cactaggttc gtgatgcacg 2500
   agcagctggc cggcagccgc tacgactcgg acagggacat ccagtttctg 2550
   caggacatca cccgcctgag ctcccccacg cgcctggctg tgattccctg 2600
   gggggtgcgc aagctgctgc ggtgggtccg gaggaactac ggcgacatgg 2650
   acatttacat caccgccagt ggcatcgacg accaggctct ggaggatgac 2700
   cggctccgga agtactacct agggaagtac cttcaggagg tgctgaaagc 2750
   atacctgatt gataaagtca gaatcaaagg ctattatgca ttcaaactgg 2800
   ctgaagagaa atctaaaccc agatttggat tcttcacatc tgattttaaa 2850
   gctaaatcct caatacaatt ttacaacaaa gtgatcagca gcaggggctt 2900
   cccttttgag aacagtagtt ctagatgcag tcagacccaa gaaaatacag 2950
   agtgcactgt ctgcttattc cttgtgcaga agaaaccact gatattcctg 3000
   ggttgttgct tcttctccac cctggttcta ctcttatcaa ttgccatttt 3050
   tcaaaggcag aagagaagaa agttttggaa agcaaaaaac ttacaacaca 3100
   taccattaaa gaaaggcaag agagttgtta gc 3132
<210> 2
   <211> 1044
   <212> PRT
   <213> Humanized
<400> 2
   Met Lys Pro Gly Cys Ala Ala Gly Ser Pro Gly Asn Glu Trp Ile
    1              5                   10                  15
   Phe Phe Ser Thr Asp Glu Ile Thr Thr Arg Tyr Arg Asn Thr Met
                   20                  25                  30
   Ser Asn Gly Gly Leu Gln Arg Ser Val Ile Leu Ser Ala Leu Ile
                   35                  40                  45
   Leu Leu Arg Ala Val Thr Gly Phe Ser Gly Asp Gly Arg Ala Ile
                   50                  55                  60
   Trp Ser Lys Asn Pro Asn Phe Thr Pro Val Asn Glu Ser Gln Leu
                   65                  70                  75
   Phe Leu Tyr Asp Thr Phe Pro Lys Asn Phe Phe Trp Gly Ile Gly
                   80                  85                  90
   Thr Gly Ala Leu Gln Val Glu Gly Ser Trp Lys Lys Asp Gly Lys
                   95                  100                 105
   Gly Pro Ser Ile Trp Asp His Phe Ile His Thr His Leu Lys Asn
                   110                 115                 120
   Val Ser Ser Thr Asn Gly Ser Ser Asp Ser Tyr Ile Phe Leu Glu
                   125                 130                 135
   Lys Asp Leu Ser Ala Leu Asp Phe Ile Gly Val Ser Phe Tyr Gln
                   140                 145                 150
   Phe Ser Ile Ser Trp Pro Arg Leu Phe Pro Asp Gly Ile Val Thr
                   155                 160                 165
   Val Ala Asn Ala Lys Gly Leu Gln Tyr Tyr Ser Thr Leu Leu Asp
   170 175 180
   Ala Leu Val Leu Arg Asn Ile Glu Pro Ile Val Thr Leu Tyr His
   185 190 195
   Trp Asp Leu Pro Leu Ala Leu Gln Glu Lys Tyr Gly Gly Trp Lys
   200 205 210
   Asn Asp Thr Ile Ile Asp Ile Phe Asn Asp Tyr Ala Thr Tyr Cys
   215 220 225
   Phe Gln Met Phe Gly Asp Arg Val Lys Tyr Trp Ile Thr Ile His
   230 235 240
   Asn Pro Tyr Leu Val Ala Trp His Gly Tyr Gly Thr Gly Met His
   245 250 255
   Ala Pro Gly Glu Lys Gly Asn Leu Ala Ala Val Tyr Thr Val Gly
   260 265 270
   His Asn Leu Ile Lys Ala His Ser Lys Val Trp His Asn Tyr Asn
   275 280 285
   Thr His Phe Arg Pro His Gln Lys Gly Trp Leu Ser Ile Thr Leu
   290 295 300
   Gly Ser His Trp Ile Glu Pro Asn Arg Ser Glu Asn Thr Met Asp
   305 310 315
   Ile Phe Lys Cys Gln Gln Ser Met Val Ser Val Leu Gly Trp Phe
   320 325 330
   Ala Asn Pro Ile His Gly Asp Gly Asp Tyr Pro Glu Gly Met Arg
   335 340 345
   Lys Lys Leu Phe Ser Val Leu Pro Ile Phe Ser Glu Ala Glu Lys
   350 355 360
   His Glu Met Arg Gly Thr Ala Asp Phe Phe Ala Phe Ser Phe Gly
   365 370 375
   Pro Asn Asn Phe Lys Pro Leu Asn Thr Met Ala Lys Met Gly Gln
   380 385 390
   Asn Val Ser Leu Asn Leu Arg Glu Ala Leu Asn Trp Ile Lys Leu
   395 400 405
   Glu Tyr Asn Asn Pro Arg Ile Leu Ile Ala Ala Asn Gly Trp Phe
   410 415 420
   Thr Asp Ser Arg Val Lys Thr Glu Asp Thr Thr Ala Ile Tyr Met
   425 430 435
   Met Lys Asn Phe Leu Ser Gln Val Leu Gln Ala Ile Arg Leu Asp
   440 445 450
   Glu Ile Arg Val Phe Gly Tyr Thr Ala Trp Ser Leu Leu Asp Gly
   455 460 465
   Phe Glu Trp Gln Asp Ala Tyr Thr Ile Arg Arg Gly Leu Phe Tyr
   470 475 480
   Val Asp Phe Asn Ser Lys Gln Lys Glu Arg Lys Pro Lys Ser Ser
   485 490 495
   Ala His Tyr Tyr Lys Gln Ile Ile Arg Glu Asn Gly Phe Ser Leu
   500 505 510
   Lys Glu Ser Thr Pro Asp Val Gln Gly Gln Phe Pro Cys Asp Phe
   515 520 525
   Ser Trp Gly Val Thr Glu Ser Val Leu Lys Pro Glu Ser Val Ala
   530 535 540
   Ser Ser Pro Gln Phe Ser Asp Pro His Leu Tyr Val Trp Asn Ala
   545 550 555
   Thr Gly Asn Arg Leu Leu His Arg Val Glu Gly Val Arg Leu Lys
   560 565 570
   Thr Arg Pro Ala Gln Cys Thr Asp Phe Val Asn Ile Lys Lys Gln
   575 580 585
   Leu Glu Met Leu Ala Arg Met Lys Val Thr His Tyr Arg Phe Ala
   590 595 600
   Leu Asp Trp Ala Ser Val Leu Pro Thr Gly Asn Leu Ser Ala Val
   605 610 615
   Asn Arg Gln Ala Leu Arg Tyr Tyr Arg Cys Val Val Ser Glu Gly
   620 625 630
   Leu Lys Leu Gly Ile Ser Ala Met Val Thr Leu Tyr Tyr Pro Thr
   635 640 645
   His Ala His Leu Gly Leu Pro Glu Pro Leu Leu His Ala Asp Gly
   650 655 660
   Trp Leu Asn Pro Ser Thr Ala Glu Ala Phe Gln Ala Tyr Ala Gly
   665 670 675
   Leu Cys Phe Gln Glu Leu Gly Asp Leu Val Lys Leu Trp Ile Thr
   680 685 690
   Ile Asn Ala Pro Asn Arg Leu Ser Asp Ile Tyr Asn Arg Ser Gly
   695 700 705
   Asn Asp Thr Tyr Gly Ala Ala His Asn Leu Leu Val Ala His Ala
   710 715 720
   Leu Ala Trp Arg Leu Tyr Asp Arg Gln Phe Arg Pro Ser Gln Arg
   725 730 735
   Gly Ala Val Ser Leu Ser Leu His Ala Asp Trp Ala Glu Pro Ala
   740 745 750
   Asn Pro Tyr Ala Asp Ser His Trp Arg Ala Ala Glu Arg Phe Leu
   755 760 765
   Gln Phe Glu Ile Ala Trp Phe Ala Glu Pro Leu Phe Lys Thr Gly
   770 775 780
   Asp Tyr Pro Ala Ala Met Arg Glu Tyr Ile Ala Ser Lys His Arg
   785 790 795
   Arg Gly Leu Ser Ser Ser Ala Leu Pro Arg Leu Thr Glu Ala Glu
   800 805 810
   Arg Arg Leu Leu Lys Gly Thr Val Asp Phe Cys Ala Leu Asn His
   815 820 825
   Phe Thr Thr Arg Phe Val Met His Glu Gln Leu Ala Gly Ser Arg
   830 835 840
   Tyr Asp Ser Asp Arg Asp Ile Gln Phe Leu Gln Asp Ile Thr Arg
   845 850 855
   Leu Ser Ser Pro Thr Arg Leu Ala Val Ile Pro Trp Gly Val Arg
   860 865 870
   Lys Leu Leu Arg Trp Val Arg Arg Asn Tyr Gly Asp Met Asp Ile
   875 880 885
   Tyr Ile Thr Ala Ser Gly Ile Asp Asp Gln Ala Leu Glu Asp Asp
   890 895 900
   Arg Leu Arg Lys Tyr Tyr Leu Gly Lys Tyr Leu Gln Glu Val Leu
   905 910 915
   Lys Ala Tyr Leu Ile Asp Lys Val Arg Ile Lys Gly Tyr Tyr Ala
   920 925 930
   Phe Lys Leu Ala Glu Glu Lys Ser Lys Pro Arg Phe Gly Phe Phe
   935 940 945
   Thr Ser Asp Phe Lys Ala Lys Ser Ser Ile Gln Phe Tyr Asn Lys
   950 955 960
   Val Ile Ser Ser Arg Gly Phe Pro Phe Glu Asn Ser Ser Ser Arg
   965 970 975
   Cys Ser Gln Thr Gln Glu Asn Thr Glu Cys Thr Val Cys Leu Phe
   980 985 990
   Leu Val Gln Lys Lys Pro Leu Ile Phe Leu Gly Cys Cys Phe Phe
   995 1000 1005
   Ser Thr Leu Val Leu Leu Leu Ser Ile Ala Ile Phe Gln Arg Gln
   1010 1015 1020
   Lys Arg Arg Lys Phe Trp Lys Ala Lys Asn Leu Gln His Ile Pro
   1025 1030 1035
   Leu Lys Lys Gly Lys Arg Val Val Ser
   1040
<210> 3
   <211> 34
   <212> DNA
   <213> Artificial sequence
<220>
   <223> sequence is synthesized
<400> 3
   cgggcgctag catgaagcca ggctgtgcgg cagg 34
<210> 4
   <211> 63
   <212> DNA
   <213> Artificial sequence
<220>
   <223> sequence is synthesized
<400> 4
   cagtggatcc ttacttatcg tcgtcatcct tgtaatcgct aacaactctc 50
   ttgcctttct ttc 63
<210> 5
   <211> 37
   <212> DNA
   <213> Artificial sequence
<220>
   <223> sequence is synthesized
<400> 5
   ccgccggata tcatgcggct gctgctggcc ctgttgg 37
<210> 6
   <211> 37
   <212> DNA
   <213> Artificial sequence
<220>
   <223> sequence is synthesized
<400> 6
   ccgccggaat tctgtctgca ccccagaccc gaagggg 37
<210> 7
   <211> 79
   <212> DNA
   <213> Artificial sequence
<220>
   <223> sequence is synthesized
<400> 7
   ggtctggggt gcagacaggt aagcctatcc ctaaccctct cctcggtctc 50
   gattctacgt aggaattcgg atccgcggc 79
<210> 8
   <211> 79
   <212> DNA
   <213> Artificial sequence
<220>
   <223> sequence is synthesized
<400> 8
   gccgcggatc cgaattccta cgtagaatcg agaccgagga gagggttagg 50
   gataggctta cctgtctgca ccccagacc 79
<210> 9
   <211> 41
   <212> DNA
   <213> Artificial sequence
<220>
   <223> sequence is synthesized
<400> 9
   gaattccacc atgaagccag gctgtgcggc aggatctcca g 41
<210> 10
   <211> 37
   <212> DNA
   <213> Artificial sequence
<220>
   <223> sequence is synthesized
<400> 10
   ggcgcgccga caaggaataa gcagacagtg cactctg 37
<210> 11
   <211> 42
   <212> DNA
   <213> Artificial sequence
<220>
   <223> sequence is synthesized
<400> 11
   ccctcgaatc ttgattgctg cgaatggctg gttcacagac ag 42
<210> 12
   <211> 42
   <212> DNA
   <213> Artificial sequence
<220>
   <223> sequence is synthesized
<400> 12
   ctgtctgtga accagccatt cgcagcaatc aagattcgag gg 42
<210> 13
   <211> 40
   <212> DNA
   <213> Artificial sequence
<220>
   <223> sequence is synthesized
<400> 13
   gctctggatc accatcaacg cgcctaaccg gctaagtgac 40
<210> 14
   <211> 40
   <212> DNA
   <213> Artificial sequence
<220>
   <223> sequence is synthesized
<400> 14
   gtcacttagc cggttaggcg cgttgatggt gatccagagc 40
<210> 15
   <211> 19
   <212> DNA
   <213> Artificial sequence
<220>
   <223> sequence is synthesized
<400> 15
   agcggattct catggaaca 19
<210> 16
   <211> 18
   <212> DNA
   <213> Artificial sequence
<220>
   <223> sequence is synthesized
<400> 16
   ctggtcagcc aggagctt 18
<210> 17
   <211> 24
   <212> DNA
   <213> Artificial sequence
<220>
   <223> sequence is synthesized
<400> 17
   tccacaagct gaaggcagac aagg 24
<210> 18
   <211> 25
   <212> DNA
   <213> Artificial sequence
<220>
   <223> sequence is synthesized
<400> 18
   gcagtcagac ccaagaaaat acaga 25
<210> 19
   <211> 24
   <212> DNA
   <213> Artificial sequence
<220>
   <223> sequence is synthesized
<400> 19
   cccaggaata tcagtggttt cttc 24
<210> 20
   <211> 23
   <212> DNA
   <213> Artificial sequence
<220>
   <223> sequence is synthesized
<400> 20
   tgcactgtct gcttattcct tgt 23
<210> 21
   <211> 21
   <212> DNA
   <213> Artificial sequence
<220>
   <223> sequence is synthesized
<400> 21
   cgagcccttt gatgacttcc t 21
<210> 22
   <211> 18
   <212> DNA
   <213> Artificial sequence
<220>
   <223> sequence is synthesized
<400> 22
   ggagcgggct gtctcaga 18
<210> 23
   <211> 21
   <212> DNA
   <213> Artificial sequence
<220>
   <223> sequence is synthesized
<400> 23
   cccagcatca tccaggccca g 21
<210> 24
   <211> 21
   <212> DNA
   <213> Artificial sequence
<220>
   <223> sequence is synthesized
<400> 24
   agtccttcca cctcgacgtt t 21
<210> 25
   <211> 23
   <212> DNA
   <213> Artificial sequence
<220>
   <223> sequence is synthesized
<400> 25
   aatcgagtct gtttccagca gaa 23
<210> 26
   <211> 20
   <212> DNA
   <213> Artificial sequence
<220>
   <223> sequence is synthesized
<400> 26
   agccccccct tccacttttt 20
<210> 27
   <211> 21
   <212> DNA
   <213> Artificial sequence
<220>
   <223> sequence is synthesized
<400> 27
   cgttaacagt gggtgccaac t 21
<210> 28
   <211> 20
   <212> DNA
   <213> Artificial sequence
<220>
   <223> sequence is synthesized
<400> 28
   cccgacggtc tctcttcaaa 20
<210> 29
   <211> 24
   <212> DNA
   <213> Artificial sequence
<220>
   <223> sequence is synthesized
<400> 29
   atgctaacgc agcagttgca aaca 24
<210> 30
   <211> 20
   <212> DNA
   <213> Artificial sequence
<220>
   <223> sequence is synthesized
<400> 30
   tgtggtgagc gaaggactga 20
<210> 31
   <211> 20
   <212> DNA
   <213> Artificial sequence
<220>
   <223> sequence is synthesized
<400> 31
   ggagtgggtt gggtggtaca 20
<210> 32
   <211> 19
   <212> DNA
   <213> Artificial sequence
<220>
   <223> sequence is synthesized
<400> 32
   ctgggcgtct tccccatgg 19
<210> 33
   <211> 18
   <212> DNA
   <213> Artificial sequence
<220>
   <223> sequence is synthesized
<400> 33
   cctgcccctt ctcaacga 18
<210> 34
   <211> 20
   <212> DNA
   <213> Artificial sequence
<220>
   <223> sequence is synthesized
<400> 34
   tccacgttgc tgatgctctt 20
<210> 35
   <211> 22
   <212> DNA
   <213> Artificial sequence
<220>
   <223> sequence is synthesized
<400> 35
   ccaagccatc cttggagcca gt 22
<210> 36
   <211> 22
   <212> DNA
   <213> Artificial sequence
<220>
   <223> sequence is synthesized
<400> 36
   cgccagcctg tcactataca aa 22
<210> 37
   <211> 20
   <212> DNA
   <213> Artificial sequence
<220>
   <223> sequence is synthesized
<400> 37
   ccagaggacc tcgactccaa 20
<210> 38
   <211> 25
   <212> DNA
   <213> Artificial sequence
<220>
   <223> sequence is synthesized
<400> 38
   cgtttccctt tggcccgaca gttct 25
<210> 39
   <211> 21
   <212> RNA
   <213> Artificial sequence
<220>
   <223> sequence is synthesized
<400> 39
   gcacacuacu acaaacagau u 21
<210> 40
   <211> 21
   <212> RNA
   <213> Artificial sequence
<220>
   <223> sequence is synthesized
<400> 40
   ucuguuugua guagugugcu u 21
<210> 41
   <211> 21
   <212> RNA
   <213> Artificial sequence
<220>
   <223> sequence is synthesized
<400> 41
   gcacgaaugg uuccagugau u 21
<210> 42
   <211> 21
   <212> RNA
   <213> Artificial sequence
<220>
   <223> sequence is synthesized
<400> 42
   ucacuggaac cauucgugcu u 21
<210> 43
   <211> 21
   <212> RNA
   <213> Artificial sequence
<220>
   <223> sequence is synthesized
<400> 43
   cgauggauau auucaaaugu u 21
<210> 44
   <211> 21
   <212> RNA
   <213> Artificial sequence
<220>
   <223> sequence is synthesized
<400> 44
   cauuugaaua uauccaucgu u 21
<210> 45
   <211> 21
   <212> RNA
   <213> Artificial sequence
<220>
   <223> sequence is synthesized
<400> 45
   ugaaauaacc acacgcuauu u 21
<210> 46
   <211> 21
   <212> RNA
   <213> Artificial sequence
<220>
   <223> sequence is synthesized
<400> 46
   auagcgugug guuauuucau u 21
<210> 47
   <211> 19
   <212> RNA
   <213> Artificial sequence
<220>
   <223> sequence is synthesized
<400> 47
   ugguuuacau guuccaaua 19
<210> 48
   <211> 19
   <212> RNA
   <213> Artificial sequence
<220>
   <223> sequence is synthesized
<400> 48
   uauuggaaca uguaaacca 19
<210> 49
   <211> 3159
   <212> DNA
   <213> Artificial sequence
<220>
   <223> sequence is synthesized
<400> 49
   atgaagccag gctgtgcggc aggatctcca gggaatgaat ggattttctt 50
   cagcactgat gaaataacca cacgctatag gaatacaatg tccaacgggg 100
   gattgcaaag atctgtcatc ctgtcagcac ttattctgct acgagctgtt 150
   actggattct ctggagatgg aagagctata tggtctaaaa atcctaattt 200
   tactccggta aatgaaagtc agctgtttct ctatgacact ttccctaaaa 250
   actttttctg ggtattgggg actggagcat tgcaagtgga agggagttgg 300
   aagaaggatg gaaaaggacc ttctatatgg gatcatttca tccacacaca 350
   ccttaaaaat gtcagcagca cgaatggttc cagtgacagt tatatttttc 400
   tggaaaaaga cttatcagcc ctggatttta taggagtttc tttttatcaa 450
   ttttcaattt cctggccaag gcttttcccc gatggaatag taacagttgc 500
   caacgcaaaa ggtctgcagt actacagtac tcttctggac gctctagtgc 550
   ttagaaacat tgaacctata gttactttat accactggga tttgcctttg 600
   gcactacaag aaaaatatgg ggggtggaaa aatgatacca taatagatat 650
   cttcaatgac tatgccacat actgtttcca gatgtttggg gaccgtgtca 700
   aatattggat tacaattcac aacccatatc tagtggcttg gcatgggtat 750
   gggacaggta tgcatgcccc tggagagaag ggaaatttag cagctgtcta 800
   cactgtggga cacaacttga tcaaggctca ctcgaaagtt tggcataact 850
   acaacacaca tttccgccca catcagaagg gttggttatc gatcacgttg 900
   ggatctcatt ggatcgagcc aaaccggtcg gaaaacacga tggatatatt 950
   caaatgtcaa caatccatgg tttctgtgct tggatggttt gccaacccta 1000
   tccatgggga tggcgactat ccagagggga tgagaaagaa gttgttctcc 1050
   gttctaccca ttttctctga agcagagaag catgagatga gaggcacagc 1100
   tgatttcttt gccttttctt ttggacccaa caacttcaag cccctaaaca 1150
   ccatggctaa aatgggacaa aatgtttcac ttaatttaag agaagcgctg 1200
   aactggatta aactggaata caacaaccct cgaatcttga ttgctgagaa 1250
   tggctggttc acagacagtc gtgtgaaaac agaagacacc acggccatct 1300
   acatgatgaa gaatttcctc agccaggtgc ttcaagcaat aaggttagat 1350
   gaaatacgag tgtttggtta tactgcctgg tctctcctgg atggctttga 1400
   atggcaggat gcttacacca tccgccgagg attattttat gtggatttta 1450
   acagtaaaca gaaagagcgg aaacctaagt cttcagcaca ctactacaaa 1500
   cagatcatac gagaaaatgg tttttcttta aaagagtcca cgccagatgt 1550
   gcagggccag tttccctgtg acttctcctg gggtgtcact gaatctgttc 1600
   ttaagcccga gtctgtggct tcgtccccac agttcagcga tcctcatctg 1650
   tacgtgtgga acgccactgg caacagactg ttgcaccgag tggaaggggt 1700
   gaggctgaaa acacgacccg ctcaatgcac agattttgta aacatcaaaa 1750
   aacaacttga gatgttggca agaatgaaag tcacccacta ccggtttgct 1800
   ctggattggg cctcggtcct tcccactggc aacctgtccg cggtgaaccg 1850
   acaggccctg aggtactaca ggtgcgtggt cagtgagggg ctgaagcttg 1900
   gcatctccgc gatggtcacc ctgtattatc cgacccacgc ccacctaggc 1950
   ctccccgagc ctctgttgca tgccgacggg tggctgaacc catcgacggc 2000
   cgaggccttc caggcctacg ctgggctgtg cttccaggag ctgggggacc 2050
   tggtgaagct ctggatcacc atcaacgagc ctaaccggct aagtgacatc 2100
   tacaaccgct ctggcaacga cacctacggg gcggcgcaca acctgctggt 2150
   ggcccacgcc ctggcctggc gcctctacga ccggcagttc aggccctcac 2200
   agcgcggggc cgtgtcgctg tcgctgcacg cggactgggc ggaacccgcc 2250
   aacccctatg ctgactcgca ctggagggcg gccgagcgct tcctgcagtt 2300
   cgagatcgcc tggttcgccg agccgctctt caagaccggg gactaccccg 2350
   cggccatgag ggaatacatt gcctccaagc accgacgggg gctttccagc 2400
   tcggccctgc cgcgcctcac cgaggccgaa aggaggctgc tcaagggcac 2450
   ggtcgacttc tgcgcgctca accacttcac cactaggttc gtgatgcacg 2500
   agcagctggc cggcagccgc tacgactcgg acagggacat ccagtttctg 2550
   caggacatca cccgcctgag ctcccccacg cgcctggctg tgattccctg 2600
   gggggtgcgc aagctgctgc ggtgggtccg gaggaactac ggcgacatgg 2650
   acatttacat caccgccagt ggcatcgacg accaggctct ggaggatgac 2700
   cggctccgga agtactacct agggaagtac cttcaggagg tgctgaaagc 2750
   atacctgatt gataaagtca gaatcaaagg ctattatgca ttcaaactgg 2800
   ctgaagagaa atctaaaccc agatttggat tcttcacatc tgattttaaa 2850
   gctaaatcct caatacaatt ttacaacaaa gtgatcagca gcaggggctt 2900
   cccttttgag aacagtagtt ctagatgcag tcagacccaa gaaaatacag 2950
   agtgcactgt ctgcttattc cttgtgcaga agaaaccact gatattcctg 3000
   ggttgttgct tcttctccac cctggttcta ctcttatcaa ttgccatttt 3050
   tcaaaggcag aagagaagaa agttttggaa agcaaaaaac ttacaacaca 3100
   taccattaaa gaaaggcaag agagttgtta gcgattacaa ggatgacgac 3150
   gataagtaa 3159
<210> 50
   <211> 3012
   <212> DNA
   <213> Artificial sequence
<220>
   <223> sequence is synthesized
<400> 50
   atgaagccag gctgtgcggc aggatctcca gggaatgaat ggattttctt 50
   cagcactgat gaaataacca cacgctatag gaatacaatg tccaacgggg 100
   gattgcaaag atctgtcatc ctgtcagcac ttattctgct acgagctgtt 150
   actggattct ctggagatgg aagagctata tggtctaaaa atcctaattt 200
   tactccggta aatgaaagtc agctgtttct ctatgacact ttccctaaaa 250
   actttttctg gggtattggg actggagcat tgcaagtgga agggagttgg 300
   aagaaggatg gaaaaggacc ttctatatgg gatcatttca tccacacaca 350
   ccttaaaaat gtcagcagca cgaatggttc cagtgacagt tatatttttc 400
   tggaaaaaga cttatcagcc ctggatttta taggagtttc tttttatcaa 450
   ttttcaattt cctggccaag gcttttcccc gatggaatag taacagttgc 500
   caacgcaaaa ggtctgcagt actacagtac tcttctggac gctctagtgc 550
   ttagaaacat tgaacctata gttactttat accactggga tttgcctttg 600
   gcactacaag aaaaatatgg ggggtggaaa aatgatacca taatagatat 650
   cttcaatgac tatgccacat actgtttcca gatgtttggg gaccgtgtca 700
   aatattggat tacaattcac aacccatatc tagtggcttg gcatgggtat 750
   gggacaggta tgcatgcccc tggagagaag ggaaatttag cagctgtcta 800
   cactgtggga cacaacttga tcaaggctca ctcgaaagtt tggcataact 850
   acaacacaca tttccgccca catcagaagg gttggttatc gatcacgttg 900
   ggatctcatt ggatcgagcc aaaccggtcg gaaaacacga tggatatatt 950
   caaatgtcaa caatccatgg tttctgtgct tggatggttt gccaacccta 1000
   tccatgggga tggcgactat ccagagggga tgagaaagaa gttgttctcc 1050
   gttctaccca ttttctctga agcagagaag catgagatga gaggcacagc 1100
   tgatttcttt gccttttctt ttggacccaa caacttcaag cccctaaaca 1150
   ccatggctaa aatgggacaa aatgtttcac ttaatttaag agaagcgctg 1200
   aactggatta aactggaata caacaaccct cgaatcttga ttgctgagaa 1250
   tggctggttc acagacagtc gtgtgaaaac agaagacacc acggccatct 1300
   acatgatgaa gaatttcctc agccaggtgc ttcaagcaat aaggttagat 1350
   gaaatacgag tgtttggtta tactgcctgg tctctcctgg atggctttga 1400
   atggcaggat gcttacacca tccgccgagg attattttat gtggatttta 1450
   acagtaaaca gaaagagcgg aaacctaagt cttcagcaca ctactacaaa 1500
   cagatcatac gagaaaatgg tttttcttta aaagagtcca cgccagatgt 1550
   gcagggccag tttccctgtg acttctcctg gggtgtcact gaatctgttc 1600
   ttaagcccga gtctgtggct tcgtccccac agttcagcga tcctcatctg 1650
   tacgtgtgga acgccactgg caacagactg ttgcaccgag tggaaggggt 1700
   gaggctgaaa acacgacccg ctcaatgcac agattttgta aacatcaaaa 1750
   aacaacttga gatgttggca agaatgaaag tcacccacta ccggtttgct 1800
   ctggattggg cctcggtcct tcccactggc aacctgtccg cggtgaaccg 1850
   acaggccctg aggtactaca ggtgcgtggt cagtgagggg ctgaagcttg 1900
   gcatctccgc gatggtcacc ctgtattatc cgacccacgc ccacctaggc 1950
   ctccccgagc ctctgttgca tgccgacggg tggctgaacc catcgacggc 2000
   cgaggccttc caggcctacg ctgggctgtg cttccaggag ctgggggacc 2050
   tggtgaagct ctggatcacc atcaacgagc ctaaccggct aagtgacatc 2100
   tacaaccgct ctggcaacga cacctacggg gcggcgcaca acctgctggt 2150
   ggcccacgcc ctggcctggc gcctctacga ccggcagttc aggccctcac 2200
   agcgcggggc cgtgtcgctg tcgctgcacg cggactgggc ggaacccgcc 2250
   aacccctatg ctgactcgca ctggagggcg gccgagcgct tcctgcagtt 2300
   cgagatcgcc tggttcgccg agccgctctt caagaccggg gactaccccg 2350
   cggccatgag ggaatacatt gcctccaagc accgacgggg gctttccagc 2400
   tcggccctgc cgcgcctcac cgaggccgaa aggaggctgc tcaagggcac 2450
   ggtcgacttc tgcgcgctca accacttcac cactaggttc gtgatgcacg 2500
   agcagctggc cggcagccgc tacgactcgg acagggacat ccagtttctg 2550
   caggacatca cccgcctgag ctcccccacg cgcctggctg tgattccctg 2600
   gggggtgcgc aagctgctgc ggtgggtccg gaggaactac ggcgacatgg 2650
   acatttacat caccgccagt ggcatcgacg accaggctct ggaggatgac 2700
   cggctccgga agtactacct agggaagtac cttcaggagg tgctgaaagc 2750
   atacctgatt gataaagtca gaatcaaagg ctattatgca ttcaaactgg 2800
   ctgaagagaa atctaaaccc agatttggat tcttcacatc tgattttaaa 2850
   gctaaatcct caatacaatt ttacaacaaa gtgatcagca gcaggggctt 2900
   cccttttgag aacagtagtt ctagatgcag tcagacccaa gaaaatacag 2950
   agtgcactgt ctgcttattc cttgtcggcg cgccccatca tcatcatcat 3000
   catcaccact aa 3012
<210> 51
   <211> 3159
   <212> DNA
   <213> Artificial sequence
<220>
   <223> sequence is synthesized
<400> 51
   atgaagccag gctgtgcggc aggatctcca gggaatgaat ggattttctt 50
   cagcactgat gaaataacca cacgctatag gaatacaatg tccaacgggg 100
   gattgcaaag atctgtcatc ctgtcagcac ttattctgct acgagctgtt 150
   actggattct ctggagatgg aagagctata tggtctaaaa atcctaattt 200
   tactccggta aatgaaagtc agctgtttct ctatgacact ttccctaaaa 250
   actttttctg gggtattggg actggagcat tgcaagtgga agggagttgg 300
   aagaaggatg gaaaaggacc ttctatatgg gatcatttca tccacacaca 350
   ccttaaaaat gtcagcagca cgaatggttc cagtgacagt tatatttttc 400
   tggaaaaaga cttatcagcc ctggatttta taggagtttc tttttatcaa 450
   ttttcaattt cctggccaag gcttttcccc gatggaatag taacagttgc 500
   caacgcaaaa ggtctgcagt actacagtac tcttctggac gctctagtgc 550
   ttagaaacat tgaacctata gttactttat accactggga tttgcctttg 600
   gcactacaag aaaaatatgg ggggtggaaa aatgatacca taatagatat 650
   cttcaatgac tatgccacat actgtttcca gatgtttggg gaccgtgtca 700
   aatattggat tacaattcac aacccatatc tagtggcttg gcatgggtat 750
   gggacaggta tgcatgcccc tggagagaag ggaaatttag cagctgtcta 800
   cactgtggga cacaacttga tcaaggctca ctcgaaagtt tggcataact 850
   acaacacaca tttccgccca catcagaagg gttggttatc gatcacgttg 900
   ggatctcatt ggatcgagcc aaaccggtcg gaaaacacga tggatatatt 950
   caaatgtcaa caatccatgg tttctgtgct tggatggttt gccaacccta 1000
   tccatgggga tggcgactat ccagagggga tgagaaagaa gttgttctcc 1050
   gttctaccca ttttctctga agcagagaag catgagatga gaggcacagc 1100
   tgatttcttt gccttttctt ttggacccaa caacttcaag cccctaaaca 1150
   ccatggctaa aatgggacaa aatgtttcac ttaatttaag agaagcgctg 1200
   aactggatta aactggaata caacaaccct cgaatcttga ttgctgcgaa 1250
   tggctggttc acagacagtc gtgtgaaaac agaagacacc acggccatct 1300
   acatgatgaa gaatttcctc agccaggtgc ttcaagcaat aaggttagat 1350
   gaaatacgag tgtttggtta tactgcctgg tctctcctgg atggctttga 1400
   atggcaggat gcttacacca tccgccgagg attattttat gtggatttta 1450
   acagtaaaca gaaagagcgg aaacctaagt cttcagcaca ctactacaaa 1500
   cagatcatac gagaaaatgg tttttcttta aaagagtcca cgccagatgt 1550
   gcagggccag tttccctgtg acttctcctg gggtgtcact gaatctgttc 1600
   ttaagcccga gtctgtggct tcgtccccac agttcagcga tcctcatctg 1650
   tacgtgtgga acgccactgg caacagactg ttgcaccgag tggaaggggt 1700
   gaggctgaaa acacgacccg ctcaatgcac agattttgta aacatcaaaa 1750
   aacaacttga gatgttggca agaatgaaag tcacccacta ccggtttgct 1800
   ctggattggg cctcggtcct tcccactggc aacctgtccg cggtgaaccg 1850
   acaggccctg aggtactaca ggtgcgtggt cagtgagggg ctgaagcttg 1900
   gcatctccgc gatggtcacc ctgtattatc cgacccacgc ccacctaggc 1950
   ctccccgagc ctctgttgca tgccgacggg tggctgaacc catcgacggc 2000
   cgaggccttc caggcctacg ctgggctgtg cttccaggag ctgggggacc 2050
   tggtgaagct ctggatcacc atcaacgagc ctaaccggct aagtgacatc 2100
   tacaaccgct ctggcaacga cacctacggg gcggcgcaca acctgctggt 2150
   ggcccacgcc ctggcctggc gcctctacga ccggcagttc aggccctcac 2200
   agcgcggggc cgtgtcgctg tcgctgcacg cggactgggc ggaacccgcc 2250
   aacccctatg ctgactcgca ctggagggcg gccgagcgct tcctgcagtt 2300
   cgagatcgcc tggttcgccg agccgctctt caagaccggg gactaccccg 2350
   cggccatgag ggaatacatt gcctccaagc accgacgggg gctttccagc 2400
   tcggccctgc cgcgcctcac cgaggccgaa aggaggctgc tcaagggcac 2450
   ggtcgacttc tgcgcgctca accacttcac cactaggttc gtgatgcacg 2500
   agcagctggc cggcagccgc tacgactcgg acagggacat ccagtttctg 2550
   caggacatca cccgcctgag ctcccccacg cgcctggctg tgattccctg 2600
   gggggtgcgc aagctgctgc ggtgggtccg gaggaactac ggcgacatgg 2650
   acatttacat caccgccagt ggcatcgacg accaggctct ggaggatgac 2700
   cggctccgga agtactacct agggaagtac cttcaggagg tgctgaaagc 2750
   atacctgatt gataaagtca gaatcaaagg ctattatgca ttcaaactgg 2800
   ctgaagagaa atctaaaccc agatttggat tcttcacatc tgattttaaa 2850
   gctaaatcct caatacaatt ttacaacaaa gtgatcagca gcaggggctt 2900
   cccttttgag aacagtagtt ctagatgcag tcagacccaa gaaaatacag 2950
   agtgcactgt ctgcttattc cttgtgcaga agaaaccact gatattcctg 3000
   ggttgttgct tcttctccac cctggttcta ctcttatcaa ttgccatttt 3050
   tcaaaggcag aagagaagaa agttttggaa agcaaaaaac ttacaacaca 3100
   taccattaaa gaaaggcaag agagttgtta gcgattacaa ggatgacgac 3150
   gataagtaa 3159
<210> 52
   <211> 3159
   <212> DNA
   <213> Artificial sequence
<220>
   <223> sequence is synthesized
<400> 52
   atgaagccag gctgtgcggc aggatctcca gggaatgaat ggattttctt 50
   cagcactgat gaaataacca cacgctatag gaatacaatg tccaacgggg 100
   gattgcaaag atctgtcatc ctgtcagcac ttattctgct acgagctgtt 150
   actggattct ctggagatgg aagagctata tggtctaaaa atcctaattt 200
   tactccggta aatgaaagtc agctgtttct ctatgacact ttccctaaaa 250
   actttttctg gggtattggg actggagcat tgcaagtgga agggagttgg 300
   aagaaggatg gaaaaggacc ttctatatgg gatcatttca tccacacaca 350
   ccttaaaaat gtcagcagca cgaatggttc cagtgacagt tatatttttc 400
   tggaaaaaga cttatcagcc ctggatttta taggagtttc tttttatcaa 450
   ttttcaattt cctggccaag gcttttcccc gatggaatag taacagttgc 500
   caacgcaaaa ggtctgcagt actacagtac tcttctggac gctctagtgc 550
   ttagaaacat tgaacctata gttactttat accactggga tttgcctttg 600
   gcactacaag aaaaatatgg ggggtggaaa aatgatacca taatagatat 650
   cttcaatgac tatgccacat actgtttcca gatgtttggg gaccgtgtca 700
   aatattggat tacaattcac aacccatatc tagtggcttg gcatgggtat 750
   gggacaggta tgcatgcccc tggagagaag ggaaatttag cagctgtcta 800
   cactgtggga cacaacttga tcaaggctca ctcgaaagtt tggcataact 850
   acaacacaca tttccgccca catcagaagg gttggttatc gatcacgttg 900
   ggatctcatt ggatcgagcc aaaccggtcg gaaaacacga tggatatatt 950
   caaatgtcaa caatccatgg tttctgtgct tggatggttt gccaacccta 1000
   tccatgggga tggcgactat ccagagggga tgagaaagaa gttgttctcc 1050
   gttctaccca ttttctctga agcagagaag catgagatga gaggcacagc 1100
   tgatttcttt gccttttctt ttggacccaa caacttcaag cccctaaaca 1150
   ccatggctaa aatgggacaa aatgtttcac ttaatttaag agaagcgctg 1200
   aactggatta aactggaata caacaaccct cgaatcttga ttgctgcgaa 1250
   tggctggttc acagacagtc gtgtgaaaac agaagacacc acggccatct 1300
   acatgatgaa gaatttcctc agccaggtgc ttcaagcaat aaggttagat 1350
   gaaatacgag tgtttggtta tactgcctgg tctctcctgg atggctttga 1400
   atggcaggat gcttacacca tccgccgagg attattttat gtggatttta 1450
   acagtaaaca gaaagagcgg aaacctaagt cttcagcaca ctactacaaa 1500
   cagatcatac gagaaaatgg tttttcttta aaagagtcca cgccagatgt 1550
   gcagggccag tttccctgtg acttctcctg gggtgtcact gaatctgttc 1600
   ttaagcccga gtctgtggct tcgtccccac agttcagcga tcctcatctg 1650
   tacgtgtgga acgccactgg caacagactg ttgcaccgag tggaaggggt 1700
   gaggctgaaa acacgacccg ctcaatgcac agattttgta aacatcaaaa 1750
   aacaacttga gatgttggca agaatgaaag tcacccacta ccggtttgct 1800
   ctggattggg cctcggtcct tcccactggc aacctgtccg cggtgaaccg 1850
   acaggccctg aggtactaca ggtgcgtggt cagtgagggg ctgaagcttg 1900
   gcatctccgc gatggtcacc ctgtattatc cgacccacgc ccacctaggc 1950
   ctccccgagc ctctgttgca tgccgacggg tggctgaacc catcgacggc 2000
   cgaggccttc caggcctacg ctgggctgtg cttccaggag ctgggggacc 2050
   tggtgaagct ctggatcacc atcaacgcgc ctaaccggct aagtgacatc 2100
   tacaaccgct ctggcaacga cacctacggg gcggcgcaca acctgctggt 2150
   ggcccacgcc ctggcctggc gcctctacga ccggcagttc aggccctcac 2200
   agcgcggggc cgtgtcgctg tcgctgcacg cggactgggc ggaacccgcc 2250
   aacccctatg ctgactcgca ctggagggcg gccgagcgct tcctgcagtt 2300
   cgagatcgcc tggttcgccg agccgctctt caagaccggg gactaccccg 2350
   cggccatgag ggaatacatt gcctccaagc accgacgggg gctttccagc 2400
   tcggccctgc cgcgcctcac cgaggccgaa aggaggctgc tcaagggcac 2450
   ggtcgacttc tgcgcgctca accacttcac cactaggttc gtgatgcacg 2500
   agcagctggc cggcagccgc tacgactcgg acagggacat ccagtttctg 2550
   caggacatca cccgcctgag ctcccccacg cgcctggctg tgattccctg 2600
   gggggtgcgc aagctgctgc ggtgggtccg gaggaactac ggcgacatgg 2650
   acatttacat caccgccagt ggcatcgacg accaggctct ggaggatgac 2700
   cggctccgga agtactacct agggaagtac cttcaggagg tgctgaaagc 2750
   atacctgatt gataaagtca gaatcaaagg ctattatgca ttcaaactgg 2800
   ctgaagagaa atctaaaccc agatttggat tcttcacatc tgattttaaa 2850
   gctaaatcct caatacaatt ttacaacaaa gtgatcagca gcaggggctt 2900
   cccttttgag aacagtagtt ctagatgcag tcagacccaa gaaaatacag 2950
   agtgcactgt ctgcttattc cttgtgcaga agaaaccact gatattcctg 3000
   ggttgttgct tcttctccac cctggttcta ctcttatcaa ttgccatttt 3050
   tcaaaggcag aagagaagaa agttttggaa agcaaaaaac ttacaacaca 3100
   taccattaaa gaaaggcaag agagttgtta gcgattacaa ggatgacgac 3150
   gataagtaa 3159
<210> 53
   <211> 11
   <212> PRT
   <213> Artificial sequence
<220>
   <223> sequence was synthesized
<400> 53
   Lys Ala Ser Gln Asp Ile Asn Ser Phe Leu Ala
   5 10
<210> 54
   <211> 7
   <212> PRT
   <213> Artificial sequence
<220>
   <223> sequence was synthesized
<400> 54
   Arg Ala Asn Arg Leu Val Ser
   5
<210> 55
   <211> 9
   <212> PRT
   <213> Mus musculus
<400> 55
   Leu Gln Tyr Asp Glu Phe Pro Leu Thr
   5
<210> 56
   <211> 10
   <212> PRT
   <213> Mus musculus
<400> 56
   Gly Phe Ser Leu Thr Thr Tyr Gly Val His
   5 10
<210> 57
   <211> 17
   <212> PRT
   <213> Mus musculus
<400> 57
   Gly Val Ile Trp Pro Gly Gly Gly Thr Asp Tyr Asn Ala Ala Phe
   1 5 10 15
   Ile Ser
<210> 58
   <211> 10
   <212> PRT
   <213> Mus musculus
<400> 58
   Val Arg Lys Glu Tyr Ala Asn Leu Tyr Ala
                    5                  10

### Sequence Listing

<110> GENENTECH, INC.
<120> KLOTHO BETA
<130> EAH/FP6840573
<140> EP
   <141> 2008-04-01
<150> EP 08826247.2
   <151> 2008-04-01
<150> PCT/US2008/059032
   <151> 2008-04-01
<150> US 60/909,699
   <151> 2007-04-02
<150> US 60/916,187
   <151> 2007-05-04
<160> 58
<210> 1
   <211> 3132
   <212> DNA
   <213> Homo sapiens
<400> 1
   atgaagccag gctgtgcggc aggatctcca gggaatgaat ggattttctt 50
   cagcactgat gaaataacca cacgctatag gaatacaatg tccaacgggg 100
   gattgcaaag atctgtcatc ctgtcagcac ttattctgct acgagctgtt 150
   actggattct ctggagatgg aagagctata tggtctaaaa atcctaattt 200
   tactccggta aatgaaagtc agctgtttct ctatgacact ttccctaaaa 250
   actttttctg gggtattggg actggagcat tgcaagtgga agggagttgg 300
   aagaaggatg gaaaaggacc ttctatatgg gatcatttca tccacacaca 350
   ccttaaaaat gtcagcagca cgaatggttc cagtgacagt tatatttttc 400
   tggaaaaaga cttatcagcc ctggatttta taggagtttc tttttatcaa 450
   ttttcaattt cctggccaag gcttttcccc gatggaatag taacagttgc 500
   caacgcaaaa ggtctgcagt actacagtac tcttctggac gctctagtgc 550
   ttagaaacat tgaacctata gttactttat accactggga tttgcctttg 600
   gcactacaag aaaaatatgg ggggtggaaa aatgatacca taatagatat 650
   cttcaatgac tatgccacat actgtttcca gatgtttggg gaccgtgtca 700
   aatattggat tacaattcac aacccatatc tagtggcttg gcatgggtat 750
   gggacaggta tgcatgcccc tggagagaag ggaaatttag cagctgtcta 800
   cactgtggga cacaacttga tcaaggctca ctcgaaagtt tggcataact 850
   acaacacaca tttccgccca catcagaagg gttggttatc gatcacgttg 900
   ggatctcatt ggatcgagcc aaaccggtcg gaaaacacga tggatatatt 950
   caaatgtcaa caatccatgg tttctgtgct tggatggttt gccaacccta 1000
   tccatgggga tggcgactat ccagagggga tgagaaagaa gttgttctcc 1050
   gttctaccca ttttctctga agcagagaag catgagatga gaggcacagc 1100
   tgatttcttt gccttttctt ttggacccaa caacttcaag cccctaaaca 1150
   ccatggctaa aatgggacaa aatgtttcac ttaatttaag agaagcgctg 1200
   aactggatta aactggaata caacaaccct cgaatcttga ttgctgcgaa 1250
   tggctggttc acagacagtc gtgtgaaaac agaagacacc acggccatct 1300
   acatgatgaa gaatttcctc agccaggtgc ttcaagcaat aaggttagat 1350
   gaaatacgag tgtttggtta tactgcctgg tctctcctgg atggctttga 1400
   atggcaggat gcttacacca tccgccgagg attattttat gtggatttta 1450
   acagtaaaca gaaagagcgg aaacctaagt cttcagcaca ctactacaaa 1500
   cagatcatac gagaaaatgg tttttcttta aaagagtcca cgccagatgt 1550
   gcagggccag tttccctgtg acttctcctg gggtgtcact gaatctgttc 1600
   ttaagcccga gtctgtggct tcgtccccac agttcagcga tcctcatctg 1650
   tacgtgtgga acgccactgg caacagactg ttgcaccgag tggaaggggt 1700
   gaggctgaaa acacgacccg ctcaatgcac agattttgta aacatcaaaa 1750
   aacaacttga gatgttggca agaatgaaag tcacccacta ccggtttgct 1800
   ctggattggg cctcggtcct tcccactggc aacctgtccg cggtgaaccg 1850
   acaggccctg aggtactaca ggtgcgtggt cagtgagggg ctgaagcttg 1900
   gcatctccgc gatggtcacc ctgtattatc cgacccacgc ccacctaggc 1950
   ctccccgagc ctctgttgca tgccgacggg tggctgaacc catcgacggc 2000
   cgaggccttc caggcctacg ctgggctgtg cttccaggag ctgggggacc 2050
   tggtgaagct ctggatcacc atcaacgcgc ctaaccggct aagtgacatc 2100
   tacaaccgct ctggcaacga cacctacggg gcggcgcaca acctgctggt 2150
   ggcccacgcc ctggcctggc gcctctacga ccggcagttc aggccctcac 2200
   agcgcggggc cgtgtcgctg tcgctgcacg cggactgggc ggaacccgcc 2250
   aacccctatg ctgactcgca ctggagggcg gccgagcgct tcctgcagtt 2300
   cgagatcgcc tggttcgccg agccgctctt caagaccggg gactaccccg 2350
   cggccatgag ggaatacatt gcctccaagc accgacgggg gctttccagc 2400
   tcggccctgc cgcgcctcac cgaggccgaa aggaggctgc tcaagggcac 2450
   ggtcgacttc tgcgcgctca accacttcac cactaggttc gtgatgcacg 2500
   agcagctggc cggcagccgc tacgactcgg acagggacat ccagtttctg 2550
   caggacatca cccgcctgag ctcccccacg cgcctggctg tgattccctg 2600
   gggggtgcgc aagctgctgc ggtgggtccg gaggaactac ggcgacatgg 2650
   acatttacat caccgccagt ggcatcgacg accaggctct ggaggatgac 2700
   cggctccgga agtactacct agggaagtac cttcaggagg tgctgaaagc 2750
   atacctgatt gataaagtca gaatcaaagg ctattatgca ttcaaactgg 2800
   ctgaagagaa atctaaaccc agatttggat tcttcacatc tgattttaaa 2850
   gctaaatcct caatacaatt ttacaacaaa gtgatcagca gcaggggctt 2900
   cccttttgag aacagtagtt ctagatgcag tcagacccaa gaaaatacag 2950
   agtgcactgt ctgcttattc cttgtgcaga agaaaccact gatattcctg 3000
   ggttgttgct tcttctccac cctggttcta ctcttatcaa ttgccatttt 3050
   tcaaaggcag aagagaagaa agttttggaa agcaaaaaac ttacaacaca 3100
   taccattaaa gaaaggcaag agagttgtta  gc 3132
<210> 2
   <211> 1044
   <212> PRT
   <213> Humanized
<400> 2
   Met Lys Pro Gly Cys Ala Ala Gly Ser Pro Gly Asn Glu Trp Ile
    1               5                  10                  15
   Phe Phe Ser Thr Asp Glu Ile Thr Thr Arg Tyr Arg Asn Thr Met
                   20                  25                  30
   Ser Asn Gly Gly Leu Gln Arg Ser Val Ile Leu Ser Ala Leu Ile
                   35                  40                  45
   Leu Leu Arg Ala Val Thr Gly Phe Ser Gly Asp Gly Arg Ala Ile
                   50                  55                  60
   Trp Ser Lys Asn Pro Asn Phe Thr Pro Val Asn Glu Ser Gln Leu
                   65                  70                  75
   Phe Leu Tyr Asp Thr Phe Pro Lys Asn Phe Phe Trp Gly Ile Gly
                   80                  85                  90
   Thr Gly Ala Leu Gln Val Glu Gly Ser Trp Lys Lys Asp Gly Lys
                   95                 100                 105
   Gly Pro Ser Ile Trp Asp His Phe Ile His Thr His Leu Lys Asn
                  110                 115                 120
   Val Ser Ser Thr Asn Gly Ser Ser Asp Ser Tyr Ile Phe Leu Glu
                  125                 130                 135
   Lys Asp Leu Ser Ala Leu Asp Phe Ile Gly Val Ser Phe Tyr Gln
                  140                 145                 150
   Phe Ser Ile Ser Trp Pro Arg Leu Phe Pro Asp Gly Ile Val Thr
                  155                 160                 165
   Val Ala Asn Ala Lys Gly Leu Gln Tyr Tyr Ser Thr Leu Leu Asp
                   170                 175                 180
   Ala Leu Val Leu Arg Asn Ile Glu Pro Ile Val Thr Leu Tyr His
                   185                 190                 195
   Trp Asp Leu Pro Leu Ala Leu Gln Glu Lys Tyr Gly Gly Trp Lys
                   200                 205                 210
   Asn Asp Thr Ile Ile Asp Ile Phe Asn Asp Tyr Ala Thr Tyr Cys
                   215                 220                 225
   Phe Gln Met Phe Gly Asp Arg Val Lys Tyr Trp Ile Thr Ile His
                   230                 235                 240
   Asn Pro Tyr Leu Val Ala Trp His Gly Tyr Gly Thr Gly Met His
                   245                 250                 255
   Ala Pro Gly Glu Lys Gly Asn Leu Ala Ala Val Tyr Thr Val Gly
                   260                 265                 270
   His Asn Leu Ile Lys Ala His Ser Lys Val Trp His Asn Tyr Asn
                   275                 280                 285
   Thr His Phe Arg Pro His Gln Lys Gly Trp Leu Ser Ile Thr Leu
                   290                 295                 300
   Gly Ser His Trp Ile Glu Pro Asn Arg Ser Glu Asn Thr Met Asp
                   305                 310                 315
   Ile Phe Lys Cys Gln Gln Ser Met Val Ser Val Leu Gly Trp Phe
                   320                 325                 330
   Ala Asn Pro Ile His Gly Asp Gly Asp Tyr Pro Glu Gly Met Arg
                   335                 340                 345
   Lys Lys Leu Phe Ser Val Leu Pro Ile Phe Ser Glu Ala Glu Lys
                   350                 355                 360
   His Glu Met Arg Gly Thr Ala Asp Phe Phe Ala Phe Ser Phe Gly
                   365                 370                 375
   Pro Asn Asn Phe Lys Pro Leu Asn Thr Met Ala Lys Met Gly Gln
                   380                 385                 390
   Asn Val Ser Leu Asn Leu Arg Glu Ala Leu Asn Trp Ile Lys Leu
                   395                 400                 405
   Glu Tyr Asn Asn Pro Arg Ile Leu Ile Ala Ala Asn Gly Trp Phe
                   410                 415                 420
   Thr Asp Ser Arg Val Lys Thr Glu Asp Thr Thr Ala Ile Tyr Met
                   425                 430                 435
   Met Lys Asn Phe Leu Ser Gln Val Leu Gln Ala Ile Arg Leu Asp
                   440                 445                 450
   Glu Ile Arg Val Phe Gly Tyr Thr Ala Trp Ser Leu Leu Asp Gly
                   455                 460                 465
   Phe Glu Trp Gln Asp Ala Tyr Thr Ile Arg Arg Gly Leu Phe Tyr
                   470                 475                 480
   Val Asp Phe Asn Ser Lys Gln Lys Glu Arg Lys Pro Lys Ser Ser
   485 490 495
   Ala His Tyr Tyr Lys Gln Ile Ile Arg Glu Asn Gly Phe Ser Leu
   500 505 510
   Lys Glu Ser Thr Pro Asp Val Gln Gly Gln Phe Pro Cys Asp Phe
   515 520 525
   Ser Trp Gly Val Thr Glu Ser Val Leu Lys Pro Glu Ser Val Ala
   530 535 540
   Ser Ser Pro Gln Phe Ser Asp Pro His Leu Tyr Val Trp Asn Ala
   545 550 555
   Thr Gly Asn Arg Leu Leu His Arg Val Glu Gly Val Arg Leu Lys
   560 565 570
   Thr Arg Pro Ala Gln Cys Thr Asp Phe Val Asn Ile Lys Lys Gln
   575 580 585
   Leu Glu Met Leu Ala Arg Met Lys Val Thr His Tyr Arg Phe Ala
   590 595 600
   Leu Asp Trp Ala Ser Val Leu Pro Thr Gly Asn Leu Ser Ala Val
   605 610 615
   Asn Arg Gln Ala Leu Arg Tyr Tyr Arg Cys Val Val Ser Glu Gly
   620 625 630
   Leu Lys Leu Gly Ile Ser Ala Met Val Thr Leu Tyr Tyr Pro Thr
   635 640 645
   His Ala His Leu Gly Leu Pro Glu Pro Leu Leu His Ala Asp Gly
   650 655 660
   Trp Leu Asn Pro Ser Thr Ala Glu Ala Phe Gln Ala Tyr Ala Gly
   665 670 675
   Leu Cys Phe Gln Glu Leu Gly Asp Leu Val Lys Leu Trp Ile Thr
   680 685 690
   Ile Asn Ala Pro Asn Arg Leu Ser Asp Ile Tyr Asn Arg Ser Gly
   695 700 705
   Asn Asp Thr Tyr Gly Ala Ala His Asn Leu Leu Val Ala His Ala
   710 715 720
   Leu Ala Trp Arg Leu Tyr Asp Arg Gln Phe Arg Pro Ser Gln Arg
   725 730 735
   Gly Ala Val Ser Leu Ser Leu His Ala Asp Trp Ala Glu Pro Ala
   740 745 750
   Asn Pro Tyr Ala Asp Ser His Trp Arg Ala Ala Glu Arg Phe Leu
   755 760 765
   Gln Phe Glu Ile Ala Trp Phe Ala Glu Pro Leu Phe Lys Thr Gly
   770 775 780
   Asp Tyr Pro Ala Ala Met Arg Glu Tyr Ile Ala Ser Lys His Arg
   785 790 795
   Arg Gly Leu Ser Ser Ser Ala Leu Pro Arg Leu Thr Glu Ala Glu
   800 805 810
   Arg Arg Leu Leu Lys Gly Thr Val Asp Phe Cys Ala Leu Asn His
   815 820 825
   Phe Thr Thr Arg Phe Val Met His Glu Gln Leu Ala Gly Ser Arg
   830 835 840
   Tyr Asp Ser Asp Arg Asp Ile Gln Phe Leu Gln Asp Ile Thr Arg
   845 850 855
   Leu Ser Ser Pro Thr Arg Leu Ala Val Ile Pro Trp Gly Val Arg
   860 865 870
   Lys Leu Leu Arg Trp Val Arg Arg Asn Tyr Gly Asp Met Asp Ile
   875 880 885
   Tyr Ile Thr Ala Ser Gly Ile Asp Asp Gln Ala Leu Glu Asp Asp
   890 895 900
   Arg Leu Arg Lys Tyr Tyr Leu Gly Lys Tyr Leu Gln Glu Val Leu
   905 910 915
   Lys Ala Tyr Leu Ile Asp Lys Val Arg Ile Lys Gly Tyr Tyr Ala
   920 925 930
   Phe Lys Leu Ala Glu Glu Lys Ser Lys Pro Arg Phe Gly Phe Phe
   935 940 945
   Thr Ser Asp Phe Lys Ala Lys Ser Ser Ile Gln Phe Tyr Asn Lys
   950 955 960
   Val Ile Ser Ser Arg Gly Phe Pro Phe Glu Asn Ser Ser Ser Arg
   965 970 975
   Cys Ser Gln Thr Gln Glu Asn Thr Glu Cys Thr Val Cys Leu Phe
   980 985 990
   Leu Val Gln Lys Lys Pro Leu Ile Phe Leu Gly Cys Cys Phe Phe
   995 1000 1005
   Ser Thr Leu Val Leu Leu Leu Ser Ile Ala Ile Phe Gln Arg Gln
   1010 1015 1020
   Lys Arg Arg Lys Phe Trp Lys Ala Lys Asn Leu Gln His Ile Pro
   1025 1030 1035
   Leu Lys Lys Gly Lys Arg Val Val Ser
   1040
<210> 3
   <211> 34
   <212> DNA
   <213> Artificial sequence
<220>
   <223> sequence is synthesized
<400> 3
   cgggcgctag catgaagcca ggctgtgcgg cagg 34
<210> 4
   <211> 63
   <212> DNA
   <213> Artificial sequence
<220>
   <223> sequence is synthesized
<400> 4
   cagtggatcc ttacttatcg tcgtcatcct tgtaatcgct aacaactctc 50
   ttgcctttct ttc 63
<210> 5
   <211> 37
   <212> DNA
   <213> Artificial sequence
<220>
   <223> sequence is synthesized
<400> 5
   ccgccggata tcatgcggct gctgctggcc ctgttgg 37
<210> 6
   <211> 37
   <212> DNA
   <213> Artificial sequence
<220>
   <223> sequence is synthesized
<400> 6
   ccgccggaat tctgtctgca ccccagaccc gaagggg 37
<210> 7
   <211> 79
   <212> DNA
   <213> Artificial sequence
<220>
   <223> sequence is synthesized
<400> 7
   ggtctggggt gcagacaggt aagcctatcc ctaaccctct cctcggtctc 50
   gattctacgt aggaattcgg atccgcggc 79
<210> 8
   <211> 79
   <212> DNA
   <213> Artificial sequence
<220>
   <223> sequence is synthesized
<400> 8
   gccgcggatc cgaattccta cgtagaatcg agaccgagga gagggttagg 50
   gataggctta cctgtctgca ccccagacc 79
<210> 9
   <211> 41
   <212> DNA
   <213> Artificial sequence
<220>
   <223> sequence is synthesized
<400> 9
   gaattccacc atgaagccag gctgtgcggc aggatctcca g 41
<210> 10
   <211> 37
   <212> DNA
   <213> Artificial sequence
<220>
   <223> sequence is synthesized
<400> 10
   ggcgcgccga caaggaataa gcagacagtg cactctg 37
<210> 11
   <211> 42
   <212> DNA
   <213> Artificial sequence
<220>
   <223> sequence is synthesized
<400> 11
   ccctcgaatc ttgattgctg cgaatggctg gttcacagac ag 42
<210> 12
   <211> 42
   <212> DNA
   <213> Artificial sequence
<220>
   <223> sequence is synthesized
<400> 12
   ctgtctgtga accagccatt cgcagcaatc aagattcgag gg 42
<210> 13
   <211> 40
   <212> DNA
   <213> Artificial sequence
<220>
   <223> sequence is synthesized
<400> 13
   gctctggatc accatcaacg cgcctaaccg gctaagtgac 40
<210> 14
   <211> 40
   <212> DNA
   <213> Artificial sequence
<220>
   <223> sequence is synthesized
<400> 14
   gtcacttagc cggttaggcg cgttgatggt gatccagagc 40
<210> 15
   <211> 19
   <212> DNA
   <213> Artificial sequence
<220>
   <223> sequence is synthesized
<400> 15
   agcggattct catggaaca 19
<210> 16
   <211> 18
   <212> DNA
   <213> Artificial sequence
<220>
   <223> sequence is synthesized
<400> 16
   ctggtcagcc aggagctt 18
<210> 17
   <211> 24
   <212> DNA
   <213> Artificial sequence
<220>
   <223> sequence is synthesized
<400> 17
   tccacaagct gaaggcagac aagg 24
<210> 18
   <211> 25
   <212> DNA
   <213> Artificial sequence
<220>
   <223> sequence is synthesized
<400> 18
   gcagtcagac ccaagaaaat acaga 25
<210> 19
   <211> 24
   <212> DNA
   <213> Artificial sequence
<220>
   <223> sequence is synthesized
<400> 19
   cccaggaata tcagtggttt cttc 24
<210> 20
   <211> 23
   <212> DNA
   <213> Artificial sequence
<220>
   <223> sequence is synthesized
<400> 20
   tgcactgtct gcttattcct tgt 23
<210> 21
   <211> 21
   <212> DNA
   <213> Artificial sequence
<220>
   <223> sequence is synthesized
<400> 21
   cgagcccttt gatgacttcc t 21
<210> 22
   <211> 18
   <212> DNA
   <213> Artificial sequence
<220>
   <223> sequence is synthesized
<400> 22
   ggagcgggct gtctcaga 18
<210> 23
   <211> 21
   <212> DNA
   <213> Artificial sequence
<220>
   <223> sequence is synthesized
<400> 23
   cccagcatca tccaggccca g 21
<210> 24
   <211> 21
   <212> DNA
   <213> Artificial sequence
<220>
   <223> sequence is synthesized
<400> 24
   agtccttcca cctcgacgtt t 21
<210> 25
   <211> 23
   <212> DNA
   <213> Artificial sequence
<220>
   <223> sequence is synthesized
<400> 25
   aatcgagtct gtttccagca gaa 23
<210> 26
   <211> 20
   <212> DNA
   <213> Artificial sequence
<220>
   <223> sequence is synthesized
<400> 26
   agccccccct tccacttttt 20
<210> 27
   <211> 21
   <212> DNA
   <213> Artificial sequence
<220>
   <223> sequence is synthesized
<400> 27
   cgttaacagt gggtgccaac t 21
<210> 28
   <211> 20
   <212> DNA
   <213> Artificial sequence
<220>
   <223> sequence is synthesized
<400> 28
   cccgacggtc tctcttcaaa 20
<210> 29
   <211> 24
   <212> DNA
   <213> Artificial sequence
<220>
   <223> sequence is synthesized
<400> 29
   atgctaacgc agcagttgca aaca 24
<210> 30
   <211> 20
   <212> DNA
   <213> Artificial sequence
<220>
   <223> sequence is synthesized
<400> 30
   tgtggtgagc gaaggactga 20
<210> 31
   <211> 20
   <212> DNA
   <213> Artificial sequence
<220>
   <223> sequence is synthesized
<400> 31
   ggagtgggtt gggtggtaca 20
<210> 32
   <211> 19
   <212> DNA
   <213> Artificial sequence
<220>
   <223> sequence is synthesized
<400> 32
   ctgggcgtct tccccatgg 19
<210> 33
   <211> 18
   <212> DNA
   <213> Artificial sequence
<220>
   <223> sequence is synthesized
<400> 33
   cctgcccctt ctcaacga 18
<210> 34
   <211> 20
   <212> DNA
   <213> Artificial sequence
<220>
   <223> sequence is synthesized
<400> 34
   tccacgttgc tgatgctctt 20
<210> 35
   <211> 22
   <212> DNA
   <213> Artificial sequence
<220>
   <223> sequence is synthesized
<400> 35
   ccaagccatc cttggagcca gt 22
<210> 36
   <211> 22
   <212> DNA
   <213> Artificial sequence
<220>
   <223> sequence is synthesized
<400> 36
   cgccagcctg tcactataca aa 22
<210> 37
   <211> 20
   <212> DNA
   <213> Artificial sequence
<220>
   <223> sequence is synthesized
<400> 37
   ccagaggacc tcgactccaa 20
<210> 38
   <211> 25
   <212> DNA
   <213> Artificial sequence
<220>
   <223> sequence is synthesized
<400> 38
   cgtttccctt tggcccgaca gttct 25
<210> 39
   <211> 21
   <212> RNA
   <213> Artificial sequence
<220>
   <223> sequence is synthesized
<400> 39
   gcacacuacu acaaacagau u 21
<210> 40
   <211> 21
   <212> RNA
   <213> Artificial sequence
<220>
   <223> sequence is synthesized
<400> 40
   ucuguuugua guagugugcu u 21
<210> 41
   <211> 21
   <212> RNA
   <213> Artificial sequence
<220>
   <223> sequence is synthesized
<400> 41
   gcacgaaugg uuccagugau u 21
<210> 42
   <211> 21
   <212> RNA
   <213> Artificial sequence
<220>
   <223> sequence is synthesized
<400> 42
   ucacuggaac cauucgugcu u 21
<210> 43
   <211> 21
   <212> RNA
   <213> Artificial sequence
<220>
   <223> sequence is synthesized
<400> 43
   cgauggauau auucaaaugu u 21
<210> 44
   <211> 21
   <212> RNA
   <213> Artificial sequence
<220>
   <223> sequence is synthesized
<400> 44
   cauuugaaua uauccaucgu u 21
<210> 45
   <211> 21
   <212> RNA
   <213> Artificial sequence
<220>
   <223> sequence is synthesized
<400> 45
   ugaaauaacc acacgcuauu u 21
<210> 46
   <211> 21
   <212> RNA
   <213> Artificial sequence
<220>
   <223> sequence is synthesized
<400> 46
   auagcgugug guuauuucau u 21
<210> 47
   <211> 19
   <212> RNA
   <213> Artificial sequence
<220>
   <223> sequence is synthesized
<400> 47
   ugguuuacau guuccaaua 19
<210> 48
   <211> 19
   <212> RNA
   <213> Artificial sequence
<220>
   <223> sequence is synthesized
<400> 48
   uauuggaaca uguaaacca 19
<210> 49
   <211> 3159
   <212> DNA
   <213> Artificial sequence
<220>
   <223> sequence is synthesized
<400> 49
   atgaagccag gctgtgcggc aggatctcca gggaatgaat ggattttctt 50
   cagcactgat gaaataacca cacgctatag gaatacaatg tccaacgggg 100
   gattgcaaag atctgtcatc ctgtcagcac ttattctgct acgagctgtt 150
   actggattct ctggagatgg aagagctata tggtctaaaa atcctaattt 200
   tactccggta aatgaaagtc agctgtttct ctatgacact ttccctaaaa 250
   actttttctg ggtattgggg actggagcat tgcaagtgga agggagttgg 300
   aagaaggatg gaaaaggacc ttctatatgg gatcatttca tccacacaca 350
   ccttaaaaat gtcagcagca cgaatggttc cagtgacagt tatatttttc 400
   tggaaaaaga cttatcagcc ctggatttta taggagtttc tttttatcaa 450
   ttttcaattt cctggccaag gcttttcccc gatggaatag taacagttgc 500
   caacgcaaaa ggtctgcagt actacagtac tcttctggac gctctagtgc 550
   ttagaaacat tgaacctata gttactttat accactggga tttgcctttg 600
   gcactacaag aaaaatatgg ggggtggaaa aatgatacca taatagatat 650
   cttcaatgac tatgccacat actgtttcca gatgtttggg gaccgtgtca 700
   aatattggat tacaattcac aacccatatc tagtggcttg gcatgggtat 750
   gggacaggta tgcatgcccc tggagagaag ggaaatttag cagctgtcta 800
   cactgtggga cacaacttga tcaaggctca ctcgaaagtt tggcataact 850
   acaacacaca tttccgccca catcagaagg gttggttatc gatcacgttg 900
   ggatctcatt ggatcgagcc aaaccggtcg gaaaacacga tggatatatt 950
   caaatgtcaa caatccatgg tttctgtgct tggatggttt gccaacccta 1000
   tccatgggga tggcgactat ccagagggga tgagaaagaa gttgttctcc 1050
   gttctaccca ttttctctga agcagagaag catgagatga gaggcacagc 1100
   tgatttcttt gccttttctt ttggacccaa caacttcaag cccctaaaca 1150
   ccatggctaa aatgggacaa aatgtttcac ttaatttaag agaagcgctg 1200
   aactggatta aactggaata caacaaccct cgaatcttga ttgctgagaa 1250
   tggctggttc acagacagtc gtgtgaaaac agaagacacc acggccatct 1300
   acatgatgaa gaatttcctc agccaggtgc ttcaagcaat aaggttagat 1350
   gaaatacgag tgtttggtta tactgcctgg tctctcctgg atggctttga 1400
   atggcaggat gcttacacca tccgccgagg attattttat gtggatttta 1450
   acagtaaaca gaaagagcgg aaacctaagt cttcagcaca ctactacaaa 1500
   cagatcatac gagaaaatgg tttttcttta aaagagtcca cgccagatgt 1550
   gcagggccag tttccctgtg acttctcctg gggtgtcact gaatctgttc 1600
   ttaagcccga gtctgtggct tcgtccccac agttcagcga tcctcatctg 1650
   tacgtgtgga acgccactgg caacagactg ttgcaccgag tggaaggggt 1700
   gaggctgaaa acacgacccg ctcaatgcac agattttgta aacatcaaaa 1750
   aacaacttga gatgttggca agaatgaaag tcacccacta ccggtttgct 1800
   ctggattggg cctcggtcct tcccactggc aacctgtccg cggtgaaccg 1850
   acaggccctg aggtactaca ggtgcgtggt cagtgagggg ctgaagcttg 1900
   gcatctccgc gatggtcacc ctgtattatc cgacccacgc ccacctaggc 1950
   ctccccgagc ctctgttgca tgccgacggg tggctgaacc catcgacggc 2000
   cgaggccttc caggcctacg ctgggctgtg cttccaggag ctgggggacc 2050
   tggtgaagct ctggatcacc atcaacgagc ctaaccggct aagtgacatc 2100
   tacaaccgct ctggcaacga cacctacggg gcggcgcaca acctgctggt 2150
   ggcccacgcc ctggcctggc gcctctacga ccggcagttc aggccctcac 2200
   agcgcggggc cgtgtcgctg tcgctgcacg cggactgggc ggaacccgcc 2250
   aacccctatg ctgactcgca ctggagggcg gccgagcgct tcctgcagtt 2300
   cgagatcgcc tggttcgccg agccgctctt caagaccggg gactaccccg 2350
   cggccatgag ggaatacatt gcctccaagc accgacgggg gctttccagc 2400
   tcggccctgc cgcgcctcac cgaggccgaa aggaggctgc tcaagggcac 2450
   ggtcgacttc tgcgcgctca accacttcac cactaggttc gtgatgcacg 2500
   agcagctggc cggcagccgc tacgactcgg acagggacat ccagtttctg 2550
   caggacatca cccgcctgag ctcccccacg cgcctggctg tgattccctg 2600
   gggggtgcgc aagctgctgc ggtgggtccg gaggaactac ggcgacatgg 2650
   acatttacat caccgccagt ggcatcgacg accaggctct ggaggatgac 2700
   cggctccgga agtactacct agggaagtac cttcaggagg tgctgaaagc 2750
   atacctgatt gataaagtca gaatcaaagg ctattatgca ttcaaactgg 2800
   ctgaagagaa atctaaaccc agatttggat tcttcacatc tgattttaaa 2850
   gctaaatcct caatacaatt ttacaacaaa gtgatcagca gcaggggctt 2900
   cccttttgag aacagtagtt ctagatgcag tcagacccaa gaaaatacag 2950
   agtgcactgt ctgcttattc cttgtgcaga agaaaccact gatattcctg 3000
   ggttgttgct tcttctccac cctggttcta ctcttatcaa ttgccatttt 3050
   tcaaaggcag aagagaagaa agttttggaa agcaaaaaac ttacaacaca 3100
   taccattaaa gaaaggcaag agagttgtta gcgattacaa ggatgacgac 3150
   gataagtaa 3159
<210> 50
   <211> 3012
   <212> DNA
   <213> Artificial sequence
<220>
   <223> sequence is synthesized
<400> 50
   atgaagccag gctgtgcggc aggatctcca gggaatgaat ggattttctt 50
   cagcactgat gaaataacca cacgctatag gaatacaatg tccaacgggg 100
   gattgcaaag atctgtcatc ctgtcagcac ttattctgct acgagctgtt 150
   actggattct ctggagatgg aagagctata tggtctaaaa atcctaattt 200
   tactccggta aatgaaagtc agctgtttct ctatgacact ttccctaaaa 250
   actttttctg gggtattggg actggagcat tgcaagtgga agggagttgg 300
   aagaaggatg gaaaaggacc ttctatatgg gatcatttca tccacacaca 350
   ccttaaaaat gtcagcagca cgaatggttc cagtgacagt tatatttttc 400
   tggaaaaaga cttatcagcc ctggatttta taggagtttc tttttatcaa 450
   ttttcaattt cctggccaag gcttttcccc gatggaatag taacagttgc 500
   caacgcaaaa ggtctgcagt actacagtac tcttctggac gctctagtgc 550
   ttagaaacat tgaacctata gttactttat accactggga tttgcctttg 600
   gcactacaag aaaaatatgg ggggtggaaa aatgatacca taatagatat 650
   cttcaatgac tatgccacat actgtttcca gatgtttggg gaccgtgtca 700
   aatattggat tacaattcac aacccatatc tagtggcttg gcatgggtat 750
   gggacaggta tgcatgcccc tggagagaag ggaaatttag cagctgtcta 800
   cactgtggga cacaacttga tcaaggctca ctcgaaagtt tggcataact 850
   acaacacaca tttccgccca catcagaagg gttggttatc gatcacgttg 900
   ggatctcatt ggatcgagcc aaaccggtcg gaaaacacga tggatatatt 950
   caaatgtcaa caatccatgg tttctgtgct tggatggttt gccaacccta 1000
   tccatgggga tggcgactat ccagagggga tgagaaagaa gttgttctcc 1050
   gttctaccca ttttctctga agcagagaag catgagatga gaggcacagc 1100
   tgatttcttt gccttttctt ttggacccaa caacttcaag cccctaaaca 1150
   ccatggctaa aatgggacaa aatgtttcac ttaatttaag agaagcgctg 1200
   aactggatta aactggaata caacaaccct cgaatcttga ttgctgagaa 1250
   tggctggttc acagacagtc gtgtgaaaac agaagacacc acggccatct 1300
   acatgatgaa gaatttcctc agccaggtgc ttcaagcaat aaggttagat 1350
   gaaatacgag tgtttggtta tactgcctgg tctctcctgg atggctttga 1400
   atggcaggat gcttacacca tccgccgagg attattttat gtggatttta 1450
   acagtaaaca gaaagagcgg aaacctaagt cttcagcaca ctactacaaa 1500
   cagatcatac gagaaaatgg tttttcttta aaagagtcca cgccagatgt 1550
   gcagggccag tttccctgtg acttctcctg gggtgtcact gaatctgttc 1600
   ttaagcccga gtctgtggct tcgtccccac agttcagcga tcctcatctg 1650
   tacgtgtgga acgccactgg caacagactg ttgcaccgag tggaaggggt 1700
   gaggctgaaa acacgacccg ctcaatgcac agattttgta aacatcaaaa 1750
   aacaacttga gatgttggca agaatgaaag tcacccacta ccggtttgct 1800
   ctggattggg cctcggtcct tcccactggc aacctgtccg cggtgaaccg 1850
   acaggccctg aggtactaca ggtgcgtggt cagtgagggg ctgaagcttg 1900
   gcatctccgc gatggtcacc ctgtattatc cgacccacgc ccacctaggc 1950
   ctccccgagc ctctgttgca tgccgacggg tggctgaacc catcgacggc 2000
   cgaggccttc caggcctacg ctgggctgtg cttccaggag ctgggggacc 2050
   tggtgaagct ctggatcacc atcaacgagc ctaaccggct aagtgacatc 2100
   tacaaccgct ctggcaacga cacctacggg gcggcgcaca acctgctggt 2150
   ggcccacgcc ctggcctggc gcctctacga ccggcagttc aggccctcac 2200
   agcgcggggc cgtgtcgctg tcgctgcacg cggactgggc ggaacccgcc 2250
   aacccctatg ctgactcgca ctggagggcg gccgagcgct tcctgcagtt 2300
   cgagatcgcc tggttcgccg agccgctctt caagaccggg gactaccccg 2350
   cggccatgag ggaatacatt gcctccaagc accgacgggg gctttccagc 2400
   tcggccctgc cgcgcctcac cgaggccgaa aggaggctgc tcaagggcac 2450
   ggtcgacttc tgcgcgctca accacttcac cactaggttc gtgatgcacg 2500
   agcagctggc cggcagccgc tacgactcgg acagggacat ccagtttctg 2550
   caggacatca cccgcctgag ctcccccacg cgcctggctg tgattccctg 2600
   gggggtgcgc aagctgctgc ggtgggtccg gaggaactac ggcgacatgg 2650
   acatttacat caccgccagt ggcatcgacg accaggctct ggaggatgac 2700
   cggctccgga agtactacct agggaagtac cttcaggagg tgctgaaagc 2750
   atacctgatt gataaagtca gaatcaaagg ctattatgca ttcaaactgg 2800
   ctgaagagaa atctaaaccc agatttggat tcttcacatc tgattttaaa 2850
   gctaaatcct caatacaatt ttacaacaaa gtgatcagca gcaggggctt 2900
   cccttttgag aacagtagtt ctagatgcag tcagacccaa gaaaatacag 2950
   agtgcactgt ctgcttattc cttgtcggcg cgccccatca tcatcatcat 3000
   catcaccact aa 3012
<210> 51
   <211> 3159
   <212> DNA
   <213> Artificial sequence
<220>
   <223> sequence is synthesized
<400> 51
   atgaagccag gctgtgcggc aggatctcca gggaatgaat ggattttctt 50
   cagcactgat gaaataacca cacgctatag gaatacaatg tccaacgggg 100
   gattgcaaag atctgtcatc ctgtcagcac ttattctgct acgagctgtt 150
   actggattct ctggagatgg aagagctata tggtctaaaa atcctaattt 200
   tactccggta aatgaaagtc agctgtttct ctatgacact ttccctaaaa 250
   actttttctg gggtattggg actggagcat tgcaagtgga agggagttgg 300
   aagaaggatg gaaaaggacc ttctatatgg gatcatttca tccacacaca 350
   ccttaaaaat gtcagcagca cgaatggttc cagtgacagt tatatttttc 400
   tggaaaaaga cttatcagcc ctggatttta taggagtttc tttttatcaa 450
   ttttcaattt cctggccaag gcttttcccc gatggaatag taacagttgc 500
   caacgcaaaa ggtctgcagt actacagtac tcttctggac gctctagtgc 550
   ttagaaacat tgaacctata gttactttat accactggga tttgcctttg 600
   gcactacaag aaaaatatgg ggggtggaaa aatgatacca taatagatat 650
   cttcaatgac tatgccacat actgtttcca gatgtttggg gaccgtgtca 700
   aatattggat tacaattcac aacccatatc tagtggcttg gcatgggtat 750
   gggacaggta tgcatgcccc tggagagaag ggaaatttag cagctgtcta 800
   cactgtggga cacaacttga tcaaggctca ctcgaaagtt tggcataact 850
   acaacacaca tttccgccca catcagaagg gttggttatc gatcacgttg 900
   ggatctcatt ggatcgagcc aaaccggtcg gaaaacacga tggatatatt 950
   caaatgtcaa caatccatgg tttctgtgct tggatggttt gccaacccta 1000
   tccatgggga tggcgactat ccagagggga tgagaaagaa gttgttctcc 1050
   gttctaccca ttttctctga agcagagaag catgagatga gaggcacagc 1100
   tgatttcttt gccttttctt ttggacccaa caacttcaag cccctaaaca 1150
   ccatggctaa aatgggacaa aatgtttcac ttaatttaag agaagcgctg 1200
   aactggatta aactggaata caacaaccct cgaatcttga ttgctgcgaa 1250
   tggctggttc acagacagtc gtgtgaaaac agaagacacc acggccatct 1300
   acatgatgaa gaatttcctc agccaggtgc ttcaagcaat aaggttagat 1350
   gaaatacgag tgtttggtta tactgcctgg tctctcctgg atggctttga 1400
   atggcaggat gcttacacca tccgccgagg attattttat gtggatttta 1450
   acagtaaaca gaaagagcgg aaacctaagt cttcagcaca ctactacaaa 1500
   cagatcatac gagaaaatgg tttttcttta aaagagtcca cgccagatgt 1550
   gcagggccag tttccctgtg acttctcctg gggtgtcact gaatctgttc 1600
   ttaagcccga gtctgtggct tcgtccccac agttcagcga tcctcatctg 1650
   tacgtgtgga acgccactgg caacagactg ttgcaccgag tggaaggggt 1700
   gaggctgaaa acacgacccg ctcaatgcac agattttgta aacatcaaaa 1750
   aacaacttga gatgttggca agaatgaaag tcacccacta ccggtttgct 1800
   ctggattggg cctcggtcct tcccactggc aacctgtccg cggtgaaccg 1850
   acaggccctg aggtactaca ggtgcgtggt cagtgagggg ctgaagcttg 1900
   gcatctccgc gatggtcacc ctgtattatc cgacccacgc ccacctaggc 1950
   ctccccgagc ctctgttgca tgccgacggg tggctgaacc catcgacggc 2000
   cgaggccttc caggcctacg ctgggctgtg cttccaggag ctgggggacc 2050
   tggtgaagct ctggatcacc atcaacgagc ctaaccggct aagtgacatc 2100
   tacaaccgct ctggcaacga cacctacggg gcggcgcaca acctgctggt 2150
   ggcccacgcc ctggcctggc gcctctacga ccggcagttc aggccctcac 2200
   agcgcggggc cgtgtcgctg tcgctgcacg cggactgggc ggaacccgcc 2250
   aacccctatg ctgactcgca ctggagggcg gccgagcgct tcctgcagtt 2300
   cgagatcgcc tggttcgccg agccgctctt caagaccggg gactaccccg 2350
   cggccatgag ggaatacatt gcctccaagc accgacgggg gctttccagc 2400
   tcggccctgc cgcgcctcac cgaggccgaa aggaggctgc tcaagggcac 2450
   ggtcgacttc tgcgcgctca accacttcac cactaggttc gtgatgcacg 2500
   agcagctggc cggcagccgc tacgactcgg acagggacat ccagtttctg 2550
   caggacatca cccgcctgag ctcccccacg cgcctggctg tgattccctg 2600
   gggggtgcgc aagctgctgc ggtgggtccg gaggaactac ggcgacatgg 2650
   acatttacat caccgccagt ggcatcgacg accaggctct ggaggatgac 2700
   cggctccgga agtactacct agggaagtac cttcaggagg tgctgaaagc 2750
   atacctgatt gataaagtca gaatcaaagg ctattatgca ttcaaactgg 2800
   ctgaagagaa atctaaaccc agatttggat tcttcacatc tgattttaaa 2850
   gctaaatcct caatacaatt ttacaacaaa gtgatcagca gcaggggctt 2900
   cccttttgag aacagtagtt ctagatgcag tcagacccaa gaaaatacag 2950
   agtgcactgt ctgcttattc cttgtgcaga agaaaccact gatattcctg 3000
   ggttgttgct tcttctccac cctggttcta ctcttatcaa ttgccatttt 3050
   tcaaaggcag aagagaagaa agttttggaa agcaaaaaac ttacaacaca 3100
   taccattaaa gaaaggcaag agagttgtta gcgattacaa ggatgacgac 3150
   gataagtaa 3159
<210> 52
   <211> 3159
   <212> DNA
   <213> Artificial sequence
<220>
   <223> sequence is synthesized
<400> 52
   atgaagccag gctgtgcggc aggatctcca gggaatgaat ggattttctt 50
   cagcactgat gaaataacca cacgctatag gaatacaatg tccaacgggg 100
   gattgcaaag atctgtcatc ctgtcagcac ttattctgct acgagctgtt 150
   actggattct ctggagatgg aagagctata tggtctaaaa atcctaattt 200
   tactccggta aatgaaagtc agctgtttct ctatgacact ttccctaaaa 250
   actttttctg gggtattggg actggagcat tgcaagtgga agggagttgg 300
   aagaaggatg gaaaaggacc ttctatatgg gatcatttca tccacacaca 350
   ccttaaaaat gtcagcagca cgaatggttc cagtgacagt tatatttttc 400
   tggaaaaaga cttatcagcc ctggatttta taggagtttc tttttatcaa 450
   ttttcaattt cctggccaag gcttttcccc gatggaatag taacagttgc 500
   caacgcaaaa ggtctgcagt actacagtac tcttctggac gctctagtgc 550
   ttagaaacat tgaacctata gttactttat accactggga tttgcctttg 600
   gcactacaag aaaaatatgg ggggtggaaa aatgatacca taatagatat 650
   cttcaatgac tatgccacat actgtttcca gatgtttggg gaccgtgtca 700
   aatattggat tacaattcac aacccatatc tagtggcttg gcatgggtat 750
   gggacaggta tgcatgcccc tggagagaag ggaaatttag cagctgtcta 800
   cactgtggga cacaacttga tcaaggctca ctcgaaagtt tggcataact 850
   acaacacaca tttccgccca catcagaagg gttggttatc gatcacgttg 900
   ggatctcatt ggatcgagcc aaaccggtcg gaaaacacga tggatatatt 950
   caaatgtcaa caatccatgg tttctgtgct tggatggttt gccaacccta 1000
   tccatgggga tggcgactat ccagagggga tgagaaagaa gttgttctcc 1050
   gttctaccca ttttctctga agcagagaag catgagatga gaggcacagc 1100
   tgatttcttt gccttttctt ttggacccaa caacttcaag cccctaaaca 1150
   ccatggctaa aatgggacaa aatgtttcac ttaatttaag agaagcgctg 1200
   aactggatta aactggaata caacaaccct cgaatcttga ttgctgcgaa 1250
   tggctggttc acagacagtc gtgtgaaaac agaagacacc acggccatct 1300
   acatgatgaa gaatttcctc agccaggtgc ttcaagcaat aaggttagat 1350
   gaaatacgag tgtttggtta tactgcctgg tctctcctgg atggctttga 1400
   atggcaggat gcttacacca tccgccgagg attattttat gtggatttta 1450
   acagtaaaca gaaagagcgg aaacctaagt cttcagcaca ctactacaaa 1500
   cagatcatac gagaaaatgg tttttcttta aaagagtcca cgccagatgt 1550
   gcagggccag tttccctgtg acttctcctg gggtgtcact gaatctgttc 1600
   ttaagcccga gtctgtggct tcgtccccac agttcagcga tcctcatctg 1650
   tacgtgtgga acgccactgg caacagactg ttgcaccgag tggaaggggt 1700
   gaggctgaaa acacgacccg ctcaatgcac agattttgta aacatcaaaa 1750
   aacaacttga gatgttggca agaatgaaag tcacccacta ccggtttgct 1800
   ctggattggg cctcggtcct tcccactggc aacctgtccg cggtgaaccg 1850
   acaggccctg aggtactaca ggtgcgtggt cagtgagggg ctgaagcttg 1900
   gcatctccgc gatggtcacc ctgtattatc cgacccacgc ccacctaggc 1950
   ctccccgagc ctctgttgca tgccgacggg tggctgaacc catcgacggc 2000
   cgaggccttc caggcctacg ctgggctgtg cttccaggag ctgggggacc 2050
   tggtgaagct ctggatcacc atcaacgcgc ctaaccggct aagtgacatc 2100
   tacaaccgct ctggcaacga cacctacggg gcggcgcaca acctgctggt 2150
   ggcccacgcc ctggcctggc gcctctacga ccggcagttc aggccctcac 2200
   agcgcggggc cgtgtcgctg tcgctgcacg cggactgggc ggaacccgcc 2250
   aacccctatg ctgactcgca ctggagggcg gccgagcgct tcctgcagtt 2300
   cgagatcgcc tggttcgccg agccgctctt caagaccggg gactaccccg 2350
   cggccatgag ggaatacatt gcctccaagc accgacgggg gctttccagc 2400
   tcggccctgc cgcgcctcac cgaggccgaa aggaggctgc tcaagggcac 2450
   ggtcgacttc tgcgcgctca accacttcac cactaggttc gtgatgcacg 2500
   agcagctggc cggcagccgc tacgactcgg acagggacat ccagtttctg 2550
   caggacatca cccgcctgag ctcccccacg cgcctggctg tgattccctg 2600
   gggggtgcgc aagctgctgc ggtgggtccg gaggaactac ggcgacatgg 2650
   acatttacat caccgccagt ggcatcgacg accaggctct ggaggatgac 2700
   cggctccgga agtactacct agggaagtac cttcaggagg tgctgaaagc 2750
   atacctgatt gataaagtca gaatcaaagg ctattatgca ttcaaactgg 2800
   ctgaagagaa atctaaaccc agatttggat tcttcacatc tgattttaaa 2850
   gctaaatcct caatacaatt ttacaacaaa gtgatcagca gcaggggctt 2900
   cccttttgag aacagtagtt ctagatgcag tcagacccaa gaaaatacag 2950
   agtgcactgt ctgcttattc cttgtgcaga agaaaccact gatattcctg 3000
   ggttgttgct tcttctccac cctggttcta ctcttatcaa ttgccatttt 3050
   tcaaaggcag aagagaagaa agttttggaa agcaaaaaac ttacaacaca 3100
   taccattaaa gaaaggcaag agagttgtta gcgattacaa ggatgacgac 3150
   gataagtaa 3159
<210> 53
   <211> 11
   <212> PRT
   <213> Artificial sequence
<220>
   <223> sequence was synthesized
<400> 53
   Lys Ala Ser Gln Asp Ile Asn Ser Phe Leu Ala
                   5                   10
<210> 54
   <211> 7
   <212> PRT
   <213> Artificial sequence
<220>
   <223> sequence was synthesized
<400> 54
   Arg Ala Asn Arg Leu Val Ser
                   5
<210> 55
   <211> 9
   <212> PRT
   <213> Mus musculus
<400> 55
   Leu Gln Tyr Asp Glu Phe Pro Leu Thr
                   5
<210> 56
   <211> 10
   <212> PRT
   <213> Mus musculus
<400> 56
   Gly Phe Ser Leu Thr Thr Tyr Gly Val His
                   5                   10
<210> 57
   <211> 17
   <212> PRT
   <213> Mus musculus
<400> 57
   Gly Val Ile Trp Pro Gly Gly Gly Thr Asp Tyr Asn Ala Ala Phe
    1               5                  10                  15
   Ile Ser
<210> 58
   <211> 10
   <212> PRT
   <213> Mus musculus
<400> 58
   Val Arg Lys Glu Tyr Ala Asn Leu Tyr Ala
                   5                   10

## Description

### RELATED APPLICATIONS

This application claims priority to U.S. Patent Application Serial No. 60/909,699 filed on April 2, 2007 and U.S. Patent Application Serial No. 60/916,187 filed on May 4 2007.

### FIELD OF THE INVENTION

The present invention relates generally to the fields of molecular biology. More specifically, the invention concerns uses of anti-KLβ agents, and detection of KLβ and/or FGF19 and/or FGFR4.

### BACKGROUND OF THE INVENTION

Klotho beta ("KLβ", "KLB" or "beta klotho") is a 130-kDa type 1 transmembrane protein with a short (29 amino acids) intracellular domain that has no predicted kinase activity (Ito et al., Mech. Dev. 98 (2000) 115-119). KLβ has two extracellular glycosidase domains that lack a characteristic glutamic acid residue essential for enzymatic activity. Klb-deficient mice (Klb - / - mice) have increased CYP7A1 expression and decreased gallbladder size, indicating that Klb - / - mice can no longer suppress bile acid synthesis (Inagaki, T et al (2005) Cell Metab 2:217-25). KLβ is predominantly expressed in liver and pancreas. *Id.* Disruption of the gene encoding KLβ in mice results in marked increases in mRNA levels of cholesterol 7alpha-hydroxylase (CYP7A1), the first and rate-limiting enzyme in the bile acid biosynthetic pathway. Ito et al (2005) J Clin Invest 115(8):2202-2208; Arrese et al (2006) Hepatology 43(1):191-193; Moschetta and Kliewer (2005) J Clin Invest 115(8): 2075-2077.

The fibroblast growth factor (FGF) family is composed of 22 structurally related polypeptides that bind to 4 receptor tyrosine kinases (FGFR1-4) and one kinase deficient receptor (FGFR5) (Eswarakumar et al (2005) Cytokine Growth Factor Rev 16, 139-149; Ornitz et al (2001) Genome Biol 2, REVIEWS3005; Sleeman et al (2001) Gene 271, 171-182). FGFs' interaction with FGFR1-4 results in receptor homodimerization and autophosphorylation, recruitment of cytosolic adaptors such as FRS2 and initiation of multiple signaling pathways (Powers et al (2000) Endocr Relat Cancer 7, 165-197; Schlessinger, J. (2004) Science 306, 1506-1507).

FGFs and FGFRs play important roles in development and tissue repair by regulating cell proliferation, migration, chemotaxis, differentiation, morphogenesis and angiogenesis (Ornitz et al (2001) Genome Biol 2, REVIEWS3005; Augusteet al (2003) Cell Tissue Res 314, 157-166; Steiling et al (2003) Curr Opin Biotechnol 14, 533-537). Several FGFs and FGFRs arc associated with the pathogenesis of breast, prostate, cervix, stomach and colon cancers (Jeffers et al (2002) Expert Opin Ther Targets 6, 469-482; Mattila et al. (2001) Oncogene 20, 2791-2804; Ruohola et al. (2001) Cancer Res 61, 4229-4237; Marsh et al (1999) Oncogene 18, 1053-1060; Shimokawa et al (2003) Cancer Res 63, 6116-6120; Jang (2001) Cancer Res 61, 3541-3543; Cappellen (1999) Nat Genet 23, 18-20; Gowardhan (2005) Br J Cancer 92, 320-327).

FGF19 is a member of the most distant of the seven subfamilies of the FGFs. FGF19 is a high affinity ligand of FGFR4 (Xie et al (1999) Cytokine 11:729-735). FGF19 is normally secreted by the biliary and intestinal epithelium. FGF19 plays a role in cholesterol homeostasis by repressing hepatic expression of cholesterol-7-α-hydroxylase 1 (Cyp7α1), the rate-limiting enzyme for cholesterol and bile acid synthesis (Gutierrez ct al (2006) Arterioscler Thromb Vasc Biol 26, 301-306; Yu et al (2000) J Biol Chem 275, 15482-15489; Holt, JA, et al. (2003) Genes Dev 17(130):158). FGF19 ectopic expression in a transgenic mouse model increases hepatocyte proliferation, promotes hepatocellular dysplasia and results in neoplasia by 10 months of age (Nicholes et al. (2002). Am J Pathol 160, 2295-2307). The mechanism of FGF19 induced hepatocellular carcinoma is thought to involve FGFR4 interaction. FGF19 overexpression in tumor tissues is described in co-owned co-pending US patent application serial number 11/673,411 (filed February 9, 2007). Transgenic mice ectopically expressing FGF19 weigh less than their wild-type littermates, due in part to decrease in white adipose tissue. Tomlinson, E et al. (2002) Endocrinology 143:1741-1747. Although FGF19 transgenic mice have increased food intake, they also have a higher metabolic rate that is independent of increases in leptin, IGF-1, growth hormone, or thyroid hormone levels. Similarly, treatment with FGF-19 increased metabolic rate and reverses dietary and leptin-deficient diabetes. FGF19 administration improved glucose tolerance and decreased serum insulin, leptin, cholesterol and triglycerides. Fu et al (2004) 145:2594-2603. Administration of recombinant FGF19 to *ob*/*ob* mice, or crossing the FGF19 transgenic mice onto the *ob*/*ob* background resulted in mice that weighed less and had lower serum glucose levels and improved glucose sensitivity compared to *ob*/*ob* mice. Id. FGF-19 is also described in, for example, Harmer et al (2004) Biochemistry 43:629-640.

FGFR4 expression is widely distributed and was reported in developing skeletal muscles, liver, lung, pancreas, adrenal, kidney and brain (Kan et al. (1999) J Biol Chem 274, 15947-15952; Nicholes et al. (2002). Am J Pathol 160, 2295-2307; Ozawa et al. (1996) Brain Res Mol Brain Res 41, 279-288; Stark et al (1991) Development 113, 641-651). FGFR4 amplification was reported in mammary and ovarian adenocarcinomas (Jaakkola et al (1993) Int J Cancer 54, 378-382). FGFR4 mutation and truncation were correlated with the malignancy and in some cases the prognosis of prostate and lung adenocarcinomas, head and neck squamous cell carcinoma, soft tissue sarcoma, astrocytoma and pituitary adenomas (Jaakkola et al (1993) Int J Cancer 54, 378-382; Morimoto (2003) Cancer 98, 2245-2250; Qian (2004) J Clin Endocrinol Metab 89, 1904-1911; Spinola et al. (2005) J Clin Oncol 23, 7307-7311; Streit et al (2004) Int J Cancer 111, 213-217; Wang (1994) Mol Cell Biol 14, 181-188; Yamada (2002) Neurol Res 24, 244-248). FGFR4 overexpression in tumor tissues is described in WO2007/13693.

It is clear that there continues to be a need for agents that have clinical attributes that arc optimal for development as therapeutic agents. The invention described herein meets this need and provides other benefits.

### SUMMARY OF THE INVENTION

It is demonstrated herein that FGF19 requires KLβ for binding to FGFR4, FGFR4 downstream signaling and down-stream gene modulation. Thus, it is shown that KLβ and its interaction with FGFR can be a unique and advantageous target for greater fine-tuning in designing prophylatic and/or therapeutic approaches against pathological conditions associated with abnormal or unwanted signaling of the FGF/FGFR pathway. Thus, the present disclosure provides methods, compositions, kits and articles of manufacture for identifying and using substances that are capable of modulating the FGF/FGFR pathways through modulation of KLβ binding to FGFR and modulation of KLβ binding to FGFs, and for modulation of biological/physiological activities associated with FGF/FGFR signaling. KLβ presents as an important and advantageous therapeutic target, and the present disclosure provides compositions and methods based on binding KLβ. KLβ binding agents, as described herein, provide important therapeutic and diagnostic agents for use in targeting pathological conditions associated with expression and/or activity of the KLβ-FGF-FGFR pathways.

In one aspect, the disclosure provides methods, compositions, kits and articles of manufacture related to KLβ binding and detection of KLβ and/or FGF19 and/or FGFR4 binding.

In one aspect, the present disclosure provides methods and compositions useful for modulating disease states associated with expression and/or activity of KLβ, such as increased expression and/or activity or undesired expression and/or activity, said methods comprising administration of an effective dose of a KLβ antagonist (such as an anti-KLβ antibody) to an individual in need of such treatment.

In one aspect, the present disclosure provides methods for treating a tumor, cancer, or cell proliferative disorder comprising administering an effective amount of a KLβ antagonist (such as an anti-KLβ antibody) to an individual in need of such treatment. In some embodiments, the tumor, cancer, or cell proliferative disorder is hepatocellular carcinoma, pancreatic cancer, non-small cell lung cancer, breast cancer, or colorectal cancer.

In one aspect, the present disclosure provides methods for killing a cell (such as a cancer or tumor cell), the methods comprising administering an effective amount of a KLβ antagonist (such as an anti-KLβ antibody) to an individual in need of such treatment. In some embodiments, the cell is a hepatocellular carcinoma cell or a pancreatic cancer cell. In some embodiments, the cell is a liver or pancreatic cell.

In one aspect, the present disclosure provides methods for reducing, inhibiting, blocking, or preventing growth of a tumor or cancer, the methods comprising administering an effective amount of a KLβ antagonist (such as an anti-KLβ antibody) to an individual in need of such treatment. In some embodiments, the tumor, cancer, or cell proliferative disorder is hepatocellular carcinoma, pancreatic cancer, non-small cell lung cancer, breast cancer, or colorectal cancer.

In one aspect, the present disclosure provides methods for treating and/or preventing a liver disorder, the methods comprising administering an effective amount of a KLβ antagonist (such as an anti-KLβ antibody) to an individual in need of such treatment. In some embodiments, the liver disorder is cirrhosis.

In one aspect, the present disclosure provides methods for treating a wasting disorder comprising administering an effective amount of a KLβ antagonist (such as an anti-KLβ antibody) to an individual in need of such treatment. In some embodiments, the individual has a tumor, a cancer, and/or a cell proliferative disorder.

In one aspect, the present disclosure provides methods for treating hypoglycemia comprising administering an effective amount of a KLβ antagonist (such as an anti-KLβ antibody) to an individual in need of such treatment.

In one aspect, the present disclosure provides methods for treating cholestasis or dysregulation of bile acid metabolism comprising administering an effective amount of a KLβ antagonist (such as an anti-KLβ antibody) to an individual in need of such treatment.

In one aspect, the present disclosure provides methods for treating obesity or an obesity-related condition comprising administration of an effective dose of a KLβ agonist to an individual in need of such treatment. In some embodiments, the obesity-related condition is diabetes mellitus, cardiovascular disease, insulin resistance, hypertension, hypercholesterolemia, thromboembolic disease (such as stroke), atherosclerosis, dyslipidemia (for example, high total cholesterol or high triglyceride levels), osteoarthritis, gallbladder disease, osteoarthritis, and sleep apnea and other respiratory disorders.

In one aspect, the present disclosure provides methods for inducing an increase in insulin sensitivity comprising administration of an effective dose of a KLβ agonist to an individual in need of such treatment.

In one aspect, the present disclosure provides methods for reducing total body mass comprising administration of an effective dose of a KLβ agonist to an individual in need of such treatment.

In one aspect, the present disclosure provides methods for treating hyperglycemia comprising administration of an effective dose of a KLβ agonist to an individual in need of such treatment.

In one aspect, the present disclosure provides methods for reducing at least one of triglyceride and free fatty acid levels comprising administration of an effective dose of a KLβ agonist to an individual in need of such treatment.

Methods of the present disclosure can be used to affect any suitable pathological state. Exemplary disorders are described herein.

In one embodiment, a cell that is targeted in a method is a cancer cell. For example, a cancer cell can be one selected from the group consisting of a hepatocellular carcinoma cell or a pancreatic cancer cell. In one embodiment, a cell that is targeted in a method of the invention is a hyperproliferative and/or hyperplastic cell. In one embodiment, a cell that is targeted in a method of the invention is a dysplastic cell. In yet another embodiment, a cell that is targeted in a method of the invention is a metastatic cell. In one embodiment, the cell that is targeted is a cirrhotic liver cell.

Methods can further comprise additional treatment steps. For example, in one embodiment, a method further comprises a step wherein a targeted cell and/or tissue (for e.g., a cancer cell) is exposed to radiation treatment or a chemotherapeutic agent.

KLβ antagonists and agonists are known in the art and some are described and exemplified herein. In some embodiments, the KLβ antagonist is a molecule which binds to KLβ and neutralizes, blocks, inhibits, abrogates, reduces or interferes with one or more aspects of KLβ-associated effect.

In some embodiments, the KLβ antagonist is an antibody. In some embodiments, the antibody is a monoclonal antibody. In some embodiments, the antibody is a polyclonal antibody. In some embodiments, the antibody is selected from the group consisting of a chimeric antibody, an affinity matured antibody, a humanized antibody, and a human antibody. In some embodiments, the antibody is an antibody fragment. In some embodiments, the antibody is a Fab, Fab', Fab'-SH, F(ab')₂, or scFv.

In one embodiment, the antibody is a chimeric antibody, for example, an antibody comprising antigen binding sequences from a non-human donor grafted to a heterologous non-human, human or humanized sequence (e.g., framework and/or constant domain sequences). In one embodiment, the non-human donor is a mouse. In one embodiment, an antigen binding sequence is synthetic, e.g. obtained by mutagenesis (e.g., phage display screening, etc.). In one embodiment, a chimeric antibody has murine V regions and human C region. In one embodiment, the murine light chain V region is fused to a human kappa light chain. In one embodiment, the murine heavy chain V region is fused to a human IgG1 C region.

Humanized antibodies useful in methods of the invention include those that have amino acid substitutions in the FR and affinity maturation variants with changes in the grafted CDRs. The substituted amino acids in the CDR or FR are not limited to those present in the donor or recipient antibody. In other embodiments, the antibodies further comprise changes in amino acid residues in the Fc region that lead to improved effector function including enhanced CDC and/or ADCC function and B-cell killing. Other antibodies include those having specific changes that improve stability. In other embodiments, the useful antibodies comprise changes in amino acid residues in the Fc region that lead to decreased effector function, e.g. decreased CDC and/or ADCC function and/or decreased B-cell killing. In some embodiments, the antibodies are characterized by decreased binding (such as absence of binding) to human complement factor C1q and/or human Fc receptor on natural killer (NK) cells. In some embodiments, the antibodies are characterized by decreased binding (such as the absence of binding) to human FcγRI, FcγRIIA, and/or FcγRIIIA. In some embodiments, the antibody is of the IgG class (e.g., IgG1 or IgG4) and comprises at least one mutation in E233, L234, L235, G236, D265, D270, N297, E318, K320, K322, A327, A330, P331 and/or P329 (numbering according to the EU index). In some embodiments, the antibodies comprise the mutation L234A/L235A or D265A/N297A.

In one aspect, the KLβ antagonist is an anti-KLβ polypeptide comprising any of the antigen binding sequences provided herein, wherein the anti-KLβ polypeptides specifically bind to KLβ.

In one aspect, the KLβ antagonist is an immunoconjugate (interchangeably termed "antibody drug conjugate" or "ADC") comprising an anti-KLβ polypeptide (such as an anti-KLβ antibody) conjugated to an agent, such as a drug.

In one aspect, the KLβ antagonist is a KLβ siRNA. Examples of KLβ siRNA are described herein.

In some embodiments, the KLβ antagonist may modulate one or more aspects of KLβ-associatcd effects, including but not limited to binding FGFR (e.g., FGFR4) (optionally in conjunction with heparin), binding FGF (e.g., FGF19) (optionally in conjunction with heparin), binding FGFR4 and FGF19 (optionally in conjunction with heparin), promoting FGF19-mediated induction of cFos, Junb and/or June (in vitro or in vivo), promoting FGFR4 and/or FGF19 downstream signaling (including but not limited to FGFR phosphorylation, FRS2 phosphorylation, ERK1/2 phosphorylation and Wnt pathway activation), and/or promotion of any biologically relevant KLβ and/or FGFR and/or FGF biological pathway, and/or promotion of a tumor, cell proliferative disorder or a cancer; and/or promotion of a disorder associated with KLβ expression and/or activity (such as increased KLβ expression and/or activity). In some embodiments, the antagonist binds (such as specifically binds to KLβ). In some embodiments, the antagonist binds to an FGFR (such as FGFR4) binding region of KLβ. In some embodiments, the antagonist binds to a FGF (e.g., FGF19) binding regions of KLβ. In some embodiments, the antagonist reduces, inhibits, and/or blocks KLβ activity in vivo and/or in vitro. In some embodiments, the antagonist competes for binding with FGFR4 (reduces and/or blocks FGFR4 binding to KLβ). In some embodiments, the antagonist competes for binding with FGF19 (reduces and/or blocks FGF19 binding to KLβ).

In another aspect, the present disclosure supplies a composition comprising one or more KLβ antagonist (such as an anti-KLβ antibody), and a carrier. This composition may further comprise a second medicament, wherein the KLβ antagonist is a first medicament. This second medicament, for cancer treatment, for example, may be another KLβ antagonist (such as an anti-KLβ antibody), chemotherapeutic agent, cytotoxic agent, anti-angiogenic agent, immunosuppressive agent, prodrug, cytokine, cytokine antagonist, cytotoxic radiotherapy, corticosteroid, anti-emetic cancer vaccine, analgesic, anti-vascular agent, or growth-inhibitory agent. In another embodiment, a second medicament is administered to the subject in an effective amount, wherein the antibody is a first medicament. This second medicament is more than one medicament, and is preferably another antibody, chemotherapeutic agent, cytotoxic agent, anti-angiogenic agent, immunosuppressive agent, prodrug, cytokine, cytokine antagonist, cytotoxic radiotherapy, corticosteroid, anti-emetic, cancer vaccine, analgesic, anti-vascular agent, or growth-inhibitory agent. More specific agents include, for example, irinotecan (CAMPTOSAR®), cetuximab (ERBITUX®), fulvestrant (FASLODEX®), vinorelbine (NAVELBINE®), EFG-receptor antagonists such as erlotinib (TARCEVA®) VEGF antagonists such as bevacizumab (AVASTIN®), vincristine (ONCOVIN®), inhibitors of mTor (a serine/threonine protein kinase) such as rapamycin and CCI-779, and anti-HER1, HER2, ErbB, and/or EGFR antagonists such as trastuzumab (HERCEPTIN®), pertuzumab (OMNITARG™), or lapatinib, and other cytotoxic agents including chemotherapeutic agents. Insome embodiments, the second medicament is an anti-estrogen drug such as tamoxifen, fulvestrant, or an aromatase inhibitor, an antagonist to vascular endothelial growth factor (VEGF) or to ErbB or the Efb receptor, or Her-1 or Her-2. In some embodiments, the second medicament is tamoxifen, letrozole, exemestane, anastrozole, irinotecan, cetuximab, fulvestrant, vinorelbine, erlotinib, bevacizumab, vincristine, imatinib, sorafenib, lapatinib, or trastuzumab, and preferably, the second medicament is erlotinib, bevacizumab, or trastuzumab.

In one aspect, the present disclosure provides an article of manufacture comprising a container; and a composition contained within the container, wherein the composition comprises one or more KLβ antagonist (such as an anti-KLβ antibody). In one embodiment, a composition comprising a KLβ antagonist further comprises a carrier, which in some embodiments is pharmaceutically acceptable. In one embodiment, an article of manufacture of the invention further comprises instructions for administering the composition (for e.g., an anti-KLβ antibody) to an individual (such as instructions for any of the methods described herein).

In one aspect, the present disclosure provides a kit comprising a first container comprising a composition comprising one or more anti-KLβ antagonist; and a second container comprising a buffer. This composition may further comprise a second medicament, wherein the KLβ antagonist is a first medicament. Exemplary second medicaments are described above and elsewhere herein. In one embodiment, the buffer is pharmaceutically acceptable. In one embodiment, a composition comprising an antibody further comprises a carrier, which in some embodiments is pharmaceutically acceptable. In one embodiment, a kit further comprises instructions for administering the composition (for e.g., the antibody) to an individual.

In another aspect, the present disclosure provides methods for detection of KLβ, the methods comprising detecting KLβ in a sample (such as a biological sample). The term "detection" as used herein includes qualitative and/or quantitative detection (measuring levels) with or without reference to a control. In some embodiment, the biological sample is from a patient having or suspected of having a tumor, cancer, and/or a cell proliferative disorder, such as hepatocellular carcinoma, pancreatic cancer, non-small cell lung cancer, breast cancer, or colorectal cancer. In some embodiments, the biological sample is from a tumor. In some embodiments, the biological sample expresses FGF (e.g., FGF19) and/or FGFR (e.g., FGFR4).

In another aspect, the present disclosure provides methods for detecting a disorder associated with KLβ expression and/or activity, the methods comprising detecting KLβ in a biological sample from an individual. In some embodiments, the KLβ expression is increased expression or abnormal expression. In some embodiments, the disorder is a tumor, cancer, and/or a cell proliferative disorder, such as hepatocellular carcinoma, pancreatic cancer, non-small cell lung cancer, breast cancer, or colorectal cancer. In some embodiment, the biological sample is serum or of a tumor.

In another aspect, the present disclosure provides methods for detecting a disorder associated with FGFR4 and KLβ expression and/or activity, the methods comprising detecting FGFR4 and KLβ in a biological sample from an individual. In some embodiments, the KLβ expression is increased expression or abnormal expression. In some embodiments, FGFR4 expression is increased expression or abnormal expression. In some embodiments, the disorder is hepatocellular carcinoma, pancreatic cancer, non-small cell lung cancer, breast cancer, or colorectal cancer. In some embodiment, the biological sample is serum or of a tumor. In some embodiments, expression of FGFR4 is detected in a first biological sample, and expression of KLβ is detected in a second biological sample.

In another aspect, the present disclosure provides methods for detecting a disorder associated with FGF19 and KLβ expression and/or activity, the methods comprising detecting FGF19 and KLβ in a biological sample from an individual. In some embodiments, the KLβ expression is increased expression or abnormal expression. In some embodiments, FGF19 expression is increased expression or abnormal expression. In some embodiments, the disorder is a tumor, cancer, and/or a cell proliferative disorder, such as hepatocellular carcinoma, pancreatic cancer, non-small cell lung cancer, breast cancer, or colorectal cancer. In some embodiment, the biological sample is serum or of a tumor. In some embodiments, expression of FGF19 is detected in a first biological sample, and expression of KLβ is detected in a second biological sample.

In another aspect, the present disclosure provides methods for detecting a disorder associated with FGFR4, FGF19, and KLβ expression and/or activity, the methods comprising detecting FGFR4, FGF19 and KLβ in a biological sample from an individual. In some embodiments, the KLβ expression is increased expression or abnormal expression. In some embodiments, FGFR4 expression is increased expression or abnormal expression. In some embodiments, the disorder is a tumor, cancer, and/or a cell proliferative disorder, such as hepatocellular carcinoma, pancreatic cancer, non-small cell lung cancer, breast cancer, or colorectal cancer. In some embodiment, the biological sample is serum or from a tumor. In some embodiments, expression of FGFR4 is detected in a first biological sample, expression of FGF19 is detected in a second biological sample, and expression of KLβ is detected in a third biological sample.

In another aspect, the present disclosure provides methods for treating an individual having or suspected of having a cancer, a tumor, and/or a cell proliferative disorder or a liver disorder (such as cirrhosis) by administering an effective amount of a KLβ antagonist (e.g., an anti-KLβ antibody), wherein a biological sample of the cancer, tumor and/or cell disorder or liver disorder expresses (i) KLβ, (ii) KLβ and FGFR4, (iii) KLβ and FGF19, or (iv) KLβ, FGFR4 and FGF19. In some embodiments, the cancer, rumor and/or cell proliferative disorder or liver disorder is hepatocellular carcinoma, pancreatic cancer, non-small cell lung cancer, breast cancer, or colorectal cancer.

In another aspect, the present disclosure provides methods for treating an individual having or suspected of having a cancer, a tumor, and/or a cell proliferative disorder or a liver disorder (such as cirrhosis) by administering an effective amount of a FGF19 antagonist (e.g., an anti-FGF19 antibody), wherein a biological sample of the cancer, tumor and/or cell disorder or liver disorder expresses (i) KLβ, (ii) KLβ and FGFR4, (iii) KLβ and FGF19, or (iv) KLβ, FGFR4 and FGF19. In some embodiments, the cancer, rumor and/or cell proliferative disorder or liver disorder is hepatocellular carcinoma, pancreatic cancer, non-small cell lung cancer, breast cancer, or colorectal cancer.

In another aspect, the present disclosure provides methods for treating an individual having or suspected of having a cancer, a tumor, and/or a cell proliferative disorder or a liver disorder (such as cirrhosis) by administering an effective amount of an FGFR4 antagonist (e.g., an anti-FGFR4 antibody), wherein a biological sample of the cancer, tumor and/or cell disorder or liver disorder expresses (i) KLβ, (ii) KLβ and FGFR4, (iii) KLβ and FGF19, or (iv) KLβ, FGFR4 and FGF19. In some embodiments, the cancer, rumor and/or cell proliferative disorder or liver disorder is hepatocellular carcinoma, pancreatic cancer, non-small cell lung cancer, breast cancer, or colorectal cancer.

In another aspect, the present disclosure provides methods for selecting treatment for an individual, the methods comprising: (a) determining (i) KLβ expression, (ii) KLβ and FGF19 expression, (iii) KLβ and FGFR4 expression, or (iv) KLβ, FGF19 and FGFR4 expression, if any, in an individual's biological sample; and (b) subsequent to step (a), selecting treatment for the individual, wherein the selection of treatment is based on the expression determined in step (a). In some embodiments, increased KLβ expression in the individual's biological sample relative to a reference value or control sample is determined. In some embodiments, decreased KLβ expression in the individual's biological sample relative to a reference value or control sample is determined in the individual. In some embodiments, KLβ expression is determined and treatment with an anti-KLβ antibody is selected. In some embodiments, KLβ expression is determined and treatment with an FGF19 antagonist (such as an anti-FGF 19 antibody) is selected. In some embodiments, KLβ expression is determined and treatment with an FRFR4 antagonist (such as an anti-FGFR4 antibody) is selected. FGFR4 antagonists are known in the art. In some embodiments, the individual has a tumor, cancer, and/or a cell proliferative disorder, such as hepatocellular carcinoma, pancreatic cancer, non-small cell lung cancer, breast cancer, or colorectal cancer.

In another aspect, the present disclosure provides methods for treating an individual having or suspected of having a cancer, a tumor, and/or a cell proliferative disorder or a liver disorder (such as cirrhosis) by administering an effective amount of an anti-KLβ antibody, further wherein (i) KLβ expression, (ii) KLβ and FGF19 expression, (iii) KLβ and FGFR4 expression, or (iv) KLβ, FGF19 and FGFR4 expression is determined in the individual's biological sample before, during or after administration of an anti-KLβ antibody. In some embodiments, the biological sample is of the cancer, tumor and/or cell proliferative disorder. In some embodiments, the biological sample is serum. In some embodiments, KLβ over-expression is determined before, during and/or after administration of an anti-KLβ antibody. In some embodiments, FGFR4 expression is determined before, during and/or after administration of an anti-KLβ antibody. Expression may be determined before; during; after; before and during; before and after; during and after; or before, during and after administration of an anti-KLβ antibody.

In another aspect, the present disclosure provides methods for treating an individual having or suspected of having a cancer, a tumor, and/or a cell proliferative disorder or a liver disorder (such as cirrhosis) by administering an effective amount of an anti-FGF19 antibody, further wherein (i) KLβ expression, (ii) KLβ and FGF19 expression, (iii) KLβ and FGFR4 expression, or (iv) KLβ, FGF19 and FGFR4 expression is determined in the individual's biological sample before, during or after administration of an anti-FGF 19 antibody. In some embodiments, the biological sample is of the cancer, tumor and/or cell proliferative disorder. In some embodiments, the biological sample is serum. In some embodiments, KLβ over-expression is determined before, during and/or after administration of an anti-FGF 19 antibody. In some embodiments, FGFR4 expression is determined before, during and/or after administration of an anti-FGF 19 antibody. Expression may be determined before; during; after; before and during; before and after; during and after; or before, during and after administration of an anti-FGF 19 antibody. Anti-FGF 19 antibodies and methods of treatment comprising use of an anti-FGF 19 antibody arc described in co-owned co-pending US patent application serial number 11/673,411 (filed February 9, 2007).

In embodiments involving detection, expression of FGFR4 downstream molecular signaling may be detected in addition to or as an alternative to detection of FGFR4 expression. In some embodiments, detection of FGFR4 downstream molecular signaling comprises one or more of detection of phosphorylation of MAPK, FRS2 or ERK1/2 (or ERK1 and/or ERK2).

In some embodiments involving detection, expression of FGFR4 comprises detection of FGFR4 gene deletion, gene amplification and/or gene mutation. In some embodiments involving detection, expression of KLβ comprises detection of KLβ gene deletion, gene amplification and/or gene mutation. In some embodiments involving detection, expression of FGF19 comprises detection of FGF19 gene deletion, gene amplification and/or gene mutation.

Some embodiments involving detection further comprise detection of Wnt pathway activation. In some embodiments, detection of Wnt pathway activation comprises one or more of tyrosine phosphorylation of β-catenin, expression of Wnt target genes, β-catenin mutation, and E-cadherin binding to β-catenin. Detection of Wnt pathway activation is known in the art, and some examples are described and exemplified herein.

Biological samples are described herein, e.g., in the definition of Biological Sample. In some embodiment, the biological sample is serum or of a tumor.

In embodiments involving detection of KLβ and/or FGFR4 and/or FGF19 expression, KLβ and/or FGFR4 and/or FGF19 polynucleotide expression and/or KLβ and/or FGFR4 and/or FGF19 polypeptide expression may be detected. In some embodiments involving detection of KLβ and/or FGFR4 and/or FGF19 expression, KLβ and/or FGFR4 and/or FGF19 mRNA expression is detected. In other embodiments, KLβ and/or FGFR4 and/or FGF19 polypeptide expression is detected using an anti-KLβ agent and/or an anti-FGFR4 agent. In some embodiments, KLβ and/or FGFR4 and/or FGF19 polypeptide expression is detected using an antibody. Any suitable antibody may be used for detection and/or diagnosis, including monoclonal and/or polyclonal antibodies, a human antibody, a chimeric antibody, an affinity-matured antibody, a humanized antibody, and/or an antibody fragment. In some embodiments, an anti-KLβ antibody described herein is used for detection. In some embodiments, KLβ and/or FGFR4 and/or FGF19 polypeptide expression is detected using immunohistochemistry (IHC). In some embodiments, polypeptide expression is scored at 2 or higher using an IHC.

In some embodiments involving detection of KLβ and/or FGFR4 and/or FGF19 expression, presence and/or absence and/or level of KLβ and/or FGFR4 and/or FGF19 expression may be detected. KLβ and/or FGFR4 and/or FGF19 expression may be increased. It is understood that absence of KLβ and/or FGFR4 and/or FGF19 expression includes insignificant, or de minimus levels. In some embodiments, target expression in the test biological sample is higher than that observed for a control biological sample (or control or reference level of expression). In some embodiments, target expression is at least about 2-fold, 5-fold, 10-fold, 20-fold, 30-fold, 40-fold, 50-fold, 75-fold, 100-fold, 150-fold higher, or higher in the test biological sample than in the control biological sample. In some embodiments, target polypeptide expression is determined in an immunohistochemistry ("IHC") assay to score at least 2 or higher for staining intensity. In some embodiments, target polypeptide expression is determined in an IHC assay to score at least 1 or higher, or at least 3 or higher for staining intensity. In some embodiments, target expression in the test biological sample is lower than that observed for a control biological sample (or control expression level).

In one aspect, the present disclosure provides methods of identifying a candidate inhibitor substance that inhibits KLβ binding to FGFR (e.g., FGFR4), said method comprising: (a) contacting a candidate substance with a first sample comprising FGFR, FGF (e.g., FGF19) and KLβ, and (b) comparing amount of FGFR biological activity in the sample with amount of FGFR biological activity in a reference sample comprising similar amounts of KLβ, FGF and FGFR as the first sample but that has not been contacted with said candidate substance, whereby a decrease in amount of FGFR biological activity in the first sample compared to the reference sample indicates that the candidate substance is capable of inhibiting KLβ binding to FGFR.

In another aspect, the present disclosure provides methods of identifying a candidate inhibitor substance that inhibits KLβ binding to FGFR (e.g., FGFR4), said method comprising: (a) contacting a candidate substance with a first sample comprising FGFR, FGF and KLβ, and (b) comparing amount of FGFR- KLβ complex in the sample with amount of FGFR- KLβ complex in a reference sample comprising similar amounts of KLβ, FGF and FGFR as the first sample but that has not been contacted with said candidate substance, whereby a decrease in amount of FGFR- KLβ complex in the first sample compared to the reference sample indicates that the candidate substance is capable of inhibiting KLβ binding to FGFR.

In another aspect, the present disclosure provides methods of determining whether a candidate substance inhibits KLβ binding to FGFR (e.g., FGFR4), said method comprising: (a) contacting a candidate substance with a first sample comprising FGFR, FGF and KLβ, and (b) comparing amount of FGFR biological activity in the sample with amount of FGFR biological activity in a reference sample comprising similar amounts of KLβ, FGF and FGFR as the first sample but that has not been contacted with said candidate substance, whereby a decrease in amount of FGFR biological activity in the first sample compared to the reference sample indicates that the candidate substance is capable of inhibiting KLβ binding to FGFR.

In another aspect, the present disclosure provides methods of determining whether a candidate substance inhibits FGF binding to KLβ, said method comprising: (a) contacting a candidate substance with a first sample comprising FGF, FGFR and KLβ, and (b) comparing amount of FGFR biological activity in the sample with amount of FGFR biological activity in a reference sample comprising similar amounts of FGF, FGFR and KLβ as the first sample but that has not been contacted with said candidate substance, whereby a decrease in amount of FGFR biological activity in the first sample compared to the reference sample indicates that the candidate substance is capable of inhibiting KLβ.

In another aspect, the present disclosure provides methods of determining whether a candidate substance promotes KLβ biological activity, said method comprising: (a) contacting a candidate substance with a first sample comprising FGFR and KLβ, and (b) comparing amount of FGFR biological activity in the sample with amount of FGFR biological activity in a reference sample comprising similar amounts of KLβ and FGFR as the first sample but that has not been contacted with said candidate substance, whereby an increase in amount of FGFR biological activity in the first sample compared to the reference sample indicates that the candidate substance is capable of promoting KLβ binding to FGFR.

FGFR biological activities are described herein. In some embodiments, FGFR, FGF, KLβ are in an amount effective for FGFR biological activity

### BRIEF DESCRIPTION OF THE FIGURES

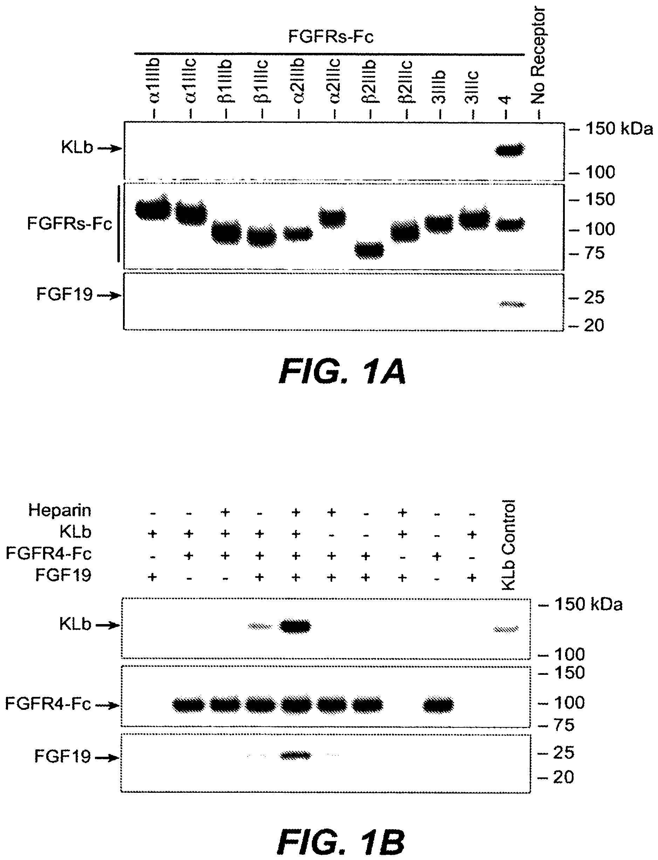

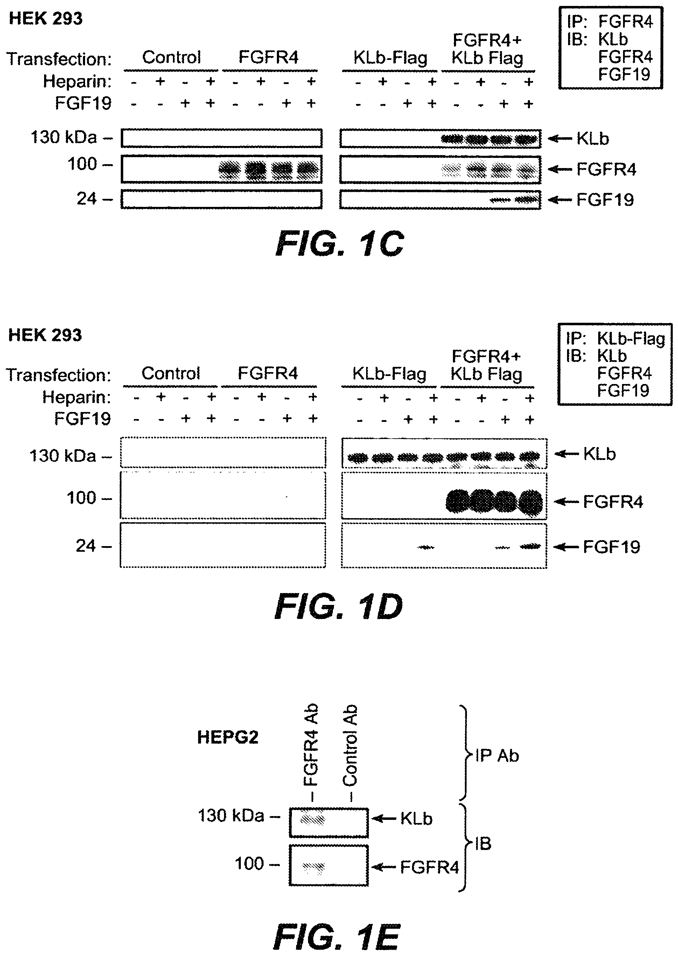

FIGURE 1: KLβ forms a complex with FGF19, FGFR4, and heparin. (A) FGF19 (0.5 µg), heparin (0.5 µg), and different FGFR-Fc fusion proteins (0.5 µg) were incubated in KLβΔTM-conditioned media for 18 hours at 4°C. The protein interactions were determined by protein A-agarose precipitation and immunoblot analyses. (B) KLβΔTM- or control-conditioned medium was incubated in the presence or the absence of FGF19 (0.5 µg), heparin (0.5 µg), or the FGFR4-Fc fusion protein (0.5 µg) for 18 hours at 4°C. The protein interactions were determined by protein A-agarose precipitation and immunoblot analyses. (C) and (D) Lysates from HEK293 cells transfected with empty (control vector), FGFR4, KLβ -Flag, or a combination of FGFR4 and KLβ -Flag expression vectors were incubated in the presence or absence of heparin and FGF19. The protein interactions were analyzed by FGFR4 (C) and KLβ -Flag (D) immunoprecipitation and immunoblotting. (E) The FGFR4-KLβ interaction in HEPG2 cells lysates was analyzed by immunoprecipitation and immunoblotting.

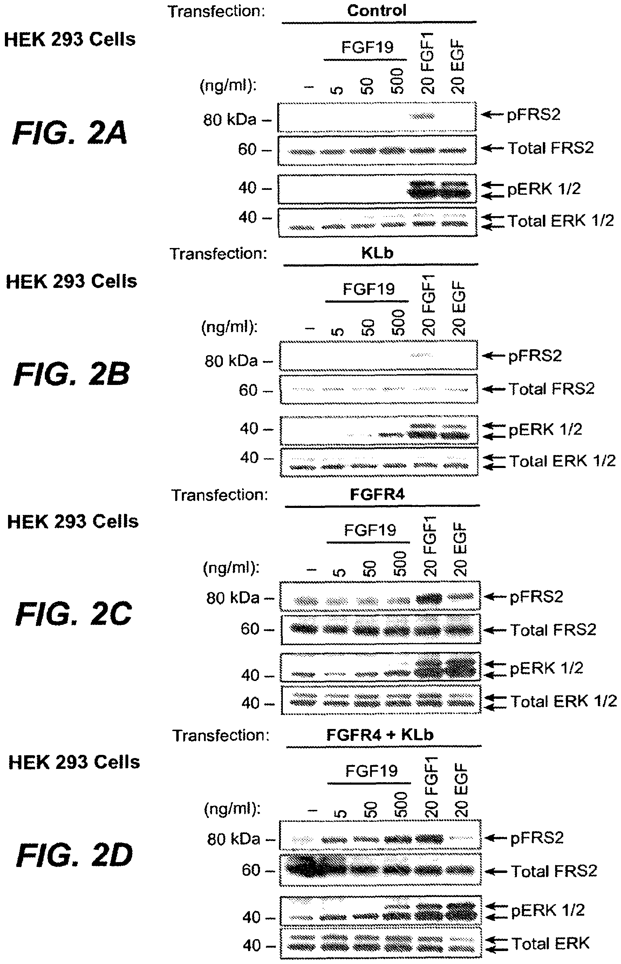

FIGURE 2: KLβ is required for FGF19 signaling. The effect of KLβ on FGF19 signaling was analyzed using HEK293 cells transfected with empty (control vector)(A), KLβ (B), FGFR4 (C), or a combination of FGFR4 and KLβ expression vectors (D). The transfected cells were incubated with vehicle (PBS) or FGF19 (0-500 ng/mL) for 10 minutes, lysed, and FRS2 and ERK1/2 phosphorylation were analyzed by immunoblot.

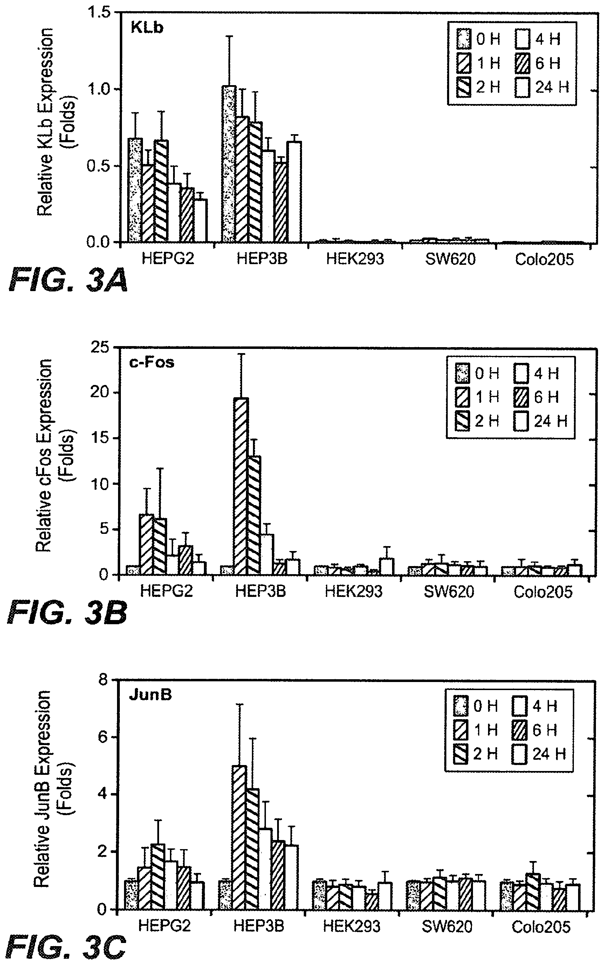

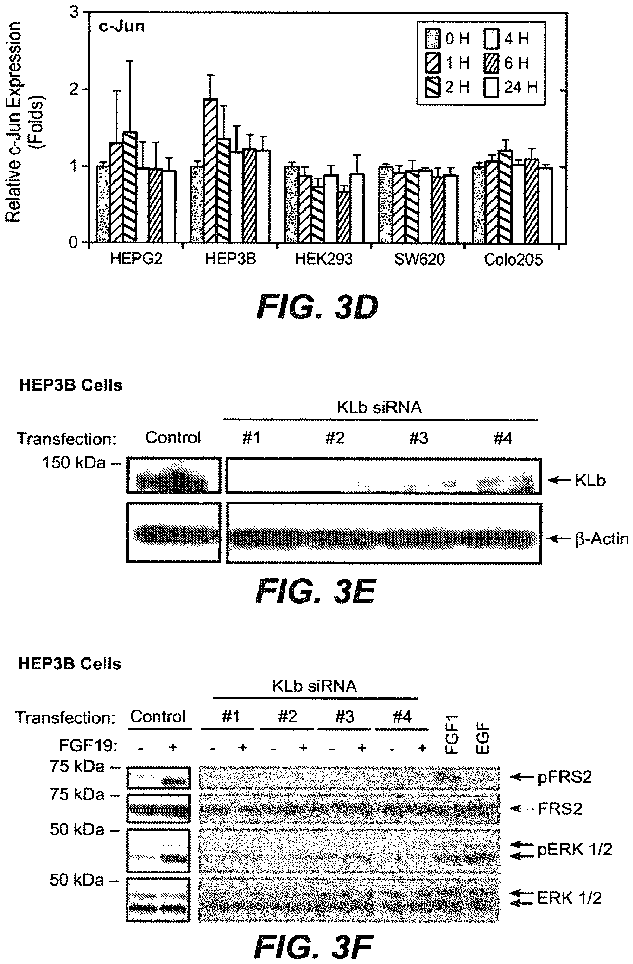

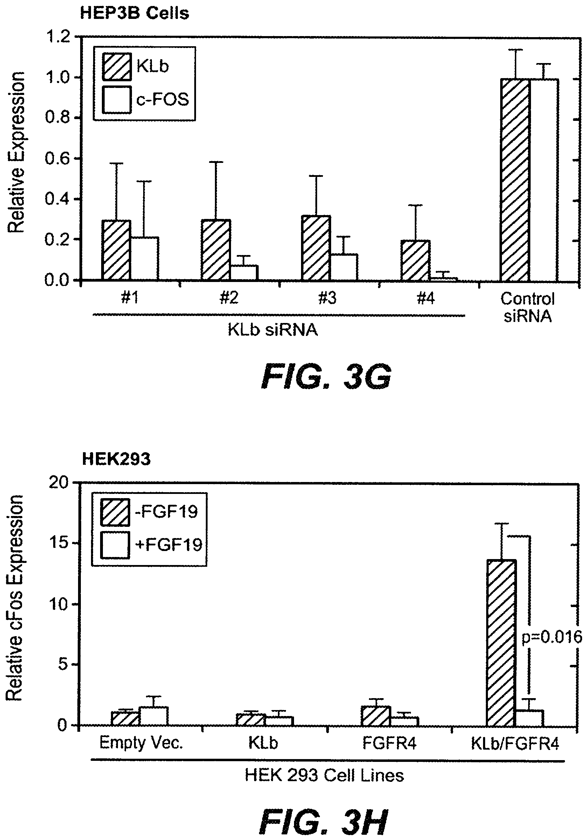

FIGURE 3: KLβ is required for FGF19 downstream modulation of gene expression. (A) FGF19 represses KLβ expression. Cell lines were incubated with FGF19 (100 ng/mL; 0-24 hours) and KLβ expression levels were analyzed by RT-PCR. All values were compared with KLβ expression levels in HEP3B cells at time 0. A triplicate set of data was analyzed for each condition. Data are presented as the mean ± SEM. (B-D) FGF19 promotes expression of c-Fos, JunB, and c-Jun. Cell lines were incubated with FGF19 (100 ng/mL; 0-24 hours) and c-Fos (B), JunB (C), and c-Jun (D) expression were analyzed by RT-PCR. The values represent the relative fold increase in the expression of a particular gene compared with its expression before exposure to FGF19. (E) KLβ siRNA transfection represses KLβ synthesis. HEP3B cells transfected with each of four different KLβ siRNAs were analyzed for KLβ expression by immunoblot. (F) Inhibition of KLβ expression by KLβ siRNA transfection inhibits FGF19 signaling. HEP3B cells transfected with each of four different KLB siRNAs were incubated with vehicle (PBS) or FGF19 (100 ng/mL) for 10 minutes and analyzed for FRS2 and ERK1/2 phosphorylation by immunoblot. (G) Inhibition of KLβ expression by KLβ siRNA transfection inhibits FGF19-mediated c-Fos induction. HEP3B cells transfected with each of four different KLβ siRNAs were incubated with FGF19 (100 ng/mL) for 90 minutes and KLβ and c-Fos expression levels were analyzed by RT-PCR. The values represent the relative expression of each particular gene compared with that of cells transfected with control siRNA. (H) HEK293 cells transfected with either empty (control vector), KLβ, FGFR4, or a combination of FGFR4 and KLβ expression vectors were incubated with PBS or FGF19 (100 ng/mL) for 90 minutes; c-Fos expression was analyzed by RT-PCR. The values represent the fold increase in c-Fos expression compared with the expression levels before cells were exposed to FGF19.

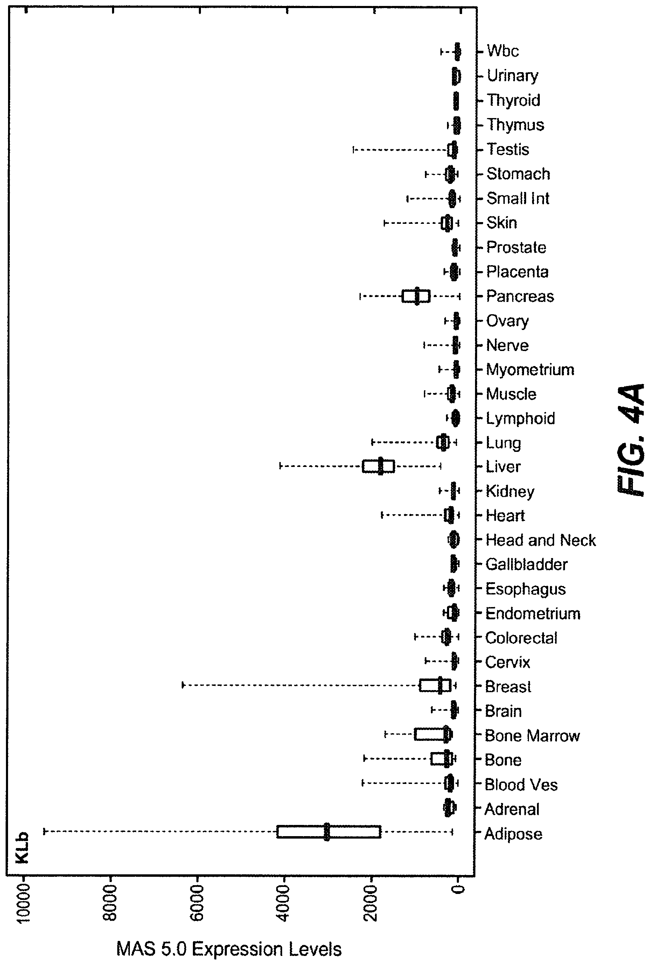

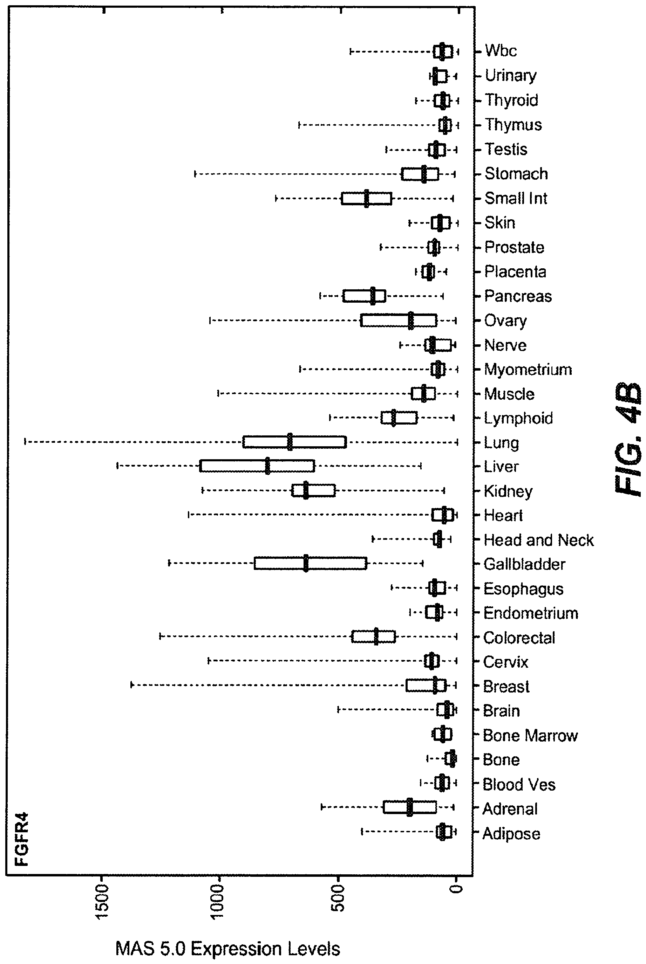

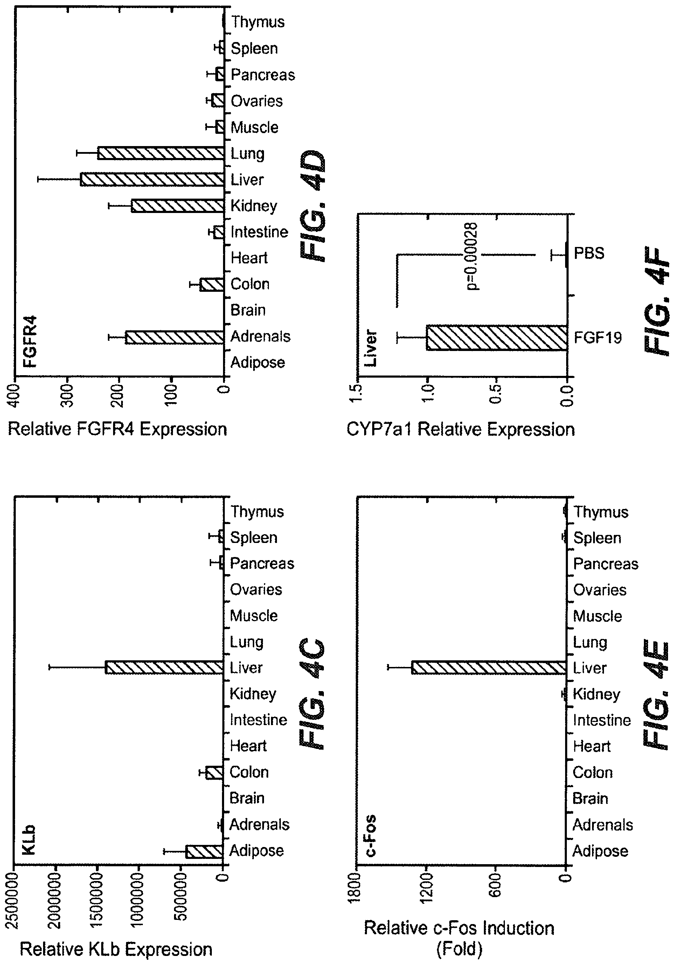

FIGURE 4: KLβ and FGFR4 distribution determine FGF19 tissue-specific activity. KLβ and FGFR4 distribution in human tissues. Whisker-box plots showing KLβ (A) and FGFR4 (B) expression in human tissues, as determined by mRNA analysis of the BioExpress database. The center line indicates the median; the box represents the inter-quartile range between the first and third quartiles. Whiskers extend from the inter-quartile to the positions of extreme values. KLβ (C) and FGFR4 (D) expression in a panel of mouse tissues were determined by RT-PCR. The value for each organ represents the mean expression (n = 3 mice), fold relative to the expression level observed in brain tissues. (E) The tissue specificity of FGF19 in vivo was determined by analyzing c-Fos expression in various organ tissues 30 minutes after mice (n = 3) were injected with PBS or FGF19 (1 mg/kg). The values represent c-Fos expression in mice injected with FGF19, compared with the expression levels in mice injected with PBS. (F) CYP7A1 expression in mouse livers 30 minutes after injection with FGF19 (1 mg/kg) or PBS. The values represent the CYP7A1 expression in mice injected with FGF19 compared with the expression found in mice injected with PBS. A triplicate set of data was analyzed for each condition. Data are presented as the mean ± SEM.

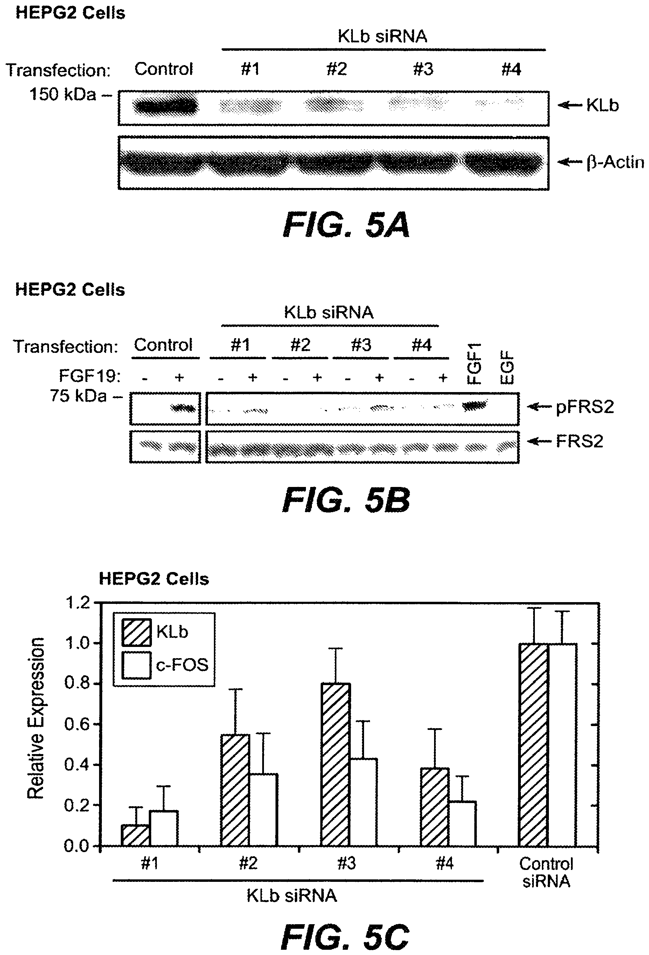

FIGURE 5: KLβ is required for FGF19 downstream modulation of gene expression in HEPG2 cells. (A) KLB siRNA transfection represses KLβ synthesis. Expression of KLβ in HEPG2 cells transfected with each of four different KLβ siRNAs was analyzed by immunoblot. (B) KLβ siRNA transfection inhibits FGF19 signaling. HEPG2 cells transfected with each of four different KLβ siRNAs were incubated with PBS or FGF19 (100 ng/mL) for 10 minutes, lysed, and analyzed for FRS2 phosphorylation levels by immunoblot. (C) KLβ siRNA transfection inhibits FGF19 mediated c-Fos induction. HEP3B cells transfected with each of four different KLβ siRNAs were incubated with FGF19 (100 ng/mL) for 90 minutes and KLβ and c-Fos expression were analyzed by RT-PCR. The values represent the expression of each gene compared with its expression in control siRNA-transfected cells.

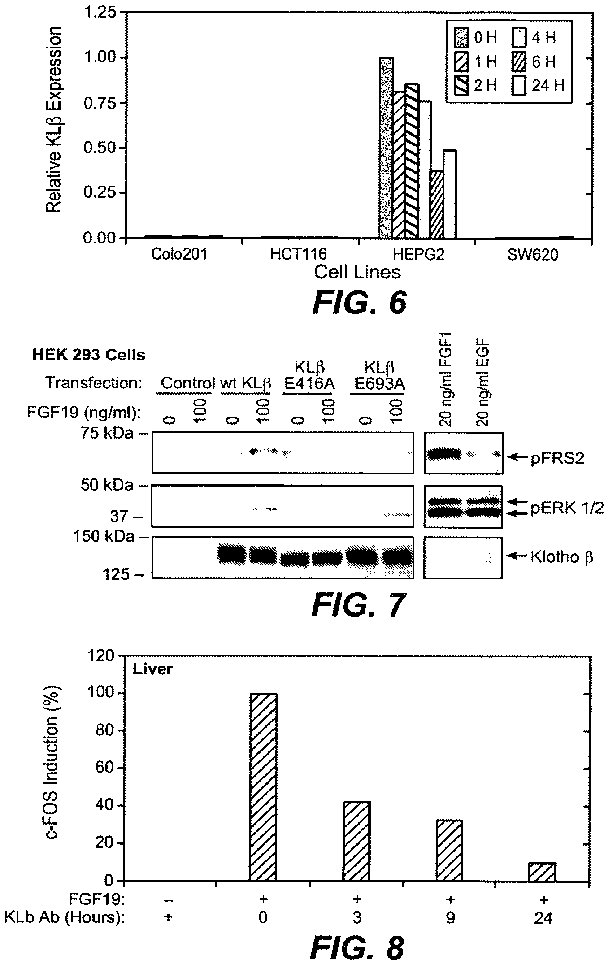

FIGURE 6: Treatment with anti-KLβ antibody inhibits FGF19-mediated c-Fos induction. HEPG2 cells were treated with a control antibody or a polyclonal anti-KLβ antibody that was raised against mouse KLβ but cross-reacts with human KLβ (10 µg/ml). FGF19 stimulated c-Fos induction was measured by RT-PCR. The anti-KLβ antibody treatment inhibited the FGF 19-mediated c-Fos induction whereas the control antibody did not have any significant effect.
FIGURE 7: KLβ active site mutation inhibits FGF19 pathway activation. HEK293 cells were untransfected or transfected with the KLβ E416A (active site) or the KLβ E693A (non-active site) mutant. FGF 19-stimulated activity was assessed by phosphorylated FRS2 and phosphorylated ERK1/2 immunodetection. FGF19 treatment (100 ng/ml; 10 min) yielded an increased in phosphorylated FRS2 and phosphorylated ERK1/2 signal in wildtype (wt) KLβ transfected cells whereas these signals were undetectable in untransfected cells. The FRS2 and ERK1/2 phosphorylation in KLβ E693A mutant transfected cells was comparable to the FRS2 and ERK1/2 phosphorylation in the wildtype KLβ-transfected cells. The FGF19 stimulation in KLβ E416A transfected cells was greatly reduced compared to the wildtype KLβ. These findings corroborate the enhancement of FGF19 signaling by KLβ and further suggest the requirement of KLβ enzymatic activity for FGF19 signaling.
FIGURE 8: KLβ antibody treatment inhibits FGF19-dependent c-Fos induction in mouse liver. FGF19-dependent c-Fos induction was measured in the liver of a mouse treated with a KLβ antibody (2.2 mg/kg) for 0, 3, 9 or 24 hours. Anti-KLβ antibody treatment for 3, 9 or 24 hours before a FGF19 injection (1 mg/kg) reduced the liver specific FGF19-mediated c-Fos induction by 58%, 68% and 91% respectively.

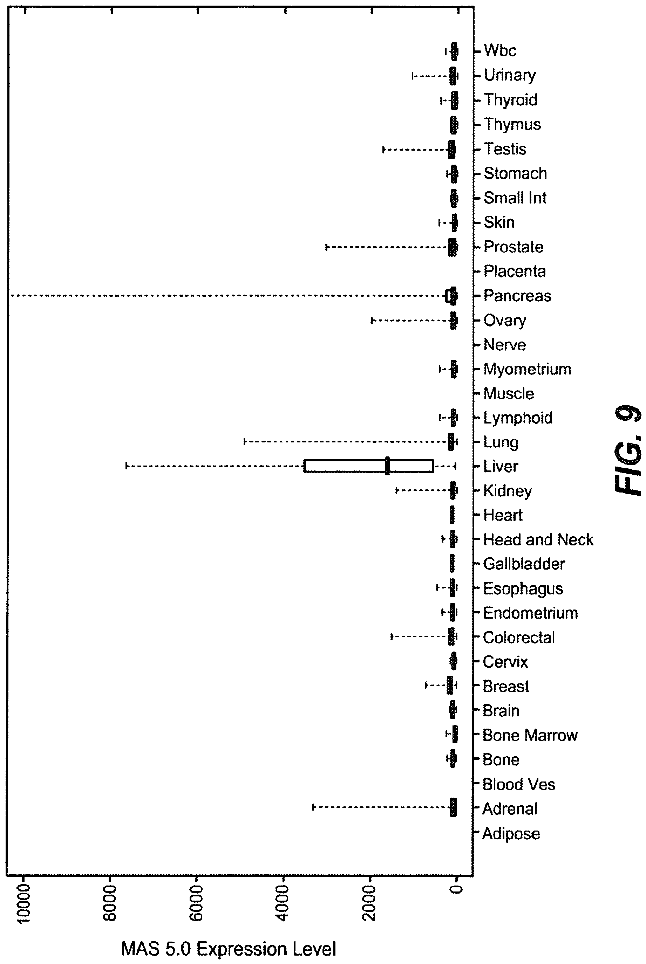

FIGURES 9: Expression of KLβ mRNA was determined in tumor tissues.

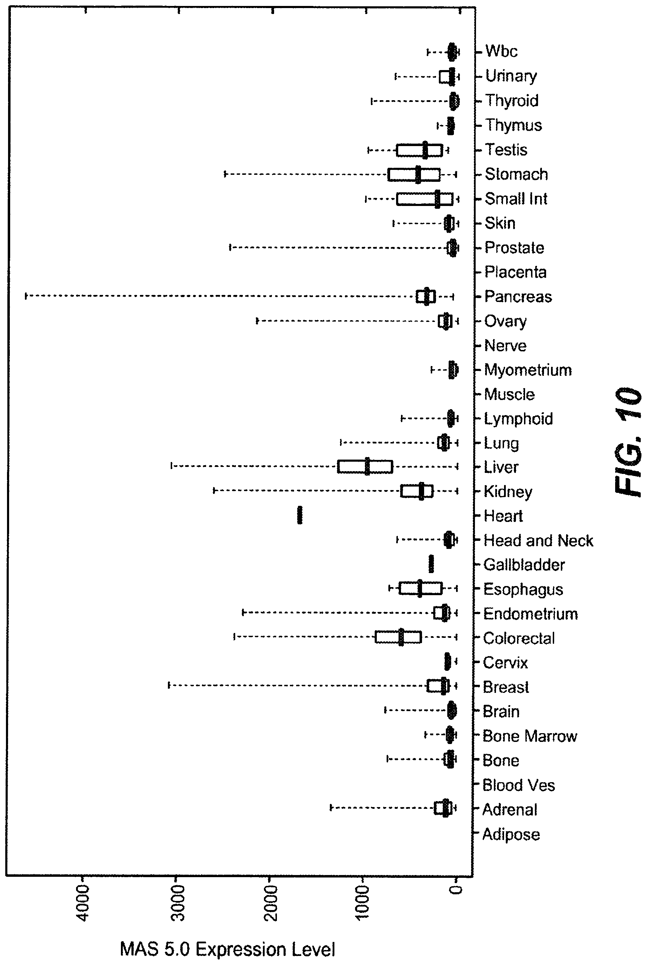

FIGURE 10: Expression of FGFR4 mRNA was determined in tumor tissues.

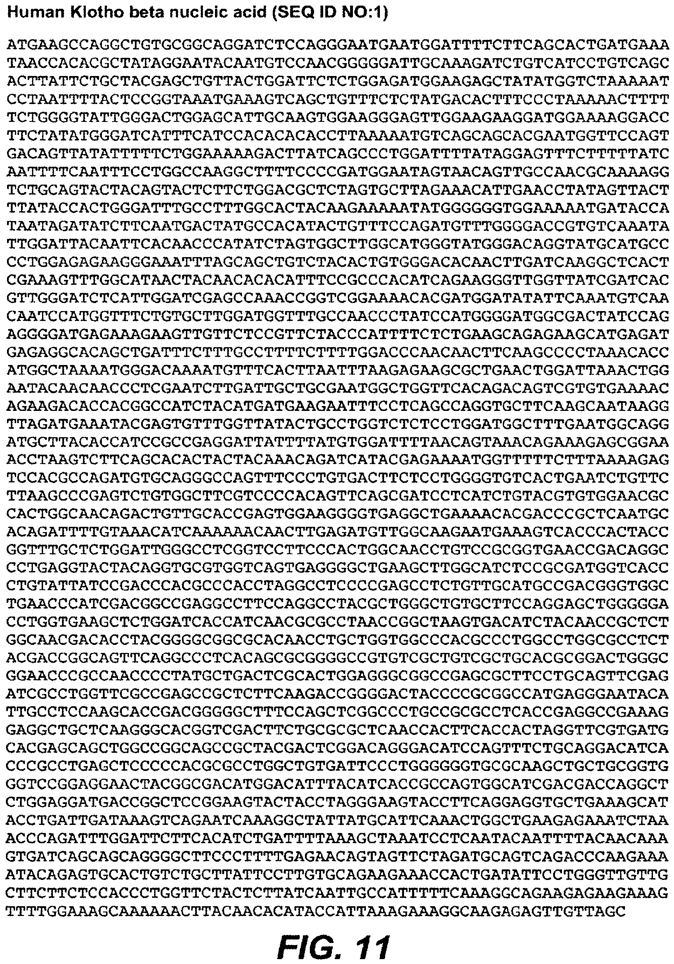

FIGURE 11: depicts an exemplary KLβ nucleic acid sequence (SEQ ID NO:1).

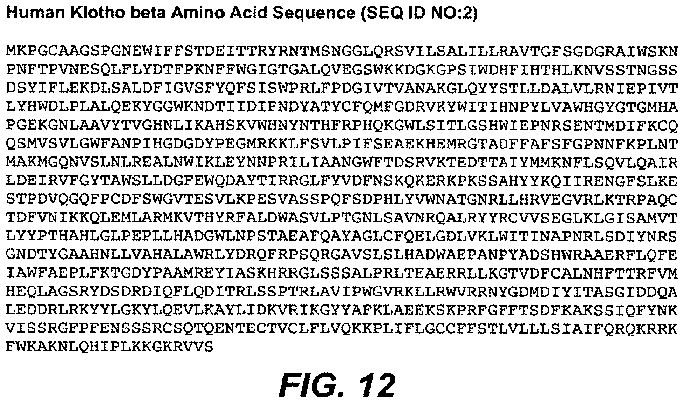

FIGURE 12: depicts an exemplary KLβ amino acid sequence (SEQ ID NO:2).

### DETAILED DESCRIPTION

In one aspect, the present disclosure provides compositions and methods based on binding KLβ. KLβ binding agents, as described herein, provide important therapeutic and diagnostic agents for use in targeting pathological conditions associated with expression and/or activity of the KLβ-FGF19-FGFR4 pathways. Accordingly, the present disclosure provides methods, compositions, kits and articles of manufacture related to KLβ binding. In another aspect, the present disclosure provides methods based on detection of KLβ of FGF (such as FGF19) and/or FGFR (such as FGFR4). The invention is defined in the claims.

### General techniques

The techniques and procedures described or referenced herein are generally well understood and commonly employed using conventional methodology by those skilled in the art, such as, for example, the widely utilized methodologies described in Sambrook et al., Molecular Cloning: A Laboratory Manual 3rd. edition (2001) Cold Spring Harbor Laboratory Press, Cold Spring Harbor, N.Y. CURRENT PROTOCOLS IN MOLECULAR BIOLOGY (F. M. Ausubel, et al. eds., (2003)); the series METHODS IN ENZYMOLOGY (Academic Press, Inc.): PCR 2: A PRACTICAL APPROACH (M. J. MacPherson, B. D. Hames and G. R. Taylor eds. (1995)), Harlow and Lane, eds. (1988) ANTIBODIES, A LABORATORY MANUAL, and ANIMAL CELL CULTURE (R. I. Freshney, ed. (1987)).

### Definitions

An "isolated" antibody is one which has been identified and separated and/or recovered from a component of its natural environment. Contaminant components of its natural environment are materials which would interfere with diagnostic or therapeutic uses for the antibody, and may include enzymes, hormones, and other proteinaceous or nonproteinaceous solutes. In preferred embodiments, the antibody will be purified (1) to greater than 95% by weight of antibody as determined by the Lowry method, and most preferably more than 99% by weight, (2) to a degree sufficient to obtain at least 15 residues of N-terminal or internal amino acid sequence by use of a spinning cup sequenator, or (3) to homogeneity by SDS-PAGE under reducing or nonreducing conditions using Coomassie blue or, preferably, silver stain. Isolated antibody includes the antibody *in situ* within recombinant cells since at least one component of the antibody's natural environment will not be present. Ordinarily, however, isolated antibody will be prepared by at least one purification step.

An "isolated" nucleic acid molecule is a nucleic acid molecule that is identified and separated from at least one contaminant nucleic acid molecule with which it is ordinarily associated in the natural source of the antibody nucleic acid. An isolated nucleic acid molecule is other than in the form or setting in which it is found in nature. Isolated nucleic acid molecules therefore are distinguished from the nucleic acid molecule as it exists in natural cells. However, an isolated nucleic acid molecule includes a nucleic acid molecule contained in cells that ordinarily express the antibody where, for example, the nucleic acid molecule is in a chromosomal location different from that of natural cells.

The term "variable domain residue numbering as in Kabat" or "amino acid position numbering as in Kabat", and variations thereof, refers to the numbering system used for heavy chain variable domains or light chain variable domains of the compilation of antibodies in Kabat et al., Sequences of Proteins of Immunological Interest, 5th Ed. Public Health Service, National Institutes of Health, Bethesda, MD. (1991). Using this numbering system, the actual linear amino acid sequence may contain fewer or additional amino acids corresponding to a shortening of, or insertion into, a FR or CDR of the variable domain. For example, a heavy chain variable domain may include a single amino acid insert (residue 52a according to Kabat) after residue 52 of H2 and inserted residues (e.g. residues 82a, 82b, and 82c, etc according to Kabat) after heavy chain FR residue 82. The Kabat numbering of residues may be determined for a given antibody by alignment at regions of homology of the sequence of the antibody with a "standard" Kabat numbered sequence.

The phrase "substantially similar," or "substantially the same", as used herein, denotes a sufficiently high degree of similarity between two numeric values (generally one associated with an antibody and the other associated with a reference/comparator antibody) such that one of skill in the art would consider the difference between the two values to be of little or no biological and/or statistical significance within the context of the biological characteristic measured by said values (e.g., Kd values). The difference between said two values is preferably less than about 50%, preferably less than about 40%, preferably less than about 30%, preferably less than about 20%, preferably less than about 10% as a function of the value for the reference/comparator antibody.

"Binding affinity" generally refers to the strength of the sum total of noncovalcnt interactions between a single binding site of a molecule (e.g., an antibody) and its binding partner (e.g., an antigen). Unless indicated otherwise, as used herein, "binding affinity" refers to intrinsic binding affinity which reflects a 1:1 interaction between members of a binding pair (e.g., antibody and antigen). The affinity of a molecule X for its partner Y can generally be represented by the dissociation constant (Kd). Affinity can be measured by common methods known in the art, including those described herein. Low-affinity antibodies generally bind antigen slowly and tend to dissociate readily, whereas high-affinity antibodies generally bind antigen faster and tend to remain bound longer. A variety of methods of measuring binding affinity are known in the art, any of which can be used for purposes of the present disclosure. Specific illustrative embodiments are described in the following.

In one embodiment, the "Kd" or "Kd value" is measured by a radiolabeled antigen binding assay (RIA) performed with the Fab version of an antibody of interest and its antigen as described by the following assay that measures solution binding affinity of Fabs for antigen by equilibrating Fab with a minimal concentration of (¹²⁵I)-labeled antigen in the presence of a titration series of unlabeled antigen, then capturing bound antigen with an anti-Fab antibody-coated plate (Chen, et al., (1999) J. Mol Biol 293:865-881). To establish conditions for the assay, microtiter plates (Dynex) are coated overnight with 5 ug/ml of a capturing anti-Fab antibody (Cappel Labs) in 50 mM sodium carbonate (pH 9.6), and subsequently blocked with 2% (w/v) bovine serum albumin in PBS for two to five hours at room temperature (approximately 23°C). In a non-adsorbant plate (Nunc #269620), 100 pM or 26 pM [¹²⁵I]-antigen are mixed with serial dilutions of a Fab of interest (e.g., consistent with assessment of an anti-VEGF antibody, Fab-12, in Presta et al., (1997) Cancer Res. 57:4593-4599). The Fab of interest is then incubated overnight; however, the incubation may continue for a longer period (e.g., 65 hours) to insure that equilibrium is reached. Thereafter, the mixtures are transferred to the capture plate for incubation at room temperature (e.g., for one hour). The solution is then removed and the plate washed eight times with 0.1% Tween-20 in PBS. When the plates have dried, 150 ul/well of scintillant (MicroScint-20; Packard) is added, and the plates are counted on a Topcount gamma counter (Packard) for ten minutes. Concentrations of each Fab that give less than or equal to 20% of maximal binding are chosen for use in competitive binding assays. According to another embodiment the Kd or Kd value is measured by using surface plasmon resonance assays using a BIAcore™-2000 or a BIAcore™-3000 (BIAcore, Inc., Piscataway, NJ) at 25C with immobilized antigen CM5 chips at ∼10 response units (RU). Briefly, carboxymethylated dextran biosensor chips (CM5, BIAcore Inc.) are activated with N-ethyl-N'-(3-dimethylaminopropyl)-carbodiimide hydrochloride (EDC) and N-hydroxysuccinimide (NHS) according to the supplier's instructions. Antigen is diluted with 10mM sodium acetate, pH 4.8, into 5ug/ml (∼0.2uM) before injection at a flow rate of 5ul/minute to achieve approximately 10 response units (RU) of coupled protein. Following the injection of antigen, IM ethanolamine is injected to block unreacted groups. For kinetics measurements, two-fold serial dilutions of Fab (0.78 nM to 500 nM) are injected in PBS with 0.05% Tween 20 (PBST) at 25°C at a flow rate of approximately 25ul/min. Association rates (kₒₙ) and dissociation rates (k_{off}) are calculated using a simple one-to-one Langmuir binding model (BIAcore Evaluation Software version 3.2) by simultaneous fitting the association and dissociation sensorgram. The equilibrium dissociation constant (Kd) is calculated as the ratio k_{off}/kₒₙ. See, e.g., Chen, Y., et al., (1999) J. Mol Biol 293:865-881. If the on-rate exceeds 10⁶ M⁻¹ S⁻¹ by the surface plasmon resonance assay above, then the on-rate can be determined by using a fluorescent quenching technique that measures the increase or decrease in fluorescence emission intensity (excitation = 295 nm; emission = 340 nm, 16 nm band-pass) at 25°C of a 20nM anti-antigen antibody (Fab form) in PBS, pH 7.2, in the presence of increasing concentrations of antigen as measured in a spectrometer, such as a stop-flow equipped spectrophometer (Aviv Instruments) or a 8000-series SLM-Aminco spectrophotometer (ThermoSpectronic) with a stir red cuvette.

An "on-rate" or "rate of association" or "association rate" or "kₒₙ" can also be determined with the same surface plasmon resonance technique described above using a BIAcore™-2000 or a BIAcore™-3000 (BIAcore, Inc., Piscataway, NJ) at 25C with immobilized antigen CM5 chips at -10 response units (RU). Briefly, carboxymethylated dextran biosensor chips (CM5, BIAcore Inc.) are activated with N-ethyl-N'- (3-dimethylaminopropyl)-carbodiimide hydrochloride (EDC) and N-hydroxysuccinimide (NHS) according to the supplier's instructions. Antigen is diluted with 10mM sodium acetate, pH 4.8, into 5ug/ml (∼0.2uM) before injection at a flow rate of 5ul/minute to achieve approximately 10 response units (RU) of coupled protein. Following the injection of antigen, 1M ethanolamine is injected to block unreacted groups. For kinetics measurements, two-fold serial dilutions of Fab (0.78 nM to 500 nM) are injected in PBS with 0.05% Tween 20 (PBST) at 25°C at a flow rate of approximately 25ul/min. Association rates (kₒₙ) and dissociation rates (k_{off}) are calculated using a simple one-to-one Langmuir binding model (BIAcore Evaluation Software version 3.2) by simultaneous fitting the association and dissociation sensorgram. The equilibrium dissociation constant (Kd) was calculated as the ratio k_{off}/kₒₙ. See, e.g., Chen, Y., et al., (1999) J. Mol Biol 293:865-881. However, if the on-rate exceeds 10⁶ M⁻¹ S⁻¹ by the surface plasmon resonance assay above, then the on-rate is preferably determined by using a fluorescent quenching technique that measures the increase or decrease in fluorescence emission intensity (excitation = 295 nm; emission = 340 nm, 16 nm band-pass) at 25°C of a 20nM anti-antigen antibody (Fab form) in PBS, pH 7.2, in the presence of increasing concentrations of antigen as measured in a spectrometer, such as a stop-flow equipped spectrophometer (Aviv Instruments) or a 8000-series SLM-Aminco spectrophotometer (ThermoSpectronic) with a stirred cuvette.

The term "vector," as used herein, is intended to refer to a nucleic acid molecule capable of transporting another nucleic acid to which it has been linked. One type of vector is a "plasmid", which refers to a circular double stranded DNA loop into which additional DNA segments may be ligated. Another type of vector is a phage vector. Another type of vector is a viral vector, wherein additional DNA segments may be ligated into the viral genome. Certain vectors are capable of autonomous replication in a host cell into which they are introduced (e.g., bacterial vectors having a bacterial origin of replication and episomal mammalian vectors). Other vectors (e.g., non-episomal mammalian vectors) can be integrated into the genome of a host cell upon introduction into the host cell, and thereby are replicated along with the host genome. Moreover, certain vectors are capable of directing the expression of genes to which they arc operatively linked. Such vectors are referred to herein as "recombinant expression vectors" (or simply, "recombinant vectors"). In general, expression vectors of utility in recombinant DNA techniques are often in the form of plasmids. In the present specification, "plasmid" and "vector" may be used interchangeably as the plasmid is the most commonly used form of vector.

"Polynucleotide," or "nucleic acid," as used interchangeably herein, refer to polymers of nucleotides of any length, and include DNA and RNA. The nucleotides can be deoxyribonucleotides, ribonucleotides, modified nucleotides or bases, and/or their analogs, or any substrate that can be incorporated into a polymer by DNA or RNA polymerase, or by a synthetic reaction. A polynucleotide may comprise modified nucleotides, such as methylated nucleotides and their analogs. If present, modification to the nucleotide structure may be imparted before or after assembly of the polymer. The sequence of nucleotides may be interrupted by non-nucleotide components. A polynucleotide may be further modified after synthesis, such as by conjugation with a label. Other types of modifications include, for example, "caps", substitution of one or more of the naturally occurring nucleotides with an analog, internucleotide modifications such as, for example, those with uncharged linkages (e.g., methyl phosphonates, phosphotriesters, phosphoamidates, carbamates, etc.) and with charged linkages (e.g., phosphorothioates, phosphorodithioates, etc.), those containing pendant moieties, such as, for example, proteins (e.g., nucleases, toxins, antibodies, signal peptides, ply-L-lysine, etc.), those with intercalators (e.g., acridine, psoralen, etc.), those containing chelators (e.g., metals, radioactive metals, boron, oxidative metals, etc.), those containing alkylators, those with modified linkages (e.g., alpha anomeric nucleic acids, etc.), as well as unmodified forms of the polynucleotide(s). Further, any of the hydroxyl groups ordinarily present in the sugars may be replaced, for example, by phosphonate groups, phosphate groups, protected by standard protecting groups, or activated to prepare additional linkages to additional nucleotides, or may be conjugated to solid or semi-solid supports. The 5' and 3' terminal OH can be phosphorylated or substituted with amines or organic capping group moieties of from 1 to 20 carbon atoms. Other hydroxyls may also be derivatized to standard protecting groups. Polynucleotides can also contain analogous forms of ribose or deoxyribose sugars that are generally known in the art, including, for example, 2'-O-methyl-, 2'-O-allyl, 2'-fluoro- or 2'-azido-ribose, carbocyclic sugar analogs, alpha-anomeric sugars, epimeric sugars such as arabinose, xyloses or lyxoses, pyranose sugars, furanose sugars, sedoheptuloses, acyclic analogs and a basic nucleoside analogs such as methyl riboside. One or more phosphodiester linkages may be replaced by alternative linking groups. These alternative linking groups include, but are not limited to, embodiments wherein phosphate is replaced by P(O)S ("thioate"), P(S)S ("dithioate"), "(O)NR₂ ("amidatc"), P(O)R, P(O)OR', CO or CH₂ ("formacetal"), in which each R or R' is independently H or substituted or unsubstituted alkyl (1-20 C) optionally containing an ether (-O-) linkage, aryl, alkenyl, cycloalkyl, cycloalkenyl or araldyl. Not all linkages in a polynucleotide need be identical. The preceding description applies to all polynucleotides referred to herein, including RNA and DNA.

"Oligonucleotide," as used herein, generally refers to short, generally single stranded, generally synthetic polynucleotides that are generally, but not necessarily, less than about 200 nucleotides in length. The terms "oligonucleotide" and "polynucleotide" are not mutually exclusive. The description above for polynucleotides is equally and fully applicable to oligonucleotides.

The term "Klotho beta" (interchangeably termed "KLβ" or "Beta Klotho" or βKlotho"), as used herein, refers, unless specifically or contextually indicated otherwise, to any native or variant (whether native or synthetic) KLβ polypeptide. The term "native sequence" specifically encompasses naturally occurring truncated or secreted forms (e.g., an extracellular domain sequence), naturally occurring variant forms (e.g., alternatively spliced forms) and naturally-occurring allelic variants. The term "wild type KLβ" generally refers to a polypeptide comprising the amino acid sequence of a naturally occurring KLβ protein. The term "wild type KLβ sequence" generally refers to an amino acid sequence found in a naturally occurring KLβ.

The term "FGF19" (interchangeably termed "Fibroblast growth factor 19"), as used herein, refers, unless specifically or contextually indicated otherwise, to any native or variant (whether native or synthetic) KLβ polypeptide. The term "native sequence" specifically encompasses naturally occurring truncated or secreted forms (e.g., an extracellular domain sequence), naturally occurring variant forms (e.g., alternatively spliced forms) and naturally-occurring allelic variants. The term "wild type KLβ" generally refers to a polypeptide comprising the amino acid sequence of a naturally occurring KLβ protein. The term "wild type KLβ sequence" generally refers to an amino acid sequence found in a naturally occurring KLβ.

A "FGF19 antagonist" refers to a molecule capable of neutralizing, blocking, inhibiting, abrogating, reducing or interfering with the activities of a FGF19 including, for example, binding KLβ (optionally in conjunction with heparin), binding FGFR4 (optionally in conjunction with heparin), binding KLβ and FGFR4 (optionally in conjunction with heparin), promoting FGF 19-mediated induction of cFos, Junb and/or June (in vitro or in vivo), promoting FGFR4 and/or FGF19 down stream signaling (including but not limited to FRS2 phosphorylation, ERK1/2 phosphorylation and Wnt pathway activation), and/or promotion of any biologically relevant FGF19 and/or FGFR4 biological pathway, and/or promotion of a tumor, cell proliferative disorder or a cancer; and/or promotion of a disorder associated with FGF19 expression and/or activity (such as increased FGF19 expression and/or activity). FGF19 antagonists include antibodies and antigen-binding fragments thereof, proteins, peptides, glycoproteins, glycopeptides, glycolipids, polysaccharides, oligosaccharides, nucleic acids, bioorganic molecules, peptidomimetics, pharmacological agents and their metabolites, transcriptional and translation control sequences, and the like. Antagonists also include small molecule inhibitors of a protein, and fusions proteins, receptor molecules and derivatives which bind specifically to protein thereby sequestering its binding to its target, antagonist variants of the protein, siRNA molecules directed to a protein, antisense molecules directed to a protein, RNA aptamers, and ribozymes against a protein. In some embodiments, the FGF19 antagonist is a molecule which binds to FGF19 and neutralizes, blocks, inhibits, abrogates, reduces or interferes with a biological activity of FGF19.

A "KLβ antagonist" refers to a molecule capable of neutralizing, blocking, inhibiting, abrogating, reducing or interfering with the activities of a KLβ including, for example, binding FGFR (e.g., FGFR4) (optionally in conjunction with heparin), binding FGF (e.g. FGF19) (optionally in conjunction with heparin), binding FGFR4 and FGF19 (optionally in conjunction with heparin), promoting FGF19-mediated induction of cFos, Junb and/or June (in vitro or in vivo), promoting FGFR4 and/or FGF19 down stream signaling (including but not limited to FRS2 phosphorylation, ERK1/2 phosphorylation and Wnt pathway activation), and/or promotion of any biologically relevant KLβ and/or FGFR4 biological pathway, and/or promotion of a tumor, cell proliferative disorder or a cancer; and/or promotion of a disorder associated with KLβ expression and/or activity (such as increased KLβ expression and/or activity). KLβ antagonists include antibodies and antigen-binding fragments thereof, proteins, peptides, glycoproteins, glycopeptides, glycolipids, polysaccharides, oligosaccharides, nucleic acids, bioorganic molecules, peptidomimetics, pharmacological agents and their metabolites, transcriptional and translation control sequences, and the like. Antagonists also include small molecule inhibitors of a protein, and fusions proteins, receptor molecules and derivatives which bind specifically to protein thereby sequestering its binding to its target, antagonist variants of the protein, siRNA molecules directed to a protein, antisense molecules directed to a protein, RNA aptamers, and ribozymes against a protein. In some embodiments, the KLβ antagonist is a molecule which binds to KLβ and neutralizes, blocks, inhibits, abrogates, reduces or interferes with a biological activity of KLβ.

The term "FGFR4" (interchangeably termed "Fibroblast growth factor receptor 4"), as used herein, refers, unless specifically or contextually indicated otherwise, to any native or variant (whether native or synthetic) FGFR4 polypeptide. The term "native sequence" specifically encompasses naturally occurring truncated or secreted forms (e.g., an extracellular domain sequence), naturally occurring variant forms (e.g., alternatively spliced forms) and naturally-occurring allelic variants. The term "wild type FGFR4" generally refers to a polypeptide comprising the amino acid sequence of a naturally occurring FGFR4 protein. The term "wild type FGFR4 sequence" generally refers to an amino acid sequence found in a naturally occurring FGFR4.

A "FGFR antagonist" refers to a molecule capable of neutralizing, blocking, inhibiting, abrogating, reducing or interfering with the activities of a FGF receptor ("FGFR") including, for example, binding KLβ (optionally in conjunction with heparin), binding FGF (e.g., FGF19) (optionally in conjunction with heparin), binding KLβ and FGF (e.g., FGF19) (optionally in conjunction with heparin), promoting FGF 19-mediated induction of cFos, Junb and/or Junc (in vitro or in vivo), promoting FGFR and/or FGF down stream signaling (including but not limited to FRS2 phosphorylation, ERK1/2 phosphorylation and Wnt pathway activation), and/or promotion of any biologically relevant FGF and/or FGFR biological pathway, and/or promotion of a tumor, cell proliferative disorder or a cancer; and/or promotion of a disorder associated with FGFR expression and/or activity (such as increased FGFR expression and/or activity). FGFR antagonists include antibodies and antigen-binding fragments thereof, proteins, peptides, glycoproteins, glycopeptides, glycolipids, polysaccharides, oligosaccharides, nucleic acids, bioorganic molecules, peptidomimetics, pharmacological agents and their metabolites, transcriptional and translation control sequences, and the like. Antagonists also include small molecule inhibitors of a protein, and fusions proteins, receptor molecules and derivatives which bind specifically to protein thereby sequestering its binding to its target, antagonist variants of the protein, siRNA molecules directed to a protein, antisense molecules directed to a protein, RNA aptamers, and ribozymes against a protein. In some embodiments, the FGFR antagonist (e.g., FGFR4 antagonist) is a molecule which binds to FGFR and neutralizes, blocks, inhibits, abrogates, reduces or interferes with a biological activity of FGFR.

The terms "antibody" and "immunoglobulin" are used interchangeably in the broadest sense and include monoclonal antibodies (for e.g., full length or intact monoclonal antibodies), polyclonal antibodies, multivalent antibodies, multispecific antibodies (e.g., bispecific antibodies so long as they exhibit the desired biological activity) and may also include certain antibody fragments (as described in greater detail herein). An antibody can be human, humanized and/or affinity matured.

The term "variable" refers to the fact that certain portions of the variable domains differ extensively in sequence among antibodies and are used in the binding and specificity of each particular antibody for its particular antigen. However, the variability is not evenly distributed throughout the variable domains of antibodies. It is concentrated in three segments called complementarity-determining regions (CDRs) or hypervariable regions both in the light-chain and the heavy-chain variable domains. The more highly conserved portions of variable domains are called the framework (FR). The variable domains of native heavy and light chains each comprise four FR regions, largely adopting a β-sheet configuration, connected by three CDRs, which form loops connecting, and in some cases forming part of, the β-sheet structure. The CDRs in each chain are held together in close proximity by the FR regions and, with the CDRs from the other chain, contribute to the formation of the antigen-binding site of antibodies (see Kabat et al., Sequences of Proteins of Immunological Interest, Fifth Edition, National Institute of Health, Bethesda, MD (1991)). The constant domains are not involved directly in binding an antibody to an antigen, but exhibit various effector functions, such as participation of the antibody in antibody-dependent cellular toxicity.

Papain digestion of antibodies produces two identical antigen-binding fragments, called "Fab" fragments, each with a single antigen-binding site, and a residual "Fc" fragment, whose name reflects its ability to crystallize readily. Pepsin treatment yields an F(ab')₂ fragment that has two antigen-combining sites and is still capable of cross-linking antigen.

"Fv" is the minimum antibody fragment which contains a complete antigen-recognition and -binding site. In a two-chain Fv species, this region consists of a dimer of one heavy- and one light-chain variable domain in tight, non-covalent association. In a single-chain Fv species, one heavy- and one light-chain variable domain can be covalently linked by a flexible peptide linker such that the light and heavy chains can associate in a "dimeric" structure analogous to that in a two-chain Fv species. It is in this configuration that the three CDRs of each variable domain interact to define an antigen-binding site on the surface of the VH-VL dimer. Collectively, the six CDRs confer antigen-binding specificity to the antibody. However, even a single variable domain (or half of an Fv comprising only three CDRs specific for an antigen) has the ability to recognize and bind antigen, although at a lower affinity than the entire binding site.

The Fab fragment also contains the constant domain of the light chain and the first constant domain (CH1) of the heavy chain. Fab' fragments differ from Fab fragments by the addition of a few residues at the carboxy terminus of the heavy chain CH1 domain including one or more cysteines from the antibody hinge region. Fab'-SH is the designation herein for Fab' in which the cysteine residue(s) of the constant domains bear a free thiol group. F(ab')₂ antibody fragments originally were produced as pairs of Fab' fragments which have hinge cysteines between them. Other chemical couplings of antibody fragments are also known.

The "light chains" of antibodies (immunoglobulins) from any vertebrate species can be assigned to one of two clearly distinct types, called kappa (κ) and lambda (λ), based on the amino acid sequences of their constant domains.

Depending on the amino acid sequence of the constant domain of their heavy chains, immunoglobulins can be assigned to different classes. There are five major classes of immunoglobulins: IgA, IgD, IgE, IgG, and IgM, and several of these can be further divided into subclasses (isotypes), e.g., IgG₁, IgG₂, IgG₃, IgG₄, IgA₁, and IgA₂. The heavy-chain constant domains that correspond to the different classes of immunoglobulins are called α, δ, ε, γ, and µ, respectively. The subunit structures and three-dimensional configurations of different classes of immunoglobulins are well known.

"Antibody fragments" comprise only a portion of an intact antibody, wherein the portion preferably retains at least one, preferably most or all, of the functions normally associated with that portion when present in an intact antibody. Examples of antibody fragments include Fab, Fab', F(ab')2, and Fv fragments; diabodies; linear antibodies; single-chain antibody molecules; and multispecific antibodies formed from antibody fragments. In one embodiment, an antibody fragment comprises an antigen binding site of the intact antibody and thus retains the ability to bind antigen. In another embodiment, an antibody fragment, for example one that comprises the Fc region, retains at least one of the biological functions normally associated with the Fc region when present in an intact antibody, such as FcRn binding, antibody half life modulation, ADCC function and complement binding. In one embodiment, an antibody fragment is a monovalent antibody that has an in vivo half life substantially similar to an intact antibody. For e.g., such an antibody fragment may comprise on antigen binding arm linked to an Fc sequence capable of conferring in vivo stability to the fragment.

The term "hypervariable region", "HVR", or "HV", when used herein refers to the regions of an antibody variable domain which are hypervariable in sequence and/or form structurally defined loops. Generally, antibodies comprise six hypervariable regions; three in the VH (H1, H2, H3), and three in the VL (L1, L2, L3). A number of hypervariable region delineations are in use and are encompassed herein. The Kabat Complementarity Determining Regions (CDRs) are based on sequence variability and are the most commonly used (Kabat et al., Sequences of Proteins of Immunological Interest, 5th Ed. Public Health Service, National Institutes of Health, Bethesda, MD. (1991)). Chothia refers instead to the location of the structural loops (Chothia and Lesk J. Mol. Biol. 196:901-917 (1987)). The AbM hypervariable regions represent a compromise between the Kabat CDRs and Chothia structural loops, and are used by Oxford Molccular's AbM antibody modeling software. The "contact" hypervariable regions are based on an analysis of the available complex crystal structures.

Hypervariable regions may comprise "extended hypervariable regions" as follows: 24-36 (L1), 46-56 (L2) and 89-97 (L3) in the VL and 26-35 (H1), 49-65 or 50 to 65 (H2) and 93-102 (H3) in the VH. The variable domain residues are numbered according to Kabat et al., supra for each of these definitions.

"Framework" or "FR" residues are those variable domain residues other than the hypervariable region residues as herein defined.

"Humanized" forms of non-human (e.g., murine) antibodies are chimeric antibodies that contain minimal sequence derived from non-human immunoglobulin. For the most part, humanized antibodies arc human immunoglobulins (recipient antibody) in which residues from a hypervariable region of the recipient are replaced by residues from a hypervariable region of a non-human species (donor antibody) such as mouse, rat, rabbit or nonhuman primate having the desired specificity, affinity, and capacity. In some instances, framework region (FR) residues of the human immunoglobulin arc replaced by corresponding non-human residues. Furthermore, humanized antibodies may comprise residues that are not found in the recipient antibody or in the donor antibody. These modifications are made to further refine antibody performance. In general, the humanized antibody will comprise substantially all of at least one, and typically two, variable domains, in which all or substantially all of the hypervariable loops correspond to those of a non-human immunoglobulin and all or substantially all of the FRs are those of a human immunoglobulin sequence. The humanized antibody optionally will also comprise at least a portion of an immunoglobulin constant region (Fc), typically that of a human immunoglobulin. For further details, see Jones et al., Nature 321:522-525 (1986); Riechmann et al., Nature 332:323-329 (1988); and Presta, Curr. Op. Struct. Biol. 2:593-596 (1992). See also the following review articles and references cited therein: Vaswani and Hamilton, Ann. Allergy, Asthma & Immunol. 1:105-115 (1998); Harris, Biochem. Soc. Transactions 23:1035-1038 (1995); Hurle and Gross, Curr. Op. Biotech. 5:428-433 (1994).

"Chimeric" antibodies (immunoglobulins) have a portion of the heavy and/or light chain identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (U.S. Patent No. 4,816,567; and Morrison et al., Proc. Natl. Acad. Sci. USA 81:6851-6855 (1984)). Humanized antibody as used herein is a subset of chimeric antibodies.

"Single-chain Fv" or "scFv" antibody fragments comprise the VH and VL domains of antibody, wherein these domains are present in a single polypeptide chain. Generally, the scFv polypeptide further comprises a polypeptide linker between the VH and VL domains which enables the scFv to form the desired structure for antigen binding. For a review of scFv see Pluckthun, in The Pharmacology of Monoclonal Antibodies, vol. 113, Rosenburg and Moore eds., Springer-Verlag, New York, pp. 269-315 (1994).

An "antigen" is a predetermined antigen to which an antibody can selectively bind. The target may be polypeptide, carbohydrate, nucleic acid, lipid, hapten or other naturally occurring or synthetic compound. Preferably, the target is a polypeptide.

The term "diabodies" refers to small antibody fragments with two antigen-binding sites, which fragments comprise a heavy-chain variable domain (VH) connected to a light-chain variable domain (VL) in the same polypeptide chain (VH - VL). By using a linker that is too short to allow pairing between the two domains on the same chain, the domains are forced to pair with the complementary domains of another chain and create two antigen-binding sites. Diabodies are described more fully in, for example, EP 404,097; WO 93/11161; and Hollinger et al., Proc. Natl. Acad. Sci. USA, 90:6444-6448 (1993).

A "human antibody" is one which possesses an amino acid sequence which corresponds to that of an antibody produced by a human and/or has been made using any of the techniques for making human antibodies as disclosed herein. This definition of a human antibody specifically excludes a humanized antibody comprising non-human antigen-binding residues.

An "affinity matured" antibody is one with one or more alterations in one or more CDRs thereof which result in an improvement in the affinity of the antibody for antigen, compared to a parent antibody which does not possess those alteration(s). Preferred affinity matured antibodies will have nanomolar or even picomolar affinities for the target. Affinity matured antibodies are produced by procedures known in the art. Marks et al. Bio/Technology 10:779-783 (1992) describes affinity maturation by VH and VL domain shuffling. Random mutagenesis of CDR and/or framework residues is described by: Barbas et al. Proc Nat. Acad. Sci, USA 91:3809-3813 (1994); Schier et al. Gene 169:147-155 (1995); Yelton et al. J. Immunol. 155:1994-2004 (1995); Jackson et al., J. Immunol. 154(7):3310-9 (1995); and Hawkins et al, J. Mol. Biol. 226:889-896 (1992).

Antibody "effector functions" refer to those biological activities attributable to the Fc region (a native sequence Fc region or amino acid sequence variant Fc region) of an antibody, and vary with the antibody isotype. Examples of antibody effector functions include: C1q binding and complement dependent cytotoxicity; Fc receptor binding; antibody-dependent cell-mediated cytotoxicity (ADCC); phagocytosis; down regulation of cell surface receptors (*e.g*. B cell receptor); and B cell activation.

Antibody-dependent cell-mediated cytotoxicity" or "ADCC" refers to a form of cytotoxicity in which secreted Ig bound onto Fc receptors (FcRs) present on certain cytotoxic cells (*e.g*. Natural Killer (NK) cells, neutrophils, and macrophages) enable these cytotoxic effector cells to bind specifically to an antigen-bearing target cell and subsequently kill the target cell with cytotoxins. The antibodies "arm" the cytotoxic cells and are absolutely required for such killing. The primary cells for mediating ADCC, NK cells, express FcγRIII only, whereas monocytes express FcγRI, FcγRII and FcγRIII. FcR expression on hematopoietic cells is summarized in Table 3 on page 464 of Ravetch and Kinet, Annu. Rev. Immunol 9:457-92 (1991). To assess ADCC activity of a molecule of interest, an *in vitro* ADCC assay, such as that described in US Patent No. 5,500,362 or 5,821,337 or Presta U.S. Patent No. 6,737,056 may be performed. Useful effector cells for such assays include peripheral blood mononuclear cells (PBMC) and Natural Killer (NK) cells. Alternatively, or additionally, ADCC activity of the molecule of interest may be assessed *in vivo,* e.g., in a animal model such as that disclosed in Clynes et al. PNAS (USA) 95:652-656 (1998).

"Human effector cells" are leukocytes which express one or more FcRs and perform effector functions. Preferably, the cells express at least FcγRIII and perform ADCC effector function. Examples of human leukocytes which mediate ADCC include peripheral blood mononuclear cells (PBMC), natural killer (NK) cells, monocytes, cytotoxic T cells and neutrophils; with PBMCs and NK cells being preferred. The effector cells may be isolated from a native source, *e.g*. from blood.

"Fc receptor" or "FcR" describes a receptor that binds to the Fc region of an antibody. The preferred FcR is a native sequence human FcR. Moreover, a preferred FcR is one which binds an IgG antibody (a gamma receptor) and includes receptors of the FcγRI, FcγRII, and FcγRIII subclasses, including allelic variants and alternatively spliced forms of these receptors. FcγRII receptors include FcγRIIA (an "activating receptor") and FcγRIIB (an "inhibiting receptor"), which have similar amino acid sequences that differ primarily in the cytoplasmic domains thereof. Activating receptor FcγRIIA contains an immunoreceptor tyrosine-based activation motif (ITAM) in its cytoplasmic domain. Inhibiting receptor FcγRIIB contains an immunoreceptor tyrosine-based inhibition motif (ITIM) in its cytoplasmic domain. (see review M. in Daëron, Annu. Rev. Immunol. 15:203-234 (1997)). FcRs are reviewed in Ravetch and Kinet, Annu. Rev. Immunol 9:457-92 (1991); Capel et al., Immunomethods 4:25-34 (1994); and de Haas et al., J. Lab. Clin. Med. 126:330-41 (1995). Other FcRs, including those to be identified in the future, are encompassed by the term "FcR" herein. The term also includes the neonatal receptor, FcRn, which is responsible for the transfer of maternal IgGs to the fetus (Guyer et al., J. Immunol. 117:587 (1976) and Kim et al., J. Immunol. 24:249 (1994)) and regulates homeostasis of immunoglobulins.
WO00/42072 (Presta) describes antibody variants with improved or diminished binding to FcRs. See, also, Shields et al. J. Biol. Chem. 9(2): 6591-6604 (2001).

Methods of measuring binding to FcRn are known (see, e.g., Ghetie 1997, Hinton 2004). Binding to human FcRn in vivo and serum half life of human FcRn high affinity binding polypeptides can be assayed, e.g., in transgenic mice or transfected human cell lines expressing human FcRn, or in primates administered with the Fc variant polypeptides.

"Complement dependent cytotoxicity" or "CDC" refers to the lysis of a target cell in the presence of complement. Activation of the classical complement pathway is initiated by the binding of the first component of the complement system (C1q) to antibodies (of the appropriate subclass) which are bound to their cognate antigen. To assess complement activation, a CDC assay, *e.g.* as described in Gazzano-Santoro et al., J. Immunol. Methods 202:163 (1996), may be performed.

Polypeptide variants with altered Fc region amino acid sequences and increased or decreased C1q binding capability are described in US patent No. 6,194,551 B1 and WO99/51642. See, also, Idusogie et al. J. Immunol. 164: 4178-4184 (2000).

A "blocking" antibody or an "antagonist" antibody is one which inhibits or reduces biological activity of the antigen it binds. Preferred blocking antibodies or antagonist antibodies substantially or completely inhibit the biological activity of the antigen.

A "biological sample" (interchangeably termed "sample" or "tissue or cell sample") encompasses a variety of sample types obtained from an individual and can be used in a diagnostic or monitoring assay. The definition encompasses blood and other liquid samples of biological origin, solid tissue samples such as a biopsy specimen or tissue cultures or cells derived therefrom, and the progeny thereof. The definition also includes samples that have been manipulated in any way after their procurement, such as by treatment with reagents, solubilization, or enrichment for certain components, such as proteins or polynucleotides, or embedding in a semi-solid or solid matrix for sectioning purposes. The term "biological sample" encompasses a clinical sample, and also includes cells in culture, cell supernatants, cell lysates, serum, plasma, biological fluid, and tissue samples. The source of the biological sample may be solid tissue as from a fresh, frozen and/or preserved organ or tissue sample or biopsy or aspirate; blood or any blood constituents; bodily fluids such as cerebral spinal fluid, amniotic fluid, peritoneal fluid, or interstitial fluid; cells from any time in gestation or development of the subject. In some embodiments, the biological sample is obtained from a primary or metastatic tumor. The biological sample may contain compounds which are not naturally intermixed with the tissue in nature such as preservatives, anticoagulants, buffers, fixatives, nutrients, antibiotics, or the like.

For the purposes herein a "section" of a tissue sample is meant a single part or piece of a tissue sample, *e.g.* a thin slice of tissue or cells cut from a tissue sample. It is understood that multiple sections of tissue samples may be taken and subjected to analysis according to the present disclosure. In some embodiments, the same section of tissue sample is analyzed at both morphological and molecular levels, or is analyzed with respect to both protein and nucleic acid.

The word "label" when used herein refers to a compound or composition which is conjugated or fused directly or indirectly to a reagent such as a nucleic acid probe or an antibody and facilitates detection of the reagent to which it is conjugated or fused. The label may itself be detectable (*e.g*., radioisotope labels or fluorescent labels) or, in the case of an enzymatic label, may catalyze chemical alteration of a substrate compound or composition which is detectable.

A "medicament" is an active drug to treat the disorder in question or its symptoms, or side effects.

A "disorder" or "disease" is any condition that would benefit from treatment with a substance/molecule or method. This includes chronic and acute disorders or diseases including those pathological conditions which predispose the mammal to the disorder in question. Non-limiting examples of disorders to be treated herein include malignant and benign tumors; carcinoma, blastoma, and sarcoma.

The terms "cell proliferative disorder" and "proliferative disorder" refer to disorders that are associated with some degree of abnormal cell proliferation. In one embodiment, the cell proliferative disorder is cancer.

"Tumor", as used herein, refers to all neoplastic cell growth and proliferation, whether malignant or benign, and all pre-cancerous and cancerous cells and tissues. The terms "cancer", "cancerous", "cell proliferative disorder", "proliferative disorder" and "tumor" are not mutually exclusive as referred to herein.

The terms "cancer" and "cancerous" refer to or describe the physiological condition in mammals that is typically characterized by unregulated cell growth/proliferation. Examples of cancer include but are not limited to, carcinoma, lymphoma, blastoma, sarcoma, and leukemia. More particular examples of such cancers include squamous cell cancer, small-cell lung cancer, pituitary cancer, esophageal cancer, astrocytoma, soft tissue sarcoma, non-small cell lung cancer, adenocarcinoma of the lung, squamous carcinoma of the lung, cancer of the peritoneum, hepatocellular cancer, gastrointestinal cancer, pancreatic cancer, glioblastoma, cervical cancer, ovarian cancer, liver cancer, bladder cancer, hepatoma, breast cancer, colon cancer, colorectal cancer, endometrial or uterine carcinoma, salivary gland carcinoma, kidney cancer, liver cancer, prostate cancer, vulval cancer, thyroid cancer, hepatic carcinoma, brain cancer, endometrial cancer, testis cancer, cholangiocarcinoma, gallbladder carcinoma, gastric cancer, melanoma, and various types of head and neck cancer. Dysregulation of angiogenesis can lead to many disorders that can be treated by compositions and methods of the invention. These disorders include both non-neoplastic and neoplastic conditions. Neoplastics include but are not limited those described above. Non-neoplastic disorders include but are not limited to undesired or aberrant hypertrophy, arthritis, rheumatoid arthritis (RA), psoriasis, psoriatic plaques, sarcoidosis, atherosclerosis, atherosclerotic plaques, diabetic and other proliferative retinopathies including retinopathy of prematurity, retrolental fibroplasia, neovascular glaucoma, age-related macular degeneration, diabetic macular edema, corneal neovascularization, corneal graft neovascularization, corneal graft rejection, retinal/choroidal neovascularization, neovascularization of the angle (rubeosis), ocular neovascular disease, vascular restenosis, artcriovenous malformations (AVM), meningioma, hemangioma, angiofibroma, thyroid hyperplasias (including Grave's disease), corneal and other tissue transplantation, chronic inflammation, lung inflammation, acute lung injury/ARDS, sepsis, primary pulmonary hypertension, malignant pulmonary effusions, cerebral edema (e.g., associated with acute stroke/ closed head injury/ trauma), synovial inflammation, pannus formation in RA, myositis ossificans, hypertropic bone formation, osteoarthritis (OA), refractory ascites, polycystic ovarian disease, endometriosis, 3rd spacing of fluid diseases (pancreatitis, compartment syndrome, bums, bowel disease), uterine fibroids, premature labor, chronic inflammation such as IBD (Crohn's disease and ulcerative colitis), renal allograft rejection, inflammatory bowel disease, nephrotic syndrome, undesired or aberrant tissue mass growth (non-cancer), hemophilic joints, hypertrophic scars, inhibition of hair growth, Osler-Weber syndrome, pyogenic granuloma retrolental fibroplasias, scleroderma, trachoma, vascular adhesions, synovitis, dermatitis, preeclampsia, ascites, pericardial effusion (such as that associated with pericarditis), and pleural effusion.

The term "wasting" disorders (e.g., wasting syndrome, cachexia, sarcopenia) refers to a disorder caused by undesirable and/or unhealthy loss of weight or loss of body cell mass. In the elderly as well as in AIDS and cancer patients, wasting disease can result in undesired loss of body weight, including both the fat and the fat-free compartments. Wasting diseases can be the result of inadequate intake of food and/or metabolic changes related to illness and/or the aging process. Cancer patients and AIDS patients, as well as patients following extensive surgery or having chronic infections, immunologic diseases, hyperthyroidism, Crohn's disease, psychogenic disease, chronic heart failure or other severe trauma, frequently suffer from wasting disease which is sometimes also referred to as cachexia, a metabolic and, sometimes, an eating disorder. Cachexia is additionally characterized by hypermetabolism and hypercatabolism. Although cachexia and wasting disease are frequently used interchangeably to refer to wasting conditions, there is at least one body of research which differentiates cachexia from wasting syndrome as a loss of fat-free mass, and particularly, body cell mass (Mayer, 1999, J. Nutr. 129(1S Suppl.):256S-259S). Sarcopenia, yet another such disorder which can affect the aging individual, is typically characterized by loss of muscle mass. End stage wasting disease as described above can develop in individuals suffering from either cachexia or sarcopenia.

As used herein, "treatment" refers to clinical intervention in an attempt to alter the natural course of the individual or cell being treated, and can be performed either for prophylaxis or during the course of clinical pathology. Desirable effects of treatment include preventing occurrence or recurrence of disease, alleviation of symptoms, diminishment of any direct or indirect pathological consequences of the disease, decreasing the rate of disease progression, amelioration or palliation of the disease state, and remission or improved prognosis. In some embodiments, antibodies arc used to delay development of a disease or disorder.

An "anti-angiogenesis agent" or "angiogenesis inhibitor" refers to a small molecular weight substance, a polynucleotide, a polypeptide, an isolated protein, a recombinant protein, an antibody, or conjugates or fusion proteins thereof, that inhibits angiogenesis, vasculogenesis, or undesirable vascular permeability, either directly or indirectly. For example, an anti-angiogenesis agent is an antibody or other antagonist to an angiogenic agent as defined above, e.g., antibodies to VEGF, antibodies to VEGF receptors, small molecules that block VEGF receptor signaling (e.g., PTK787/ZK2284, SU6668, SUTENT/SU1 1248 (sunitinib malate), AMG706). Anti-angiogensis agents also include native angiogenesis inhibitors, e.g., angiostatin, endostatin, etc. See, e.g., Klagsbrun and D'Amore, Annu. Rev. Physiol., 53:217-39 (1991); Streit and Detmar, Oncogene, 22:3172-3179 (2003) (e.g., Table 3 listing anti-angiogenic therapy in malignant melanoma); Ferrara & Alitalo, Nature Medicine 5(12):1359-1364 (1999); Tonini et al., Oncogene, 22:6549-6556 (2003) (e.g., Table 2 listing antiangiogenic factors); and, Sato Int. J. Clin. Oncol., 8:200-206 (2003) (e.g., Table 1 lists Anti-angiogenic agents used in clinical trials).

An "individual" is a vertebrate, preferably a mammal, more preferably a human. Mammals include, but are not limited to, farm animals (such as cows), sport animals, pets (such as cats, dogs and horses), primates, mice and rats.

"Mammal" for purposes of treatment refers to any animal classified as a mammal, including humans, domestic and farm animals, and zoo, sports, or pet animals, such as dogs, horses, cats, cows, *etc.* Preferably, the mammal is human.

An "effective amount" refers to an amount effective, at dosages and for periods of time necessary, to achieve the desired therapeutic or prophylactic result.

A "therapeutically effective amount" of a substance/molecule of the invention, agonist or antagonist may vary according to factors such as the disease state, age, sex, and weight of the individual, and the ability of the substance/molecule, agonist or antagonist to elicit a desired response in the individual. A therapeutically effective amount is also one in which any toxic or detrimental effects of the substance/molecule, agonist or antagonist are outweighed by the therapeutically beneficial effects. A "prophylactically effective amount" refers to an amount effective, at dosages and for periods of time necessary, to achieve the desired prophylactic result. Typically but not necessarily, since a prophylactic dose is used in subjects prior to or at an earlier stage of disease, the prophylactically effective amount will be less than the therapeutically effective amount.

"Conditions related to obesity" refer to conditions which are the result of or which are exasperated by obesity, such as, but not limited to dermatological disorders such as infections, varicose veins, Acanthosis nigricans, and eczema, exercise intolerance, type II diabetes mellitus, insulin resistance, hypertension, hypercholesterolemia, cholelithiasis, osteoarthritis, orthopedic injury, thromboembolic disease, cancer, and coronary (or cardiovascular) heart disease, particular those cardiovascular conditions associated with high triglycerides and free fatty acids in an individual.

"Obesity" refers to a condition whereby a mammal has a Body Mass Index (BMI), which is calculated as weight (kg) per height² (meters), of at least 25.9. Conventionally, those persons with normal weight have a BMI of 19.9 to less than 25.9. The obesity herein may be due to any cause, whether genetic or environmental. Examples of disorders that may result in obesity or be the cause of obesity include overeating and bulimia, polycystic ovarian disease, craniopharyngioma, the Prader-Willi Syndrome, Frohlich's syndrome, Type II diabetes, GH-deficient subjects, normal variant short stature, Turner's syndrome, and other pathological conditions showing reduced metabolic activity or a decrease in resting energy expenditure as a percentage of total fat-free mass, e.g., children with acute lymphoblastic leukemia.

The term "cytotoxic agent" as used herein refers to a substance that inhibits or prevents the function of cells and/or causes destruction of cells. The term is intended to include radioactive isotopes (e.g., At²¹¹, I¹³¹, I¹²⁵, Y⁹⁰, Re¹⁸⁶, Re¹⁸⁸, Sm¹⁵³, Bi²¹², P³² and radioactive isotopes of Lu), chemotherapeutic agents e.g. methotrexate, adriamicin, vinca alkaloids (vincristine, vinblastine, etoposide), doxorubicin, melphalan, mitomycin C, chlorambucil, daunorubicin or other intercalating agents, enzymes and fragments thereof such as nucleolytic enzymes, antibiotics, and toxins such as small molecule toxins or enzymatically active toxins of bacterial, fungal, plant or animal origin, including fragments and/or variants thereof, and the various antitumor or anticancer agents disclosed below. Other cytotoxic agents are described below. A tumoricidal agent causes destruction of tumor cells.

A "chemotherapeutic agent" is a chemical compound useful in the treatment of cancer. Examples of chemotherapeutic agents include alkylating agents such as thiotepa and CYTOXAN® cyclosphosphamide; alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethiylenethiophosphoramide and trimethylolomelamine; acetogenins (especially bullatacin and bullatacinone); delta-9-tetrahydrocannabinol (dronabinol, MARINOL®); beta-lapachonc; lapachol; colchicines; betulinic acid; a camptothecin (including the synthetic analogue topotecan (HYCAMTIN®), CPT-11 (irinotecan, CAMPTOSAR®), acetylcamptothecin, scopolectin, and 9-aminocamptothecin); bryostatin; callystatin; CC-1065 (including its adozelesin, carzelesin and bizelesin synthetic analogues); podophyllotoxin; podophyllinic acid; teniposide; cryptophycins (particularly cryptophycin 1 and cryptophycin 8); dolastatin; duocarmycin (including the synthetic analogues, KW-2189 and CB1-TM1); eleutherobin; pancratistatin; a sarcodictyin; spongistatin; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustinc, nimustine, and ranimnustine; antibiotics such as the enediyne antibiotics (e. g., calicheamicin, especially calicheamicin gammaII and calicheamicin omegaII (see, e.g., Agnew, Chem Intl. Ed. Engl., 33: 183-186 (1994)); dynemicin, including dynemicin A; an esperamicin; as well as neocarzinostatin chromophore and related chromoprotein enediyne antiobiotic chromophores), aclacinomysins, actinomycin, authramycin, azaserine, bleomycins, cactinomycin, carabicin, carminomycin, carzinophilin, chromomycinis, dactinomycin, daunorubicin, detorubicin, 6-diazo-5-oxo-L-norleucine, ADRIAMYCIN® doxorubicin (including morpholino-doxorubicin, cyanomorpholino-doxorubicin, 2-pyrrolino-doxorubicin and deoxydoxorubicin), cpirubicin, esorubicin, idarubicin, marcellomycin, mitomycins such as mitomycin C, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elfomithine; elliptinium acetate; an epothilone; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; 2-ethylhydrazide; procarbazine; PSK® polysaccharide complex (JHS Natural Products, Eugene, OR); razoxane; rhizoxin; sizofiran; spirogermanium; tenuazonic acid; triaziquone; 2,2',2"-trichlorotriethylamine; trichothecenes (especially T-2 toxin, vcrracurin A, roridin A and anguidinc); urethan; vindesine (ELDISINE®, FILDESIN®); dacarbazine; mannomustine; mitobronitol; mitolactol; pipobroman; gacytosine; arabinoside ("Ara-C"); thiotepa; taxoids, e.g., TAXOL® paclitaxel (Bristol-Myers Squibb Oncology, Princeton, N.J.), ABRAXANETM Cremophor-free, albumin-engineered nanoparticle formulation of paclitaxel (American Pharmaceutical Partners, Schaumberg, Illinois), and TAXOTERE® doxetaxel (Rhône-Poulenc Rorer, Antony, France); chloranbucil; gemcitabine (GEMZAR®; 6-thioguanine; mercaptopurine; methotrexate; platinum analogs such as cisplatin and carboplatin; vinblastine (VELBAN®); platinum; etoposide (VP-16); ifosfamide; mitoxantrone; vincristine (ONCOVIN®); oxaliplatin; leucovovin; vinorelbinc (NAVELBINE®); novantrone; edatrexate; daunomycin; aminopterin; ibandronate; topoisomerase inhibitor RFS 2000; difluorometlhylornithine (DMFO); retinoids such as retinoic acid; capecitabine (XELODA®); pharmaceutically acceptable salts, acids or derivatives of any of the above; as well as combinations of two or more of the above such as CHOP, an abbreviation for a combined therapy of cyclophosphamide, doxorubicin, vincristine, and prednisolone, and FOLFOX, an abbreviation for a treatment regimen with oxaliplatin (ELOXATINTM) combined with 5-FU and leucovovin.

Also included in this definition are anti-hormonal agents that act to regulate, reduce, block, or inhibit the effects of hormones that can promote the growth of cancer, and are often in the form of systemic, or whole-body treatment. They may be hormones themselves. Examples include anti-estrogens and selective estrogen receptor modulators (SERMs), including, for example, tamoxifen (including NOLVADEX®) tamoxifen), EVISTA® raloxifene, droloxifene, 4-hydroxytamoxifen, trioxifene, keoxifene, LY117018, onapristone, and FARESTON® toremifene; anti-progesterones; estrogen receptor down-regulators (ERDs); agents that function to suppress or shut down the ovaries, for example, leutinizing hormone-releasing hormone (LHRH) agonists such as LUPRON® and ELIGARD® leuprolide acetate, goserelin acetate, buserelin acetate and tripterelin; other anti-androgens such as flutamide, nilutamide and bicalutamide; and aromatase inhibitors that inhibit the enzyme aromatase, which regulates estrogen production in the adrenal glands, such as, for example, 4(5)-imidazoles, aminoglutethimide, MEGASE® megestrol acetate, AROMASIN® exemestane, formestanie, fadrozole, RIVISOR® vorozole, FEMARA® letrozole, and ARIMIDEX® anastrozole. In addition, such definition of chemotherapeutic agents includes bisphosphonatcs such as clodronate (for example, BONEFOS® or OSTAC®), DIDROCAL® etidronate, NE-58095, ZOMETA® zoledronic acid/zoledronate, FOSAMAX® alendronate, AREDIA® pamidronate, SKELID® tiludronate, or ACTONEL® risedronate; as well as troxacitabine (a 1,3-dioxolane nucleoside cytosine analog); antisense oligonucleotides, particularly those that inhibit expression of genes in signaling pathways implicated in abherant cell proliferation, such as, for example, PKC-alpha, Raf, H-Ras, and epidermal growth factor receptor (EGF-R); vaccines such as THERATOPE® vaccine and gene therapy vaccines, for example, ALLOVECTIN® vaccine, LEUVECTIN® vaccine, and VAXID® vaccine; LURTOTECAN® topoisomerase 1 inhibitor; ABARELIX® rmRH; lapatinib ditosylate (an ErbB-2 and EGFR dual tyrosine kinase small-molecule inhibitor also known as GW572016); and pharmaceutically acceptable salts, acids or derivatives of any of the above.

A "growth inhibitory agent" when used herein refers to a compound or composition which inhibits growth of a cell (such as a cell expressing KLβ) either in vitro or in vivo. Thus, the growth inhibitory agent may be one which significantly reduces the percentage of cells (such as a cell expressing KLβ) in S phase. Examples of growth inhibitory agents include agents that block cell cycle progression (at a place other than S phase), such as agents that induce G1 arrest and M-phase arrest. Classical M-phase blockers include the vincas (vincristine and vinblastine), taxanes, and topoisomerase II inhibitors such as doxorubicin, epirubicin, daunorubicin, etoposide, and bleomycin. Those agents that arrest G1 also spill over into S-phase arrest, for example, DNA alkylating agents such as tamoxifen, prednisone, dacarbazine, mechlorethamine, cisplatin, methotrexate, 5-fluorouracil, and ara-C. Further information can be found in The Molecular Basis of Cancer, Mendelsohn and Israel, eds., Chapter 1, entitled "Cell cycle regulation, oncogenes, and antineoplastic drugs" by Murakami et al. (WB Saunders: Philadelphia, 1995), especially p. 13. The taxanes (paclitaxel and docetaxel) are anticancer drugs both derived from the yew tree. Docetaxel (TAXOTERE®, Rhone-Poulenc Rorer), derived from the European yew, is a semisynthetic analogue of paclitaxel (TAXOL®), Bristol-Myers Squibb). Paclitaxel and docetaxel promote the assembly of microtubules from tubulin dimers and stabilize microtubules by preventing depolymerization, which results in the inhibition of mitosis in cells.

"Doxorubicin" is an anthracycline antibiotic. The full chemical name of doxorubicin is (8S-cis)-10-[(3-amino-2,3,6-trideoxy-α-L-lyxo-hexapyranosyl)oxy]-7,8,9,10-tetrahydro-6,8,11-trihydroxy-8-(hydroxyacetyl)-1-methoxy-5,12-naphthacenedione.

The term "Fc region-comprising polypeptide" refers to a polypeptide, such as an antibody or immunoadhesin (see definitions below), which comprises an Fc region. The C-terminal lysine (residue 447 according to the EU numbering system) of the Fc region may be removed, for example, during purification of the polypeptide or by recombinant engineering the nucleic acid encoding the polypeptide. Accordingly, a composition comprising a polypeptide having an Fc region according to this invention can comprise polypeptides with K447, with all K447 removed, or a mixture of polypeptides with and without the K447 residue.

### KLβ

KLβ is a transmembrane protein comprising an extracellular domain containing two regions with homology to those in family 1 glycosidases, a transmembrane domain, and a short intracellular hydrophilic tail at the carboxy terminus. Human KLβ protein is a 1043 amino acid protein and contains the following regions: signal peptide (amino acids 1-51); glycosidase (amino acids 77-508); glycosidase (amino acids 517-967); transmembrane (amino acids 996-1012); and cytoplasmic domain (amino acids 1013-1043). KLβ nucleic acid and amino acid sequences are known in the art and are further discussed herein. Nucleic acid sequence encoding the KLβ can be designed using the amino acid sequence of the desired region of KLβ. Alternatively, the cDNA sequence (or fragments thereof) of KLβ can be used. The accession number of human KLβ is NM_175737, and the accession number of mouse KLβ is NM_031180. Additional exemplary KLβ sequences are, e.g., shown in Figures 17 and 18, and described, for example, in Ito et al. (2000) Mech Dev 98:115-119.

### KLβ Modulators

Modulators of KLβ are molecules that modulate the activity of KLβ, e.g., agonists and antagonists. The term "KLβ agonist" is defined in the context of the biological role of KLβ. In certain embodiments, agonists possess the biological activities of a KLβ, as defined above. In some embodiments, KLβ agonists bind FGFR4 (optionally in conjunction with heparin), bind FGF19 (optionally in conjunction with heparin), bind FGFR4 and FGF19 (optionally in conjunction with heparin), promote FGF19-mediated induction of cFos, Junb and/or Junc (in vitro or in vivo), promote FGFR4 and/or FGF19 down stream signaling (including but not limited to FRS2 phosphorylation, ERK1/2 phosphorylation and Wnt pathway activation), and/or promotion of any biologically relevant KLβ and/or FGFR4 biological pathway.

KLβ modulators are known in the art, and some are described and exemplified herein. An exemplary and non-limiting list of KLβ antagonists (such as an anti-KLβ antibody) contemplated is provided herein under "Definitions."

The modulators useful in the present disclosure can be characterized for their physical/chemical properties and biological functions by various assays known in the art. In some embodiments, KLβ antagonists are characterized for any one or more of: binding to KLβ, reduction or blocking of FGFR4 activation, reduction or blocking of FGFR4 receptor downstream molecular signaling, inhibition of KLβ enzymatic activity (such as KLβ glycosidase activity), disruption or blocking of binding to FGF19, reduction and/or blocking of FGF19 downstream molecular signaling, and/or treatment and/or prevention of a tumor, cell proliferative disorder or a cancer (such as hepatocellular carcinoma); and/or treatment or prevention of a disorder associated with KLβ expression and/or activity. Methods for characterizing KLβ antagonists and agonists are known in the art, and some are described and exemplified herein.

### FGFR Modulators

Modulators of FGFR are molecules that modulate the activity of FGFR, e.g., agonists and antagonists. A "FGFR antagonist" refers to a molecule capable of neutralizing, blocking, inhibiting, abrogating, reducing or interfering with the activities of a FGF receptor ("FGFR") including, for example, binding KLβ (optionally in conjunction with heparin), binding FGF (e.g., FGF19) (optionally in conjunction with heparin), binding KLβ and FGF (e.g., FGF19) (optionally in conjunction with heparin), promoting FGF19-mediated induction of cFos, Junb and/or Junc (in vitro or in vivo), promoting FGFR and/or FGF down stream signaling (including but not limited to FRS2 phosphorylation, ERK1/2 phosphorylation and Wnt pathway activation), and/or promotion of any biologically relevant FGF and/or FGFR biological pathway, and/or promotion of a tumor, cell proliferative disorder or a cancer; and/or promotion of a disorder associated with FGFR expression and/or activity (such as increased FGFR expression and/or activity). FGFR antagonists include antibodies and antigen-binding fragments thereof, proteins, peptides, glycoproteins, glycopeptides, glycolipids, polysaccharides, oligosaccharides, nucleic acids, bioorganic molecules, peptidomimetics, pharmacological agents and their metabolites, transcriptional and translation control sequences, and the like. Antagonists also include small molecule inhibitors of a protein, and fusions proteins, receptor molecules and derivatives which bind specifically to protein thereby sequestering its binding to its target, antagonist variants of the protein, siRNA molecules directed to a protein, antisense molecules directed to a protein, RNA aptamers, and ribozymes against a protein. In some embodiments, the FGFR antagonist (e.g., FGFR4 antagonist) is a molecule which binds to FGFR and neutralizes, blocks, inhibits, abrogates, reduces or interferes with a biological activity of FGFR.

FGFR modulators are known in the art. For example, FGFR small molecule inhibitors are described in Manetti, F. and Botta, M., Curr. Pharm. Des., 9, 567-581 (2003). An example of a FGFR4 small molecule inhibitor is PD173074 (Pfizer, Inc. Groton CT). An exemplary and non-limiting list of FGFR antagonists (such as an anti-FGFR antibody) contemplated is provided herein under "Definitions." Methods for characterizing FGFR antagonists are known in the art, and some are described and exemplified herein.

### FGF19 Antagonists

A "FGF19 antagonist" refers to a molecule capable of neutralizing, blocking, inhibiting, abrogating, reducing or interfering with the activities of a FGF19 including, for example, binding KLβ (optionally in conjunction with heparin), binding FGFR4 (optionally in conjunction with heparin), binding KLβ and FGFR4 (optionally in conjunction with heparin), promoting FGF19-mediated induction of cFos, Junb and/or Junc (in vitro or in vivo), promoting FGFR4 and/or FGF19 down stream signaling (including but not limited to FRS2 phosphorylation, ERK1/2 phosphorylation and Wnt pathway activation), and/or promotion of any biologically relevant FGF19 and/or FGFR4 biological pathway, and/or promotion of a tumor, cell proliferative disorder or a cancer; and/or promotion of a disorder associated with FGF19 expression and/or activity (such as increased FGF19 expression and/or activity). FGF19 antagonists include antibodies and antigen-binding fragments thereof, proteins, peptides, glycoproteins, glycopeptides, glycolipids, polysaccharides, oligosaccharides, nucleic acids, bioorganic molecules, peptidomimetics, pharmacological agents and their metabolites, transcriptional and translation control sequences, and the like. Antagonists also include small molecule inhibitors of a protein, and fusions proteins, receptor molecules and derivatives which bind specifically to protein thereby sequestering its binding to its target, antagonist variants of the protein, siRNA molecules directed to a protein, antisense molecules directed to a protein, RNA aptamers, and ribozymes against a protein. In some embodiments, the FGF19 antagonist is a molecule which binds to FGF19 and neutralizes, blocks, inhibits, abrogates, reduces or interferes with a biological activity of FGF19.

FGF19 antagonists are known in the art. An exemplary and non-limiting list of FGF19 antagonists (such as an anti-FGFR antibody) contemplated is provided herein under "Definitions." Methods for characterizing FGFR antagonists are known in the art, and some are described and exemplified herein.

### Antibodies

The antibodies are preferably monoclonal, although polyclonal antibodies may also be useful and are exemplified herein. Also encompassed within the scope of the invention are Fab, Fab', Fab'-SH and F(ab')₂ fragments of the antibodies provided herein. These antibody fragments can be created by traditional means, such as enzymatic digestion, or may be generated by recombinant techniques. Such antibody fragments may be chimeric or humanized. These fragments are useful for the diagnostic and therapeutic purposes set forth below. Anti- KLβ antibodies are known in the art, e.g., antibodies disclosed in Ito et al (2005) J Clin Invest 115(8): 2202-2208; R&D Systems Catalog No. MAB3738. Anti-FGF19 antibodies are disclosed in, e.g., WO2007/13693. The anti-FGF19 antibody may be an antibody comprising (a) a light chain comprising (i) HVR-L1 comprising the sequence KASQDINSFLA (SEQ ID NO:53); (ii) HVR-L2 comprising the sequence RANRLVS (SEQ ID NO:54); and (iii) HVR-L3 comprising the sequence LQYDEFPLT (SEQ ID NO:55), and (b) a heavy chain comprising (i) HVR-H1 comprising the sequence GFSLTTYGVH (SEQ ID NO:56); (ii) HVR-H2 comprising the sequence GVIWPGGGTDYNAAFIS (SEQ ID NO:57); and (iii) HVR-H3 comprising the sequence VRKEYANLYA (SEQ ID NO:58).

Monoclonal antibodies are obtained from a population of substantially homogeneous antibodies, i.e., the individual antibodies comprising the population are identical except for possible naturally occurring mutations that may be present in minor amounts. Thus, the modifier "monoclonal" indicates the character of the antibody as not being a mixture of discrete antibodies.

The monoclonal antibodies can be made using the hybridoma method first described by Kohler et al., Nature, 256:495 (1975), or may be made by recombinant DNA methods (U.S. Patent No. 4,816,567).

In the hybridoma method, a mouse or other appropriate host animal, such as a hamster, is immunized to elicit lymphocytes that produce or are capable of producing antibodies that will specifically bind to the protein used for immunization. Antibodies to a given target generally arc raised in animals by multiple subcutaneous (sc) or intraperitoneal (ip) injections of target immunogen and an adjuvant. Target polypeptide may be prepared using methods well-known in the art, some of which are further described herein. For example, recombinant production of protein is described below. In one embodiment, animals are immunized with a derivative of antigen that contains the extracellular domain (ECD) of the target fused to the Fc portion of an immunoglobulin heavy chain. In one embodiment, animals are immunized with a target polypeptide-IgG 1 fusion protein. Animals ordinarily are immunized against immunogenic conjugates or derivatives of target polypeptide with monophosphoryl lipid A (MPL)/trehalose dicrynomycolate (TDM) (Ribi Immunochem. Research, Inc., Hamilton, MT) and the solution is injected intradermally at multiple sites. Two weeks later the animals are boosted. 7 to 14 days later animals are bled and the serum is assayed for anti-antigen titer. Animals are boosted until titer plateaus.

Alternatively, lymphocytes may be immunized *in vitro.* Lymphocytes then are fused with myeloma cells using a suitable fusing agent, such as polyethylene glycol, to form a hybridoma cell (Goding, Monoclonal Antibodies: Principles and Practice, pp.59-103 (Academic Press, 1986)).

The hybridoma cells thus prepared are seeded and grown in a suitable culture medium that preferably contains one or more substances that inhibit the growth or survival of the unfused, parental myeloma cells. For example, if the parental myeloma cells lack the enzyme hypoxanthine guanine phosphoribosyl transferase (HGPRT or HPRT), the culture medium for the hybridomas typically will include hypoxanthine, aminopterin, and thymidine (HAT medium), which substances prevent the growth of HGPRT-deficient cells.

Preferred myeloma cells are those that fuse efficiently, support stable high-level production of antibody by the selected antibody-producing cells, and are sensitive to a medium such as HAT medium. Among these, preferred myeloma cell lines are murine myeloma lines, such as those derived from MOPC-21 and MPC-11 mouse tumors available from the Salk Institute Cell Distribution Center, San Diego, California USA, and SP-2 or X63-Ag8-653 cells available from the American Type Culture Collection, Rockville, Maryland USA. Human myeloma and mouse-human heteromyeloma cell lines also have been described for the production of human monoclonal antibodies (Kozbor, J. Immunol., 133:3001 (1984); Brodeur et al., Monoclonal Antibody Production Techniques and Applications, pp. 51-63 (Marcel Dekker, Inc., New York, 1987)).

Culture medium in which hybridoma cells are growing is assayed for production of monoclonal antibodies directed against antigen. Preferably, the binding specificity of monoclonal antibodies produced by hybridoma cells is determined by immunoprecipitation or by an in vitro binding assay, such as radioimmunoassay (RIA) or enzyme-linked immunoadsorbent assay (ELISA).

The binding affinity of the monoclonal antibody can, for example, be determined by the Scatchard analysis of Munson et al., Anal. Biochem., 107:220 (1980).

After hybridoma cells are identified that produce antibodies of the desired specificity, affinity, and/or activity, the clones may be subcloned by limiting dilution procedures and grown by standard methods (Goding, Monoclonal Antibodies: Principles and Practice, pp.59-103 (Academic Press, 1986)). Suitable culture media for this purpose include, for example, D-MEM or RPMI-1640 medium. In addition, the hybridoma cells may be grown in vivo as ascites tumors in an animal.

The monoclonal antibodies secreted by the subclones are suitably separated from the culture medium, ascites fluid, or serum by conventional immunoglobulin purification procedures such as, for example, protein A-Sepharose, hydroxylapatite chromatography, gel electrophoresis, dialysis, or affinity chromatography.

The antibodies can be made by using combinatorial libraries to screen for synthetic antibody clones with the desired activity or activities. In principle, synthetic antibody clones are selected by screening phage libraries containing phage that display various fragments of antibody variable region (Fv) fused to phage coat protein. Such phage libraries are panned by affinity chromatography against the desired antigen. Clones expressing Fv fragments capable of binding to the desired antigen are adsorbed to the antigen and thus separated from the non-binding clones in the library. The binding clones are then eluted from the antigen, and can be further enriched by additional cycles of antigen adsorption/elution. Any of the desired antibodies can be obtained by designing a suitable antigen screening procedure to select for the phage clone of interest followed by construction of a full length antibody clone using the Fv sequences from the phage clone of interest and suitable constant region (Fc) sequences described in Kabat et al., Sequences of Proteins of Immunological Interest, Fifth Edition, NIH Publication 91-3242, Bethesda MD (1991), vols. 1-3.

The antigen-binding domain of an antibody is formed from two variable (V) regions of about 110 amino acids, one each from the light (VL) and heavy (VH) chains, that both present three hypervariable loops or complementarity-determining regions (CDRs). Variable domains can be displayed functionally on phage, either as single-chain Fv (scFv) fragments, in which VH and VL are covalently linked through a short, flexible peptide, or as Fab fragments, in which they are each fused to a constant domain and interact non-covalently, as described in Winter et al., Ann. Rev. Immunol., 12: 433-455 (1994). As used herein, scFv encoding phage clones and Fab encoding phage clones are collectively referred to as "Fv phage clones" or "Fv clones".

Repertoires of VH and VL genes can be separately cloned by polymerase chain reaction (PCR) and recombined randomly in phage libraries, which can then be searched for antigen-binding clones as described in Winter et al., Ann. Rev. Immunol., 12: 433-455 (1994). Libraries from immunized sources provide high-affinity antibodies to the immunogen without the requirement of constructing hybridomas. Alternatively, the naive repertoire can be cloned to provide a single source of human antibodies to a wide range of non-self and also self antigens without any immunization as described by Griffiths et al., EMBO J, 12: 725-734 (1993). Finally, naive libraries can also be made synthetically by cloning the unrearranged V-gene segments from stem cells, and using PCR primers containing random sequence to encode the highly variable CDR3 regions and to accomplish rearrangement in vitro as described by Hoogenboom and Winter, J. Mol. Biol., 227: 381-388 (1992).

Filamentous phage is used to display antibody fragments by fusion to the minor coat protein pIII. The antibody fragments can be displayed as single chain Fv fragments, in which VH and VL domains are connected on the same polypeptide chain by a flexible polypeptide spacer, e.g. as described by Marks et al., J. Mol. Biol., 222: 581-597 (1991), or as Fab fragments, in which one chain is fused to pIII and the other is secreted into the bacterial host cell periplasm where assembly of a Fab-coat protein structure which becomes displayed on the phage surface by displacing some of the wild type coat proteins, e.g. as described in Hoogenboom et al., Nucl. Acids Res., 19: 4133-4137 (1991).

In general, nucleic acids encoding antibody gene fragments are obtained from immune cells harvested from humans or animals. If a library biased in favor of anti-antigen clones is desired, the subject is immunized with antigen polypeptide to generate an antibody response, and spleen cells and/or circulating B cells other peripheral blood lymphocytes (PBLs) are recovered for library construction. In a preferred embodiment, a human antibody gene fragment library biased in favor of anti-human clones is obtained by generating an anti-huma antibody response in transgenic mice carrying a functional human immunoglobulin gene array (and lacking a functional endogenous antibody production system) such that immunization gives rise to B cells producing human antibodies against antigen. The generation of human antibody-producing transgenic mice is described below.

Additional enrichment for anti-antigen reactive cell populations can be obtained by using a suitable screening procedure to isolate B cells expressing antigen-specific membrane bound antibody, e.g., by cell separation with antigen affinity chromatography or adsorption of cells to fluorochrome-labeled antigen protein followed by flow-activatcd cell sorting (FACS).

Alternatively, the use of spleen cells and/or B cells or other PBLs from an unimmunized donor provides a better representation of the possible antibody repertoire, and also permits the construction of an antibody library using any animal (human or non-human) species in which antigen is not antigenic. For libraries incorporating in vitro antibody gene construction, stem cells are harvested from the subject to provide nucleic acids encoding unrearranged antibody gene segments. The immune cells of interest can be obtained from a variety of animal species, such as human, mouse, rat, lagomorpha, luprine, canine, feline, porcine, bovine, equine, and avian species, etc.

Nucleic acid encoding antibody variable gene segments (including VH and VL segments) are recovered from the cells of interest and amplified. In the case of rearranged VH and VL gene libraries, the desired DNA can be obtained by isolating genomic DNA or mRNA from lymphocytes followed by polymerase chain reaction (PCR) with primers matching the 5' and 3' ends of rearranged VH and VL genes as described in Orlandi et al., Proc. Natl. Acad. Sci. (USA), 86: 3833-3837 (1989), thereby making diverse V gene repertoires for expression. The V genes can be amplified from cDNA and genomic DNA, with back primers at the 5' end of the exon encoding the mature V-domain and forward primers based within the J-segment as described in Orlandi et al. (1989) and in Ward et al., Nature, 341: 544-546 (1989). However, for amplifying from cDNA, back primers can also be based in the leader exon as described in Jones et al., Biotechnol., 9: 88-89 (1991), and forward primers within the constant region as described in Sastry et al., Proc. Natl. Acad. Sci. (USA), 86: 5728-5732 (1989). To maximize complementarity, degeneracy can be incorporated in the primers as described in Orlandi et al. (1989) or Sastry et al. (1989). Preferably, the library diversity is maximized by using PCR primers targeted to each V-gene family in order to amplify all available VH and VL arrangements present in the immune cell nucleic acid sample, e.g. as described in the method of Marks et al., J. Mol. Biol., 222: 581-597 (1991) or as described in the method of Orum et al., Nucleic Acids Res., 21: 4491-4498 (1993). For cloning of the amplified DNA into expression vectors, rare restriction sites can be introduced within the PCR primer as a tag at one end as described in Orlandi et al. (1989), or by further PCR amplification with a tagged primer as described in Clackson et al., Nature, 352: 624-628 (1991).

Repertoires of synthetically rearranged V genes can be derived in vitro from V gene segments. Most of the human VH-gene segments have been cloned and sequenced (reported in Tomlinson et al., J. Mol. Biol., 227: 776-798 (1992)), and mapped (reported in Matsuda et al., Nature Genet., 3: 88-94 (1993); these cloned segments (including all the major conformations of the H1 and H2 loop) can be used to generate diverse VH gene repertoires with PCR primers encoding H3 loops of diverse sequence and length as described in Hoogenboom and Winter, J. Mol. Biol., 227: 381-388 (1992). VH repertoires can also be made with all the sequence diversity focused in a long H3 loop of a single length as described in Barbas et al., Proc. Natl. Acad. Sci. USA, 89: 4457-4461 (1992). Human Vκ and Vλ segments have been cloned and sequenced (reported in Williams and Winter, Eur. J. Immunol., 23: 1456-1461 (1993)) and can be used to make synthetic light chain repertoires. Synthetic V gene repertoires, based on a range of VH and VL folds, and L3 and H3 lengths, will encode antibodies of considerable structural diversity. Following amplification of V-gene encoding DNAs, germline V-gene segments can be rearranged in vitro according to the methods of Hoogenboom and Winter, J. Mol. Biol., 227: 381-388 (1992).

Repertoires of antibody fragments can be constructed by combining VH and VL gene repertoires together in several ways. Each repertoire can be created in different vectors, and the vectors recombined in vitro, e.g., as described in Hogrefe et al., Gene, 128: 119-126 (1993), or in vivo by combinatorial infection, e.g., the loxP system described in Waterhouse et al., Nucl. Acids Res., 21: 2265-2266 (1993). The in vivo recombination approach exploits the two-chain nature of Fab fragments to overcome the limit on library size imposed by E. coli transformation efficiency. Naive VH and VL repertoires are cloned separately, one into a phagemid and the other into a phage vector. The two libraries are then combined by phage infection of phagemid-containing bacteria so that each cell contains a different combination and the library size is limited only by the number of cells present (about 10¹² clones). Both vectors contain in vivo recombination signals so that the VH and VL genes are recombined onto a single replicon and are co-packaged into phage virions. These huge libraries provide large numbers of diverse antibodies of good affinity (K_{d}⁻¹ of about 10⁻⁸ M).

Alternatively, the repertoires may be cloned sequentially into the same vector, e.g. as described in Barbas et al., Proc. Natl. Acad. Sci. USA, 88: 7978-7982 (1991), or assembled together by PCR and then cloned, e.g. as described in Clackson et al., Nature, 352: 624-628 (1991). PCR assembly can also be used to join VH and VL DNAs with DNA encoding a flexible peptide spacer to form single chain Fv (scFv) repertoires. In yet another technique, "in cell PCR assembly" is used to combine VH and VL genes within lymphocytes by PCR and then clone repertoires of linked genes as described in Embleton et al., Nucl. Acids Res., 20: 3831-3837 (1992).

The antibodies produced by naive libraries (either natural or synthetic) can be of moderate affinity (K_{d}⁻¹ of about 10⁶ to 10⁷ M⁻¹), but affinity maturation can also be mimicked in vitro by constructing and reselecting from secondary libraries as described in Winter et al. (1994), supra. For example, mutation can be introduced at random in vitro by using error-prone polymerase (reported in Leung et al., Technique, 1: 11-15 (1989)) in the method of Hawkins et al., J. Mol. Biol., 226: 889-896 (1992) or in the method of Gram et al., Proc. Natl. Acad. Sci USA, 89: 3576-3580 (1992). Additionally, affinity maturation can be performed by randomly mutating one or more CDRs, e.g. using PCR with primers carrying random sequence spanning the CDR of interest, in selected individual Fv clones and screening for higher affinity clones. WO 9607754 (published 14 March 1996) described a method for inducing mutagenesis in a complementarity determining region of an immunoglobulin light chain to create a library of light chain genes. Another effective approach is to recombine the VH or VL domains selected by phage display with repertoires of naturally occurring V domain variants obtained from unimmunized donors and screen for higher affinity in several rounds of chain reshuffling as described in Marks et al., Biotechnol., 10: 779-783 (1992). This technique allows the production of antibodies and antibody fragments with affinities in the 10⁻⁹ M range.

Nucleic acid sequence encoding a antigen can be designed using the amino acid sequence of the desired region of antigen. Alternatively, the cDNA sequence (or fragments thereof) may be used. DNAs encoding antigen can be prepared by a variety of methods known in the art. These methods include, but are not limited to, chemical synthesis by any of the methods described in Engels et al., Agnew. Chem. Int. Ed. Engl., 28: 716-734 (1989), such as the triester, phosphite, phosphoramidite and H-phosphonate methods. In one embodiment, codons preferred by the expression host cell are used in the design of the DNA. Alternatively, DNA encoding antigen can be isolated from a genomic or cDNA library.

Following construction of the DNA molecule encoding antigen, the DNA molecule is operably linked to an expression control sequence in an expression vector, such as a plasmid, wherein the control sequence is recognized by a host cell transformed with the vector. In general, plasmid vectors contain replication and control sequences which are derived from species compatible with the host cell. The vector ordinarily carries a replication site, as well as sequences which encode proteins that are capable of providing phenotypic selection in transformed cells. Suitable vectors for expression in prokaryotic and eukaryotic host cells are known in the art and some are further described herein. Eukaryotic organisms, such as yeasts, or cells derived from multicellular organisms, such as mammals, may be used.

Optionally, the DNA encoding antigen is operably linked to a secretory leader sequence resulting in secretion of the expression product by the host cell into the culture medium. Examples of secretory leader sequences include stII, ecotin, lamB, herpes GD, lpp, alkaline phosphatase, invertase, and alpha factor. Also suitable for use herein is the 36 amino acid leader sequence of protein A (Abrahmsen et al., EMBO J., 4: 3901 (1985)).

Host cells are transfected and preferably transformed with the above-described expression or cloning vectors of this invention and cultured in conventional nutrient media modified as appropriate for inducing promoters, selecting transformants, or amplifying the genes encoding the desired sequences.

Transfection refers to the taking up of an expression vector by a host cell whether or not any coding sequences are in fact expressed. Numerous methods of transfection are known to the ordinarily skilled artisan, for example, CaPO₄ precipitation and electroporation. Successful transfection is generally recognized when any indication of the operation of this vector occurs within the host cell. Methods for transfection are well known in the art, and some are further described herein.

Transformation means introducing DNA into an organism so that the DNA is replicable, either as an extrachromosomal element or by chromosomal integrant. Depending on the host cell used, transformation is done using standard techniques appropriate to such cells. Methods for transformation are well known in the art, and some are further described herein.

Prokaryotic host cells used to produce antigen can be cultured as described generally in Sambrook et al., supra.

The mammalian host cells used to produce antigen can be cultured in a variety of media, which is well known in the art and some of which is described herein.

The host cells referred to in this disclosure encompass cells in in vitro culture as well as cells that are within a host animal.

Purification of antigen may be accomplished using art-recognized methods.

The purified antigen can be attached to a suitable matrix such as agarose beads, acrylamide beads, glass beads, cellulose, various acrylic copolymers, hydroxyl methacrylate gels, polyacrylic and polymethacrylic copolymers, nylon, neutral and ionic carriers, and the like, for use in the affinity chromatographic separation of phage display clones. Attachment of the protein to the matrix can be accomplished by the methods described in Methods in Enzymology, vol. 44 (1976). A commonly employed technique for attaching protein ligands to polysaccharide matrices, e.g. agarose, dextran or cellulose, involves activation of the carrier with cyanogen halides and subsequent coupling of the peptide ligand's primary aliphatic or aromatic amines to the activated matrix.

Alternatively, antigen can be used to coat the wells of adsorption plates, expressed on host cells affixed to adsorption plates or used in cell sorting, or conjugated to biotin for capture with streptavidin-coated beads, or used in any other art-known method for panning phage display libraries.

The phage library samples are contacted with immobilized antigen under conditions suitable for binding of at least a portion of the phage particles with the adsorbent. Normally, the conditions, including pH, ionic strength, temperature and the like are selected to mimic physiological conditions. The phages bound to the solid phase are washed and then eluted by acid, e.g. as described in Barbas et al., Proc. Natl. Acad. Sci USA, 88: 7978-7982 (1991), or by alkali, e.g. as described in Marks et al., J. Mol. Biol., 222: 581-597 (1991), or by KLβ antigen competition, e.g. in a procedure similar to the antigen competition method of Clackson et al., Nature, 352: 624-628 (1991). Phages can be enriched 20-1,000-fold in a single round of selection. Moreover, the enriched phages can be grown in bacterial culture and subjected to further rounds of selection.

The efficiency of selection depends on many factors, including the kinetics of dissociation during washing, and whether multiple antibody fragments on a single phage can simultaneously engage with antigen. Antibodies with fast dissociation kinetics (and weak binding affinities) can be retained by use of short washes, multivalent phage display and high coating density of antigen in solid phase. The high density not only stabilizes the phage through multivalent interactions, but favors rebinding of phage that has dissociated. The selection of antibodies with slow dissociation kinetics (and good binding affinities) can be promoted by use of long washes and monovalent phage display as described in Bass et al., Proteins, 8: 309-314 (1990) and in WO 92/09690, and a low coating density of antigen as described in Marks et al., Biotechnol., 10: 779-783 (1992).

It is possible to select between phage antibodies of different affinities, even with affinities that differ slightly, for antigen. However, random mutation of a selected antibody (e.g. as performed in some of the affinity maturation techniques described above) is likely to give rise to many mutants, most binding to antigen, and a few with higher affinity. With limiting antigen, rare high affinity phage could be competed out. To retain all the higher affinity mutants, phages can be incubated with excess biotinylated antigen, but with the biotinylated antigen at a concentration of lower molarity than the target molar affinity constant for antigen. The high affinity-binding phages can then be captured by streptavidin-coated paramagnetic beads. Such "equilibrium capture" allows the antibodies to be selected according to their affinities of binding, with sensitivity that permits isolation of mutant clones with as little as two-fold higher affinity from a great excess of phages with lower affinity. Conditions used in washing phages bound to a solid phase can also be manipulated to discriminate on the basis of dissociation kinetics. Anti- antigen clones may also be activity selected.

DNA encoding the hybridoma-derived monoclonal antibodies or phage display Fv clones is readily isolated and sequenced using conventional procedures (e.g. by using oligonucleotide primers designed to specifically amplify the heavy and light chain coding regions of interest from hybridoma or phage DNA template). Once isolated, the DNA can be placed into expression vectors, which are then transfected into host cells such as E. coli cells, simian COS cells, Chinese hamster ovary (CHO) cells, or myeloma cells that do not otherwise produce immunoglobulin protein, to obtain the synthesis of the desired monoclonal antibodies in the recombinant host cells. Review articles on recombinant expression in bacteria of antibody-encoding DNA include Skerra et al., Curr. Opinion in Immunol., 5: 256 (1993) and Pluckthun, Immunol. Revs, 130: 151 (1992).

DNA encoding the Fv clones can be combined with known DNA sequences encoding heavy chain and/or light chain constant regions (e.g. the appropriate DNA sequences can be obtained from Kabat et al., supra) to form clones encoding full or partial length heavy and/or light chains. It will be appreciated that constant regions of any isotype can be used for this purpose, including IgG, IgM, IgA, IgD, and IgE constant regions, and that such constant regions can be obtained from any human or animal species. A Fv clone derived from the variable domain DNA of one animal (such as human) species and then fused to constant region DNA of another animal species to form coding sequcnce(s) for "hybrid", full length heavy chain and/or light chain is included in the definition of "chimeric" and "hybrid" antibody as used herein. In a preferred embodiment, a Fv clone derived from human variable DNA is fused to human constant region DNA to form coding sequence(s) for all human, full or partial length heavy and/or light chains.

DNA encoding anti- antigen antibody derived from a hybridoma can also be modified, for example, by substituting the coding sequence for human heavy- and light-chain constant domains in place of homologous murine sequences derived from the hybridoma clone (e.g. as in the method of Morrison et al., Proc. Natl. Acad. Sci. USA, 81: 6851-6855 (1984)). DNA encoding a hybridoma or Fv clone-derived antibody or fragment can be further modified by covalently joining to the immunoglobulin coding sequence all or part of the coding sequence for a non-immunoglobulin polypeptide. In this manner, "chimeric" or "hybrid" antibodies are prepared that have the binding specificity of the Fv clone or hybridoma clone-derived antibodies.

### Antibody Fragments

The present invention encompasses antibody fragments. In certain circumstances there are advantages of using antibody fragments, rather than whole antibodies. The smaller size of the fragments allows for rapid clearance, and may lead to improved access to solid tumors.

Various techniques have been developed for the production of antibody fragments. Traditionally, these fragments were derived via proteolytic digestion of intact antibodies (see, e.g., Morimoto et al., Journal of Biochemical and Biophysical Methods 24:107-117 (1992); and Brennan et al., Science, 229:81 (1985)). However, these fragments can now be produced directly by recombinant host cells. Fab, Fv and ScFv antibody fragments can all be expressed in and secreted from E. coli, thus allowing the facile production of large amounts of these fragments. Antibody fragments can be isolated from the antibody phage libraries discussed above. Alternatively, Fab'-SH fragments can be directly recovered from E. coli and chemically coupled to form F(ab')₂ fragments (Carter et al., Bio/Technology 10:163-167 (1992)). According to another approach, F(ab')₂ fragments can be isolated directly from recombinant host cell culture. Fab and F(ab')₂ fragment with increased in vivo half-life comprising a salvage receptor binding epitope residues are described in U.S. Pat. No. 5,869,046. Other techniques for the production of antibody fragments will be apparent to the skilled practitioner. In other embodiments, the antibody of choice is a single chain Fv fragment (scFv). See WO 93/16185; U.S. Pat. Nos. 5,571,894; and 5,587,458. Fv and sFv are the only species with intact combining sites that are devoid of constant regions; thus, they are suitable for reduced nonspecific binding during in vivo use. sFv fusion proteins may be constructed to yield fusion of an effector protein at either the amino or the carboxy terminus of an sFv. See Antibody Engineering, ed. Borrebaeck, supra. The antibody fragment may also be a "linear antibody", e.g., as described in U.S. Pat. No. 5,641,870 for example. Such linear antibody fragments may be monospecific or bispecific.

### Humanized Antibodies

The present invention encompasses humanized antibodies. Various methods for humanizing non-human antibodies are known in the art. For example, a humanized antibody can have one or more amino acid residues introduced into it from a source which is non-human. These non-human amino acid residues are often referred to as "import" residues, which are typically taken from an "import" variable domain. Humanization can be essentially performed following the method of Winter and co-workers (Jones et al. (1986) Nature 321:522-525; Riechmann et al. (1988) Nature 332:323-327; Verhoeyen et al. (1988) Science 239:1534-1536), by substituting hypervariable region sequences for the corresponding sequences of a human antibody. Accordingly, such "humanized" antibodies are chimeric antibodies (U.S. Patent No. 4,816,567) wherein substantially less than an intact human variable domain has been substituted by the corresponding sequence from a non-human species. In practice, humanized antibodies are typically human antibodies in which some hypervariable region residues and possibly some FR residues are substituted by residues from analogous sites in rodent antibodies.

The choice of human variable domains, both light and heavy, to be used in making the humanized antibodies is very important to reduce antigenicity. According to the so-called "best-fit" method, the sequence of the variable domain of a rodent antibody is screened against the entire library of known human variable-domain sequences. The human sequence which is closest to that of the rodent is then accepted as the human framework for the humanized antibody (Sims et al. (1993) J. Immunol. 151:2296; Chothia et al. (1987) J. Mol. Biol. 196:901. Another method uses a particular framework derived from the consensus sequence of all human antibodies of a particular subgroup of light or heavy chains. The same framework may be used for several different humanized antibodies (Carter et al. (1992) Proc. Natl. Acad. Sci. USA, 89:4285; Presta et al. (1993) J. Immunol., 151:2623.

It is further important that antibodies be humanized with retention of high affinity for the antigen and other favorable biological properties. To achieve this goal, according to one method, humanized antibodies are prepared by a process of analysis of the parental sequences and various conceptual humanized products using three-dimensional models of the parental and humanized sequences. Three-dimensional immunoglobulin models are commonly available and arc familiar to those skilled in the art. Computer programs are available which illustrate and display probable three-dimensional conformational structures of selected candidate immunoglobulin sequences. Inspection of these displays permits analysis of the likely role of the residues in the functioning of the candidate immunoglobulin sequence, i.e., the analysis of residues that influence the ability of the candidate immunoglobulin to bind its antigen. In this way, FR residues can be selected and combined from the recipient and import sequences so that the desired antibody characteristic, such as increased affinity for the target(s), is achieved. In general, the hypervariable region residues are directly and most substantially involved in influencing antigen binding.

### Human antibodies

Human anti-KLβ antibodies can be constructed by combining Fv clone variable domain sequence(s) selected from human-derived phage display libraries with known human constant domain sequences(s) as described above. Alternatively, human monoclonal anti-KLβ antibodies can be made by the hybridoma method. Human myeloma and mouse-human heteromyeloma cell lines for the production of human monoclonal antibodies have been described, for example, by Kozbor J. Immunol., 133: 3001 (1984); Brodeur et al., Monoclonal Antibody Production Techniques and Applications, pp. 51-63 (Marcel Dekker, Inc., New York, 1987); and Boerner et al., J. Immunol., 147: 86 (1991).

It is now possible to produce transgenic animals (e.g. mice) that are capable, upon immunization, of producing a full repertoire of human antibodies in the absence of endogenous immunoglobulin production. For example, it has been described that the homozygous deletion of the antibody heavy-chain joining region (JH) gene in chimeric and germ-line mutant mice results in complete inhibition of endogenous antibody production. Transfer of the human germ-line immunoglobulin gene array in such germ-line mutant mice will result in the production of human antibodies upon antigen challenge. See, e.g., Jakobovits et al., Proc. Natl. Acad. Sci USA, 90: 2551 (1993); Jakobovits et al., Nature, 362: 255 (1993); Bruggermann et al., Year in Immunol., 7: 33 (1993).

Gene shuffling can also be used to derive human antibodies from non-human, e.g. rodent, antibodies, where the human antibody has similar affinities and specificities to the starting non-human antibody. According to this method, which is also called "epitope imprinting", either the heavy or light chain variable region of a non-human antibody fragment obtained by phage display techniques as described above is replaced with a repertoire of human V domain genes, creating a population of non-human chain/human chain scFv or Fab chimeras. Selection with antigen results in isolation of a non-human chain/human chain chimeric scFv or Fab wherein the human chain restores the antigen binding site destroyed upon removal of the corresponding non-human chain in the primary phage display clone, i.e. the epitope governs (imprints) the choice of the human chain partner. When the process is repeated in order to replace the remaining non-human chain, a human antibody is obtained (see PCT WO 93/06213 published April 1, 1993). Unlike traditional humanization of non-human antibodies by CDR grafting, this technique provides completely human antibodies, which have no FR or CDR residues of non-human origin.

### Bispecific Antibodies

Bispecific antibodies are monoclonal, preferably human or humanized, antibodies that have binding specificities for at least two different antigens. In one embodiment, one of the binding specificities is for KLβ and the other is for any other antigen. Exemplary bispecific antibodies may bind to two different epitopes of the KLβ protein. Bispecific antibodies may also be used to localize cytotoxic agents to cells which express KLβ. These antibodies possess a KLβ-binding arm and an arm which binds the cytotoxic agent (e.g. saporin, anti-interferon-α, vinca alkaloid, ricin A chain, methotrexate or radioactive isotope hapten). Bispecific antibodies can be prepared as full length antibodies or antibody fragments (e.g. F(ab')₂ bispecific antibodies).

Methods for making bispecific antibodies are known in the art. Traditionally, the recombinant production of bispecific antibodies is based on the co-expression of two immunoglobulin heavy chain-light chain pairs, where the two heavy chains have different specificities (Milstein and Cuello, Nature, 305: 537 (1983)). Because of the random assortment of immunoglobulin heavy and light chains, these hybridomas (quadromas) produce a potential mixture of 10 different antibody molecules, of which only one has the correct bispecific structure. The purification of the correct molecule, which is usually done by affinity chromatography steps, is rather cumbersome, and the product yields are low. Similar procedures are disclosed in WO 93/08829 published May 13, 1993, and in Traunecker et al., EMBO J., 10: 3655 (1991).

According to a different and more preferred approach, antibody variable domains with the desired binding specificities (antibody-antigen combining sites) are fused to immunoglobulin constant domain sequences. The fusion preferably is with an immunoglobulin heavy chain constant domain, comprising at least part of the hinge, CH2, and CH3 regions. It is preferred to have the first heavy-chain constant region (CH1), containing the site necessary for light chain binding, present in at least one of the fusions. DNAs encoding the immunoglobulin heavy chain fusions and, if desired, the immunoglobulin light chain, are inserted into separate expression vectors, and are co-transfected into a suitable host organism. This provides for great flexibility in adjusting the mutual proportions of the three polypeptide fragments in embodiments when unequal ratios of the three polypeptide chains used in the construction provide the optimum yields. It is, however, possible to insert the coding sequences for two or all three polypeptide chains in one expression vector when the expression of at least two polypeptide chains in equal ratios results in high yields or when the ratios are of no particular significance.

In a preferred embodiment of this approach, the bispecific antibodies are composed of a hybrid immunoglobulin heavy chain with a first binding specificity in one arm, and a hybrid immunoglobulin heavy chain-light chain pair (providing a second binding specificity) in the other arm. It was found that this asymmetric structure facilitates the separation of the desired bispecific compound from unwanted immunoglobulin chain combinations, as the presence of an immunoglobulin light chain in only one half of the bispecific molecule provides for a facile way of separation. This approach is disclosed in WO 94/04690. For further details of generating bispecific antibodies see, for example, Suresh et al., Methods in Enzymology, 121:210 (1986).

According to another approach, the interface between a pair of antibody molecules can be engineered to maximize the percentage of heterodimers which are recovered from recombinant cell culture. The preferred interface comprises at least a part of the C_{H}3 domain of an antibody constant domain. In this method, one or more small amino acid side chains from the interface of the first antibody molecule are replaced with larger side chains (e.g. tyrosine or tryptophan). Compensatory "cavities" of identical or similar size to the large side chain(s) are created on the interface of the second antibody molecule by replacing large amino acid side chains with smaller ones (e.g. alanine or threonine). This provides a mechanism for increasing the yield of the heterodimer over other unwanted end-products such as homodimers.

Bispecific antibodies include cross-linked or "heteroconjugate" antibodies. For example, one of the antibodies in the heteroconjugate can be coupled to avidin, the other to biotin. Such antibodies have, for example, been proposed to target immune system cells to unwanted cells (US Patent No. 4,676,980), and for treatment of HIV infection (WO 91/00360, WO 92/00373, and EP 03089). Heteroconjugate antibodies may be made using any convenient cross-linking methods. Suitable cross-linking agents are well known in the art, and are disclosed in US Patent No. 4,676,980, along with a number of cross-linking techniques.

Techniques for generating bispecific antibodies from antibody fragments have also been described in the literature. For example, bispecific antibodies can be prepared using chemical linkage. Brennan et al., Science, 229: 81 (1985) describe a procedure wherein intact antibodies are proteolytically cleaved to generate F(ab')₂ fragments. These fragments are reduced in the presence of the dithiol complexing agent sodium arsenite to stabilize vicinal dithiols and prevent intermolecular disulfide formation. The Fab' fragments generated are then converted to thionitrobenzoate (TNB) derivatives. One of the Fab'-TNB derivatives is then reconverted to the Fab'-thiol by reduction with mercaptoethylamine and is mixed with an equimolar amount of the other Fab'-TNB derivative to form the bispecific antibody. The bispecific antibodies produced can be used as agents for the selective immobilization of enzymes.

Recent progress has facilitated the direct recovery of Fab'-SH fragments from E. coli, which can be chemically coupled to form bispecific antibodies. Shalaby et al., J. Exp. Med., 175: 217-225 (1992) describe the production of a fully humanized bispecific antibody F(ab')₂ molecule. Each Fab' fragment was separately secreted from E. coli and subjected to directed chemical coupling in vitro to form the bispecific antibody. The bispecific antibody thus formed was able to bind to cells overexpressing the HER2 receptor and normal human T cells, as well as trigger the lytic activity of human cytotoxic lymphocytes against human breast tumor targets.

Various techniques for making and isolating bispecific antibody fragments directly from recombinant cell culture have also been described. For example, bispecific antibodies have been produced using leucine zippers. Kostelny et al., J. Immunol., 148(5): 1547-1553 (1992). The leucine zipper peptides from the Fos and Jun proteins were linked to the Fab' portions of two different antibodies by gene fusion. The antibody homodimers were reduced at the hinge region to form monomers and then re-oxidized to form the antibody heterodimers. This method can also be utilized for the production of antibody homodimers. The "diabody" technology described by Hollinger et al., Proc. Natl. Acad. Sci. USA, 90:6444-6448 (1993) has provided an alternative mechanism for making bispecific antibody fragments. The fragments comprise a heavy-chain variable domain (VH) connected to a light-chain variable domain (VL) by a linker which is too short to allow pairing between the two domains on the same chain. Accordingly, the VH and VL domains of one fragment arc forced to pair with the complementary VL and VH domains of another fragment, thereby forming two antigen-binding sites. Another strategy for making bispecific antibody fragments by the use of single-chain Fv (sFv) dimers has also been reported. See Gruber et al., J. Immunol., 152:5368 (1994).

Antibodies with more than two valencies are contemplated. For example, trispecific antibodies can be prepared. Tutt et al. J. Immunol. 147: 60 (1991).

### Multivalent Antibodies

A multivalent antibody may be internalized (and/or catabolized) faster than a bivalent antibody by a cell expressing an antigen to which the antibodies bind. The antibodies of the present invention can be multivalent antibodies (which are other than of the IgM class) with three or more antigen binding sites (e.g. tetravalent antibodies), which can be readily produced by recombinant expression of nucleic acid encoding the polypeptide chains of the antibody. The multivalent antibody can comprise a dimerization domain and three or more antigen binding sites. The preferred dimerization domain comprises (or consists of) an Fc region or a hinge region. In this scenario, the antibody will comprise an Fc region and three or more antigen binding sites amino-terminal to the Fc region. The preferred multivalent antibody herein comprises (or consists of) three to about eight, but preferably four, antigen binding sites. The multivalent antibody comprises at least one polypeptide chain (and preferably two polypeptide chains), wherein the polypeptide chain(s) comprise two or more variable domains. For instance, the polypeptide chain(s) may comprise VD1-(X1)n -VD2-(X2)n -Fc, wherein VD1 is a first variable domain, VD2 is a second variable domain, Fc is one polypeptide chain of an Fc region, X1 and X2 represent an amino acid or polypeptide, and n is 0 or 1. For instance, the polypeptide chain(s) may comprise: VH-CH1-flexible linker-VH-CH1-Fc region chain; or VH-CH1-VH-CH1-Fc region chain. The multivalent antibody herein preferably further comprises at least two (and preferably four) light chain variable domain polypeptides. The multivalent antibody herein may, for instance, comprise from about two to about eight light chain variable domain polypeptides. The light chain variable domain polypeptides contemplated here comprise a light chain variable domain and, optionally, further comprise a CL domain.

### Antibody Variants

In some embodiments, amino acid sequence modification(s) of the antibodies described herein arc contemplated. For example, it may be desirable to improve the binding affinity and/or other biological properties of the antibody. Amino acid sequence variants of the antibody are prepared by introducing appropriate nucleotide changes into the antibody nucleic acid, or by peptide synthesis. Such modifications include, for example, deletions from, and/or insertions into and/or substitutions of, residues within the amino acid sequences of the antibody. Any combination of deletion, insertion, and substitution is made to arrive at the final construct, provided that the final construct possesses the desired characteristics. The amino acid alterations may be introduced in the subject antibody amino acid sequence at the time that sequence is made.

A useful method for identification of certain residues or regions of the antibody that arc preferred locations for mutagenesis is called "alanine scanning mutagenesis" as described by Cunningham and Wells (1989) Science, 244:1081-1085. Here, a residue or group of target residues are identified (e.g., charged residues such as arg, asp, his, lys, and glu) and replaced by a neutral or negatively charged amino acid (most preferably alanine or polyalanine) to affect the interaction of the amino acids with antigen. Those amino acid locations demonstrating functional sensitivity to the substitutions then are refined by introducing further or other variants at, or for, the sites of substitution. Thus, while the site for introducing an amino acid sequence variation is predetermined, the nature of the mutation per se need not be predetermined. For example, to analyze the performance of a mutation at a given site, ala scanning or random mutagenesis is conducted at the target codon or region and the expressed immunoglobulins arc screened for the desired activity.

Amino acid sequence insertions include amino- and/or carboxyl-terminal fusions ranging in length from one residue to polypeptides containing a hundred or more residues, as well as intrasequence insertions of single or multiple amino acid residues. Examples of terminal insertions include an antibody with an N-terminal methionyl residue or the antibody fused to a cytotoxic polypeptide. Other insertional variants of the antibody molecule include the fusion to the N- or C-terminus of the antibody to an enzyme (e.g. for ADEPT) or a polypeptide which increases the serum half-life of the antibody.

Another type of amino acid variant of the antibody alters the original glycosylation pattern of the antibody. Such altering includes deleting one or more carbohydrate moieties found in the antibody, and/or adding one or more glycosylation sites that are not present in the antibody.

Glycosylation of polypeptides is typically cither N-linked or O-linked. N-linked refers to the attachment of the carbohydrate moiety to the side chain of an asparagine residue. The tripcptidc sequences asparagine-X-serine and asparagine-X-threonine, where X is any amino acid except proline, arc the recognition sequences for enzymatic attachment of the carbohydrate moiety to the asparagine side chain. Thus, the presence of either of these tripeptide sequences in a polypeptide creates a potential glycosylation site. O-linked glycosylation refers to the attachment of one of the sugars N-aceylgalactosamine, galactose, or xylose to a hydroxyamino acid, most commonly serine or threonine, although 5-hydroxyproline or 5-hydroxylysine may also be used.

Addition of glycosylation sites to the antibody is conveniently accomplished by altering the amino acid sequence such that it contains one or more of the above-described tripeptide sequences (for N-linked glycosylation sites). The alteration may also be made by the addition of, or substitution by, one or more serine or threonine residues to the sequence of the original antibody (for O-linked glycosylation sites).

Where the antibody comprises an Fc region, the carbohydrate attached thereto may be altered. For example, antibodies with a mature carbohydrate structure that lacks fucose attached to an Fc region of the antibody are described in US Pat Appl No US 2003/0157108 (Presta, L.). See also US 2004/0093621 (Kyowa Hakko Kogyo Co., Ltd). Antibodies with a bisecting N-acetylglucosamine (GlcNAc) in the carbohydrate attached to an Fc region of the antibody are referenced in WO 2003/011878, Jean-Mairet et al. and US Patent No. 6,602,684, Umana et al. Antibodies with at least one galactose residue in the oligosaccharide attached to an Fc region of the antibody are reported in WO 1997/30087, Patel et al. See, also, WO 1998/58964 (Raju, S.) and WO 1999/22764 (Raju, S.) concerning antibodies with altered carbohydrate attached to the Fc region thereof. See also US 2005/0123546 (Umana et al.) on antigen-binding molecules with modified glycosylation.

The preferred glycosylation variant herein comprises an Fc region, wherein a carbohydrate structure attached to the Fc region lacks fucose. Such variants have improved ADCC function. Optionally, the Fc region further comprises one or more amino acid substitutions therein which further improve ADCC, for example, substitutions at positions 298, 333, and/or 334 of the Fc region (Eu numbering of residues). Examples of publications related to "defucosylated" or "fucose-deficient" antibodies include: US 2003/0157108; WO 2000/61739; WO 2001/29246; US 2003/0115614; US 2002/0164328; US 2004/0093621; US 2004/0132140; US 2004/0110704; US 2004/0110282; US 2004/0109865; WO 2003/085119; WO 2003/084570; WO 2005/035586; WO 2005/035778; WO2005/053742; Okazaki et al. J. Mol. Biol. 336:1239-1249 (2004); Yamane-Ohnuki et al. Biotech. Bioeng. 87: 614 (2004). Examples of cell lines producing defucosylated antibodies include Lec13 CHO cells deficient in protein fucosylation (Ripka et al. Arch. Biochcm. Biophys. 249:533-545 (1986); US Pat Appl No US 2003/0157108 A1, Presta, L; and WO 2004/056312 A1, Adams et al., especially at Example 11), and knockout cell lines, such as alpha-1,6-fucosyltransferase gene, FUT8, knockout CHO cells (Yamane-Ohnuki et al. Biotech. Bioeng. 87: 614 (2004)).

Another type of variant is an amino acid substitution variant. These variants have at least one amino acid residue in the antibody molecule replaced by a different residue. The sites of greatest interest for substitutional mutagenesis include the hypervariable regions, but FR alterations are also contemplated. Conservative substitutions are shown in Table 1 under the heading of "preferred substitutions". If such substitutions result in a change in biological activity, then more substantial changes, denominated "exemplary substitutions" in Table 1, or as further described below in reference to amino acid classes, may be introduced and the products screened.

**Table 1**

| **Original Residue** | **Exemplary Substitutions** | **Preferred Substitutions** |
|---|---|---|
| Ala (A) | Val; Leu; Ile | Val |
| Arg (R) | Lys; Gln; Asn | Lys |
| Asn (N) | Gln; His; Asp, Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |
| His (H) | Asn; Gln; Lys; Arg | Arg |
| Ile (I) | Leu; Val; Met; Ala; Phe; Norleucine | Leu |
| Leu (L) | Norleucine; Ile; Val; Met; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser(S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Norleucine | Leu |

Substantial modifications in the biological properties of the antibody are accomplished by selecting substitutions that differ significantly in their effect on maintaining (a) the structure of the polypeptide backbone in the area of the substitution, for example, as a sheet or helical conformation, (b) the charge or hydrophobicity of the molecule at the target site, or (c) the bulk of the side chain. Naturally occurring residues are divided into groups based on common side-chain properties:
(1) hydrophobic: norleucine, met, ala, val, leu, ile;
(2) neutral hydrophilic: Cys, Ser, Thr, Asn, Gln;
(3) acidic: asp, glu;
(4) basic: his, lys, arg;
(5) residues that influence chain orientation: gly, pro; and
(6) aromatic: trp, tyr, phe.

Non-conscrvative substitutions will entail exchanging a member of one of these classes for another class.

One type of substitutional variant involves substituting one or more hypervariable region residues of a parent antibody (*e.g*. a humanized or human antibody). Generally, the resulting variant(s) selected for further development will have improved biological properties relative to the parent antibody from which they are generated. A convenient way for generating such substitutional variants involves affinity maturation using phage display. Briefly, several hypervariable region sites (*e.g.* 6-7 sites) are mutated to generate all possible amino acid substitutions at each site. The antibodies thus generated are displayed from filamentous phage particles as fusions to the gene III product of M13 packaged within each particle. The phage-displayed variants are then screened for their biological activity (*e.g.* binding affinity) as herein disclosed. In order to identify candidate hypervariable region sites for modification, alanine scanning mutagenesis can be performed to identify hypervariable region residues contributing significantly to antigen binding. Alternatively, or additionally, it may be beneficial to analyze a crystal structure of the antigen-antibody complex to identify contact points between the antibody and antigen. Such contact residues and neighboring residues are candidates for substitution according to the techniques elaborated herein. Once such variants are generated, the panel of variants is subjected to screening as described herein and antibodies with superior properties in one or more relevant assays may be selected for further development.

Nucleic acid molecules encoding amino acid sequence variants of the antibody arc prepared by a variety of methods known in the art. These methods include, but are not limited to, isolation from a natural source (in the case of naturally occurring amino acid sequence variants) or preparation by oligonucleotide-mediated (or site-directed) mutagenesis, PCR mutagenesis, and cassette mutagenesis of an earlier prepared variant or a non-variant version of the antibody.

It may be desirable to introduce one or more amino acid modifications in an Fc region of the immunoglobulin polypeptide, thereby generating a Fc region variant. The Fc region variant may comprise a human Fc region sequence (*e.g*., a human IgG1, IgG2, IgG3 or IgG4 Fc region) comprising an amino acid modification (*e.g.* a substitution) at one or more amino acid positions including that of a hinge cysteine.

In accordance with this description and the teachings of the art, it is contemplated that in some embodiments, an antibody used in methods of the invention may comprise one or more alterations as compared to the wild type counterpart antibody, e.g. in the Fc region. These antibodies would nonetheless retain substantially the same characteristics required for therapeutic utility as compared to their wild type counterpart. For example, it is thought that certain alterations can be made in the Fc region that would result in altered (i.e., either improved or diminished) C1q binding and/or Complement Dependent Cytotoxicity (CDC), e.g., as described in WO99/51642. See also Duncan & Winter Nature 322:738-40 (1988); US Patent No. 5,648,260; US Patent No. 5,624,821; and WO94/29351 concerning other examples of Fc region variants. WO00/42072 (Prcsta) and WO 2004/056312 (Lowman) describe antibody variants with improved or diminished binding to FcRs. See, also, Shields et al. J. Biol. Chem. 9(2): 6591-6604 (2001). Antibodies with increased half lives and improved binding to the neonatal Fc receptor (FcRn), which is responsible for the transfer of maternal IgGs to the fetus (Guyer et al., J. Immunol. 117:587 (1976) and Kim et al., J. Immunol. 24:249 (1994)), are described in US2005/0014934A1 (Hinton et al.). These antibodies comprise an Fc reg on with one or more substitutions therein which improve binding of the Fc region to FcRn. Polypeptide variants with altered Fc region amino acid sequences and increased or decreased C1q binding capability are described in US patent No. 6,194,551B1, WO99/51642. See, also, Idusogie et al. J. Immunol. 164: 4178-4184 (2000).

### Antibody Derivatives

The antibodies of the present invention can be further modified to contain additional nonproteinaceous moieties that are known in the art and readily available. Preferably, the moieties suitable for dcrivatization of the antibody are water soluble polymers. Non-limiting examples of water soluble polymers include, but are not limited to, polyethylene glycol (PEG), copolymers of ethylene glycol/propylene glycol, carboxymethylcellulose, dextran, polyvinyl alcohol, polyvinyl pyrrolidone, poly-1,3-dioxolane, poly-1,3,6-trioxane, ethylene/maleic anhydride copolymer, polyaminoacids (either homopolymers or random copolymers), and dextran or poly(n-vinyl pyrrolidone)polyethylene glycol, propropylene glycol homopolymers, prolypropylene oxide/ethylene oxide co-polymers, polyoxyethylated polyols (e.g., glycerol), polyvinyl alcohol, and mixtures thereof. Polyethylene glycol propionaldehyde may have advantages in manufacturing due to its stability in water. The polymer may be of any molecular weight, and may be branched or unbranched. The number of polymers attached to the antibody may vary, and if more than one polymers are attached, they can be the same or different molecules. In general, the number and/or type of polymers used for derivatization can be determined based on considerations including, but not limited to, the particular properties or functions of the antibody to be improved, whether the antibody derivative will be used in a therapy under defined conditions, etc.

### Vectors, Host Cells and Recombinant Methods

For recombinant production of an antibody, the nucleic acid encoding it is isolated and inserted into a replicable vector for further cloning (amplification of the DNA) or for expression. DNA encoding the antibody is readily isolated and sequenced using conventional procedures (e.g., by using oligonucleotide probes that are capable of binding specifically to genes encoding the heavy and light chains of the antibody). Many vectors are available. The choice of vector depends in part on the host cell to be used. Generally, preferred host cells are of either prokaryotic or eukaryotic (generally mammalian) origin. It will be appreciated that constant regions of any isotype can be used for this purpose, including IgG, IgM, IgA, IgD, and IgE constant regions, and that such constant regions can be obtained from any human or animal species.

### a. Generating antibodies using prokaryotic host cells:

### i. Vector Construction

Polynucleotide sequences encoding polypeptide components of the antibody can be obtained using standard recombinant techniques. Desired polynucleotide sequences may be isolated and sequenced from antibody producing cells such as hybridoma cells. Alternatively, polynucleotides can be synthesized using nucleotide synthesizer or PCR techniques. Once obtained, sequences encoding the polypeptides are inserted into a recombinant vector capable of replicating and expressing heterologous polynucleotides in prokaryotic hosts. Many vectors that are available and known in the art can be used for the purpose of the present invention. Selection of an appropriate vector will depend mainly on the size of the nucleic acids to be inserted into the vector and the particular host cell to be transformed with the vector. Each vector contains various components, depending on its function (amplification or expression of heterologous polynucleotide, or both) and its compatibility with the particular host cell in which it resides. The vector components generally include, but are not limited to: an origin of replication, a selection marker gene, a promoter, a ribosome binding site (RBS), a signal sequence, the heterologous nucleic acid insert and a transcription termination sequence.

In general, plasmid vectors containing replicon and control sequences which are derived from species compatible with the host cell are used in connection with these hosts. The vector ordinarily carries a replication site, as well as marking sequences which are capable of providing phenotypic selection in transformed cells. For example, E. coli is typically transformed using pBR322, a plasmid derived from an E. coli species. pBR322 contains genes encoding ampicillin (Amp) and tetracycline (Tet) resistance and thus provides easy means for identifying transformed cells. pBR322, its derivatives, or other microbial plasmids or bacteriophage may also contain, or be modified to contain, promoters which can be used by the microbial organism for expression of endogenous proteins. Examples of pBR322 derivatives used for expression of particular antibodies are described in detail in Carter et al., U.S. Patent No. 5,648,237.

In addition, phage vectors containing replicon and control sequences that are compatible with the host microorganism can be used as transforming vectors in connection with these hosts. For example, bacteriophage such as λGEM.TM.-11 may be utilized in making a recombinant vector which can be used to transform susceptible host cells such as E. coli LE392.

The expression vector may comprise two or more promoter-cistron pairs, encoding each of the polypeptide components. A promoter is an untranslated regulatory sequence located upstream (5') to a cistron that modulates its expression. Prokaryotic promoters typically fall into two classes, inducible and constitutive. Inducible promoter is a promoter that initiates increased levels of transcription of the cistron under its control in response to changes in the culture condition, e.g. the presence or absence of a nutrient or a change in temperature.

A large number of promoters recognized by a variety of potential host cells are well known. The selected promoter can be operably linked to cistron DNA encoding the light or heavy chain by removing the promoter from the source DNA via restriction enzyme digestion and inserting the isolated promoter sequence into the vector. Both the native promoter sequence and many heterologous promoters may be used to direct amplification and/or expression of the target genes. In some embodiments, heterologous promoters are utilized, as they generally permit greater transcription and higher yields of expressed target gene as compared to the native target polypeptide promoter.

Promoters suitable for use with prokaryotic hosts include the PhoA promoter, the β-galactamase and lactose promoter systems, a tryptophan (trp) promoter system and hybrid promoters such as the tac or the trc promoter. However, other promoters that are functional in bacteria (such as other known bacterial or phage promoters) are suitable as well. Their nucleotide sequences have been published, thereby enabling a skilled worker operably to ligate them to cistrons encoding the target light and heavy chains (Siebenlist et al. (1980) Cell 20: 269) using linkers or adaptors to supply any required restriction sites.

In one aspect, each cistron within the recombinant vector comprises a secretion signal sequence component that directs translocation of the expressed polypeptides across a membrane. In general, the signal sequence may be a component of the vector, or it may be a part of the target polypeptide DNA that is inserted into the vector. The signal sequence selected for the purpose of this invention should be one that is recognized and processed (i.e. cleaved by a signal peptidase) by the host cell. For prokaryotic host cells that do not recognize and process the signal sequences native to the heterologous polypeptides, the signal sequence is substituted by a prokaryotic signal sequence selected, for example, from the group consisting of the alkaline phosphatase, penicillinase, Ipp, or heat-stable enterotoxin II (STII) leaders, LamB, PhoE, PelB, OmpA and MBP. In one embodiment, the signal sequences used in both cistrons of the expression system are STII signal sequences or variants thereof.

In another aspect, the production of the immunoglobulins according to the invention can occur in the cytoplasm of the host cell, and therefore does not require the presence of secretion signal sequences within each cistron. In that regard, immunoglobulin light and heavy chains are expressed, folded and assembled to form functional immunoglobulins within the cytoplasm. Certain host strains (e.g., the E. coli trxB- strains) provide cytoplasm conditions that are favorable for disulfide bond formation, thereby permitting proper folding and assembly of expressed protein subunits. Proba and Pluckthun Gene, 159:203 (1995).

Prokaryotic host cells suitable for expressing antibodies include Archaebacteria and Eubacteria, such as Gram-negative or Gram-positive organisms. Examples of useful bacteria include Escherichia (e.g., E. coli), Bacilli (e.g., B. subtilis), Enterobacteria, Pseudomonas species (e.g., P. aeruginosa), Salmonella typhimurium, Serratia marcescans, Klebsiella, Proteus, Shigella, Rhizobia, Vitreoscilla, or Paracoccus. In one embodiment, gram-negative cells are used. In one embodiment, E. coli cells are used as hosts for the invention. Examples of E. coli strains include strain W3110 (Bachmann, Cellular and Molecular Biology, vol. 2 (Washington, D.C.: American Society for Microbiology, 1987), pp. 1190-1219; ATCC Deposit No. 27,325) and derivatives thereof, including strain 33D3 having genotype W3110 ΔfhuA (ΔtonA) ptr3 lac Iq lacL8 ΔompTΔ(nmpc-fepE) degP41 kanR (U.S. Pat. No. 5,639,635). Other strains and derivatives thereof, such as E. coli 294 (ATCC 31,446), E. coli B, E. coliλ 1776 (ATCC 31,537) and E. coli RV308(ATCC 31,608) are also suitable. These examples are illustrative rather than limiting. Methods for constructing derivatives of any of the above-mentioned bacteria having defined genotypes are known in the art and described in, for example, Bass et al., Proteins, 8:309-314 (1990). It is generally necessary to select the appropriate bacteria taking into consideration replicability of the replicon in the cells of a bacterium. For example, E. coli, Serratia, or Salmonella species can be suitably used as the host when well known plasmids such as pBR322, pBR325, pACYC177, or pKN410 are used to supply the replicon. Typically the host cell should secrete minimal amounts of proteolytic enzymes, and additional protease inhibitors may desirably be incorporated in the cell culture.

### ii. Antibody Production

Host cells are transformed with the above-described expression vectors and cultured in conventional nutrient media modified as appropriate for inducing promoters, selecting transformants, or amplifying the genes encoding the desired sequences.

Transformation means introducing DNA into the prokaryotic host so that the DNA is replicable, either as an extrachromosomal element or by chromosomal integrant. Depending on the host cell used, transformation is done using standard techniques appropriate to such cells. The calcium treatment employing calcium chloride is generally used for bacterial cells that contain substantial cell-wall barriers. Another method for transformation employs polyethylene glycol/DMSO. Yet another technique used is electroporation.

Prokaryotic cells used to produce the polypeptides are grown in media known in the art and suitable for culture of the selected host cells. Examples of suitable media include luria broth (LB) plus necessary nutrient supplements. In some embodiments, the media also contains a selection agent, chosen based on the construction of the expression vector, to selectively permit growth of prokaryotic cells containing the expression vector. For example, ampicillin is added to media for growth of cells expressing ampicillin resistant gene.

Any necessary supplements besides carbon, nitrogen, and inorganic phosphate sources may also be included at appropriate concentrations introduced alone or as a mixture with another supplement or medium such as a complex nitrogen source. Optionally the culture medium may contain one or more reducing agents selected from the group consisting of glutathione, cysteine, cystamine, thioglycollate, dithiocrythritol and dithiothreitol.

The prokaryotic host cells arc cultured at suitable temperatures. For E. coli growth, for example, the preferred temperature ranges from about 20°C to about 39°C, more preferably from about 25°C to about 37°C, even more preferably at about 30°C. The pH of the medium may be any pH ranging from about 5 to about 9, depending mainly on the host organism. For E. coli, the pH is preferably from about 6.8 to about 7.4, and more preferably about 7.0.

If an inducible promoter is used in the expression vector, protein expression is induced under conditions suitable for the activation of the promoter. In one aspect, PhoA promoters are used for controlling transcription of the polypeptides. Accordingly, the transformed host cells are cultured in a phosphate-limiting medium for induction. Preferably, the phosphate-limiting medium is the C.R.A.P medium (see, e.g., Simmons et al., J. Immunol. Methods (2002), 263:133-147). A variety of other inducers may be used, according to the vector construct employed, as is known in the art.

In one embodiment, the expressed polypeptides of the present invention are secreted into and recovered from the periplasm of the host cells. Protein recovery typically involves disrupting the microorganism, generally by such means as osmotic shock, sonication or lysis. Once cells are disrupted, cell debris or whole cells may be removed by centrifugation or filtration. The proteins may be further purified, for example, by affinity resin chromatography. Alternatively, proteins can be transported into the culture media and isolated therein. Cells may be removed from the culture and the culture supernatant being filtered and concentrated for further purification of the proteins produced. The expressed polypeptides can be further isolated and identified using commonly known methods such as polyacrylamide gel electrophoresis (PAGE) and Western blot assay.

In one aspect, antibody production is conducted in large quantity by a fermentation process. Various large-scale fed-batch fermentation procedures are available for production of recombinant proteins. Large-scale fermentations have at least 1000 liters of capacity, preferably about 1,000 to 100,000 liters of capacity. These fermentors use agitator impellers to distribute oxygen and nutrients, especially glucose (the preferred carbon/energy source). Small scale fermentation refers generally to fermentation in a fermentor that is no more than approximately 100 liters in volumetric capacity, and can range from about 1 liter to about 100 liters.

In a fermentation process, induction of protein expression is typically initiated after the cells have been grown under suitable conditions to a desired density, e.g., an OD550 of about 180-220, at which stage the cells are in the early stationary phase. A variety of inducers may be used, according to the vector construct employed, as is known in the art and described above. Cells may be grown for shorter periods prior to induction. Cells are usually induced for about 12-50 hours, although longer or shorter induction time may be used.

To improve the production yield and quality of the polypeptides, various fermentation conditions can be modified. For example, to improve the proper assembly and folding of the secreted antibody polypeptides, additional vectors overexpressing chaperone proteins, such as Dsb proteins (DsbA, DsbB, DsbC, DsbD and or DsbG) or FkpA (a peptidylprolyl cis,trans-isomerase with chaperone activity) can be used to co-transform the host prokaryotic cells. The chaperone proteins have been demonstrated to facilitate the proper folding and solubility of heterologous proteins produced in bacterial host cells. Chen et al. (1999) J Bio Chem 274:19601-19605; Georgiou et al., U.S. Patent No. 6,083,715; Georgiou et al., U.S. Patent No. 6,027,888; Bothmann and Pluckthun (2000) J. Biol. Chem. 275:17100-17105; Ramm and Pluckthun (2000) J. Biol. Chem. 275:17106-17113; Arie et al. (2001) Mol. Microbiol. 39:199-210.

To minimize proteolysis of expressed heterologous proteins (especially those that are proteolytically sensitive), certain host strains deficient for proteolytic enzymes can be used for the present invention. For example, host cell strains may be modified to effect genetic mutation(s) in the genes encoding known bacterial proteases such as Protease III, OmpT, DegP, Tsp, Protease I, Protease Mi, Protease V, Protease VI and combinations thereof. Some E. coli protease-deficient strains are available and described in, for example, Joly et al. (1998), supra; Georgiou et al., U.S. Patent No. 5,264,365; Georgiou et al., U.S. Patent No. 5,508,192; Hara et al., Microbial Drug Resistance, 2:63-72 (1996).

In one embodiment, E. coli strains deficient for proteolytic enzymes and transformed with plasmids overexpressing one or more chaperone proteins are used as host cells in the expression system.

### iii. Antibody Purification

Standard protein purification methods known in the art can be employed. The following procedures arc exemplary of suitable purification procedures: fractionation on immunoaffinity or ion-exchange columns, ethanol precipitation, reverse phase HPLC, chromatography on silica or on a cation-cxchangc resin such as DEAE, chromatofocusing, SDS-PAGE, ammonium sulfate precipitation, and gel filtration using, for example, Sephadex G-75.

In one aspect, Protein A immobilized on a solid phase is used for immunoaffinity purification of the full length antibody products. Protein A is a 41kD cell wall protein from Staphylococcus aureas which binds with a high affinity to the Fc region of antibodies. Lindmark ct al (1983) J. Immunol. Mcth. 62:1-13. The solid phase to which Protein A is immobilized is preferably a column comprising a glass or silica surface, more preferably a controlled pore glass column or a silicic acid column. In some applications, the column has been coated with a reagent, such as glycerol, in an attempt to prevent nonspecific adherence of contaminants.

As the first step of purification, the preparation derived from the cell culture as described above is applied onto the Protein A immobilized solid phase to allow specific binding of the antibody of interest to Protein A. The solid phase is then washed to remove contaminants non-specifically bound to the solid phase. Finally the antibody of interest is recovered from the solid phase by elution.

### b. Generating antibodies using eukaryotic host cells:

The vector components generally include, but are not limited to, one or more of the following: a signal sequence, an origin of replication, one or more marker genes, an enhancer element, a promoter, and a transcription termination sequence.

### (i) Signal sequence component

A vector for use in a eukaryotic host cell may also contain a signal sequence or other polypeptide having a specific cleavage site at the N-terminus of the mature protein or polypeptide of interest. The heterologous signal sequence selected preferably is one that is recognized and processed (i.e., cleaved by a signal peptidase) by the host cell. In mammalian cell expression, mammalian signal sequences as well as viral secretory leaders, for example, the herpes simplex gD signal, are available.

The DNA for such precursor region is ligated in reading frame to DNA encoding the antibody.

### (ii) Origin of replication

Generally, an origin of replication component is not needed for mammalian expression vectors. For example, the SV40 origin may typically be used only because it contains the early promoter.

### (iii) Selection gene component

Expression and cloning vectors may contain a selection gene, also termed a selectable marker. Typical selection genes encode proteins that (a) confer resistance to antibiotics or other toxins, e.g., ampicillin, neomycin, methotrexate, or tetracycline, (b) complement auxotrophic deficiencies, where relevant, or (c) supply critical nutrients not available from complex media.

One example of a selection scheme utilizes a drug to arrest growth of a host cell. Those cells that are successfully transformed with a heterologous gene produce a protein conferring drug resistance and thus survive the selection regimen. Examples of such dominant selection use the drugs neomycin, mycophenolic acid and hygromycin.

Another example of suitable selectable markers for mammalian cells are those that enable the identification of cells competent to take up the antibody nucleic acid, such as DHFR, thymidine kinase, metallothionein-I and -II, preferably primate metallothionein genes, adenosine deaminase, ornithine decarboxylase, etc.

For example, cells transformed with the DHFR selection gene are first identified by culturing all of the transformants in a culture medium that contains methotrexate (Mtx), a competitive antagonist of DHFR. An appropriate host cell when wild-type DHFR is employed is the Chinese hamster ovary (CHO) cell line deficient in DHFR activity (e.g., ATCC CRL-9096).

Alternatively, host cells (particularly wild-type hosts that contain endogenous DHFR) transformed or co-transformed with DNA sequences encoding an antibody, wild-type DHFR protein, and another selectable marker such as aminoglycoside 3'-phosphotransferase (APH) can be selected by cell growth in medium containing a selection agent for the selectable marker such as an aminoglycosidic antibiotic, e.g., kanamycin, neomycin, or G418. See U.S. Patent No. 4,965,199.

### (iv) Promoter component

Expression and cloning vectors usually contain a promoter that is recognized by the host organism and is operably linked to the antibody polypeptide nucleic acid. Promoter sequences arc known for eukaryotes. Virtually alleukaryotic genes have an AT-rich region located approximately 25 to 30 bases upstream from the site where transcription is initiated. Another sequence found 70 to 80 bases upstream from the start of transcription of many genes is a CNCAAT region where N may be any nucleotide. At the 3' end of most eukaryotic genes is an AATAAA sequence that may be the signal for addition of the poly A tail to the 3' end of the coding sequence. All of these sequences are suitably inserted into eukaryotic expression vectors.

Antibody polypeptide transcription from vectors in mammalian host cells is controlled, for example, by promoters obtained from the genomes of viruses such as polyoma virus, fowlpox virus, adenovirus (such as Adenovirus 2), bovine papilloma virus, avian sarcoma virus, cytomegalovirus, a retrovirus, hepatitis-B virus and Simian Virus 40 (SV40), from heterologous mammalian promoters, e.g., the actin promoter or an immunoglobulin promoter, from heat-shock promoters, provided such promoters are compatible with the host cell systems.

The early and late promoters of the SV40 virus are conveniently obtained as an SV40 restriction fragment that also contains the SV40 viral origin of replication. The immediate early promoter of the human cytomegalovirus is conveniently obtained as a HindIII E restriction fragment. A system for expressing DNA in mammalian hosts using the bovine papilloma virus as a vector is disclosed in U.S. Patent No. 4,419,446. A modification of this system is described in U.S. Patent No. 4,601,978. Alternatively, the Rous Sarcoma Virus long terminal repeat can be used as the promoter.

### (v) Enhancer element component

Transcription of DNA encoding the antibody polypeptide of this invention by higher eukaryotes is often increased by inserting an enhancer sequence into the vector. Many enhancer sequences are now known from mammalian genes (globin, elastase, albumin, α-fctoprotcin, and insulin). Typically, however, one will use an enhancer from a eukaryotic cell virus. Examples include the SV40 enhancer on the late side of the replication origin (bp 100-270), the cytomegalovirus early promoter enhancer, the polyoma enhancer on the late side of the replication origin, and adenovirus enhancers. See also Yaniv, Nature 297:17-18 (1982) on enhancing elements for activation of eukaryotic promoters. The enhancer may be spliced into the vector at a position 5' or 3' to the antibody polypeptide-encoding sequence, but is preferably located at a site 5' from the promoter.

### (vi) Transcription termination component

Expression vectors used in eukaryotic host cells will typically also contain sequences necessary for the termination of transcription and for stabilizing the mRNA. Such sequences are commonly available from the 5' and, occasionally 3', untranslated regions of eukaryotic or viral DNAs or cDNAs. These regions contain nucleotide segments transcribed as polyadenylated fragments in the untranslated portion of the mRNA encoding an antibody. One useful transcription termination component is the bovine growth hormone polyadenylation region. See WO94/11026 and the expression vector disclosed therein.

### (vii) Selection and transformation of host cells

Suitable host cells for cloning or expressing the DNA in the vectors herein include higher eukaryote cells described herein, including vertebrate host cells. Propagation of vertebrate cells in culture (tissue culture) has become a routine procedure. Examples of useful mammalian host cell lines are monkey kidney CV1 line transformed by SV40 (COS-7, ATCC CRL 1651); human embryonic kidney line (293 or 293 cells subcloned for growth in suspension culture, Graham et al., J. Gen Virol. 36:59 (1977)) ; baby hamster kidney cells (BHK, ATCC CCL 10); Chinese hamster ovary cells/-DHFR (CHO, Urlaub et al., Proc. Natl. Acad. Sci. USA 77:4216 (1980)); mouse sertoli cells (TM4, Mather, Biol. Reprod. 23:243-251 (1980) ); monkey kidney cells (CV1 ATCC CCL 70); African green monkey kidney cells (VERO-76, ATCC CRL-1587); human cervical carcinoma cells (HELA, ATCC CCL 2); canine kidney cells (MDCK, ATCC CCL 34); buffalo rat liver cells (BRL 3A, ATCC CRL 1442); human lung cells (W138, ATCC CCL 75); human liver cells (Hep G2, HB 8065); mouse mammary tumor (MMT 060562, ATCC CCL51); TRI cells (Mather et al., Annals N.Y. Acad. Sci. 383:44-68 (1982)); MRC 5 cells; FS4 cells; and a human hepatoma line (Hep G2).

Host cells are transformed with the above-described expression or cloning vectors for antibody production and cultured in conventional nutrient media modified as appropriate for inducing promoters, selecting transformants, or amplifying the genes encoding the desired sequences.

### (viii) Culturing the host cells

The host cells used to produce an antibody may be cultured in a variety of media. Commercially available media such as Ham's F10 (Sigma), Minimal Essential Medium ((MEM), (Sigma), RPMI-1640 (Sigma), and Dulbecco's Modified Eagle's Medium ((DMEM), Sigma) are suitable for culturing the host cells. In addition, any of the media described in Ham et al., Meth. Enz. 58:44 (1979), Barnes et al., Anal. Biochem. 102:255 (1980), U.S. Pat. Nos. 4,767,704; 4,657,866; 4,927,762; 4,560,655; or 5,122,469; WO 90/03430; WO 87/00195; or U.S. Patent Re. 30,985 may be used as culture media for the host cells. Any of these media may be supplemented as necessary with hormones and/or other growth factors (such as insulin, transferrin, or epidermal growth factor), salts (such as sodium chloride, calcium, magnesium, and phosphate), buffers (such as HEPES), nucleotides (such as adenosine and thymidine), antibiotics (such as GENTAMYCIN™ drug), trace elements (defined as inorganic compounds usually present at final concentrations in the micromolar range), and glucose or an equivalent energy source. Any other necessary supplements may also be included at appropriate concentrations that would be known to those skilled in the art. The culture conditions, such as temperature, pH, and the like, are those previously used with the host cell selected for expression, and will be apparent to the ordinarily skilled artisan.

### (ix) Purification of antibody

When using recombinant techniques, the antibody can be produced intracellularly, or directly secreted into the medium. If the antibody is produced intracellularly, as a first step, the particulate debris, either host cells or lysed fragments, are removed, for example, by centrifugation or ultrafiltration. Where the antibody is secreted into the medium, supernatants from such expression systems are generally first concentrated using a commercially available protein concentration filter, for example, an Amicon or Millipore Pellicon ultrafiltration unit. A protease inhibitor such as PMSF may be included in any of the foregoing steps to inhibit proteolysis and antibiotics may be included to prevent the growth of adventitious contaminants.

The antibody composition prepared from the cells can be purified using, for example, hydroxylapatite chromatography, gel electrophoresis, dialysis, and affinity chromatography, with affinity chromatography being the preferred purification technique. The suitability of protein A as an affinity ligand depends on the species and isotype of any immunoglobulin Fc domain that is present in the antibody. Protein A can be used to purify antibodies that are based on human γ1,γ2, or γ4 heavy chains (Lindmark et al., J. Immunol. Meth. 62:1-13 (1983)). Protein G is recommended for all mouse isotypes and for human γ3 (Guss et al., EMBO J. 5:15671575 (1986)). The matrix to which the affinity ligand is attached is most often agarose, but other matrices are available. Mechanically stable matrices such as controlled pore glass or poly(styrenedivinyl)benzene allow for faster flow rates and shorter processing times than can be achieved with agarose. Where the antibody comprises a CH3 domain, the Bakerbond ABX™resin (J. T. Baker, Phillipsburg, NJ) is useful for purification. Other techniques for protein purification such as fractionation on an ion-exchange column, ethanol precipitation, Reverse Phase HPLC, chromatography on silica, chromatography on heparin SEPHAROSE™ chromatography on an anion or cation exchange resin (such as a polyaspartic acid column), chromatofocusing, SDS-PAGE, and ammonium sulfate precipitation are also available depending on the antibody to be recovered.

Following any preliminary purification step(s), the mixture comprising the antibody of interest and contaminants may be subjected to low pH hydrophobic interaction chromatography using an elution buffer at a pH between about 2.5-4.5, preferably performed at low salt concentrations (e.g., from about 0-0.25M salt).

### Immunoconjugates

The invention also provides immunoconjugates (interchangeably termed "antibody-drug conjugates" or "ADC"), comprising any of the anti-KLβ antibodies described herein conjugated to a cytotoxic agent such as a chemotherapeutic agent, a drug, a growth inhibitory agent, a toxin (e.g., an enzymatically active toxin of bacterial, fungal, plant, or animal origin, or fragments thereof), or a radioactive isotope (i.e., a radioconjugate).

The use of antibody-drug conjugates for the local delivery of cytotoxic or cytostatic agents, i.e. drugs to kill or inhibit tumor cells in the treatment of cancer (Syrigos and Epenetos (1999) Anticancer Research 19:605-614; Niculescu-Duvaz and Springer (1997) Adv. Drg Del. Rev. 26:151-172; U.S. patent 4,975,278) allows targeted delivery of the drug moiety to tumors, and intracellular accumulation therein, where systemic administration of these unconjugated drug agents may result in unacceptable levels of toxicity to normal cells as well as the tumor cells sought to be eliminated (Baldwin et al., (1986) Lancet pp. (Mar. 15, 1986):603-05; Thorpe, (1985) "Antibody Carriers Of Cytotoxic Agents In Cancer Therapy: A Review," in Monoclonal Antibodies '84: Biological And Clinical Applications, A. Pinchera et al. (ed.s), pp. 475-506). Maximal efficacy with minimal toxicity is sought thereby. Both polyclonal antibodies and monoclonal antibodies have been reported as useful in these strategies (Rowland et al., (1986) Cancer Immunol. Immunothcr., 21:183-87). Drugs used in these methods include daunomycin, doxorubicin, methotrexate, and vindesine (Rowland et al., (1986) supra). Toxins used in antibody-toxin conjugates include bacterial toxins such as diphtheria toxin, plant toxins such as ricin, small molecule toxins such as geldanamycin (Mandler et al (2000) Jour. of the Nat. Cancer Inst. 92(19): 1573-1581; Mandler et al (2000) Bioorganic & Med. Chem. Letters 10: 1025-1028; Mandler et al (2002) Bioconjugate Chem. 13:786-791), maytansinoids (EP 1391213; Liu et al., (1996) Proc. Natl. Acad. Sci. USA 93:8618-8623), and calicheamicin (Lode et al (1998) Cancer Res. 58:2928; Hinman et al (1993) Cancer Res. 53:3336-3342). The toxins may effect their cytotoxic and cytostatic effects by mechanisms including tubulin binding, DNA binding, or topoisomcrase inhibition. Some cytotoxic drugs tend to be inactive or less active when conjugated to large antibodies or protein receptor ligands.

ZEVALIN® (ibritumomab tiuxetan, Biogen/Idec) is an antibody-radioisotope conjugate composed of a murine IgG1 kappa monoclonal antibody directed against the CD20 antigen found on the surface of normal and malignant B lymphocytes and ¹¹¹In or ⁹⁰Y radioisotope bound by a thiourea linker-chelator (Wiseman ct al (2000) Eur. Jour. Nucl. Med. 27(7):766-77; Wiseman et al (2002) Blood 99(12):4336-42; Witzig et al (2002) J. Clin. Oncol. 20(10):2453-63; Witzig ct al (2002) J. Clin. Oncol. 20(15):3262-69). Although ZEVALIN has activity against B-cell non-Hodgkin's Lymphoma (NHL), administration results in severe and prolonged cytopenias in most patients. MYLOTARG™ (gemtuzumab ozogamicin, Wyeth Pharmaceuticals), an antibody drug conjugate composed of a hu CD33 antibody linked to calicheamicin, was approved in 2000 for the treatment of acute myeloid leukemia by injection (Drugs of the Future (2000) 25(7):686; US Patent Nos. 4970198; 5079233; 5585089; 5606040; 5693762; 5739116; 5767285; 5773001). Cantuzumab mertansine (Immunogen, Inc.), an antibody drug conjugate composed of the huC242 antibody linked via the disulfide linker SPP to the maytansinoid drug moiety, DM1, is advancing into Phase II trials for the treatment of cancers that express CanAg, such as colon, pancreatic, gastric, and others. MLN-2704 (Millennium Pharm., BZL Biologies, Immunogen Inc.), an antibody drug conjugate composed of the anti-prostate specific membrane antigen (PSMA) monoclonal antibody linked to the maytansinoid drug moiety, DM1, is under development for the potential treatment of prostate tumors. The auristatin peptides, auristatin E (AE) and monomethylauristatin (MMAE), synthetic analogs of dolastatin, were conjugated to chimeric monoclonal antibodies cBR96 (specific to Lewis Y on carcinomas) and cAC10 (specific to CD30 on hematological malignancies) (Doronina et al (2003) Nature Biotechnology 21(7):778-784) and are under therapeutic development.

Chemotherapeutic agents useful in the generation of immunoconjugates are described herein (eg., above). Enzymatically active toxins and fragments thereof that can be used include diphtheria A chain, nonbinding active fragments of diphtheria toxin, exotoxin A chain (from Pseudomonas aeruginosa), ricin A chain, abrin A chain, modeccin A chain, alpha-sarcin, Aleurites fordii proteins, dianthin proteins, Phytolaca americana proteins (PAPI, PAPII, and PAP-S), momordica charantia inhibitor, curcin, crotin, sapaonaria officinalis inhibitor, gelonin, mitogellin, restrictocin, phenomycin, enomycin, and the tricothecenes. See, e.g., WO 93/21232 published October 28, 1993. A variety of radionuclides are available for the production of radioconjugated antibodies. Examples include ²¹²Bi, ¹³¹I, ¹³¹In, ⁹⁰Y, and ¹⁸⁶Re. Conjugates of the antibody and cytotoxic agent are made using a variety of bifunctional protein-coupling agents such as N-succinimidyl-3-(2-pyridyldithiol) propionate (SPDP), iminothiolane (IT), bifunctional derivatives of imidoesters (such as dimethyl adipimidate HCl), active esters (such as disuccinimidyl suberate), aldehydes (such as glutaraldehyde), bis-azido compounds (such as bis (p-azidobenzoyl) hexanediamine), bis-diazonium derivatives (such as bis-(p-diazoniumbenzoyl)-ethylenediamine), diisocyanates (such as toluene 2,6-diisocyanate), and bis-active fluorine compounds (such as 1,5-difluoro-2,4-dinitrobenzene). For example, a ricin immunotoxin can be prepared as described in Vitetta et al., Science, 238: 1098 (1987). Carbon- 14-labeled 1-isothiocyanatobenzyl-3-methyldiethylene triaminepentaacetic acid (MX-DTPA) is an exemplary chelating agent for conjugation of radionucleotide to the antibody. See WO94/11026.

Conjugates of an antibody and one or more small molecule toxins, such as a calicheamicin, maytansinoids, dolastatins, aurostatins, a trichothecene, and CC1065, and the derivatives of these toxins that have toxin activity, are also contemplated herein.

### i. Maytansine and maytansinoids

In some embodiments, the immunoconjugate comprises an antibody (full length or fragments) conjugated to one or more maytansinoid molecules.

Maytansinoids are mitototic inhibitors which act by inhibiting tubulin polymerization. Maytansine was first isolated from the east African shrub Maytenus scrrata (U.S. Patent No. 3,896,11 1). Subsequently, it was discovered that certain microbes also produce maytansinoids, such as maytansinol and C-3 maytansinol esters (U.S. Patent No. 4,151,042). Synthetic maytansinol and derivatives and analogues thereof are disclosed, for example, in U.S. Patent Nos. 4,137,230; 4,248,870; 4,256,746; 4,260,608; 4,265,814; 4,294,757; 4,307,016; 4,308,268; 4,308,269; 4,309,428; 4,313,946; 4,315,929; 4,317,821; 4,322,348; 4,331,598; 4,361,650; 4,364,866; 4,424,219; 4,450,254; 4,362,663; and 4,371,533.

Maytansinoid drug moieties are attractive drug moieties in antibody drug conjugates because they are: (i) relatively accessible to prepare by fermentation or chemical modification, derivatization of fermentation products, (ii) amenable to derivatization with functional groups suitable for conjugation through the non-disulfide linkers to antibodies, (iii) stable in plasma, and (iv) effective against a variety of tumor cell lines.

Immunoconjugates containing maytansinoids, methods of making same, and their therapeutic use are disclosed, for example, in U.S. Patent Nos. 5,208,020, 5,416,064 and European Patent EP 0 425 235 B1. Liu et al., Proc. Natl. Acad. Sci. USA 93:8618-8623 (1996) described immunoconjugates comprising a maytansinoid designated DM1 linked to the monoclonal antibody C242 directed against human colorectal cancer. The conjugate was found to be highly cytotoxic towards cultured colon cancer cells, and showed antitumor activity in an in vivo tumor growth assay. Chari et al., Cancer Research 52:127-131 (1992) describe immunoconjugates in which a maytansinoid was conjugated via a disulfide linker to the murine antibody A7 binding to an antigen on human colon cancer cell lines, or to another murine monoclonal antibody TA.1 that binds the HER-2/neu oncogene. The cytotoxicity of the TA.1-maytansinoid conjugate was tested in vitro on the human breast cancer cell line SK-BR-3, which expresses 3 x 10⁵ HER-2 surface antigens per cell. The drug conjugate achieved a degree of cytotoxicity similar to the free maytansinoid drug, which could be increased by increasing the number of maytansinoid molecules per antibody molecule. The A7-maytansinoid conjugate showed low systemic cytotoxicity in mice.

Antibody-maytansinoid conjugates are prepared by chemically linking an antibody to a maytansinoid molecule without significantly diminishing the biological activity of either the antibody or the maytansinoid molecule. See, e.g., U.S. Patent No. 5,208,020. An average of 3-4 maytansinoid molecules conjugated per antibody molecule has shown efficacy in enhancing cytotoxicity of target cells without negatively affecting the function or solubility of the antibody, although even one molecule of toxin/antibody would be expected to enhance cytotoxicity over the use of naked antibody. Maytansinoids arc well known in the art and can be synthesized by known techniques or isolated from natural sources. Suitable maytansinoids arc disclosed, for example, in U.S. Patent No. 5,208,020 and in the other patents and nonpatent publications referred to hereinabove. Preferred maytansinoids are maytansinol and maytansinol analogues modified in the aromatic ring or at other positions of the maytansinol molecule, such as various maytansinol esters.

There are many linking groups known in the art for making antibody-maytansinoid conjugates, including, for example, those disclosed in U.S. Patent No. 5,208,020 or EP Patent 0 425 235 B1, Chari et al., Cancer Research 52:127-131 (1992), and U.S. Patent Application No. 10/960,602, filed Oct. 8, 2004. Antibody-maytansinoid conjugates comprising the linker component SMCC may be prepared as disclosed in U.S. Patent Application No. 10/960,602, filed Oct. 8, 2004. The linking groups include disulfide groups, thioether groups, acid labile groups, photolabile groups, peptidase labile groups, or esterase labile groups, as disclosed in the above-identified patents, disulfide and thioether groups being preferred. Additional linking groups are described and exemplified herein.

Conjugates of the antibody and maytansinoid may be made using a variety of bifunctional protein coupling agents such as N-succinimidyl-3-(2-pyridyldithio) propionate (SPDP), succinimidyl-4-(N-maleimidomethyl) cyclohexane-1-carboxylate (SMCC), iminothiolane (IT), bifunctional derivatives of imidoesters (such as dimethyl adipimidate HCl), active esters (such as disuccinimidyl suberate), aldehydes (such as glutaraldehyde), bis-azido compounds (such as bis (p-azidobenzoyl) hexanediamine), bis-diazonium derivatives (such as bis-(p-diazoniumbenzoyl)-ethylenediamine), diisocyanates (such as toluene 2,6-diisocyanate), and bis-active fluorine compounds (such as 1,5-difluoro-2,4-dinitrobenzene). Particularly preferred coupling agents include N-succinimidyl-3-(2-pyridyldithio) propionate (SPDP) (Carlsson et al., Biochem. J. 173:723-737 (1978)) and N-succinimidyl-4-(2-pyridylthio)pentanoate (SPP) to provide for a disulfide linkage.

The linker may be attached to the maytansinoid molecule at various positions, depending on the type of the link. For example, an ester linkage may be formed by reaction with a hydroxyl group using conventional coupling techniques. The reaction may occur at the C-3 position having a hydroxyl group, the C-14 position modified with hydroxymethyl, the C-15 position modified with a hydroxyl group, and the C-20 position having a hydroxyl group. In a preferred embodiment, the linkage is formed at the C-3 position of maytansinol or a maytansinol analogue.

### ii. Auristatins and dolastatins

In some embodiments, the immunoconjugate comprises an antibody conjugated to dolastatins or dolostatin peptidic analogs and derivatives, the auristatins (US Patent Nos. 5635483; 5780588). Dolastatins and auristatins have been shown to interfere with microtubule dynamics, GTP hydrolysis, and nuclear and cellular division (Woyke et al (2001) Antimicrob. Agents and Chemother. 45(12):3580-3584) and have anticancer (US 5663149) and antifungal activity (Pettit et al (1998) Antimicrob. Agents Chemother. 42:2961-2965). The dolastatin or auristatin drug moiety may be attached to the antibody through the N (amino) terminus or the C (carboxyl) terminus of the peptidic drug moiety (WO 02/088172).

Exemplary auristatin embodiments include the N-terminus linked monomethylauristatin drug moieties DE and DF, disclosed in "Monomethylvaline Compounds Capable of Conjugation to Ligands", US Ser. No. 10/983,340, filed Nov. 5, 2004.

Typically, peptide-based drug moieties can be prepared by forming a peptide bond between two or more amino acids and/or peptide fragments. Such peptide bonds can be prepared, for example, according to the liquid phase synthesis method (see E. Schröder and K. Lübke, "The Peptides", volume 1, pp 76-136, 1965, Academic Press) that is well known in the field of peptide chemistry. The auristatin/dolastatin drug moieties may be prepared according to the methods of: US 5635483; US 5780588; Pettit et al (1989) J. Am. Chem. Soc. 111:5463-5465; Pettit et al (1998) Anti-Cancer Drug Design 13:243-277; Pettit, G.R., et al. Synthesis, 1996, 719-725; and Pettit et al (1996) J. Chem. Soc. Perkin Trans. 1 5:859-863. See also Doronina (2003) Nat Biotechnol 21(7):778-784; "Monomethylvaline Compounds Capable of Conjugation to Ligands", US Ser. No. 10/983,340, filed Nov. 5, 2004, (disclosing, e.g., linkers and methods of preparing monomethylvaline compounds such as MMAE and MMAF conjugated to linkers).

### iii. Calicheamicin

In other embodiments, the immunoconjugate comprises an antibody conjugated to one or more calicheamicin molecules. The calicheamicin family of antibiotics are capable of producing double-stranded DNA breaks at sub-picomolar concentrations. For the preparation of conjugates of the calicheamicin family, see U.S. patents 5,712,374, 5,714,586, 5,739,116, 5,767,285, 5,770,701, 5,770,710, 5,773,001, 5,877,296 (all to American Cyanamid Company). Structural analogues of calicheamicin which may be used include, but are not limited to, γ₁^{I}, α₂^{I}, α₃^{I}, N-acetyl-γ₁^{I}, PSAG and θ^{I}₁ (Hinman et al., Cancer Research 53:3336-3342 (1993), Lode et al., Cancer Research 58:2925-2928 (1998) and the aforementioned U.S. patents to American Cyanamid). Another anti-tumor drug that the antibody can be conjugated is QFA which is an antifolate. Both calicheamicin and QFA have intracellular sites of action and do not readily cross the plasma membrane. Therefore, cellular uptake of these agents through antibody mediated internalization greatly enhances their cytotoxic effects.

### iv. Other cytotoxic agents

Other antitumor agents that can be conjugated to the antibodies include BCNU, streptozoicin, vincristine and 5-fluorouracil, the family of agents known collectively LL-E33288 complex described in U.S. patents 5,053,394, 5,770,710, as well as esperamicins (U.S. patent 5,877,296).

Enzymatically active toxins and fragments thereof which can be used include diphtheria A chain, nonbinding active fragments of diphtheria toxin, exotoxin A chain (from Pseudomonas aeruginosa), ricin A chain, abrin A chain, modeccin A chain, alpha-sarcin, Aleurites fordii proteins, dianthin proteins, Phytolaca americana proteins (PAPI, PAPII, and PAP-S), momordica charantia inhibitor, curcin, crotin, sapaonaria officinalis inhibitor, gelonin, mitogellin, restrictocin, phenomycin, enomycin and the tricothecenes. See, for example, WO 93/21232 published October 28, 1993.

The present invention further contemplates an immunoconjugate formed between an antibody and a compound with nucleolytic activity (e.g., a ribonuclease or a DNA endonuclease such as a deoxyribonuclease; DNase).

For selective destruction of the tumor, the antibody may comprise a highly radioactive atom. A variety of radioactive isotopes are available for the production of radioconjugated antibodies. Examples include At²¹¹, I¹³¹, I¹²⁵, Y⁹⁰, Re¹⁸⁶, Re¹⁸⁸, Sm¹⁵³, Bi²¹², P³², Pb²¹² and radioactive isotopes of Lu. When the conjugate is used for detection, it may comprise a radioactive atom for scintigraphic studies, for example tc^{99m} or I¹²³, or a spin label for nuclear magnetic resonance (NMR) imaging (also known as magnetic resonance imaging, mri), such as iodine-123 again, iodine-131, indium-111, fluorine-19, carbon-13, nitrogen-15, oxygen-17, gadolinium, manganese or iron.

The radio- or other labels may be incorporated in the conjugate in known ways. For example, the peptide may be biosynthesized or may be synthesized by chemical amino acid synthesis using suitable amino acid precursors involving, for example, fluorine-19 in place of hydrogen. Labels such as tc^{99m} or I¹²³, .Re¹⁸⁶, Re¹⁸⁸ and In¹¹¹ can be attached via a cysteine residue in the peptide. Yttrium-90 can be attached via a lysine residue. The IODOGEN method (Fraker et al (1978) Biochem. Biophys. Res. Commun. 80: 49-57 can be used to incorporate iodine-123. "Monoclonal Antibodies in Immunoscintigraphy" (Chatal,CRC Press 1989) describes other methods in detail.

Conjugates of the antibody and cytotoxic agent may be made using a variety of bifunctional protein coupling agents such as N-succinimidyl-3-(2-pyridyldithio) propionate (SPDP), succinimidyl-4-(N-malcimidomethyl) cyclohexane-1-carboxylate (SMCC), iminothiolane (IT), bifunctional derivatives of imidoesters (such as dimethyl adipimidate HCl), active esters (such as disuccinimidyl suberate), aldehydes (such as glutaraldehyde), bis-azido compounds (such as bis (p-azidobenzoyl) hexanediamine), bis-diazonium derivatives (such as bis-(p-diazoniumbenzoyl)-ethylenediamine), diisocyanates (such as toluene 2,6-diisocyanate), and bis-active fluorine compounds (such as 1,5-difluoro-2,4-dinitrobenzene). For example, a ricin immunotoxin can be prepared as described in Vitetta et al., Science 238:1098 (1987). Carbon-14-labeled 1-isothiocyanatobenzyl-3-methyldiethylene triaminepentaacetic acid (MX-DTPA) is an exemplary chelating agent for conjugation of radionucleotide to the antibody. See WO94/11026. The linker may be a "cleavable linker" facilitating release of the cytotoxic drug in the cell. For example, an acid-labile linker, peptidase-sensitive linker, photolabile linker, dimethyl linker or disulfide-containing linker (Chari et al., Cancer Research 52:127-131 (1992); U.S. Patent No. 5,208,020) may be used.

The compounds expressly contemplate, but are not limited to, ADC prepared with cross-linker reagents: BMPS, EMCS, GMBS, HBVS, LC-SMCC, MBS, MPBH, SBAP, SIA, SIAB, SMCC, SMPB, SMPH, sulfo-EMCS, sulfo-GMBS, sulfo-KMUS, sulfo-MBS, sulfo-SIAB, sulfo-SMCC, and sulfo-SMPB, and SVSB (succinimidyl-(4-vinylsulfone)benzoate) which are commercially available (e.g., from Pierce Biotechnology, Inc., Rockford, IL., U.S.A). See pages 467-498, 2003-2004 Applications Handbook and Catalog.

### v. Preparation of antibody drug conjugates

In the antibody drug conjugates (ADC), an antibody (Ab) is conjugated to one or more drug moieties (D), e.g. about 1 to about 20 drug moieties per antibody, through a linker (L). The ADC of Formula I may be prepared by several routes, employing organic chemistry reactions, conditions, and reagents known to those skilled in the art, including: (1) reaction of a nucleophilic group of an antibody with a bivalent linker reagent, to form Ab-L, via a covalent bond, followed by reaction with a drug moiety D; and (2) reaction of a nucleophilic group of a drug moiety with a bivalent linker reagent, to form D-L, via a covalent bond, followed by reaction with the nucleophilic group of an antibody. Additional methods for preparing ADC are described herein.

Ab-(L-D)ₚ I

The linker may be composed of one or more linker components. Exemplary linker components include 6-maleimidocaproyl ("MC"), maleimidopropanoyl ("MP"), valine-citrulline ("val-cit"), alanine-phenylalanine ("ala-phe"), p-aminobenzyloxycarbonyl ("PAB"), N-Succinimidyl 4-(2-pyridylthio) pentanoate ("SPP"), N-Succinimidyl 4-(N-malcimidomethyl) cyclohexane-1 carboxylate ("SMCC'), and N-Succinimidyl (4-iodo-acetyl) aminobenzoate ("SIAB"). Additional linker components are known in the art and some are described herein. See also "Monomethylvaline Compounds Capable of Conjugation to Ligands", US Ser. No. 10/983,340, filed Nov. 5, 2004.

In some embodiments, the linker may comprise amino acid residues. Exemplary amino acid linker components include a dipeptide, a tripeptide, a tetrapeptide or a pentapeptide. Exemplary dipeptides include: valine-citrulline (vc or val-cit), alanine-phenylalanine (af or ala-phe). Exemplary tripeptides include: glycine-valine-citrulline (gly-val-cit) and glycine-glycine-glycine (gly-gly-gly). Amino acid residues which comprise an amino acid linker component include those occurring naturally, as well as minor amino acids and non-naturally occurring amino acid analogs, such as citrulline. Amino acid linker components can be designed and optimized in their selectivity for enzymatic cleavage by a particular enzymes, for example, a tumor-associated protease, cathepsin B, C and D, or a plasmin protease.

Nucleophilic groups on antibodies include, but are not limited to: (i) N-terminal amine groups, (ii) side chain amine groups, e.g. lysine, (iii) side chain thiol groups, e.g. cysteine, and (iv) sugar hydroxyl or amino groups where the antibody is glycosylated. Amine, thiol, and hydroxyl groups are nucleophilic and capable of reacting to form covalent bonds with electrophilic groups on linker moieties and linker reagents including: (i) active esters such as NHS esters, HOBt esters, haloformates, and acid halides; (ii) alkyl and benzyl halides such as haloacctamides; (iii) aldehydes, ketones, carboxyl, and maleimide groups. Certain antibodies have reducible interchain disulfides, i.e. cysteine bridges. Antibodies may be made reactive for conjugation with linker reagents by treatment with a reducing agent such as DTT (dithiothreitol). Each cysteine bridge will thus form, theoretically, two reactive thiol nucleophiles. Additional nucleophilic groups can be introduced into antibodies through the reaction of lysines with 2-iminothiolanc (Traut's reagent) resulting in conversion of an amine into a thiol. Reactive thiol groups may be introduced into the antibody (or fragment thereof) by introducing one, two, three, four, or more cysteine residues (e.g., preparing mutant antibodies comprising one or more non-native cysteine amino acid residues).

Antibody drug conjugates may also be produced by modification of the antibody to introduce electrophilic moieties, which can react with nucleophilic substituents on the linker reagent or drug. The sugars of glycosylated antibodies may be oxidized, e.g. with periodate oxidizing reagents, to form aldehyde or ketone groups which may react with the amine group of linker reagents or drug moieties. The resulting imine Schiff base groups may form a stable linkage, or may be reduced, e.g. by borohydridc reagents to form stable amine linkages. In one embodiment, reaction of the carbohydrate portion of a glycosylated antibody with either glactosc oxidase or sodium meta-periodate may yield carbonyl (aldehyde and ketone) groups in the protein that can react with appropriate groups on the drug (Hermanson, Bioconjugate Techniques). In another embodiment, proteins containing N-terminal serine or threonine residues can react with sodium meta-periodate, resulting in production of an aldehyde in place of the first amino acid (Geoghegan & Stroh, (1992) Bioconjugate Chem. 3:138-146; US 5362852). Such aldehyde can be reacted with a drug moiety or linker nucleophile.

Likewise, nucleophilic groups on a drug moiety include, but are not limited to: amine, thiol, hydroxyl, hydrazide, oxime, hydrazine, thiosemicarbazone, hydrazine carboxylate, and arylhydrazide groups capable of reacting to form covalent bonds with electrophilic groups on linker moieties and linker reagents including: (i) active esters such as NHS esters, HOBt esters, haloformates, and acid halides; (ii) alkyl and benzyl halides such as haloacetamides; (iii) aldehydes, ketones, carboxyl, and maleimide groups.

Alternatively, a fusion protein comprising the antibody and cytotoxic agent may be made, e.g., by recombinant techniques or peptide synthesis. The length of DNA may comprise respective regions encoding the two portions of the conjugate either adjacent one another or separated by a region encoding a linker peptide which does not destroy the desired properties of the conjugate.

In yet another embodiment, the antibody may be conjugated to a "receptor" (such streptavidin) for utilization in tumor pre-targeting wherein the antibody-receptor conjugate is administered to the patient, followed by removal of unbound conjugate from the circulation using a clearing agent and then administration of a "ligand" (e.g., avidin) which is conjugated to a cytotoxic agent (e.g., a radionucleotide).

### Binding oligopeptides

Binding oligopeptides are oligopeptides that bind, preferably specifically, to KLβ, FGFR or KLβ-FGFR complex as described herein. Binding oligopeptides may be chemically synthesized using known oligopeptide synthesis methodology or may be prepared and purified using recombinant technology. Binding oligopeptides are usually at least about 5 amino acids in length, alternatively at least about 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100 amino acids in length or more, wherein such oligopeptides that are capable of binding, preferably specifically, to a polypeptide as described herein. Binding oligopeptides may be identified without undue experimentation using well known techniques. In this regard, it is noted that techniques for screening oligopeptide libraries for oligopeptides that are capable of specifically binding to a polypeptide target are well known in the art (see, e.g., U.S. Patent Nos. 5,556,762, 5,750,373, 4,708,871, 4,833,092, 5,223,409, 5,403,484, 5,571,689, 5,663,143; PCT Publication Nos. WO 84/03506 and WO84/03564; Geysen et al., Proc. Natl. Acad. Sci. U.S.A., 81:3998-4002 (1984); Geysen et al., Proc. Natl. Acad. Sci. U.S.A., 82:178-182 (1985); Geysen et al., in Synthetic Peptides as Antigens, 130-149 (1986); Geysen et al., J. Immunol. Meth., 102:259-274 (1987); Schoofs et al., J. Immunol., 140:611-616 (1988), Cwirla, S. E. et al. (1990) Proc. Natl. Acad. Sci. USA, 87:6378; Lowman, H.B. et al. (1991) Biochemistry, 30:10832; Clackson, T. et al. (1991) Nature, 352: 624; Marks, J. D. et al. (1991), J. Mol. Biol., 222:581; Kang, A.S. et al. (1991) Proc. Natl. Acad. Sci. USA, 88:8363, and Smith, G. P. (1991) Current Opin. Biotechnol., 2:668).

In this regard, bacteriophage (phage) display is one well known technique which allows one to screen large oligopeptide libraries to identify member(s) of those libraries which are capable of specifically binding to a polypeptide target. Phage display is a technique by which variant polypeptides arc displayed as fusion proteins to the coat protein on the surface of bacteriophage particles (Scott, J.K. and Smith, G. P. (1990) Science, 249: 386). The utility of phage display lies in the fact that large libraries of selectively randomized protein variants (or randomly cloned cDNAs) can be rapidly and efficiently sorted for those sequences that bind to a target molecule with high affinity. Display of peptide (Cwirla, S. E. et al. (1990) Proc. Natl. Acad. Sci. USA, 87:6378) or protein (Lowman, H.B. et al. (1991) Biochemistry, 30:10832; Clackson, T. et al. (1991) Nature, 352: 624; Marks, J. D. et al. (1991), J. Mol. Biol., 222:581; Kang, A.S. et al. (1991) Proc. Natl. Acad. Sci. USA, 88:8363) libraries on phage have been used for screening millions of polypeptides or oligopeptides for ones with specific binding properties (Smith, G. P. (1991) Current Opin. Biotechnol., 2:668). Sorting phage libraries of random mutants requires a strategy for constructing and propagating a large number of variants, a procedure for affinity purification using the target receptor, and a means of evaluating the results of binding enrichments. U.S. Patent Nos. 5,223,409, 5,403,484, 5,571,689, and 5,663,143.

Although most phage display methods have used filamentous phage, lambdoid phage display systems (WO 95/34683; U.S. 5,627,024), T4 phage display systems (Ren et al., Gene, 215: 439 (1998); Zhu et al., Cancer Research, 58(15): 3209-3214 (1998); Jiang et al., Infection & Immunity, 65(11): 4770-4777 (1997); Ren et al., Gene, 195(2):303-311 (1997); Ren, Protein Sci., 5: 1833 (1996); Efimov et al., Virus Genes, 10: 173 (1995)) and T7 phage display systems (Smith and Scott, Methods in Enzymology, 217: 228-257 (1993); U.S. 5,766,905) are also known.

Many other improvements and variations of the basic phage display concept have now been developed. These improvements enhance the ability of display systems to screen peptide libraries for binding to selected target molecules and to display functional proteins with the potential of screening these proteins for desired properties. Combinatorial reaction devices for phage display reactions have been developed (WO 98/14277) and phage display libraries have been used to analyze and control bimolecular interactions (WO 98/20169; WO 98/20159) and properties of constrained helical peptides (WO 98/20036). WO 97/35196 describes a method of isolating an affinity ligand in which a phage display library is contacted with one solution in which the ligand will bind to a target molecule and a second solution in which the affinity ligand will not bind to the target molecule, to selectively isolate binding ligands. WO 97/46251 describes a method of biopanning a random phage display library with an affinity purified antibody and then isolating binding phage, followed by a micropanning process using microplate wells to isolate high affinity binding phage. The use of *Staphylococcus aureus* protein A as an affinity tag has also been reported (Li et al. (1998) Mol Biotech., 9:187). WO 97/47314 describes the use of substrate subtraction libraries to distinguish enzyme specificities using a combinatorial library which may be a phage display library. A method for selecting enzymes suitable for use in detergents using phage display is described in WO 97/09446. Additional methods of selecting specific binding proteins are described in U.S. Patent Nos. 5,498,538, 5,432,018, and WO 98/15833.

Methods of generating peptide libraries and screening these libraries are also disclosed in U.S. Patent Nos. 5,723,286, 5,432,018, 5,580,717, 5,427,908, 5,498,530, 5,770,434, 5,734,018, 5,698,426, 5,763,192, and 5,723,323.

### Binding small molecules

Binding small molecules are preferably organic molecules other than oligopeptides or antibodies as defined herein that bind, preferably specifically, to KLβ, FGFR or KLβ /FGFR complex as described herein. Binding organic small molecules may be identified and chemically synthesized using known methodology (see, e.g., PCT Publication Nos. WO00/00823 and WO00/39585). Binding organic small molecules are usually less than about 2000 daltons in size, alternatively less than about 1500, 750, 500, 250 or 200 daltons in size, wherein such organic small molecules that are capable of binding, preferably specifically, to a polypeptide as described herein may be identified without undue experimentation using well known techniques. In this regard, it is noted that techniques for screening organic small molecule libraries for molecules that are capable of binding to a polypeptide target are well known in the art (see, e.g., PCT Publication Nos. WO00/00823 and WO00/39585). Binding organic small molecules may be, for example, aldehydes, ketones, oximes, hydrazones, semicarbazones, carbazides, primary amines, secondary amines, tertiary amines, N-substituted hydrazines, hydrazides, alcohols, ethers, thiols, thioethers, disulfides, carboxylic acids, esters, amides, ureas, carbamates, carbonates, ketals, thioketals, acetals, thioacetals, aryl halides, aryl sulfonates, alkyl halides, alkyl sulfonates, aromatic compounds, heterocyclic compounds, anilines, alkenes, alkynes, diols, amino alcohols, oxazolidines, oxazolines, thiazolidines, thiazolines, enamines, sulfonamides, epoxides, aziridines, isocyanates, sulfonyl chlorides, diazo compounds, acid chlorides, or the like.

### Screening for antibodies, oligopeptides and organic small molecules with desired properties

In some embodiments, the antagonists bind KLβ, and in some embodiments, may modulate one or more aspects of KLβ-associated effects, including but not limited to may modulate one or more aspects of KLβ-associated effects, including but not limited to binding FGFR4 (optionally in conjunction with heparin), binding FGF19 (optionally in conjunction with heparin), binding FGFR4 and FGF19 (optionally in conjunction with heparin), promoting FGF 19-mediatcd induction of cFos, Junb and/or Junc (in vitro or in vivo), promoting FGFR4 and/or FGF19 down stream signaling (including but not limited to FRS2 phosphorylation, ERK1/2 phosphorylation and Wnt pathway activation), and/or promotion of any biologically relevant KLβ and/or FGFR4 biological pathway, and/or promotion of a tumor, cell proliferative disorder or a cancer; and/or promotion of a disorder associated with KLβ expression and/or activity (such as increased KLβ expression and/or activity).

The purified antibodies can be further characterized by a series of assays including, but not limited to, N-terminal sequencing, amino acid analysis, non-denaturing size exclusion high pressure liquid chromatography (HPLC), mass spectrometry, ion exchange chromatography and papain digestion.

In certain embodiments of the invention, the antibodies produced herein are analyzed for their biological activity. In some embodiments, the antibodies of the present invention are tested for their antigen binding activity. The antigen binding assays that are known in the art and can be used herein include without limitation any direct or competitive binding assays using techniques such as western blots, radioimmunoassays, ELISA (enzyme linked immunosorbent assay), "sandwich" immunoassays, immunoprecipitation assays, fluorescent immunoassays, and protein A immunoassays. Illustrative antigen binding assay are provided below in the Examples section.

Anti-KLp antibodies possessing the properties described herein can be obtained by screening anti-KLp hybridoma clones for the desired properties by any convenient method.

Other functional assays to determine the binding capacity of anti-KLp antibodies are known in the art, some of which are exemplified herein.

Anti-FGFR4 antibodies possessing the properties described herein can be obtained by screening anti-FGFR4 hybridoma clones for the desired properties by any convenient method.

Other functional assays to determine the binding capacity of anti-FGFR4 antibodies are known in the art, some of which are exemplified herein.

To screen for antibodies, oligopeptides or other organic small molecules which bind to an epitope on a polypeptide bound by an antibody of interest, a routine cross-blocking assay such as that described in Antibodies, A Laboratory Manual, Cold Spring Harbor Laboratory, Ed Harlow and David Lane (1988), can be performed. This assay can be used to determine if a test antibody, oligopeptide or other organic small molecule binds the same site or epitope as a known antibody. Alternatively, or additionally, epitope mapping can be performed by methods known in the art. For example, the antibody sequence can be mutagenized such as by alanine scanning, to identify contact residues. The mutant antibody is initially tested for binding with polyclonal antibody to ensure proper folding. In a different method, peptides corresponding to different regions of a polypeptide can be used in competition assays with the test antibodies or with a test antibody and an antibody with a characterized or known epitope.

In some embodiments, the present invention contemplates altered antibodies that possess some but not all effector functions, which make it a desired candidate for many applications in which the half life of the antibody in vivo is important yet certain effector functions (such as complement and ADCC) arc unnecessary or deleterious. In certain embodiments, the Fc activities of the produced immunoglobulin are measured to ensure that only the desired properties are maintained. In vitro and/or in vivo cytotoxicity assays can be conducted to confirm the reduction/depletion of CDC and/or ADCC activities. For example, Fc receptor (FcR) binding assays can be conducted to ensure that the antibody lacks FcγR binding (hence likely lacking ADCC activity), but retains FcRn binding ability. The primary cells for mediating ADCC, NK cells, express FcγRIII only, whereas monocytes express FcγRI, FcγRII and FcγRIII. FcR expression on hematopoietic cells is summarized in Table 3 on page 464 of Ravetch and Kinet, Annu. Rev. Immunol 9:457-92 (1991). An example of an in vitro assay to assess ADCC activity of a molecule of interest is described in US Patent No. 5,500,362 or 5,821,337. Useful effector cells for such assays include peripheral blood mononuclear cells (PBMC) and Natural Killer (NK) cells. Alternatively, or additionally, ADCC activity of the molecule of interest may be assessed in vivo, e.g., in a animal model such as that disclosed in Clynes et al. PNAS (USA) 95:652-656 (1998). C1q binding assays may also be carried out to confirm that the antibody is unable to bind C1q and hence lacks CDC activity. To assess complement activation, a CDC assay, e.g. as described in Gazzano-Santoro et al., J. Immunol. Methods 202:163 (1996), may be performed. FcRn binding and in vivo clearance/half life determinations can also be performed using methods known in the art.

In some embodiments, altered antibodies that possess increased effector functions and/or increased half-life are useful.

### Polypeptides and Nucleic Acids

Nucleotide sequences have various applications in the art of molecular biology, as well as uses for therapy, etc. Polypeptide-encoding nucleic acid will also be useful for the preparation of polypeptides by the recombinant techniques described herein, wherein those polypeptides may find use, for example, in the preparation of antibodies as described herein.

A full-length native sequence polypeptide gene, or portions thereof, may be used as hybridization probes for a cDNA library to isolate other cDNAs (for instance, those encoding naturally-occurring variants of a polypeptide or a polypeptide from other species) which have a desired sequence identity to a native polypeptide sequence disclosed herein. Optionally, the length of the probes will be about 20 to about 50 bases. The hybridization probes may be derived from at least partially novel regions of the full length native nucleotide sequence wherein those regions may be determined without undue experimentation or from genomic sequences including promoters, enhancer elements and introns of native sequence polypeptide. By way of example, a screening method will comprise isolating the coding region of the polypeptide gene using the known DNA sequence to synthesize a selected probe of about 40 bases. Hybridization probes may be labeled by a variety of labels, including radionucleotides such as ³²P or ³⁵S, or enzymatic labels such as alkaline phosphatase coupled to the probe via avidin/biotin coupling systems. Labeled probes having a sequence complementary to that of the polypeptide gene of the present invention can be used to screen libraries of human cDNA, genomic DNA or mRNA to determine which members of such libraries the probe hybridizes to. Hybridization techniques are described in further detail in the Examples below. Any EST sequences disclosed in the present application may similarly be employed as probes, using the methods disclosed herein.

Other useful fragments of the polypeptide-encoding nucleic acids include antisense or sense oligonucleotides comprising a singe-stranded nucleic acid sequence (either RNA or DNA) capable of binding to target a polypeptide mRNA (sense) or a polypeptide DNA (antisense) sequence. Antisense or sense oligonucleotides, according to the present invention, comprise a fragment of the coding region of a DNA encoding hepsin, pro-HGF or binding fragments as described herein. Such a fragment generally comprises at least about 14 nucleotides, preferably from about 14 to 30 nucleotides. The ability to derive an antisense or a sense oligonucleotide, based upon a cDNA sequence encoding a given protein is described in, for example, Stein and Cohen (Cancer Res. 48:2659, 1988) and van der Krol et al. (BioTechniques 6:958, 1988).

Binding of antisense or sense oligonucleotides to target nucleic acid sequences results in the formation of duplexes that block transcription or translation of the target sequence by one of several means, including enhanced degradation of the duplexes, premature termination of transcription or translation, or by other means. Such methods are encompassed by the present invention. The antisense oligonucleotides thus may be used to block expression of a protein, wherein the protein may play a role in the induction of cancer in mammals. Antisense or sense oligonucleotides further comprise oligonucleotides having modified sugar-phosphodiester backbones (or other sugar linkages, such as those described in WO 91/06629) and wherein such sugar linkages are resistant to endogenous nucleases. Such oligonucleotides with resistant sugar linkages are stable *in vivo* (i.e., capable of resisting enzymatic degradation) but retain sequence specificity to be able to bind to target nucleotide sequences.

Preferred intragenic sites for antisense binding include the region incorporating the translation initiation/start codon (5'-AUG / 5'-ATG) or termination/stop codon (5'-UAA, 5'-UAG and 5-UGA / 5'-TAA, 5'-TAG and 5'-TGA) of the open reading frame (ORF) of the gene. These regions refer to a portion of the mRNA or gene that encompasses from about 25 to about 50 contiguous nucleotides in either direction (i.e., 5' or 3') from a translation initiation or termination codon. Other preferred regions for antisense binding include: introns; exons; intron-exon junctions; the open reading frame (ORF) or "coding region," which is the region between the translation initiation codon and the translation termination codon; the 5' cap of an mRNA which comprises an N7-methylated guanosine residue joined to the 5'-most residue of the mRNA via a 5'-5' triphosphate linkage and includes 5' cap structure itself as well as the first 50 nucleotides adjacent to the cap; the 5' untranslated region (5'UTR), the portion of an mRNA in the 5' direction from the translation initiation codon, and thus including nucleotides between the 5' cap site and the translation initiation codon of an mRNA or corresponding nucleotides on the gene; and the 3' untranslated region (3'UTR), the portion of an mRNA in the 3' direction from the translation termination codon, and thus including nucleotides between the translation termination codon and 3' end of an mRNA or corresponding nucleotides on the gene.

Specific examples of preferred antisense compounds useful for inhibiting expression of a polypeptide include oligonucleotides containing modified backbones or non-natural internucleoside linkages. Oligonucleotides having modified backbones include those that retain a phosphorus atom in the backbone and those that do not have a phosphorus atom in the backbone. For the purposes of this specification, and as sometimes referenced in the art, modified oligonucleotides that do not have a phosphorus atom in their internucleoside backbone can also be considered to be oligonucleosides. Preferred modified oligonucleotide backbones include, for example, phosphorothioates, chiral phosphorothioates, phosphorodithioates, phosphotriesters, aminoalkylphosphotri-esters, methyl and other alkyl phosphonates including 3'-alkylene phosphonates, 5'-alkylenc phosphonates and chiral phosphonates, phosphinates, phosphoramidates including 3'-amino phosphoramidate and aminoalkylphosphoramidates, thionophosphoramidates, thionoalkylphosphonates, thionoalkylphosphotriesters, selenophosphates and borano-phosphates having normal 3'-5' linkages, 2'-5' linked analogs of these, and those having inverted polarity wherein one or more internucleotide linkages is a 3' to 3', 5' to 5' or 2' to 2' linkage. Preferred oligonucleotides having inverted polarity comprise a single 3' to 3' linkage at the 3'-most internucleotide linkage i.e. a single inverted nucleoside residue which may be abasic (the nucleobase is missing or has a hydroxyl group in place thereof). Various salts, mixed salts and free acid forms are also included. Representative United States patents that teach the preparation of phosphorus-containing linkages include, but are not limited to, U.S. Pat. Nos.: 3,687,808; 4,469,863; 4,476,301; 5,023,243; 5,177,196; 5,188,897; 5,264,423; 5,276,019; 5,278,302; 5,286,717; 5,321,131; 5,399,676; 5,405,939; 5,453,496; 5,455,233; 5,466,677; 5,476,925; 5,519,126; 5,536,821; 5,541,306; 5,550,111; 5,563,253; 5,571,799; 5,587,361; 5,194,599; 5,565,555; 5,527,899; 5,721,218; 5,672,697 and 5,625,050.

Preferred modified oligonucleotide backbones that do not include a phosphorus atom therein have backbones that are formed by short chain alkyl or cycloalkyl internucleoside linkages, mixed heteroatom and alkyl or cycloalkyl internucleoside linkages, or one or more short chain heteroatomic or heterocyclic internucleoside linkages. These include those having morpholino linkages (formed in part from the sugar portion of a nucleoside); siloxane backbones; sulfide, sulfoxide and sulfone backbones; formacetyl and thioformacetyl backbones; methylene formacetyl and thioformacetyl backbones; riboacetyl backbones; alkene containing backbones; sulfamate backbones; methyleneimino and methylenehydrazino backbones; sulfonate and sulfonamide backbones; amide backbones; and others having mixed N, O, S and CH.sub.2 component parts. Representative United States patents that teach the preparation of such oligonucleosides include, but are not limited to,. U.S. Pat. Nos.: 5,034,506; 5,166,315; 5,185,444; 5,214,134; 5,216,141; 5,235,033; 5,264,562; 5,264,564; 5,405,938; 5,434,257; 5,466,677; 5,470,967; 5,489,677; 5,541,307; 5,561,225; 5,596,086; 5,602,240; 5,610,289; 5,602,240; 5,608,046; 5,610,289; 5,618,704; 5,623,070; 5,663,312; 5,633,360; 5,677,437; 5,792,608; 5,646,269 and 5,677,439.

In other preferred antisense oligonucleotides, both the sugar and the internucleoside linkage, i.e., the backbone, of the nucleotide units are replaced with novel groups. The base units are maintained for hybridization with an appropriate nucleic acid target compound. One such oligomeric compound, an oligonucleotide mimetic that has been shown to have excellent hybridization properties, is referred to as a peptide nucleic acid (PNA). In PNA compounds, the sugar-backbone of an oligonucleotide is replaced with an amide containing backbone, in particular an aminoethylglycine backbone. The nucleobases are retained and are bound directly or indirectly to aza nitrogen atoms of the amide portion of the backbone. Representative United States patents that teach the preparation of PNA compounds include, but are not limited to, U.S. Pat. Nos.: 5,539,082; 5,714,331; and 5,719,262. Further teaching of PNA compounds can be found in Nielsen et al., Science, 1991, 254, 1497-1500.

Preferred antisense oligonucleotides incorporate phosphorothioate backbones and/or heteroatom backbones, and in particular -CH₂-NH-O-CH₂-, -CH₂-N(CH₃)-O-CH₂- [known as a methylene (methylimino) or MMI backbone], -CH₂-O-N(CH₃)-CH₂-, -CH₂-N(CH₃)-N(CH₃)-CH₂- and -O-N(CH₃)-CH₂-CH₂- [wherein the native phosphodiester backbone is represented as -O-P-O-CH₂-] described in the above referenced U.S. Pat. No. 5,489,677, and the amide backbones of the above referenced U.S. Pat. No. 5,602,240. Also preferred are antisense oligonucleotides having morpholino backbone structures of the above-referenced U.S. Pat. No. 5,034,506.

Modified oligonucleotides may also contain one or more substituted sugar moieties. Preferred oligonucleotides comprise one of the following at the 2' position: OH; F; O-alkyl, S-alkyl, or N-alkyl; O-alkenyl, S-alkeynyl, or N-alkenyl; O-alkynyl, S-alkynyl or N-alkynyl; or O-alkyl-O-alkyl, wherein the alkyl, alkenyl and alkynyl may be substituted or unsubstituted C₁ to C₁₀ alkyl or C₂ to C₁₀ alkenyl and alkynyl. Particularly preferred are O[(CH₂)ₙO]ₘCH₃, O(CH₂)ₙOCH₃, O(CH₂)ₙNH₂, O(CH₂)ₙCH₃, O(CH₂)ₙONH₂, and O(CH₂)ₙON[(CH₂)ₙCH₃)₂, where n and m are from 1 to about 10. Other preferred antisense oligonucleotides comprise one of the following at the 2' position: C₁ to C₁₀ lower alkyl, substituted lower alkyl, alkenyl, alkynyl, alkaryl, aralkyl, O-alkaryl or O-aralkyl, SH, SCH₃, OCN, Cl, Br, CN, CF₃, OCF₃, SOCH₃, SO₂ CH₃, ONO₂, NO₂, N₃, NH₂, heterocycloalkyl, heterocycloalkaryl, aminoalkylamino, polyalkylamino, substituted silyl, an RNA cleaving group, a reporter group, an intercalator, a group for improving the pharmacokinetic properties of an oligonucleotide, or a group for improving the pharmacodynamic properties of an oligonucleotide, and other substituents having similar properties. A preferred modification includes 2'-methoxyethoxy (2'-O-CH₂CH₂OCH₃, also known as 2'-O-(2-methoxyethyl) or 2'-MOE) (Martin et al., Helv. Chim. Acta, 1995, 78, 486-504) i.e., an alkoxyalkoxy group. A further preferred modification includes 2'-dimethylaminooxyethoxy, i.e., a O(CH₂)₂ON(CH₃)₂ group, also known as 2'-DMAOE, as described in examples hereinbelow, and 2'-dimethylaminoethoxyethoxy (also known in the art as 2'-O-dimethylaminoethoxyethyl or 2'-DMAEOE), i.e., 2'-O-CH₂-O-CH₂-N(CH₂).

A further prefered modification includes Locked Nucleic Acids (LNAs) in which the 2'-hydroxyl group is linked to the 3' or 4' carbon atom of the sugar ring thereby forming a bicyclic sugar moiety. The linkage is preferably a methelyne (-CH₂-)ₙ group bridging the 2' oxygen atom and the 4' carbon atom wherein n is 1 or 2. LNAs and preparation thereof are described in WO 98/39352 and WO 99/14226.

Other preferred modifications include 2'-methoxy (2'-O-CH₃), 2'-aminopropoxy (2'-OCH₂CH₂CH₂ NH₂), 2'-allyl (2'-CH₂-CH=CH₂), 2'-O-allyl (2'-O-CH₂-CH=CH₂) and 2'-fluoro (2'-F). The 2'-modification may be in the arabino (up) position or ribo (down) position. A preferred 2'-arabino modification is 2'-F. Similar modifications may also be made at other positions on the oligonucleotide, particularly the 3' position of the sugar on the 3' terminal nucleotide or in 2'-5' linked oligonucleotides and the 5' position of 5' terminal nucleotide. Oligonucleotides may also have sugar mimetics such as cyclobutyl moieties in place of the pentofuranosyl sugar. Representative United States patents that teach the preparation of such modified sugar structures include, but are not limited to, U.S. Pat. Nos.: 4,981,957; 5,118,800; 5,319,080; 5,359,044; 5,393,878; 5,446,137; 5,466,786; 5,514,785; 5,519,134; 5,567,811; 5,576,427; 5,591,722; 5,597,909; 5,610,300; 5,627,053; 5,639,873; 5,646,265; 5,658,873; 5,670,633; 5,792,747; and 5,700,920.

Oligonucleotides may also include nucleobase (often referred to in the art simply as "base") modifications or substitutions. As used herein, "unmodified" or "natural" nucleobases include the purine bases adenine (A) and guanine (G), and the pyrimidine bases thymine (T), cytosine (C) and uracil (U). Modified nucleobases include other synthetic and natural nucleobases such as 5-methylcytosine (5-me-C), 5-hydroxymethyl cytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine, 5-halouracil and cytosine, 5-propynyl (-C=C-CH3 or -CH₂-C=CH) uracil and cytosine and other alkynyl derivatives of pyrimidine bases, 6-azo uracil, cytosine and thymine, 5-uracil (pseudouracil), 4-thiouracil, 8-halo, 8-amino, 8-thiol, 8-thioalkyl, 8-hydroxyl and other 8-substituted adenines and guanines, 5-halo particularly 5-bromo, 5-trifluoromethyl and other 5-substituted uracils and cytosines, 7-methylguanine and 7-methyladenine, 2-F-adenine, 2-amino-adenine, 8-azaguanine and 8-azaadeninc, 7-deazaguanine and 7-deazaadenine and 3-deazaguanine and 3-deazaadenine. Further modified nucleobases include tricyclic pyrimidines such as phenoxazine cytidine(1H-pyrimido[5,4-b][1,4]benzoxazin-2(3H)-one), phenothiazine cytidine (1H-pyrimido[5,4-b][1,4]benzothiazin-2(3H)-one), G-clamps such as a substituted phenoxazine cytidine (e.g. 9-(2-aminoethoxy)-H-pyrimido[5,4-b][1,4]benzoxazin-2(3H)-one), carbazole cytidine (2H-pyrimido[4,5-b]indol-2-one), pyridoindole cytidine (H-pyrido[3',2':4,5]pyrrolo[2,3-d]pyrimidin-2-one). Modified nucleobases may also include those in which the purine or pyrimidine base is replaced with other heterocycles, for example 7-deaza-adenine, 7-deazaguanosine, 2-aminopyridine and 2-pyridone. Further nucleobases include those disclosed in U.S. Pat. No. 3,687,808, those disclosed in The Concise Encyclopedia Of Polymer Science And Engineering, pages 858-859, Kroschwitz, J. I., ed. John Wiley & Sons, 1990, and those disclosed by Englisch et al., Angewandte Chemie, International Edition, 1991, 30, 613. Certain of these nucleobases are particularly useful for increasing the binding affinity of the oligomeric compounds of the invention. These include 5-substituted pyrimidines, 6-azapyrimidines and N-2, N-6 and O-6 substituted purines, including 2-aminopropyladenine, 5-propynyluracil and 5-propynylcytosine. 5-methylcytosine substitutions have been shown to increase nucleic acid duplex stability by 0.6-1.2.degree. C. (Sanghvi et al, Antisense Research and Applications, CRC Press, Boca Raton, 1993, pp. 276-278) and are preferred base substitutions, even more particularly when combined with 2'-O-methoxyethyl sugar modifications. Representative United States patents that teach the preparation of modified nucleobases include, but are not limited to: U.S. Pat. No. 3,687,808, as well as U.S. Pat. Nos.: 4,845,205; 5,130,302; 5,134,066; 5,175,273; 5,367,066; 5,432,272; 5,457,187; 5,459,255; 5,484,908; 5,502,177; 5,525,711; 5,552,540; 5,587,469; 5,594,121, 5,596,091; 5,614,617; 5,645,985; 5,830,653; 5,763,588; 6,005,096; 5,681,941 and 5,750,692.

Another modification of antisense oligonucleotides chemically linking to the oligonucleotide one or more moieties or conjugates which enhance the activity, cellular distribution or cellular uptake of the oligonucleotide. The compounds of the invention can include conjugate groups covalently bound to functional groups such as primary or secondary hydroxyl groups. Conjugate groups of the invention include intercalators, reporter molecules, polyamines, polyamides, polyethylene glycols, polyethers, groups that enhance the pharmacodynamic properties of oligomers, and groups that enhance the pharmacokinetic properties of oligomers. Typical conjugates groups include cholesterols, lipids, cation lipids, phospholipids, cationic phospholipids, biotin, phenazine, folate, phenanthridine, anthraquinonc, acridine, fluoresceins, rhodamines, coumarins, and dyes. Groups that enhance the pharmacodynamic properties, in the context of this invention, include groups that improve oligomer uptake, enhance oligomer resistance to degradation, and/or strengthen sequence-specific hybridization with RNA. Groups that enhance the pharmacokinetic properties, in the context of this invention, include groups that improve oligomer uptake, distribution, metabolism or excretion. Conjugate moieties include but are not limited to lipid moieties such as a cholesterol moiety (Letsinger et al., Proc. Natl. Acad. Sci. USA, 1989, 86, 6553-6556), cholic acid (Manoharan et al., Bioorg. Med. Chem. Let., 1994, 4, 1053-1060), a thioether, e.g., hexyl-S-tritylthiol (Manoharan et al., Ann. N.Y. Acad. Sci., 1992, 660, 306-309; Manoharan et al., Bioorg. Med. Chem. Let., 1993, 3, 2765-2770), a thiocholesterol (Oberhauser et al., Nucl. Acids Res., 1992, 20, 533-538), an aliphatic chain, e.g., dodecandiol or undecyl residues (Saison-Behmoaras et al., EMBO J., 1991, 10, 1111-1118; Kabanov et al., FEBS Lett., 1990, 259, 327-330; Svinarchuk et al., Biochimie, 1993, 75, 49-54), a phospholipid, e.g., di-hexadecyl-rac-glycerol or triethyl-ammonium 1,2-di-O-hexadecyl-rac-glycero-3-H-phosphonate (Manoharan et al., Tetrahedron Lett., 1995, 36, 3651-3654; Shea et al., Nucl. Acids Res., 1990, 18, 3777-3783), a polyamine or a polyethylene glycol chain (Manoharan et al., Nucleosides & Nucleotides, 1995, 14, 969-973), or adamantane acetic acid (Manoharan et al., Tetrahedron Lett., 1995, 36, 3651-3654), a palmityl moiety (Mishra et al., Biochim. Biophys. Acta, 1995, 1264, 229-237), or an octadecylamine or hexylamino-carbonyl-oxycholesterol moiety. Oligonucleotides of the invention may also be conjugated to active drug substances, for example, aspirin, warfarin, phenylbutazone, ibuprofen, suprofen, fenbufen, ketoprofen, (S)-(+)-pranoprofen, carprofen, dansylsarcosine, 2,3,5-triiodobcnzoic acid, flufenamic acid, folinic acid, a benzothiadiazide, chlorothiazide, a diazepine, indomethicin, a barbiturate, a cephalosporin, a sulfa drug, an antidiabetic, an antibacterial or an antibiotic. Oligonucleotide-drug conjugates and their preparation are described in U.S. patent application Ser. No. 09/334,130 (filed Jun. 15, 1999) and United States patents Nos.: 4,828,979; 4,948,882; 5,218,105; 5,525,465; 5,541,313; 5,545,730; 5,552,538; 5,578,717, 5,580,731; 5,580,731; 5,591,584; 5,109,124; 5,118,802; 5,138,045; 5,414,077; 5,486,603; 5,512,439; 5,578,718; 5,608,046; 4,587,044; 4,605,735; 4,667,025; 4,762,779; 4,789,737; 4,824,941; 4,835,263; 4,876,335; 4,904,582; 4,958,013; 5,082,830; 5,112,963; 5,214,136; 5,082,830; 5,112,963; 5,214,136; 5,245,022; 5,254,469; 5,258,506; 5,262,536; 5,272,250; 5,292,873; 5,317,098; 5,371,241, 5,391,723; 5,416,203, 5,451,463; 5,510,475; 5,512,667; 5,514,785; 5,565,552; 5,567,810; 5,574,142; 5,585,481; 5,587,371; 5,595,726; 5,597,696; 5,599,923; 5,599,928 and 5,688,941.

It is not necessary for all positions in a given compound to be uniformly modified, and in fact more than one of the aforementioned modifications may be incorporated in a single compound or even at a single nucleoside within an oligonucleotide. The present invention also includes antisense compounds which arc chimeric compounds. "Chimeric" antisense compounds or "chimeras," in the context of this invention, are antisense compounds, particularly oligonucleotides, which contain two or more chemically distinct regions, each made up of at least one monomer unit, i.e., a nucleotide in the case of an oligonucleotide compound. These oligonucleotides typically contain at least one region wherein the oligonucleotide is modified so as to confer upon the oligonucleotide increased resistance to nuclease degradation, increased cellular uptake, and/or increased binding affinity for the target nucleic acid. An additional region of the oligonucleotide may serve as a substrate for enzymes capable of cleaving RNA:DNA or RNA:RNA hybrids. By way of example, RNase H is a cellular endonuclease which cleaves the RNA strand of an RNA:DNA duplex. Activation of RNase H, therefore, results in cleavage of the RNA target, thereby greatly enhancing the efficiency of oligonucleotide inhibition of gene expression. Consequently, comparable results can often be obtained with shorter oligonucleotides when chimeric oligonucleotides are used, compared to phosphorothioate deoxyoligonucleotides hybridizing to the same target region. Chimeric antisense compounds of the invention may be formed as composite structures of two or more oligonucleotides, modified oligonucleotides, oligonucleosides and/or oligonucleotide mimetics as described above. Preferred chimeric antisense oligonucleotides incorporate at least one 2' modified sugar (preferably 2'-O-(CH₂)₂-O-CH₃) at the 3' terminal to confer nuclease resistance and a region with at least 4 contiguous 2'-H sugars to confer RNase H activity. Such compounds have also been referred to in the art as hybrids or gapmers. Preferred gapmers have a region of 2' modified sugars (preferably 2'-O-(CH₂)₂-O-CH₃) at the 3'-terminal and at the 5' terminal separated by at least one region having at least 4 contiguous 2'-H sugars and preferably incorporate phosphorothioate backbone linkages. Representative United States patents that teach the preparation of such hybrid structures include, but are not limited to, U.S. Pat. Nos. 5,013,830; 5,149,797; 5,220,007; 5,256,775; 5,366,878; 5,403,711; 5,491,133; 5,565,350; 5,623,065; 5,652,355; 5,652,356; and 5,700,922.

The antisense compounds used in accordance with this disclosure may be conveniently and routinely made through the well-known technique of solid phase synthesis. Equipment for such synthesis is sold by several vendors including, for example, Applied Biosystems (Foster City, Calif.). Any other means for such synthesis known in the art may additionally or alternatively be employed. It is well known to use similar techniques to prepare oligonucleotides such as the phosphorothioates and alkylated derivatives. The compounds of the invention may also be admixed, encapsulated, conjugated or otherwise associated with other molecules, molecule structures or mixtures of compounds, as for example, liposomes, receptor targeted molecules, oral, rectal, topical or other formulations, for assisting in uptake, distribution and/or absorption. Representative United States patents that teach the preparation of such uptake, distribution and/or absorption assisting formulations include, but arc not limited to, U.S. Pat. Nos. 5,108,921; 5,354,844; 5,416,016; 5,459,127; 5,521,291; 5,543,158; 5,547,932; 5,583,020; 5,591,721; 4,426,330; 4,534,899; 5,013,556; 5,108,921; 5,213,804; 5,227,170; 5,264,221; 5,356,633; 5,395,619; 5,416,016; 5,417,978; 5,462,854; 5,469,854; 5,512,295; 5,527,528; 5,534,259; 5,543,152; 5,556,948; 5,580,575; and 5,595,756.

Other examples of sense or antisense oligonucleotides include those oligonucleotides which are covalently linked to organic moieties, such as those described in WO 90/10048, and other moieties that increases affinity of the oligonucleotide for a target nucleic acid sequence, such as poly-(L-lysine). Further still, intercalating agents, such as ellipticine, and alkylating agents or metal complexes may be attached to sense or antisense oligonucleotides to modify binding specificities of the antisense or sense oligonucleotide for the target nucleotide sequence.

Antisense or sense oligonucleotides may be introduced into a cell containing the target nucleic acid sequence by any gene transfer method, including, for example, CaPO₄-mediated DNA transfection, electroporation, or by using gene transfer vectors such as Epstcin-Barr virus. In a preferred procedure, an antisense or sense oligonucleotide is inserted into a suitable retroviral vector. A cell containing the target nucleic acid sequence is contacted with the recombinant retroviral vector, either *in vivo* or *ex vivo.* Suitable retroviral vectors include, but are not limited to, those derived from the murine retrovirus M-MuLV, N2 (a retrovirus derived from M-MuLV), or the double copy vectors designated DCT5A, DCT5B and DCT5C (see WO 90/13641).

Sense or antisense oligonucleotides also may be introduced into a cell containing the target nucleotide sequence by formation of a conjugate with a ligand binding molecule, as described in WO 91/04753. Suitable ligand binding molecules include, but are not limited to, cell surface receptors, growth factors, other cytokines, or other ligands that bind to cell surface receptors. Preferably, conjugation of the ligand binding molecule does not substantially interfere with the ability of the ligand binding molecule to bind to its corresponding molecule or receptor, or block entry of the sense or antisense oligonucleotide or its conjugated version into the cell.

Alternatively, a sense or an antisense oligonucleotide may be introduced into a cell containing the target nucleic acid sequence by formation of an oligonucleotide-lipid complex, as described in WO 90/10448. The sense or antisense oligonucleotide-lipid complex is preferably dissociated within the cell by an endogenous lipase.

Antisense or sense RNA or DNA molecules are generally at least about 5 nucleotides in length, alternatively at least about 6, 7, 8, 9, 10, 11, 12, 13, 14, 15, 16, 17, 18, 19, 20, 21, 22, 23, 24, 25, 26, 27, 28, 29, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 195, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990, or 1000 nucleotides in length, wherein in this context the term "about" means the referenced nucleotide sequence length plus or minus 10% of that referenced length.

The probes may also be employed in PCR techniques to generate a pool of sequences for identification of closely related polypeptide coding sequences.

Nucleotide sequences encoding a polypeptide can also be used to construct hybridization probes for mapping the gene which encodes that polypeptide and for the genetic analysis of individuals with genetic disorders. The nucleotide sequences provided herein may be mapped to a chromosome and specific regions of a chromosome using known techniques, such as *in situ* hybridization, linkage analysis against known chromosomal markers, and hybridization screening with libraries.

The polypeptide can be used in assays to identify other proteins or molecules involved in a binding interaction with the polypeptide. By such methods, inhibitors of the receptor/ligand binding interaction can be identified. Proteins involved in such binding interactions can also be used to screen for peptide or small molecule inhibitors of the binding interaction. Screening assays can be designed to find lead compounds that mimic the biological activity of a native polypeptide or a receptor for the polypeptide. Such screening assays will include assays amenable to high-throughput screening of chemical libraries, making them particularly suitable for identifying small molecule drug candidates. Small molecules contemplated include synthetic organic or inorganic compounds. The assays can be performed in a variety of formats, including protein-protein binding assays, biochemical screening assays, immunoassays and cell based assays, which are well characterized in the art.

Nucleic acids which encode a polypeptide or its modified forms can also be used to generate either transgenic animals or "knock out" animals which, in turn, are useful in the development and screening of therapeutically useful reagents. A transgenic animal (e.g., a mouse or rat) is an animal having cells that contain a transgene, which transgene was introduced into the animal or an ancestor of the animal at a prenatal, e.g., an embryonic stage. A transgene is a DNA which is integrated into the genome of a cell from which a transgenic animal develops. In one embodiment, cDNA encoding a polypeptide can be used to clone genomic DNA encoding the polypeptide in accordance with established techniques and the genomic sequences used to generate transgenic animals that contain cells which express DNA encoding the polypeptide. Methods for generating transgenic animals, particularly animals such as mice or rats, have become conventional in the art and are described, for example, in U.S. Patent Nos. 4,736,866 and 4,870,009. Typically, particular cells would be targeted for polypeptide transgene incorporation with tissue-specific enhancers. Transgenic animals that include a copy of a transgene encoding a polypeptide introduced into the germ line of the animal at an embryonic stage can be used to examine the effect of increased expression of DNA encoding a polypeptide. Such animals can be used as tester animals for reagents thought to confer protection from, for example, pathological conditions associated with its overexpression. In accordance with this facet of the invention, an animal is treated with the reagent and a reduced incidence of the pathological condition, compared to untreated animals bearing the transgene, would indicate a potential therapeutic intervention for the pathological condition.

Alternatively, non-human homologues of a polypeptide can be used to construct a a gene "knock out" animal which has a defective or altered gene encoding the polypeptide as a result of homologous recombination between the endogenous gene encoding the polypeptide and altered genomic DNA encoding the polypeptide introduced into an embryonic stem cell of the animal. For example, cDNA encoding the polypeptide can be used to clone genomic DNA encoding the polypeptide in accordance with established techniques. A portion of the genomic DNA encoding the polypeptide can be deleted or replaced with another gene, such as a gene encoding a selectable marker which can be used to monitor integration. Typically, several kilobases of unaltered flanking DNA (both at the 5' and 3' ends) are included in the vector [see e.g., Thomas and Capecchi, Cell, 51:503 (1987) for a description of homologous recombination vectors]. The vector is introduced into an embryonic stem cell line (e.g., by electroporation) and cells in which the introduced DNA has homologously recombined with the endogenous DNA are selected [see e.g., Li et al., Cell, 69:915 (1992)]. The selected cells are then injected into a blastocyst of an animal (e.g., a mouse or rat) to form aggregation chimeras [see e.g., Bradley, in Teratocarcinomas and Embryonic Stem Cells: A Practical Approach, E. J. Robertson, ed. (IRL, Oxford, 1987), pp. 113-152]. A chimeric embryo can then be implanted into a suitable pseudopregnant female foster animal and the embryo brought to term to create a "knock out" animal. Progeny harboring the homologously recombined DNA in their germ cells can be identified by standard techniques and used to breed animals in which all cells of the animal contain the homologously recombined DNA. Knockout animals can be characterized for instance, for their ability to defend against certain pathological conditions and for their development of pathological conditions due to absence of the polypeptide.

Nucleic acid encoding the polypeptides may also be used in gene therapy. In gene therapy applications, genes are introduced into cells in order to achieve *in vivo* synthesis of a therapeutically effective genetic product, for example for replacement of a defective gene. "Gene therapy" includes both conventional gene therapy where a lasting effect is achieved by a single treatment, and the administration of gene therapeutic agents, which involves the one time or repeated administration of a therapeutically effective DNA or mRNA. Antisense RNAs and DNAs can be used as therapeutic agents for blocking the expression of certain genes *in vivo.* It has already been shown that short antisense oligonucleotides can be imported into cells where they act as inhibitors, despite their low intracellular concentrations caused by their restricted uptake by the cell membrane. (Zamecnik et al., Proc. Natl. Acad. Sci. USA 83:4143-4146 [1986]). The oligonucleotides can be modified to enhance their uptake, e.g. by substituting their negatively charged phosphodiester groups by uncharged groups.

There are a variety of techniques available for introducing nucleic acids into viable cells. The techniques vary depending upon whether the nucleic acid is transferred into cultured cells *in vitro,* or *in vivo* in the cells of the intended host. Techniques suitable for the transfer of nucleic acid into mammalian cells *in vitro* include the use of liposomes, electroporation, microinjection, cell fusion, DEAE-dextran, the calcium phosphate precipitation method, etc. The currently preferred *in vivo* gene transfer techniques include transfection with viral (typically retroviral) vectors and viral coat protein-liposome mediated transfection (Dzau et al., Trends in Biotechnology 11, 205-210 [1993]). In some situations it is desirable to provide the nucleic acid source with an agent that targets the target cells, such as an antibody specific for a cell surface membrane protein or the target cell, a ligand for a receptor on the target cell, etc. Where liposomes are employed, proteins which bind to a cell surface membrane protein associated with endocytosis may be used for targeting and/or to facilitate uptake, e.g. capsid proteins or fragments thereof tropic for a particular cell type, antibodies for proteins which undergo internalization in cycling, proteins that target intracellular localization and enhance intracellular half-life. The technique of receptor-mediated endocytosis is described, for example, by Wu et al., J. Biol. Chem. 262, 4429-4432 (1987); and Wagner ct al., Proc. Natl. Acad. Sci. USA 87, 3410-3414 (1990). For review of gene marking and gene therapy protocols see Anderson et al., Science 256, 808-813 (1992).

### Methods involving screening

The inventions encompass methods of screening compounds to identify those that prevent the effect of the polypeptide (antagonists) or promote the effect of the polypeptide (agonist). Screening assays for antagonist drug candidates are designed to identify compounds that bind or complex with the polypeptides encoded by the genes identified herein, or otherwise interfere with the interaction of the encoded polypeptides with other cellular proteins, including e.g., inhibiting the expression of the polypeptide from cells. Such screening assays will include assays amenable to high-throughput screening of chemical libraries, making them particularly suitable for identifying small molecule drug candidates.

The assays can be performed in a variety of formats, including protein-protein binding assays, biochemical screening assays, immunoassays, and cell-based assays, which are well characterized in the art.

All assays for antagonists are common in that they call for contacting the drug candidate with a polypeptide encoded by a nucleic acid identified herein under conditions and for a time sufficient to allow these two components to interact.

In binding assays, the interaction is binding and the complex formed can be isolated or detected in the reaction mixture. In a particular embodiment, the polypeptide or the drug candidate is immobilized on a solid phase, e.g., on a microtiter plate, by covalcnt or non-covalent attachments. Non-covalent attachment generally is accomplished by coating the solid surface with a solution of the polypeptide and drying. Alternatively, an immobilized antibody, e.g., a monoclonal antibody, specific for the polypeptide to be immobilized can be used to anchor it to a solid surface. The assay is performed by adding the non-immobilized component, which may be labeled by a detectable label, to the immobilized component, e.g., the coated surface containing the anchored component. When the reaction is complete, the non-reacted components are removed, e.g., by washing, and complexes anchored on the solid surface are detected. When the originally non-immobilized component carries a detectable label, the detection of label immobilized on the surface indicates that complexing occurred. Where the originally non-immobilized component does not carry a label, complexing can be detected, for example, by using a labeled antibody specifically binding the immobilized complex.

If the candidate compound interacts with but does not bind to a polypeptide, its interaction with that polypeptide can be assayed by methods well known for detecting protein-protein interactions. Such assays include traditional approaches, such as, e.g., cross-linking, co-immunoprccipitation, and co-purification through gradients or chromatographic columns. In addition, protein-protein interactions can be monitored by using a yeast-based genetic system described by Fields and co-workers (Fields and Song, Nature (London), 340:245-246 (1989); Chien et al., Proc. Natl. Acad. Sci. USA, 88:9578-9582 (1991)) as disclosed by Chevray and Nathans, Proc. Natl. Acad. Sci. USA, 89: 5789-5793 (1991). Many transcriptional activators, such as yeast GAL4, consist of two physically discrete modular domains, one acting as the DNA-binding domain, the other one functioning as the transcription-activation domain. The yeast expression system described in the foregoing publications (generally referred to as the "two-hybrid system") takes advantage of this property, and employs two hybrid proteins, one in which the antigen is fused to the DNA-binding domain of GAL4, and another, in which candidate activating proteins are fused to the activation domain. The expression of a GAL1-*lacZ* reporter gene under control of a GAL4-activated promoter depends on reconstitution of GAL4 activity via protein-protein interaction. Colonies containing interacting polypeptides are detected with a chromogenic substrate for β-galactosidase. A complete kit (MATCHMAKER™) for identifying protein-protein interactions between two specific proteins using the two-hybrid technique is commercially available from Clontech. This system can also be extended to map protein domains involved in specific protein interactions as well as to pinpoint amino acid residues that are crucial for these interactions.

Compounds that interfere with the interaction of a gene encoding a polypeptide identified herein and other intra- or extracellular components can be tested as follows: usually a reaction mixture is prepared containing the product of the gene and the intra- or extracellular component under conditions and for a time allowing for the interaction and binding of the two products. To test the ability of a candidate compound to inhibit binding, the reaction is run in the absence and in the presence of the test compound. In addition, a placebo may be added to a third reaction mixture, to serve as positive control. The binding (complex formation) between the test compound and the intra- or extracellular component present in the mixture is monitored as described hereinabove. The formation of a complex in the control reaction(s) but not in the reaction mixture containing the test compound indicates that the test compound interferes with the interaction of the test compound and its reaction partner.

To assay for antagonists, the polypeptide may be added to a cell along with the compound to be screened for a particular activity and the ability of the compound to inhibit the activity of interest in the presence of the polypeptide indicates that the compound is an antagonist to the polypeptide. Alternatively, antagonists may be detected by combining the polypeptide and a potential antagonist with membrane-bound polypeptide receptors or encoded receptors under appropriate conditions for a competitive inhibition assay. The polypeptide can be labeled, such as by radioactivity, such that the number of polypeptide molecules bound to the receptor can be used to determine the effectiveness of the potential antagonist. The gene encoding the receptor can be identified by numerous methods known to those of skill in the art, for example, ligand panning and FACS sorting. Coligan et al., Current Protocols in Immun., 1(2): Chapter 5 (1991). Preferably, expression cloning is employed wherein polyadenylated RNA is prepared from a cell responsive to the polypeptide and a cDNA library created from this RNA is divided into pools and used to transfect COS cells or other cells that are not responsive to the polypeptide. Transfected cells that are grown on glass slides are exposed to labeled polypeptide. The polypeptide can be labeled by a variety of means including iodination or inclusion of a recognition site for a site-specific protein kinase. Following fixation and incubation, the slides are subjected to autoradiographic analysis. Positive pools are identified and sub-pools are prepared and re-transfected using an interactive sub-pooling and re-screening process, eventually yielding a single clone that encodes the putative receptor.

As an alternative approach for receptor identification, labeled polypeptide can be photoaffinity-linked with cell membrane or extract preparations that express the receptor molecule. Cross-linked material is resolved by PAGE and exposed to X-ray film. The labeled complex containing the receptor can be excised, resolved into peptide fragments, and subjected to protein micro-sequencing. The amino acid sequence obtained from micro- sequencing would be used to design a set of degenerate oligonucleotide probes to screen a cDNA library to identify the gene encoding the putative receptor.

In another assay for antagonists, mammalian cells or a membrane preparation expressing the receptor would be incubated with labeled polypeptide in the presence of the candidate compound. The ability of the compound to enhance or block this interaction could then be measured.

More specific examples of potential antagonists include an oligonucleotide that binds to the fusions of immunoglobulin with a polypeptide, and, in particular, antibodies including, without limitation, poly- and monoclonal antibodies and antibody fragments, single-chain antibodies, anti-idiotypic antibodies, and chimeric or humanized versions of such antibodies or fragments, as well as human antibodies and antibody fragments. Alternatively, a potential antagonist may be a closely related protein, for example, a mutated form of the polypeptide that recognizes the receptor but imparts no effect, thereby competitively inhibiting the action of the polypeptide.

Another potential antagonist is an antisense RNA or DNA construct prepared using antisense technology, where, e.g., an antisense RNA or DNA molecule acts to block directly the translation of mRNA by hybridizing to targeted mRNA and preventing protein translation. Antisense technology can be used to control gene expression through triple-helix formation or antisense DNA or RNA, both of which methods are based on binding of a polynucleotide to DNA or RNA. For example, the 5' coding portion of the polynucleotide sequence, which encodes the mature polypeptides herein, can be used to design an antisense RNA oligonucleotide of from about 10 to 40 base pairs in length. A DNA oligonucleotide is designed to be complementary to a region of the gene involved in transcription (triple helix - see Lee et al., Nucl. Acids Res., 6:3073 (1979); Cooney et al., Science, 241: 456 (1988); Dervan et al., Science, 251:1360 (1991)), thereby preventing transcription and the production of the polypeptide. The antisense RNA oligonucleotide hybridizes to the mRNA *in vivo* and blocks translation of the mRNA molecule into the polypeptide (antisense - Okano, Neurochem., 56:560 (1991); Oligodeoxynucleotides as Antisense Inhibitors of Gene Expression (CRC Press: Boca Raton, FL, 1988). The oligonucleotides described above can also be delivered to cells such that the antisense RNA or DNA may be expressed *in vivo* to inhibit production of the polypeptide. When antisense DNA is used, oligodeoxyribonucleotides derived from the translation-initiation site, e.g., between about -10 and +10 positions of the target gene nucleotide sequence, are preferred.

Potential antagonists include small molecules that bind to the active site, the receptor binding site, or growth factor or other relevant binding site of the polypeptide, thereby blocking the normal biological activity of the polypeptide. Examples of small molecules include, but are not limited to, small peptides or peptide-like molecules, preferably soluble peptides, and synthetic non-peptidyl organic or inorganic compounds.

Ribozymes are enzymatic RNA molecules capable of catalyzing the specific cleavage of RNA. Ribozymes act by sequence-specific hybridization to the complementary target RNA, followed by endonucleolytic cleavage. Specific ribozyme cleavage sites within a potential RNA target can be identified by known techniques. For further details see, e.g., Rossi, Current Biology, 4:469-471 (1994), and PCT publication No. WO 97/33551 (published September 18, 1997).

Nucleic acid molecules in triple-helix formation used to inhibit transcription should be single-stranded and composed of deoxynucleotides. The base composition of these oligonucleotides is designed such that it promotes triple-helix formation via Hoogsteen base-pairing rules, which generally require sizeable stretches of purines or pyrimidines on one strand of a duplex. For further details see, e.g., PCT publication No. WO 97/33551, *supra.*

These small molecules can be identified by any one or more of the screening assays discussed hereinabove and/or by any other screening techniques well known for those skilled in the art.

Isolated polypeptide-encoding nucleic acid can be used for recombinantly producing polypeptide using techniques well known in the art and as described herein. In turn, the produced polypeptides can be employed for generating antibodies using techniques well known in the art and as described herein.

Antibodies specifically binding a polypeptide identified herein, as well as other molecules identified by the screening assays disclosed hereinbefore, can be administered for the treatment of various disorders, including cancer, in the form of pharmaceutical compositions.

If the polypeptide is intracellular and whole antibodies are used as inhibitors, internalizing antibodies are preferred. However, lipofections or liposomes can also be used to deliver the antibody, or an antibody fragment, into cells. Where antibody fragments are used, the smallest inhibitory fragment that specifically binds to the binding domain of the antigen is preferred. For example, based upon the variable-region sequences of an antibody, peptide molecules can be designed that retain the ability to bind the antigen sequence. Such peptides can be synthesized chemically and/or produced by recombinant DNA technology. See, *e.g.,* Marasco et al., Proc. Natl. Acad. Sci. USA, 90: 7889-7893 (1993).

### Pharmaceutical Formulations

Therapeutic formulations comprising an antibody arc prepared for storage by mixing the antibody having the desired degree of purity with optional physiologically acceptable carriers, excipients or stabilizers (Remington: The Science and Practice of Pharmacy 20th edition (2000)), in the form of aqueous solutions, lyophilized or other dried formulations. Acceptable carriers, excipients, or stabilizers are nontoxic to recipients at the dosages and concentrations employed, and include buffers such as phosphate, citrate, histidine and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g., Zn-protein complexes); and/or non-ionic surfactants such as TWEEN™, PLURONICS™ or polyethylene glycol (PEG).

The formulation herein may also contain more than one active compound as necessary for the particular indication being treated, preferably those with complementary activities that do not adversely affect each other. Such molecules are suitably present in combination in amounts that are effective for the purpose intended.

The active ingredients may also be entrapped in microcapsule prepared, for example, by coacervation techniques or by interfacial polymerization, for example, hydroxymethylcellulose or gelatin-microcapsule and poly-(methylmethacylate) microcapsule, respectively, in colloidal drug delivery systems (for example, liposomes, albumin microspheres, microemulsions, nano-particlcs and nanocapsules) or in macrocmulsions. Such techniques are disclosed in Remington: The Science and Practice of Pharmacy 20th edition (2000).

The formulations to be used for *in vivo* administration must be sterile. This is readily accomplished by filtration through sterile filtration membranes.

Sustained-release preparations may be prepared. Suitable examples of sustained-release preparations include semipermeable matrices of solid hydrophobic polymers containing the immunoglobulin, which matrices are in the form of shaped articles, e.g., films, or microcapsule. Examples of sustained-release matrices include polyesters, hydrogels (for example, poly(2-hydroxyethyl-methacrylate), or poly(vinylalcohol)), polylactides (U.S. Pat. No. 3,773,919), copolymers of L-glutamic acid and γ ethyl-L-glutamate, non-degradable ethylene-vinyl acetate, degradable lactic acid-glycolic acid copolymers such as the LUPRON DEPOT™ (injectable microspheres composed of lactic acid-glycolic acid copolymer and leuprolide acetate), and poly-D-(-)-3-hydroxybutyric acid. While polymers such as ethylene-vinyl acetate and lactic acid-glycolic acid enable release of molecules for over 100 days, certain hydrogels release proteins for shorter time periods. When encapsulated immunoglobulins remain in the body for a long time, they may denature or aggregate as a result of exposure to moisture at 37°C, resulting in a loss of biological activity and possible changes in immunogenicity. Rational strategies can be devised for stabilization depending on the mechanism involved. For example, if the aggregation mechanism is discovered to be intermolecular S-S bond formation through thio-disulfide interchange, stabilization may be achieved by modifying sulfhydryl residues, lyophilizing from acidic solutions, controlling moisture content, using appropriate additives, and developing specific polymer matrix compositions.

It is further contemplated that an agent useful in the invention can be introduced to an individual by gene therapy. Gene therapy refers to therapy performed by the administration of a nucleic acid to an individual. In gene therapy applications, genes are introduced into cells in order to achieve in vivo synthesis of a therapeutically effective genetic product, for example for replacement of a defective gene. "Gene therapy" includes both conventional gene therapy where a lasting effect is achieved by a single treatment, and the administration of gene therapeutic agents, which involves the one time or repeated administration of a therapeutically effective DNA or mRNA. Antisense RNAs and DNAs can be used as therapeutic agents for blocking the expression of certain genes in vivo. See, e.g., KLβ-SiRNA described in the Examples. It has already been shown that short antisense oligonucleotides can be imported into cells where they act as inhibitors, despite their low intracellular concentrations caused by their restricted uptake by the cell membrane. (Zamecnik et al., Proc. Natl. Acad. Sci. USA 83:4143-4146 (1986)). The oligonucleotides can be modified to enhance their uptake, e.g. by substituting their negatively charged phosphodiester groups by uncharged groups. For general reviews of the methods of gene therapy, see, for example, Goldspiel et al. Clinical Pharmacy 12:488-505 (1993); Wu and Wu Biotherapy 3:87-95 (1991); Tolstoshev Ann. Rev. Pharmacol. Toxicol. 32:573-596 (1993); Mulligan Science 260:926-932 (1993); Morgan and Anderson Ann. Rev. Biochem. 62:191-217 (1993); and May TIBTECH 11:155-215 (1993). Methods commonly known in the art of recombinant DNA technology which can be used are described in Ausubel et al. eds. (1993) Current Protocols in Molecular Biology, John Wiley & Sons, NY; and Kriegler (1990) Gene Transfer and Expression, A Laboratory Manual, Stockton Press, NY.

### Uses

KLβ modulators may be used in, for example, *in vitro, ex vivo* and *in vivo* therapeutic methods.

The invention provides methods and compositions useful for modulating disease states associated with expression and/or activity of KLβ, such as increased expression and/or activity or undesired expression and/or activity, said methods comprising administration of an effective dose of a KLβ antagonist (such as an anti-KLβ antibody) to an individual in need of such treatment.

In one aspect, the invention provides methods for modulating disease states associated with expression and/or activity of KLβ and FGF19, such as increased expression and/or activity or undesired expression and/or activity, said methods comprising administration of an effective dose of a KLβ antagonist (such as an anti-KLβ antibody) to an individual in need of such treatment.

In one aspect, the invention provides methods for modulating disease states associated with expression and/or activity of KLβ and FGFR4, such as increased expression and/or activity or undesired expression and/or activity, said methods comprising administration of an effective dose of a KLβ antagonist (such as an anti-KLβ antibody) to an individual in need of such treatment.

One of ordinary skill can determine whether disorders are associated with expression and/or activity of KLβ, FGF19 and/or FGFR4 using methods known in the art and methods disclosed herein.

It is understood that any suitable KLβ antagonist (such as an anti-KLβ antibody) may be used in methods of treatment, including monoclonal and/or polyclonal antibodies, a human antibody, a chimeric antibody, an affinity-matured antibody, a humanized antibody, and/or an antibody fragment.

Moreover, at least some of the antibodies can bind antigen from other species. Accordingly, the antibodies can be used to bind specific antigen activity, e.g., in a cell culture containing the antigen, in human subjects or in other mammalian subjects having the antigen with which an antibody cross-reacts (e.g. chimpanzee, baboon, marmoset, cynomolgus and rhesus, pig or mouse). In one embodiment, the antibody can be used for inhibiting antigen activities by contacting the antibody with the antigen such that antigen activity is inhibited. Preferably, the antigen is a human protein molecule.

In one embodiment, an antibody can be used in a method for binding an antigen in an individual suffering from a disorder associated with increased antigen expression and/or activity, comprising administering to the subject an antibody such that the antigen in the subject is bound. Preferably, the antigen is a human protein molecule and the subject is a human subject. Alternatively, the subject can be a mammal expressing the antigen with which an antibody binds. Still further the subject can be a mammal into which the antigen has been introduced (e.g., by administration of the antigen or by expression of an antigen transgene). An antibody can be administered to a human subject for therapeutic purposes. Moreover, an antibody can be administered to a non-human mammal expressing an antigen with which the immunoglobulin cross-reacts (e.g., a primate, pig or mouse) for veterinary purposes or as an animal model of human disease. Regarding the latter, such animal models may be useful for evaluating the therapeutic efficacy of antibodies (e.g., testing of dosages and time courses of administration).

The antibodies can be used to treat, inhibit, delay progression of, prevent/delay recurrence of, ameliorate, or prevent diseases, disorders or conditions associated with expression and/or activity of one or more antigen molecules.

In certain embodiments, an immunoconjugate comprising an antibody conjugated with one or more cytotoxic agcnt(s) is administered to the patient. In some embodiments, the immunoconjugate and/or antigen to which it is bound is/are internalized by the cell, resulting in increased therapeutic efficacy of the immunoconjugate in killing the target cell to which it binds. In one embodiment, the cytotoxic agent targets or interferes with nucleic acid in the target cell. In one embodiment, the cytotoxic agent targets or interferes with microtubule polymerization. Examples of such cytotoxic agents include any of the chemotherapeutic agents noted herein (such as a maytansinoid, auristatin, dolastatin, or a calicheamicin), a radioactive isotope, or a ribonuclease or a DNA endonuclease.

In any of the methods herein, one may administer to the subject or patient along with the KLβ antagonist an effective amount of a second medicament (where the antibody herein is a first medicament), which is another active agent that can treat the condition in the subject that requires treatment. For instance, a KLβ antagonist may be co-administered with another KLβ antagonist, an antibody, chemotherapeutic agent(s) (including cocktails of chemotherapeutic agents), anti-angiogenic agent(s), immunosuppressive agents(s), cytokine(s), cytokine antagonist(s), and/or growth-inhibitory agent(s). The type of such second medicament depends on various factors, including the type of disorder, such as cancer or an autoimmune disorder, the severity of the disease, the condition and age of the patient, the type and dose of first medicament employed, etc.

Where a KLβ antagonist inhibits tumor growth, for example, it may be particularly desirable to combine it with one or more other therapeutic agents that also inhibit tumor growth. For instance, a KLβ antagonist may be combined with an anti-angiogenic agent, such as an anti-VEGF antibody (e.g., AVASTIN®) and/or anti-ErbB antibodies (e.g. HERCEPTIN® trastuzumab anti-HER2 antibody or an anti-HER2 antibody that binds to Domain II of HER2, such as OMNITARG™ pertuzumab anti-HER2 antibody) in a treatment scheme, e.g. in treating any of the disease described herein, including hepatocellular carcinoma and pancreatic cancer. Alternatively, or additionally, the patient may receive combined radiation therapy (e.g. external beam irradiation or therapy with a radioactive labeled agent, such as an antibody). Such combined therapies noted above include combined administration (where the two or more agents are included in the same or separate formulations), and separate administration, in which case, administration of the antibody can occur prior to, and/or following, administration of the adjunct therapy or therapies. In addition, combining a KLβ antagonist with a relatively non-cytotoxic agent such as another biologic molecule, e.g., an antibody is expected to reduce cytotoxicity versus combining the KLβ antagonist with a chemotherapeutic agent of other agent that is highly toxic to cells.

Treatment with a combination of a KLβ antagonist with one or more second medicaments preferably results in an improvement in the signs or symptoms of cancer. For instance, such therapy may result in an improvement in survival (overall survival and/or progression-free survival) relative to a patient treated with the second medicament only (e.g., a chemotherapeutic agent only), and/or may result in an objective response *(partial or complete, preferably complete). Moreover, treatment with the combination of a KLβ antagonist and one or more second medicament(s) preferably results in an additive, and more preferably synergistic (or greater than additive), therapeutic benefit to the patient. Preferably, in this combination method the timing between at least one administration of the second medicament and at least one administration of the KLβ antagonist is about one month or less, more preferably, about two weeks or less.

For treatment of cancers, the second medicament is preferably another KLβ antagonist, an antibody, chemotherapeutic agent (including cocktails of chemotherapeutic agents), anti-angiogenic agent, immunosuppressive agent, prodrug, cytokine, cytokine antagonist, cytotoxic radiotherapy, corticosteroid, anti-emetic, cancer vaccine, analgesic, anti-vascular agent, and/or growth-inhibitory agent. The cytotoxic agent includes an agent interacting with DNA, the antimetabolites, the topoisomerase I or II inhibitors, or the spindle inhibitor or stabilizer agents (e.g., preferably vinca alkaloid, more preferably selected from vinblastine, deoxyvinblastine, vincristine, vindesine, vinorelbine, vinepidine, vinfosiltine, vinzolidine and vinfunine), or any agent used in chemotherapy such as 5-FU, a taxane, doxorubicin, or dexamethasone.

In another embodiment, the second medicament is an antibody used to treat cancers such as those directed against the extracellular domain of the HER2/neu receptor, e.g., trastuzumab, or one of its functional fragments, pan-HER inhibitor, a Src inhibitor, a MEK inhibitor, or an EGFR inhibitor (e.g., an anti-EGFR antibody (such as one inhibiting the tyrosine kinase activity of the EGFR), which is preferably the mouse monoclonal antibody 225, its mouse-man chimeric derivative C225, or a humanized antibody derived from this antibody 225 or derived natural agents, dianilinophthalimides, pyrazolo- or pyrrolopyridopyrimidines, quinazilines, gefitinib, erlotinib, cetuximab, ABX-EFG, canertinib, EKB-569 and PKI-166), or dual-EGFR/HER-2 inhibitor such as lapatanib. Additional second medicaments include alemtuzumab (CAMPATH™), FavID (IDKLH), CD20 antibodies with altered glycosylation, such as GA-101/GLYCART™, oblimersen (GENASENSE™), thalidomide and analogs thereof, such as lenalidomide (REVLIMID™), imatinib, sorafenib, ofatumumab (HUMAX-CD20™), anti-CD40 antibody, e.g. SGN-40, and anti-CD-80 antibody, e.g. galiximab.

The anti-emetic agent is preferably ondansetron hydrochloride, granisetron hydrochloride, metroclopramide, domperidone, haloperidol, cyclizine, lorazepam, prochlorperazine, dexamethasone, levomepromazine, or tropisetron. The vaccine is preferably GM-CSF DNA and cell-based vaccines, dendritic cell vaccine, recombinant viral vaccines, heat shock protein (HSP) vaccines, allogeneic or autologous tumor vaccines. The analgesic agent preferably is ibuprofen, naproxen, choline magnesium trisalicylate, or oxycodone hydrochloride. The anti-vascular agent preferably is bevacizumab, or rhuMAb-VEGF. Further second medicaments include anti-proliferative agents such a farnesyl protein transferase inhibitors, anti-VEGF inhibitors, p53 inhibitors, or PDGFR inhibitors. The second medicament herein includes also biologic-targetcd therapy such as treatment with antibodies as well as small-molecule-targeted therapy, for example, against certain receptors.

Many anti-angiogenic agents have been identified and are known in the art, including those listed herein, e.g., listed under Definitions, and by, e.g., Carmeliet and Jain, Nature 407:249-257 (2000); Ferrara et al., Nature Reviews:Drug Discovery, 3:391-400 (2004); and Sato Int. J. Clin. Oncol., 8:200-206 (2003). See also, US Patent Application US20030055006. In one embodiment, an anti-KLβ antibody is used in combination with an anti-VEGF neutralizing antibody (or fragment) and/or another VEGF antagonist or a VEGF receptor antagonist including, but not limited to, for example, soluble VEGF receptor (e.g., VEGFR-1, VEGFR-2, VEGFR-3, neuropillins (e.g., NRP1, NRP2)) fragments, aptamers capable of blocking VEGF or VEGFR, neutralizing anti-VEGFR antibodies, low molecule weight inhibitors of VEGFR tyrosine kinases (RTK), antisense strategies for VEGF, ribozymes against VEGF or VEGF receptors, antagonist variants of VEGF; and any combinations thereof. Alternatively, or additionally, two or more angiogenesis inhibitors may optionally be co-administered to the patient in addition to VEGF antagonist and other agent. In certain embodiment, one or more additional therapeutic agents, e.g., anti-cancer agents, can be administered in combination with a KLβ antagonist (such as an anti-KLβ antibody), the VEGF antagonist, and an anti-angiogenesis agent.

Chemotherapeutic agents useful herein are described supra, e.g., in the definition of "chemotherapeutic agent".

Exemplary second medicaments include an alkylating agent, a folate antagonist, a pyrimidine antagonist, a cytotoxic antibiotic, a platinum compound or platinum-based compound, a taxane, a vinca alkaloid, a c-Kit inhibitor, a topoisomerase inhibitor, an anti-angiogenesis inhibitor such as an anti-VEGF inhibitor, a HER-2 inhibitor, an EGFR inhibitor or dual EGFR/HER-2 kinase inhibitor, an anti-estrogen such as fulvestrant, and a hormonal therapy agent, such as carboplatin, cisplatin, gemcitabine, capecitabine, epirubicin, tamoxifen, an aromatase inhibitor, and prednisone. Most preferably, the cancer is colorectal cancer and the second medicament is an EGFR inhibitor such as erlotinib, an anti-VEGF inhibitor such as bevacizumab, or is cetuximab, arinotecan, irinotecan, or FOLFOX, or the cancer is breast cancer an the second medicament is an anti-estrogen modulator such as fulvestrant, tamoxifen or an aromatase inhibitor such as letrozole, exemestane, or anastrozole, or is a VEGF inhibitor such as bevacizumab, or is a chemotherapeutic agent such as doxorubicin, and/or a taxane such as paclitaxel, or is an anti-HER-2 inhibitor such as trastuzumab, or a dual EGFR/HER-2 kinase inhibitor such as lapatinib or a HER-2 downregulator such as 17AAG (geldanamycin derivative that is a heat shock protein [Hsp] 90 poison) (for example, for breast cancers that have progressed on trastuzumab). In other embodiments, the cancer is lung cancer, such as small-cell lung cancer, and the second medicament is a VEGF inhibitor such as bevacizumab, or an EGFR inhibitor such as, e.g., erlotinib or a c-Kit inhibitor such as e.g., imatinib. In other embodiments, the cancer is liver cancer, such as hepatocellular carcinoma, and the second medicament is an EGFR inhibitor such as erlotinib, a chemotherapeutic agent such as doxorubicin or irinotecan, a taxane such as paclitaxel, thalidomide and/or interferon. Further, a preferred chemotherapeutic agent for front-line therapy of cancer is taxotere, alone in combination with other second medicaments. Most preferably, if chemotherapy is administered, it is given first, followed by the antibodies.

Such second medicaments may be administered within 48 hours after the antibodies are administered, or within 24 hours, or within 12 hours, or within 3-12 hours after said agent, or may be administered over a pre-selected period of time, which is preferably about 1 to 2 days. Further, the dose of such agent may be sub-therapeutic.

The KLβ antagonist can be administered concurrently, sequentially, or alternating with the second medicament or upon non-responsiveness with other therapy. Thus, the combined administration of a second medicament includes co-administration (concurrent administration), using separate formulations or a single pharmaceutical formulation, and consecutive administration in either order, wherein preferably there is a time period while both (or all) medicaments simultaneously exert their biological activities. All these second medicaments may be used in combination with each other or by themselves with the first medicament, so that the express "second medicament" as used herein does not mean it is the only medicament besides the first medicament, respectively. Thus, the second medicament need not be one medicament, but may constitute or comprise more than one such drug.

These second medicaments as set forth herein may be used in the same dosages and with administration routes as the first medicaments, or about from 1 to 99% of the dosages of the first medicaments. If such second medicaments are used at all, preferably, they are used in lower amounts than if the first medicament were not present, especially in subsequent dosings beyond the initial dosing with the first medicament, so as to eliminate or reduce side effects caused thereby.

The invention also provides methods and compositions for inhibiting or preventing relapse tumor growth or relapse cancer cell growth. Relapse tumor growth or relapse cancer cell growth is used to describe a condition in which patients undergoing or treated with one or more currently available therapies (e.g., cancer therapies, such as chemotherapy, radiation therapy, surgery, hormonal therapy and/or biological therapy/immunotherapy, anti-VEGF antibody therapy, particularly a standard therapeutic regimen for the particular cancer) is not clinically adequate to treat the patients or the patients are no longer receiving any beneficial effect from the therapy such that these patients need additional effective therapy. As used herein, the phrase can also refer to a condition of the "non-responsive/refractory" patient, e.g., which describe patients who respond to therapy yet suffer from side effects, develop resistance, do not respond to the therapy, do not respond satisfactorily to the therapy, etc. In various embodiments, a cancer is relapse tumor growth or relapse cancer cell growth where the number of cancer cells has not been significantly reduced, or has increased, or tumor size has not been significantly reduced, or has increased, or fails any further reduction in size or in number of cancer cells. The determination of whether the cancer cells are relapse tumor growth or relapse cancer cell growth can be made either in vivo or in vitro by any method known in the art for assaying the effectiveness of treatment on cancer cells, using the art-accepted meanings of "relapse" or "refractory" or "non-responsive" in such a context. A tumor resistant to anti-VEGF treatment is an example of a relapse tumor growth.

The invention provides methods of blocking or reducing relapse tumor growth or relapse cancer cell growth in an individual by administering one or more KLβ antagonist (such as an anti-KLβ antibody) to block or reduce the relapse tumor growth or relapse cancer cell growth in subject. In certain embodiments, the KLβ antagonist can be administered subsequent to the cancer therapeutic. In certain embodiments, the KLβ antagonist is administered simultaneously with cancer therapy. Alternatively, or additionally, the KLβ antagonist therapy alternates with another cancer therapy, which can be performed in any order. The invention also encompasses methods for administering one or more inhibitory antibodies to prevent the onset or recurrence of cancer in patients predisposed to having cancer. Generally, the subject was or is concurrently undergoing cancer therapy. In one embodiment, the cancer therapy is treatment with an anti-angiogenesis agent, e.g., a VEGF antagonist. The anti-angiogenesis agent includes those known in the art and those found under the Definitions herein. In one embodiment, the anti-angiogenesis agent is an anti-VEGF neutralizing antibody or fragment (e.g., humanized A4.6.1, AVASTIN ® (Genentech, South San Francisco, CA), Y0317, M4, G6, B20, 2C3, etc.). See, e.g., U.S. Patents 6,582,959, 6,884,879, 6,703,020; WO98/45332; WO 96/30046; WO94/10202; EP 0666868B1; US Patent Applications 20030206899, 20030190317, 20030203409, and 20050112126; Popkov et al., Journal of Immunological Methods 288:149-164 (2004); and, WO2005012359. Additional agents can be administered in combination with VEGF antagonist and a KLβ antagonist for blocking or reducing relapse tumor growth or relapse cancer cell growth.

The KLβ antagonists (and adjunct therapeutic agent) is/are administered by any suitable means, including parenteral, subcutaneous, intraperitoneal, intrapulmonary, and intranasal, and, if desired for local treatment, intralesional administration. Parenteral infusions include intramuscular, intravenous, intraarterial, intraperitoneal, or subcutaneous administration. In addition, the KLβ antagonists are suitably administered by pulse infusion, particularly with declining doses of the antibody. Dosing can be by any suitable route, e.g. by injections, such as intravenous or subcutaneous injections, depending in part on whether the administration is brief or chronic.

The KLβ antagonist composition will be formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners. The KLβ antagonist need not be, but is optionally formulated with one or more agents currently used to prevent or treat the disorder in question. The effective amount of such other agents depends on the amount of KLβ antagonist present in the formulation, the type of disorder or treatment, and other factors discussed above. These are generally used in the same dosages and with administration routes as used hereinbefore or about from 1 to 99% of the heretofore employed dosages.

For the prevention or treatment of disease, the appropriate dosage of a KLβ antagonist (when used alone or in combination with other agents) will depend on the type of disease to be treated, the type of antibody, the severity and course of the disease, whether the antibody is administered for preventive or therapeutic purposes, previous therapy, the patient's clinical history and response to the antibody, and the discretion of the attending physician. The antibody is suitably administered to the patient at one time or over a series of treatments. Depending on the type and severity of the disease, about 1 µg/kg to 15 mg/kg (e.g. 0.1 mg/kg-10mg/kg) of an anti-KLβ antibody is an initial candidate dosage for administration to the patient, whether, for example, by one or more separate administrations, or by continuous infusion. One typical daily dosage might range from about 1 µg/kg to 100 mg/kg or more, depending on the factors mentioned above. For repeated administrations over several days or longer, depending on the condition, the treatment is sustained until a desired suppression of disease symptoms occurs. One exemplary dosage of the antibody would be in the range from about 0.05mg/kg to about 10mg/kg. Thus, one or more doses of about 0.5mg/kg, 2.0mg/kg, 4.0mg/kg or 10mg/kg (or any combination thereof) may be administered to the patient. Such doses may be administered intermittently, *e.g.* every week or every three weeks (*e.g.* such that the patient receives from about two to about twenty, e.g. about six doses of the antibody). An initial higher loading dose, followed by one or more lower doses may be administered. An exemplary dosing regimen comprises administering an initial loading dose of about 4 mg/kg, followed by a weekly maintenance dose of about 2 mg/kg of the antibody. However, other dosage regimens may be useful. The progress of this therapy is easily monitored by conventional techniques and assays.

### Uses comprising detection of KLβ

In another aspect, the present disclosure provides methods for detection of KLβ, the methods comprising detecting KLβ in a sample. The term "detection" as used herein includes qualitative and/or quantitative detection (measuring levels) with or without reference to a control.

In one aspect, the disclosure provides methods for detecting a disorder associated with KLβ expression and/or activity, the methods comprising detecting KLβ in a biological sample from an individual. In some embodiments, the KLβ expression is increased expression or abnormal expression. In some embodiments, the disorder is a tumor, cancer, and/or a cell proliferative disorder (such as hepatocellular carcinoma and pancreatic cancer), a liver disorder (such as cirrhosis), or any disorder described herein. In some embodiment, the biological sample is serum or of a tumor.

For example, a sample may be assayed for a target antigen (e.g., KLβ, FGF19, and/or FGFR4) by obtaining the sample from a desired source, admixing the sample with anti-target antigen antibody to allow the antibody to form antibody/target antigen complex with any present in the mixture, and detecting any antibody/ target antigen complex present in the mixture. The biological sample may be prepared for assay by methods known in the art which are suitable for the particular sample. The methods of admixing the sample with antibodies and the methods of detecting antibody/ target antigen complex are chosen according to the type of assay used. Such assays include immunohistochemistry, competitive and sandwich assays, and steric inhibition assays. For sample preparation, a tissue or cell sample from a mammal (typically a human patient) may be used. Examples of samples include, but are not limited to, cancer cells such as colon, breast, prostate, ovary, lung, stomach, pancreas, lymphoma, and leukemia cancer cells. Target antigen may also be measured in serum. The sample can be obtained by a variety of procedures known in the art including, but not limited to surgical excision, aspiration or biopsy. The tissue may be fresh or frozen. In one embodiment, the sample is fixed and embedded in paraffin or the like. The tissue sample may be fixed (i.e. preserved) by conventional methodology (See e.g., "Manual of Histological Staining Method of the Armed Forces Institute of Pathology," 3rd edition (1960) Lee G. Luna, HT (ASCP) Editor, The Blakston Division McGraw-Hill Book Company, New York; The Armed Forces Institute of Pathology Advanced Laboratory Methods in Histology and Pathology (1994) Ulreka V. Mikel, Editor, Armed Forces Institute of Pathology, American Registry of Pathology, Washington, D.C.). One of ordinary skill in the art will appreciate that the choice of a fixative is determined by the purpose for which the sample is to be histologically stained or otherwise analyzed. One of ordinary skill in the art will also appreciate that the length of fixation depends upon the size of the tissue sample and the fixative used. By way of example, neutral buffered formalin, Bouin's or paraformaldehyde, may be used to fix a sample. Generally, the sample is first fixed and is then dehydrated through an ascending series of alcohols, infiltrated and embedded with paraffin or other sectioning media so that the tissue sample may be sectioned. Alternatively, one may section the tissue and fix the sections obtained. By way of example, the tissue sample may be embedded and processed in paraffin by conventional methodology (See e.g., "Manual of Histological Staining Method of the Armed Forces Institute of Pathology", *supra*). Examples of paraffin that may be used include, but are not limited to, Paraplast, Broloid, and Tissuemay. Once the tissue sample is embedded, the sample may be sectioned by a microtome or the like (See e.g., "Manual of Histological Staining Method of the Armed Forces Institute of Pathology", *supra*). By way of example for this procedure, sections may range from about three microns to about five microns in thickness. Once sectioned, the sections may be attached to slides by several standard methods. Examples of slide adhesives include, but are not limited to, silane, gelatin, poly-L-lysine and the like. By way of example, the paraffin embedded sections may be attached to positively charged slides and/or slides coated with poly-L-lysine. If paraffin has been used as the embedding material, the tissue sections are generally deparaffinized and rehydrated to water. The tissue sections may be deparaffinized by several conventional standard methodologies. For example, xylenes and a gradually descending series of alcohols may be used (See *e.g.,* "Manual of Histological Staining Method of the Armed Forces Institute of Pathology", *supra*). Alternatively, commercially available deparaffinizing non-organic agents such as Hemo-De7 (CMS, Houston, Texas) may be used.

Anti-KLβ antibodies are useful in assays detecting KLβ expression (such as diagnostic or prognostic assays) in specific cells or tissues wherein the antibodies are labeled as described below and/or are immobilized on an insoluble matrix. However, it is understood that any suitable anti-KLβ antibody may be used in embodiments involving detection and diagnosis. Some methods for making anti-KLβ antibodies arc described herein and methods for making anti-KLβ antibodies are well known in the art, e.g., antibodies disclosed in Ito et al (2005) J Clin Invest 115(8): 2202-2208; R&D Systems Catalog Nos. MAB3738 and AF2619.

Analytical methods a for target antigen all use one or more of the following reagents: labeled target antigen analogue, immobilized target antigen analogue, labeled anti-target antigen antibody, immobilized anti-target antigen antibody and steric conjugates. The labeled reagents also are known as "tracers."

The label used is any detectable functionality that does not interfere with the binding of target antigen and anti-target antigen antibody. Numerous labels are known for use in immunoassay, examples including moieties that may be detected directly, such as fluorochrome, chemiluminescent, and radioactive labels, as well as moieties, such as enzymes, that must be reacted or derivatized to be detected.

The label used is any detectable functionality that does not interfere with the binding of target antigen and anti-target antigen antibody. Numerous labels are known for use in immunoassay, examples including moieties that may be detected directly, such as fluorochrome, chemiluminescent, and radioactive labels, as well as moieties, such as enzymes, that must be reacted or derivatized to be detected. Examples of such labels include the radioisotopes ³²P, ¹⁴C, ¹²⁵I, ³H, and ¹³¹I, fluorophores such as rare earth chelates or fluorescein and its derivatives, rhodamine and its derivatives, dansyl, umbelliferone, luceriferases, e.g., firefly luciferase and bacterial luciferase (U.S. Pat. No. 4,737,456), luciferin, 2,3-dihydrophthalazinediones, horseradish peroxidase (HRP), alkaline phosphatase, β-galactosidase, glucoamylase, lysozyme, saccharide oxidases, e.g., glucose oxidase, galactose oxidase, and glucose-6-phosphate dehydrogenase, heterocyclic oxidases such as uricase and xanthine oxidase, coupled with an enzyme that employs hydrogen peroxide to oxidize a dye precursor such as HRP, lactoperoxidase, or microperoxidase, biotin/avidin, spin labels, bacteriophage labels, stable free radicals, and the like.

Conventional methods are available to bind these labels covalently to proteins or polypeptides. For instance, coupling agents such as dialdehydes, carbodiimides, dimaleimides, bis-imidates, bis-diazotized benzidine, and the like may be used to tag the antibodies with the above-described fluorescent, chemiluminescent, and enzyme labels. See, for example, U.S. Pat. Nos. 3,940,475 (fluorimetry) and 3,645,090 (enzymes); Hunter et al., Nature, 144: 945 (1962); David et al., Biochemistry, 13: 1014-1021 (1974); Pain et al., J. Immunol. Methods, 40: 219-230 (1981); and Nygrcn, J. Histochem. and Cytochem., 30: 407-412 (1982). Preferred labels herein are enzymes such as horseradish peroxidase and alkaline phosphatase. The conjugation of such label, including the enzymes, to the antibody is a standard manipulative procedure for one of ordinary skill in immunoassay techniques. See, for example, O'Sullivan et al., "Methods for the Preparation of Enzyme-antibody Conjugates for Use in Enzyme Immunoassay," in Methods in Enzymology, ed. J.J. Langone and H. Van Vunakis, Vol. 73 (Academic Press, New York, New York, 1981), pp. 147-166*.*

*Immobili*zation of reagents is required for certain assay methods. Immobilization entails separating the anti-target antigen antibody from any target antigen that remains free in solution. This conventionally is accomplished by either insolubilizing the anti-target antigen antibody or target antigen analogue before the assay procedure, as by adsorption to a water-insoluble matrix or surface (Bennich et al.., U.S. 3,720,760), by covalent coupling (for example, using glutaraldehyde cross-linking), or by insolubilizing the anti-target antigen antibody or target antigen analogue afterward, e.g., by immunoprecipitation.

The expression of proteins in a sample may be examined using immunohistochemistry and staining protocols. Immunohistochemical staining of tissue sections has been shown to be a reliable method of assessing or detecting presence of proteins in a sample. Immunohistochemistry ("IHC") techniques utilize an antibody to probe and visualize cellular antigens *in situ,* generally by chromogenic or fluorescent methods. For sample preparation, a tissue or cell sample from a mammal (typically a human patient) may be used. The sample can be obtained by a variety of procedures known in the art including, but not limited to surgical excision, aspiration or biopsy. The tissue may be fresh or frozen. In one embodiment, the sample is fixed and embedded in paraffin or the like. The tissue sample may be fixed (*i.e.* preserved) by conventional methodology. One of ordinary skill in the art will appreciate that the choice of a fixative is determined by the purpose for which the sample is to be histologically stained or otherwise analyzed. One of ordinary skill in the art will also appreciate that the length of fixation depends upon the size of the tissue sample and the fixative used.

IHC may be performed in combination with additional techniques such as morphological staining and/or fluorescence *in-situ* hybridization. Two general methods of IHC are available; direct and indirect assays. According to the first assay, binding of antibody to the target antigen (e.g., KLβ) is determined directly. This direct assay uses a labeled reagent, such as a fluorescent tag or an enzyme-labeled primary antibody, which can be visualized without further antibody interaction. In a typical indirect assay, unconjugated primary antibody binds to the antigen and then a labeled secondary antibody binds to the primary antibody. Where the secondary antibody is conjugated to an enzymatic label, a chromogenic or fluorogenic substrate is added to provide visualization of the antigen. Signal amplification occurs because several secondary antibodies may react with different epitopes on the primary antibody.

The primary and/or secondary antibody used for immunohistochemistry typically will be labeled with a detectable moiety. Numerous labels are available which can be generally grouped into the following categories:

Aside from the sample preparation procedures discussed above, further treatment of the tissue section prior to, during or following IHC may be desired, For example, epitope retrieval methods, such as heating the tissue sample in citrate buffer may be carried out (see, e.g., Leong et al. Appl. Immunohistochem. 4(3):201 (1996)).

Following an optional blocking step, the tissue section is exposed to primary antibody for a sufficient period of time and under suitable conditions such that the primary antibody binds to the antigen in the tissue sample. Appropriate conditions for achieving this can be determined by routine experimentation. The extent of binding of antibody to the sample is determined by using any one of the detectable labels discussed above. Preferably, the label is an enzymatic label (e.g. HRPO) which catalyzes a chemical alteration of the chromogenic substrate such as 3,3'-diaminobenzidine chromogen. Preferably the enzymatic label is conjugated to antibody which binds specifically to the primary antibody (e.g. the primary antibody is rabbit polyclonal antibody and secondary antibody is goat anti-rabbit antibody).

Specimens thus prepared may be mounted and coverslipped. Slide evaluation is then determined, e.g. using a microscope, and staining intensity criteria, routinely used in the art, may be employed.

Other assay methods, known as competitive or sandwich assays, are well established and widely used in the commercial diagnostics industry.

Competitive assays rely on the ability of a tracer target antigen analogue to compete with the test sample target antigen for a limited number of anti-target antigen antibody antigen-binding sites. The anti-target antigen antibody generally is insolubilized before or after the competition and then the tracer and target antigen bound to the anti-target antigen antibody are separated from the unbound tracer and target antigen. This separation is accomplished by decanting (where the binding partner was preinsolubilized) or by centrifuging (where the binding partner was precipitated after the competitive reaction). The amount of test sample target antigen is inversely proportional to the amount of bound tracer as measured by the amount of marker substance. Dose-response curves with known amounts of target antigen are prepared and compared with the test results to quantitatively determine the amount of target antigen present in the test sample. These assays are called ELISA systems when enzymes are used as the detectable markers.

Another species of competitive assay, called a "homogeneous" assay, docs not require a phase separation. Here, a conjugate of an enzyme with the target antigen is prepared and used such that when anti-target antigen antibody binds to the target antigen the presence of the anti-target antigen antibody modifies the enzyme activity. In this case, the target antigen or its immunologically active fragments arc conjugated with a bifunctional organic bridge to an enzyme such as peroxidase. Conjugates are selected for use with anti-target antigen antibody so that binding of the anti-target antigen antibody inhibits or potentiates the enzyme activity of the label. This method *per se* is widely practiced under the name of EMIT.

Steric conjugates are used in steric hindrance methods for homogeneous assay. These conjugates are synthesized by covalently linking a low-molecular-weight hapten to a small target antigen fragment so that antibody to hapten is substantially unable to bind the conjugate at the same time as anti-target antigen antibody. Under this assay procedure the target antigen present in the test sample will bind anti-target antigen antibody, thereby allowing anti-hapten to bind the conjugate, resulting in a change in the character of the conjugate hapten, e.g., a change in fluorescence when the hapten is a fluorophore.

Sandwich assays particularly are useful for the determination of target antigen or anti-target antigen antibodies. In sequential sandwich assays an immobilized anti-target antigen antibody is used to adsorb test sample target antigen, the test sample is removed as by washing, the bound target antigen is used to adsorb a second, labeled anti-target antigen antibody and bound material is then separated from residual tracer. The amount of bound tracer is directly proportional to test sample target antigen. In "simultaneous" sandwich assays the test sample is not separated before adding the labeled anti-target antigen. A sequential sandwich assay using an anti-target antigen monoclonal antibody as one antibody and a polyclonal anti-target antigen antibody as the other is useful in testing samples for target antigen.

The foregoing are merely exemplary detection assays for target antigen. Other methods now or hereafter developed that use anti-target antigen antibody for the determination of target antigen are included within the scope hereof, including the bioassays described herein.

In one aspect, the invention provides methods to detect (e.g., presence or absence of or amount) a polynucleotide(s) (e.g., target polynucleotides) in a biological sample from an individual, such as a human subject. A variety of methods for detecting polynucleotides can be employed and include, for example, RT-PCR, taqman, amplification methods, polynucleotide microarray, and the like.

Methods for the detection of polynucleotides (such as mRNA) arc well known and include, for example, hybridization assays using complementary DNA probes (such as *in situ* hybridization using labeled target riboprobes), Northern blot and related techniques, and various nucleic acid amplification assays (such as RT-PCR using complementary primers specific for target, and other amplification type detection methods, such as, for example, branched DNA, SPIA, Ribo-SPIA, SISBA, TMA and the like).

Biological samples from mammals can be conveniently assayed for, e.g., target mRNAs using Northern, dot blot or PCR analysis. For example, RT-PCR assays such as quantitative PCR assays are well known in the art. In an illustrative embodiment, a method for detecting target mRNA in a biological sample comprises producing cDNA from the sample by reverse transcription using at least one primer; amplifying the cDNA so produced using a target polynucleotide as sense and antisense primers to amplify target cDNAs therein; and detecting the presence or absence of the amplified target cDNA. In addition, such methods can include one or more steps that allow one to determine the amount (levels) of target mRNA in a biological sample (e.g. by simultaneously examining the levels a comparative control mRNA sequence of a housekeeping gene such as an actin family member). Optionally, the sequence of the amplified target cDNA can be determined.

Probes and/or primers may be labeled with a detectable marker, such as, for example, a radioisotope, fluorescent compound, bioluminescent compound, a chemiluminescent compound, metal chelator or enzyme. Such probes and primers can be used to detect the presence of target polynucleotides in a sample and as a means for detecting a cell expressing antigens. As will be understood by the skilled artisan, a great many different primers and probes may be prepared (e.g., based on the sequences provided in herein) and used effectively to amplify, clone and/or determine the presence or absence of and/or amount of target mRNAs.

Optional methods of the invention include protocols comprising detection of polynucleotides, such as target polynucleotide, in a tissue or cell sample using microarray technologies. For example, using nucleic acid microarrays, test and control mRNA samples from test and control tissue samples are reverse transcribed and labeled to generate cDNA probes. The probes are then hybridized to an array of nucleic acids immobilized on a solid support. The array is configured such that the sequence and position of each member of the array is known. For example, a selection of genes that have potential to be expressed in certain disease states may be arrayed on a solid support. Hybridization of a labeled probe with a particular array member indicates that the sample from which the probe was derived expresses that gene. Differential gene expression analysis of disease tissue can provide valuable information. Microarray technology utilizes nucleic acid hybridization techniques and computing technology to evaluate the mRNA expression profile of thousands of genes within a single experiment, (see, e.g., WO 01/75166 published October 11, 2001; (See, for example, U.S. 5,700,637, U.S. Patent 5,445,934, and U.S. Patent 5,807,522, Lockart, Nature Biotechnology, 14:1675-1680 (1996); Cheung, V.G. et al., Nature Genetics 21(Suppl): 15-19 (1999) for a discussion of array fabrication). DNA microarrays are miniature arrays containing gene fragments that are either synthesized directly onto or spotted onto glass or other substrates. Thousands of genes are usually represented in a single array. A typical microarray experiment involves the following steps: 1. preparation of fluorescently labeled target from RNA isolated from the sample, 2. hybridization of the labeled target to the microarray, 3. washing, staining, and scanning of the array, 4. analysis of the scanned image and 5. generation of gene expression profiles. Currently two main types of DNA microarrays are being used: oligonucleotide (usually 25 to 70 mers) arrays and gene expression arrays containing PCR products prepared from cDNAs. In forming an array, oligonucleotides can be either prefabricated and spotted to the surface or directly synthesized on to the surface (in situ).

The Affymetrix GeneChip® system is a commercially available microarray system which comprises arrays fabricated by direct synthesis of oligonucleotides on a glass surface. Probe/Gene Arrays: Oligonucleotides, usually 25 mers, are directly synthesized onto a glass wafer by a combination of semiconductor-based photolithography and solid phase chemical synthesis technologies. Each array contains up to 400,000 different oligos and each oligo is present in millions of copies. Since oligonucleotide probes are synthesized in known locations on the array, the hybridization patterns and signal intensities can be interpreted in terms of gene identity and relative expression levels by the Affymetrix Microarray Suite software. Each gene is represented on the array by a series of different oligonucleotide probes. Each probe pair consists of a perfect match oligonucleotide and a mismatch oligonucleotide. The perfect match probe has a sequence exactly complimentary to the particular gene and thus measures the expression of the gene. The mismatch probe differs from the perfect match probe by a single base substitution at the center base position, disturbing the binding of the target gene transcript. This helps to determine the background and nonspecific hybridization that contributes to the signal measured for the perfect match oligo. The Microarray Suite software subtracts the hybridization intensities of the mismatch probes from those of the perfect match probes to determine the absolute or specific intensity value for each probe set. Probes are chosen based on current information from GenBank and other nucleotide repositories. The sequences are believed to recognize unique regions of the 3' end of the gene. A GeneChip Hybridization Oven ("rotisserie" oven) is used to carry out the hybridization of up to 64 arrays at one time. The fluidics station performs washing and staining of the probe arrays. It is completely automated and contains four modules, with each module holding one probe array. Each module is controlled independently through Microarray Suite software using preprogrammed fluidics protocols. The scanner is a confocal laser fluorescence scanner which measures fluorescence intensity emitted by the labeled cRNA bound to the probe arrays. The computer workstation with Microarray Suite software controls the fluidics station and the scanner. Microarray Suite software can control up to eight fluidics stations using preprogrammed hybridization, wash, and stain protocols for the probe array. The software also acquires and converts hybridization intensity data into a presence/absence call for each gene using appropriate algorithms. Finally, the software detects changes in gene expression between experiments by comparison analysis and formats the output into .txt files, which can be used with other software programs for further data analysis.

In some embodiments, gene deletion, gene mutation, or gene amplification is detected (eg, KLβ and/or FGFR4 and/or FGF19 gene deletion, gene mutation, or gene amplification). Gene deletion, gene mutation, or amplification may be measured by any one of a wide variety of protocols known in the art, for example, by conventional Southern blotting, Northern blotting to quantitate the transcription of mRNA (Thomas, Proc. Natl. Acad. Sci. USA, 77:5201-5205 (1980)), dot blotting (DNA analysis), *or in situ* hybridization (e.g., FISH), using an appropriately labeled probe, cytogenetic methods or comparative genomic hybridization (CGH) using an appropriately labeled probe. In addition, these methods may be employed to detect target gene deletion, ligand mutation, or gene amplification. As used herein, "detecting KLβ expression" encompasses detection of KLβ gene deletion, gene mutation or gene amplification.

Additionally, one can examine the methylation status of the target gene in a tissue or cell sample. Aberrant demethylation and/or hypermethylation of CpG islands in gene 5' regulatory regions frequently occurs in immortalized and transformed cells, and can result in altered expression of various genes. A variety of assays for examining methylation status of a gene are well known in the art. For example, one can utilize, in Southern hybridization approaches, methylation-sensitive restriction enzymes which cannot cleave sequences that contain methylated CpG sites to assess the methylation status of CpG islands. In addition, MSP (methylation specific PCR) can rapidly profile the methylation status of all the CpG sites present in a CpG island of a given gene. This procedure involves initial modification of DNA by sodium bisulfite (which will convert all unmethylated cytosines to uracil) followed by amplification using primers specific for methylated versus unmethylated DNA. Protocols involving methylation interference can also be found for example in Current Protocols In Molecular Biology, Unit 12, Frederick M. Ausubcl et al. eds., 1995; De Marzo et al., Am. J. Pathol. 155(6): 1985-1992 (1999); Brooks et al, Cancer Epidemiol. Biomarkers Prev., 1998, 7:531-536); and Lethe et al., Int. J. Cancer 76(6): 903-908 (1998). As used herein, "detecting KLβ expression" encompasses detection of KLβ gene methylation.

In some embodiments, using methods known in the art, including those described herein, the polynucleotide and/or polypeptide expression of one or more targets can be detected. By way of example, the IHC techniques described above may be employed to detect the presence of one or more such molecules in the sample. As used herein, "in conjunction" is meant to encompass any simultaneous and/or sequential detection. Thus, it is contemplated that in embodiments in which a biological sample is being examined not only for the presence of a first target, but also for the presence of Fa second target, separate slides may be prepared from the same tissue or sample, and each slide tested with a reagent that binds to the first and/or second target, respectively. Alternatively, a single slide may be prepared from the tissue or cell sample, and antibodies directed to the first and second target, respectively, may be used in connection with a multi-color staining protocol to allow visualization and detection of the first and second target.

Biological samples are described herein, e.g., in the definition of Biological Sample. In some embodiment, the biological sample is serum or of a tumor.

### Articles of Manufacture

In another aspect of the present disclosure, an article of manufacture containing materials useful for the treatment, prevention and/or diagnosis of the disorders described above is provided. The article of manufacture comprises a container and a label or package insert on or associated with the container. Suitable containers include, for example, bottles, vials, syringes, etc. The containers may be formed from a variety of materials such as glass or plastic. The container holds a composition which is by itself or when combined with another composition(s) effective for treating, preventing and/or diagnosing the condition and may have a sterile access port (for example the container may be an intravenous solution bag or a vial having a stopper pierceable by a hypodermic injection needle). At least one active agent in the composition is an antibody. The label or package insert indicates that the composition is used for treating the condition of choice, such as cancer. Moreover, the article of manufacture may comprise (a) a first container with a composition contained therein, wherein the composition comprises a KLβ antagonist (such as an anti-KLβ antibody); and (b) a second container with a composition contained therein. The article of manufacture in this embodiment of the invention may further comprise a package insert indicating that the first and second antibody compositions can be used to treat a particular condition, e.g. cancer. Alternatively, or additionally, the article of manufacture may further comprise a second (or third) container comprising a pharmaceutically-acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

The following are examples of the methods and compositions of the present disclosure. It is understood that various other embodiments may be practiced, given the general description provided above.

### EXAMPLES

The following materials and methods were used in Examples 1-15.

### DNA Constructs:

*Full Length Human Klotho Beta Construct:* Total RNA from HepG2 hepatocellular carcinoma cell line was extracted using RNeasy kit (Quiagen). The human Klotho beta (KLβ) was cloned by reverse transcriptase PCR (RT-PCR) using the SuperScript III One-Step RT-PCR kit (Invitrogen) and the following primers:
Forward primer 5'-CGGGCGCTAGCATGAAGCCAGGCTGTGCGGCAGG-3' (SEQ ID NO:3)
Reverse primer 5'-CAGTGGATCCTTACTTATCGTCGTCATCCTTGTAATCGCTAACAACTCTCTTGCCTT TCTTTC-3'(SEQ ID NO:4)

The resulting KLβ PCR product was digested with NheI and BamHI and ligated into pIRESpuro3 (Clontech) to obtain the full-length human KLβ c-terminal flag tagged construct (pCMVhKLβ-Flag (SEQ ID NO: 49)).

*Full Length Human FGFR4 Construct:* Total RNA from HepG2 hepatocellular carcinoma cell line was extracted using RNeasy kit (Quiagen). The human FGFR4 cDNA was cloned by reverse transcriptase PCR (RT-PCR) using the SuperScriptIII One-Step RT-PCR kit (Invitrogen) and the following primers:
Forward primer 5'-CCGCCGGATATCATGCGGCTGCTGCTGGCCCTGTTGG-3' (SEQ ID NO:5)
Reverse primer 5'-CCGCCGGAATTCTGTCTGCACCCCAGACCCGAAGGGG-3' (SEQ ID NO:6)

The resulting FGFR4 PCR product was digested with EcoRV and EcoRI and ligated into pIRESpuro3 (Clontech) to obtain the full-length human FGFR4.

*Construct Human FGFR4 With C-Terminal Flag Tag:* The C terminal flag tag was added into human pIRESpuro3FGFR4 using Stratagene XL QuickChange Site-Direct Mutagenesis kit and the following primers:
Forward primer: 5'-GGT CTG GGG TGC AGA CAG GTA AGC CTA TCC CTA ACC CTC TCC TCG GTC TCG ATT CTA CGT AGG AAT TCG GAT CCG CGG C-3' (SEQ ID NO:7)
Reverse primer: 5'-GCC GCG GAT CCG AAT TCC TAC GTA GAA TCG AGA CCG AGG AGA GGG TTA GGG ATA GGC TTA CCT GTC TGC ACC CCA GAC C-3' (SEQ ID NO:8)

*Construct Human Secreted KLβ With C-Terminal His Tag:* The human secreted KLβ extracellular domain was obtained by PCR using pCMVHuKLβ-Flag as the template and the following primers:
Forward primer: 5'-GAA TTC CAC CAT GAA GCC AGG CTG TGC GGC AGG ATC TCC AG-3' (SEQ ID NO:9)
Reverse primer: 5'-GGC GCG CCG ACA AGG AAT AAG CAG ACA GTG CAC TCT G-3' (SEQ ID NO:10)

The resulting secreted PCR product was digested with EcoRI and AscI and ligated into pRK5_c-His (DNA540910) to obtain pRK5HuKLβΔTM-His (SEQ ID NO:50).

*Construct Human KLβ E416A and E693A:* The human KLβ E416A c-terminal flag construct (pCMVhKLβ-Flag E416A (SEQ ID NO:51)) was obtained by mutation of E416 to A416 in pCMVHuKLβ_Flag using the XL QuickChange Site-Direct Mutagenesis kit (Stratagene) and the following primers:
Forward primer: 5'-CCC TCG AAT CTT GAT TGC TGC GAA TGG CTG GTT CAC AGA CAG-3' (SEQ ID NO:11)
Reverse primer: 5'-CTG TCT GTG AAC CAG CCA TTC GCA GCA ATC AAG ATT CGA GGG-3' (SEQ ID NO: 12)

The human KLβ E693A c-terminal flag construct (pCMVhKLβ-Flag E693A (SEQ ID NO:52))was obtained by mutation of E693 to A693 in pCMVHuKLβ_Flag using the XL QuickChange Site-Direct Mutagenesis kit (Stratagene) and the following primers:
Forward primer: 5'-GCT CTG GAT CAC CAT CAA CGC GCC TAA CCG GCT AAG TGA C-3' (SEQ ID NO:13)
Reverse primer: 5'-GTC ACT TAG CCG GTT AGG CGC GTT GAT GGT GAT CCA GAG C-3' (SEQ ID NO:14)

### hKLβΔTM Conditioned Media

HEK 293 cells were transfected with an empty or a C-terminal his-tagged human Klotho beta extracellular domain (hKLβΔTM) containing expression vector. After transfection the cells were maintained in serum free PS25 media for 72-96 hours. The resulting media was filtered, supplemented to 40 mM HEPES pH 7.2, concentrated 4 fold and evaluated for hKLβΔTM content by Western blot using a monoclonal antibody that binds hKLβ (R&D Systems, catalog no. MAB3738)

### Coprecipitation

The concentrated control or hKLβΔTM conditioned medium were supplemented with Triton-X 100 (Calbiochem) to a final concentration of 0.5% and incubated with or without 0.5 µg/ml FGFR-IgG (R&D Systems, catalog numbers as follows: FGFR1 alpha IIIb, 655-FR-050; FGFR1 alpha III, 658-FR-050; FGFR1 beta IIIb, 765-FR-050; FGFR1 beta IIIc, 661-FR-050; FGFR2 alpha III, 663-FR-050; FGFR2 alpha IIIc, 712-FR-050; FGFR2 beta IIIb, 665-FR-050; FGFR2 beta IIIc, 684-FR-050; FGFR3 IIIb, 1264-FR-050; FGFR3 IIIc, 766-FR-050; FGFR4, 685-FR-050), 0.5 µg/ml heparin (Sigma), 1 µg/ml FGF19 (R&D Systems), 10 µl EZ view Red Protein A affinity gel (Sigma) at 4 °C for 18 h. The affinity matrix was centrifuged and washed three times with PBS/0.5% Triton-X100 and once with PBS. The pellet was eluted with SDS-PAGE sample buffer containing 5% β-mercapto ethanol and analyzed by Western blot using an anti- Klothoβ monoclonal antibody (R&D Systems catalog no. MAB3738), an FGF19 antibody (clone 1A6; Genentech Inc.), an anti-FGFR4 antibody (clone 8G11; Genentech Inc.) or a HRP conjugated anti-human IgG antibody (Jackson Immunochemical).

### Cell Culture and Stable Cell Lines

HEK 293 (ATCC Accession No. CRL-1573), HepG2 (ATCC Accession No. HB-8065) and Hep 3B (ATCC Accession No. HB-8064) cells were obtained from American Type Culture Collection and maintained in F-12:DMEM mix (50:50) supplemented with 10% fetal bovine serum (FBS) and 2 mM L-glutamine. HEK 293 cells stably expressing empty vector, human fibroblast growth factor receptor 4 (hFGFR4) R388-V5, hFGFR4 G388-V5, human Klotho b-FLAG (hKLβ-FLAG), hFGFR4 R388-V5 and hKLβ-FLAG, or hFGFR4 R388-V5 and hKLβ-FLAG were created and grown in selective medium containing 500 µg/ml geneticin and 2.5 µg/ml puromycin.

### Time Course of Gene Expression in FGF19 Treated Cells

Cells were plated at 10⁶ cells/well in a 6-well plate and grown overnight in complete media. Cells were washed twice with PBS once with serum free medium and maintained overnight in serum free media. The next day cells were treated with 20ng/ml FGF19 for 1, 2, 4, 6, or 24 hours and at the end of treatment the RNA was extracted using the RNeasy kit (Qiagen). The relative expression level of c-fos, c-jun, junB and KLβ was determined by Taqman.

### Semi-Quantitative RT-PCR

Total RNA was extracted using RNeasy kit (Quiagen). Specific primers and fluorogenic probes were used to amplify and quantitate gene expression. The gene specific signals were normalized to the RPL19 housekeeping gene. Triplicate sets of data were averaged for each condition. All TaqMan RT-PCR reagents were purchased from Applied Biosystems (Foster City, California). Data are presented as mean +/- SEM. Taqman Primers and probes (report dye was FAM and quencher dye was TAMRA). Primer sequences were as follows:

| | |
|---|---|
| RPL19 forward primer: | AGC GGA TTC TCA TGG AAC A (SEQ ID NO:15) |
| RPL19 reverse primer: | CTG GTC AGC CAG GAG CTT (SEQ ID NO:16) |
| RPL19 probe: | TCC ACA AGC TGA AGG CAG ACA AGG (SEQ ID NO:17) |
| hKLβ forward primer: | GCA GTC AGA CCC AAG AAA ATA CAG A (SEQ ID NO:18) |
| hKLβ reverse primer: | CCC AGG AAT ATC AGT GGT TTC TTC (SEQ ID NO:19) |
| hKLβ reverse probe: | TGC ACT GTC TGC TTA TTC CTT GT (SEQ ID NO:20) |
| hc-fos forward primer: | CGA GCC CTT TGA TGA CTT CCT (SEQ ID NO:21) ) |
| hc-fos reverse primer: | GGA GCG GGC TGT CTC AGA (SEQ ID NO:22) |
| hc-fos probe: | CCC AGC ATC ATC CAG GCC CAG (SEQ ID NO:23) |
| hjunb forward primer: | AGT CCT TCC ACC TCG ACG TTT (SEQ ID NO:24) |
| hjunb reverse primer: | AAT CGA GTC TGT TTC CAG CAG AA (SEQ ID NO:25) |
| hjunb probe: | AGC CCC CCC TTC CAC TTT TT (SEQ ID NO:26) |
| hc-jun forward primer: | CGT TAA CAG TGG GTG CCA ACT (SEQ ID NO:27) |
| hc-jun reverse primer: | CCC GAC GGT CTC TCT TCA AA (SEQ ID NO:28) |
| hc-jun probe: | ATG CTA ACG CAG CAG TTG CAA ACA (SEQ ID NO:29) |
| mKLβ forward: | TGT GGT GAG CGA AGG ACT GA (SEQ ID NO:30) |
| mKLβ reverse: | GGA GTG GGT TGG GTG GTA CA (SEQ ID NO:31) |
| mKLβ probe: | CTG GGC GTC TTC CCC ATG G (SEQ ID NO:32) |
| mc-fos forward: | CCT GCC CCT TCT CAA CGA (SEQ ID NO:33) |
| mc-fos reverse: | TCC ACG TTG CTG ATG CTC TT (SEQ ID NO:34) |
| mc-fos probe: | CCA AGC CAT CCT TGG AGC CAG T (SEQ ID NO:35) |
| mFGFR4 forward: | CGC CAG CCT GTC ACT ATA CAA A (SEQ ID NO:36) |
| mFGFR4 reverse: | CCA GAG GAC CTC GAC TCC AA (SEQ ID NO:37) |
| mFGFR4 probe: | CGT TTC CCT TTG GCC CGA CAG TTC T (SEQ ID NO:38) |

### siRNA Transfection in HEPG2 and HEP3B Cells:

KLβ and GAPDH siRNA oligos were obtained from Dharmacon.

**KLβ siRNA:**

| | |
|---|---|
| Duplex1: | Sense: 5'-GCACACUACUACAAACAGAUU-3' (SEQ ID NO:39) |
| | Anti-sense: 5'-UCUGUUUGUAGUAGUGUGCUU-3' (SEQ ID NO:40) |
| Duplex2: | Sense: 5'-GCACGAAUGGUUCCAGUGAUU-3' (SEQ ID NO:41) |
| | Anti-sense: 5'-UCACUGGAACCAUUCGUGCUU-3' (SEQ ID NO:42) |
| Duplex3: | Sense: 5'-CGAUGGAUAUAUUCAAAUGUU-3' (SEQ ID NO:43) |
| | Anti-sense: 5'-CAUUUGAAUAUAUCCAUCGUU-3' (SEQ ID NO:44) |
| Duplex4: | Sense: 5'-UGAAAUAACCACACGCUAUUU-3' (SEQ ID NO:45) |
| | Anti-sense: 5'-AUAGCGUGUGGUUAUUUCAUU-3' (SEQ ID NO:46) |
| GAPDH | siRNA: Sense: 5'-UGGUUUACAUGUUCCAAUA-3' (SEQ ID NO:47) |
| | Antisense: 5'-UAUUGGAACAUGUAAACCA-3'(SEQ ID NO:48) |

The various siRNA duplex were transfected using the DharmaFECT transfection kit (Dharmacon) and following the manufacturer's recommended protocol. Twenty-four hours post-transfection, the cells were washed twice with PBS and once with serum free media and maintained in serum free media overnight. The following days the cells were treated with 20 ng/ml FGF19 (R&D Systems) for 2 hours. The RNA samples were prepared with a RNeasy kit (Qiagen). The relative levels of c-fos, RPL19, and KLβ expression were determined by Taqman.

### In vitro KLβ Antibody Treatment

HEPG2 cells were plated at 10⁶ cells/well in a 6-well plate and grown overnight in complete media. Cells were washed 3 times with serum free media containing 0.1% BSA and maintained in the same media overnight. The next day the cells were treated with 10 µg/ml KLβ specific polyclonal antibody (R&D Systems; cat# AF2619) or a control antibody for 4 h. The cells were then treated with 100 ng/ml FGF19 for 2 hours and the RNA was extracted using the RNeasy kit (Qiagen). The relative expression level of c-fos was determined by Taqman.

### Co-Immunoprecipitation

HEK 293 cells transiently (24-48 hour transfection) or stably expressing empty vector, hFGFR4 R388-V5, hKLβ-FLAG, or hFGFR4 R388-V5 and hKLβ-FLAG were lysed with RIPA lysis buffer (PBS containing 1% Triton X-100 and 1% NP-40) supplemented with Complete EDTA-free protease inhibitor cocktail (Roche). Total protein concentrations were determined by BCA protein assay (Pierce). Equal amounts of total protein for each sample lysate were immunoprccipitated with EZview Red anti-FLAG M2 affinity gel (Sigma) or anti-V5 agarose affinity gel (Sigma) at 4°C overnight. Immunoprecipitated proteins were washed three times with TBST and then incubated without or with 1 mg/ml FGF-19 (R&D Systems) in F-12:DMEM mix (50:50) supplemented with 0.5% FBS and 0.5% Triton X-100 at 4°C for 3 hours. The beads were then washed once with Krebs-Ringer-HEPES (KRH) buffer containing 1% Triton X-100 and three times with KRH buffer. Immunoprecipitated proteins were eluted by addition of 1X NuPAGE LDS sample buffer (Invitrogen) and boiling for 5 minutes. Eluted proteins in sample buffer were recovered and 1X NuPAGE reducing agent (Invitrogen) was added and then boiled for 10 minutes. Protein samples were electrophoresed on 4-12% NuPAGE Bis-Tris gels followed by transfer to nitrocellulose membranes and subjected to subsequent immunoblot analyses using anti-hKLβ antibody (1 mg/ml, R&D Systems ; catalog no. MAB3738), anti-hFGFR4 antibody (1 mg/ml, clone 8G1, Genentech), or anti-FGF-19 antibody (1 mg/ml, clone 1A6, Genentech). Signal was detected using ECL Plus substrate (GE Healthcare).

### FGF Pathway Activation

HEK 293 cells transiently (24 hour transfection) or stably expressing empty vector, hFGFR4 R388-V5, hFGFR4 G388-V5, hKLβ-FLAG, hFGFR4 R388-V5 and hKLβ-FLAG, or hFGFR4 G388-V5 and hKLβ-FLAG were treated with 0, 1, 10, or 100 ng/ml of FGF-19 (R&D Systems), 20 ng/ml of FGF-1 (FGF acidic, R&D Systems), or 20 ng/ml epidermal growth factor (Roche) for 10 minutes. Cells were lysed with RIPA lysis buffer (Upstate) supplemented with Complete EDTA-free protease inhibitor cocktail (Roche) and phosphatase inhibitor cocktails 1 and 2 (Sigma). Total protein concentrations were determined by BCA protein assay (Pierce). For analysis of FRS2 and ERK1/2 phosphorylation, equal amounts of total protein were electrophoresed on 10% NuPAGE Bis-Tris gels (Invitrogen) followed by transfer to nitrocellulose membranes and subsequent immunoblot analyses using anti-phospho-FRS2 antibody (1:1,000, Cell Signaling Technology) or anti-ERK1/2 antibody (1:1,000, Cell Signaling Technology). For detection of total FRS2 and ERK1/2, membranes were stripped and reprobed with anti-FRS2 antibody (1 mg/ml, Upstate Biotechnology) or anti-ERK1/2 antibody (1:1,000, Cell Signaling Technology). Signal was detected using ECL Plus substrate (GE Healthcare).

### In vivo Experiments

All animal protocols were approved by an Institutional Animal Care and Use Committee. Five- to six-week-old Female FVB mice were obtained from Charles River Laboratories. The mice were provided standard feed and water *ad libitum* until 12 hours prior to treatment at which time feed was removed. Mice were injected intravenously with vehicle (PBS) or with 1 mg/kg FGF19. When indicated, mice were injected intravenously with 2.2 mg/kg KLβ antibody (R&D Systems; cat# AF2619) 3, 9 or 24 hours before the intravenous FGF19 inoculation. After 30 min, mice from all groups were sacrificed and tissue samples were collected, frozen in liquid nitrogen, and stored at - 70°C. Total RNA from frozen tissue samples was prepared using the RNAeasy kit (Qiagen). Groups of 3-5 animals were analyzed for each condition. Data are presented as the mean ± SEM and were analyzed by the Student t-test.

### In silico expression analysis

For KLβ and FGFR4 expression analysis, plots are based on normalized gene expression data extracted from the BioExpress™ database (Gene Logic, Inc., Gaithersburg, MD, USA). The KLβ expression reported here corresponds to the signal given by the probe number 244276_at and 204579_at, respectively, in human tissues analyzed on Affymetrix GeneChips. The bold center line indicates the median; the box (white, normal; gray, tumor) represents the interquartile range between the first and third quartiles. The distribution of the values for a given samples is indicated by broken lines. The human sample collection has been described by the originator of the BioExpress™ database (Shen-Ong GL et al. Cancer Res 2003; 63: 3296-301. The respective hybridizations were performed on Affymetrix HG-U133P oligonucleotide chips (Affymetrix, Inc., Santa Clara, CA, USA): Briefly, these chips are based on 25-mer oligonucleotides and allow the detection of more than 33,000 well-substantiated human genes, with probe sets of 11 oligonucleotides used per transcript.

### Example 1: KLβ extracellular domain and FGF19 binding specificities are restricted to FGFR4.

FGF19, heparin and FGFRs-Fc fusion proteins were incubated in conditioned medium containing KLβΔTM. The protein interactions were then analyzed by co-precipitation. KLβ and FGF19 co-associated only with FGFR4 and were pulled down only with FGFR4-Fc (Fig. 1A). These data indicated that the binding specificities of KLβ extracellular domain and FGF19 are restricted to FGFR4 and that KLβ extracellular domain, FGF19 and FGFR4 likely form a tripartite complex.

### Example 2: KLβ binding to FGFR4 is promoted by FGF19 and heparin.

To evaluate the contribution of each component to complex formation, the co-precipitation assay was used. Control or KLβΔTM containing conditioned medium was incubated in the presence or the absence of FGFR4-Fc, FGF19, and heparin. In the absence of heparin and FGF19, no interaction was detected between KLβ and FGFR4-Fc (Fig. 1B). Heparin was a weak promoter, whereas FGF19 was a strong promoter of the KLβ-FGFR4 interaction. The maximal level of stabilization of the KLβ-FGFR4-Fc interaction occurred in the presence of both heparin and FGF19. Conversely, FGF19 binding to FGFR4-Fc required the presence of heparin or KLβ. The maximal level of FGF19 binding to FGFR4 occurred when both heparin and KLβ were included in the reaction. These data demonstrate that KLβ is sufficient to support FGF19 binding to FGFR4. It also shows that KLβ promotes the previously demonstrated, heparin-dependent interaction of FGF19 with FGFR4 (Xie et al (1999) Cytokine 11 :729-35). Therefore, each individual component contributes to the stability of the FGF 19-FGFR4-KLβ-heparin complex.

Compared with the paracrine FGF family members, FGF19 has a low heparin-binding affinity that allows it to act in an endocrine fashion without being tethered to the pericellular proteoglycan of the secreting cells (Choi, M et al (2006) Nat Med 12: 1253-5; Harmer, NJ et al (2004) Biochem 43:629-40; Inagaki, Y et al (2005) Cell Metab 2:217-25; Lundasen, T et al (2006) J Intern Med 260:530-6). The topology of the FGF19 heparin-binding site prevents FGF19 from forming hydrogen bonds with heparin when FGF19 is bound to its receptor Goetz, R et al (2007) Mol Cell Biol. 27:3417-28). Therefore KLβ may act as an FGFR4 co-receptor that stabilizes the weak FGF19-FGFR4-heparin interaction.

### Example 3: Interaction of transfected FGFR4 and KLβ at the cell surface.

To test whether KLβ and FGFR4 also participate in the formation of a complex with FGF19 and heparin at the cell surface we evaluated the ability of FGFR4 and KLβ to immunoprecipitate FGF19 from lysates of transiently or stably transfected cells in the presence or the absence of heparin. No detectable FGF19 was co-precipitated from lysates of cells transfected with only a control or an FGFR4-expression vector (Fig 1C and 1D). FGFR4 and KLβ pulled down FGF19 from KLβ-transfected cell lysate only in the presence of heparin indicating that KLβ transfection promotes heparin-dependent FGF19 binding to the endogenous HEK293 FGFR4.

These findings suggest that FGFR4 and KLβ exist as a preformed complex and that their interaction is not enhanced by FGF19.

### Example 4: Interaction of endogenous FGFR4 and KLβ at the cell surface.

The KLβ and FGFR4 transmembrane domains could directly interact with each other or promote the interaction of the proteins by tethering them to the cell surface. To test the hypothesis that KLβ and FGFR4 form a constitutive complex at the cell surface, we evaluated whether KLβ co-immunoprecipitated with FGFR4 from HEPG2 cell lysates in the absence of FGF or heparin. Incubation of HEPG2 cell lysates with an antibody against FGFR4 immunoprecipitated FGFR4 and KLB, whereas no protein was immunoprecipitated with the control antibody (Fig. 1E), showing that the endogenous transmembrane KLβ and FGFR4 form a constitutive heparin- and ligand-independent complex. The KLβ-FGFR4 cell surface complex might alter the heparin- and ligand- induced receptor dimerization that was previously described for paracrine FGFs (Plonikov, A (1999) Cell 98:641-50; Schlessinger, J et al (2000) Mol Cell 6:743-50). These data indicate that endogenous KLβ and FGFR4 interact at the cell surface. Applicants note that Figure 4 of Applicant's prior application, US Serial No. 60/909,699, filed April 2, 2007, (corresponding to Fig. 1E of the present application) shows a molecular weight mark at 150kDa, while Figure 1E shows a 130kDa molecular weight mark. The mislabeling of Figure 4 of the '699 application was an inadvertent obvious error, as KLβ is known to be a 130-kDa protein (see, e.g., Ito et al., Mech. Dev. 98 (2000) 115-1 19).

### Example 5: FGF19 binding to FGFR4 and KLβ at the cell surface.

To test whether KLβ and FGFR4 also participate in the formation of a complex with FGF19 and heparin at the cell surface we evaluated the ability of FGFR4 and KLβ to immunoprecipitate FGF 19 from lysates of transiently or stably transfected cells in the presence or the absence of heparin. No detectable FGF19 was co-precipitated from lysates of cells transfected with only a control or an FGFR4-expression vector (Fig 1C and 1D). FGFR4 and KLβ pulled down FGF19 from KLB-transfected cell lysate only in the presence of heparin indicating that KLβ transfection promotes heparin-dependent FGF19 binding to the endogenous HEK293 FGFR4.

In lysates from of KLβ- and FGFR4-co-transfected cells, KLβ and FGFR4 readily pulled down FGF19. This interaction was further stabilized in the presence of heparin (Fig 1 C and 1D). These data show that KLβ is required for FGF 19 binding to the cell surface FGFR4 and that heparin promotes this interaction. In addition, FGFR4 and KLβ readily interacted in a heparin- and ligand-independent manner in co-transfected cells. This result contrasts with the heparin- and ligand-dependent complex formation observed with the secreted chimeric FGFR4 and KLβ proteins. This discrepancy indicates a role for the KLβ and FGFR4 transmembrane domains in the complex formation.

These findings suggest that FGF19 binds to KLβ and FGFR4 at the cell surface and that heparin enhances this interaction.

### Example 6: FGF19 represses Klotho beta expression

The effect of FGF19 on KLβ expression in various cell lines was evaluated. We detected high KLβ expression in liver cell lines (HepG2 and Hep3B) but only traces in kidney (HEK293) or colon cell lines (SW620 and Colo205; Fig. 3A). Upon exposure to FGF19, KLβ expression in HepG2 and Hep3B was gradually repressed, to 50-60% the level of unexposed cells after 6 hours, and remained at this level for at least 24 hours. Exposure to FGF19 did not affect KLβ expression levels in the other cell lines. The repression of KLβ expression by FGF19 might be a regulatory negative feedback mechanism in liver cells.

### Example 7: FGF19 is required for FGF19 downstream modulation of gene expression

Because a plethora of physiological and pathological stimuli induce the genes of the Fos and Jun family in a wide variety of cell types we tested whether FGF19 modulates c-Fos, JunB, and c-Jun expression in various cell lines (Ashida, R et al (2005) Inflammapharmacology 13: 113-25; Hess, J et al (2004) Biochemistry 43:629-40; Shaulian, E et al (2002) Nat Cell Biol 4:E131-6). FGF19 upregulated c-Fos and JunB expression, as well as c-Jun expression to a lesser extent, in KLβ-expressing cells (HepG2 and HEP3B; Fig, 3 B-D). The induction c-Fos, JunB and c-Jun expression occurred within 30 minutes of exposure to FGF19 and in most cases expression returned to basal levels after 6 hours. JunB expression remained elevated for at least 24 hours in HEP3B cells (Fig. 3C).

These data indicate that FGF19 induced c-Fos, Junb and c-Jun expression only in KLβ expressing cells.

### Example 8: KLβ knock-down inhibits FGF19-dependent c-fos induction.

To test whether KLβ promotes FGF19 signaling and c-Fos induction in HEP3B and HEPG2 cells, we inhibited KLβ expression using specific siRNAs. KLβ siRNA transfection significantly reduced KLβ mRNA and protein expression in HEP3B (Fig. 3G and E) and HEPG2 cells (Fig. 5A). The individual transfection of four different KLβ siRNAs significantly attenuated FGF19-mediated FRS2 and ERK1/2 phosphorylation (Fig. 3F, 5B). In addition, transfection of HEP3B cells with KLβ siRNA transfection inhibited FGF19 mediated c-Fos induction by 62%-80%, compared to the control cells (Fig. 3G). Similarly, transfection of HEPG2 cells with KLβ siRNA reduced the levels of FGF19-dependent FRS2 and ERK1/2 phosphorylation as well as c-Fos induction as compared with the control cells (Fig. 5C). These results indicate that KLβ expression is required for FGF19-dependent pathway activation and c-Fos induction. These results indicate that KLβ expression is required for FGF 19-dependent c-Fos induction.

To further assess the participation of KLβ in FGF 19-mediated c-Fos induction, we transfected HEK293 cells with empty, KLβ-, FGFR4-, or a combination of KLβ- and FGFR4-expression vectors and exposed the cells to FGF19. Only cells transfected with both KLβ and FGFR4 expression vectors induced c-Fos in response to FGF19 (Fig. 3H). These data indicate that KLβ is required for FGF19 pathway activation and modulation of gene regulation.

### Example 9: Treatment with an anti-KLβ antibody inhibits FGF19-dependent c-Fos Induction.

To further evaluate the contribution of KLβ to the FGF19-dependent c-Fos induction, HEPG2 cells were treated with an anti-KLβ antibody (raised against mouse KLβ, but cross-reactive with human KLβ) or a control antibody before treatment of the cells with FGF19. Anti-KLp antibody treatment reduced FGF19-dependent c-Fos induction by 80% whereas the control antibody did not show any significant effect (FIG. 6).

These results demonstrate that targeting KLβ with a specific antibody inhibits FGF19 activity.

### Example 10: KLβ is required for FGF19 signaling.

To test whether KLβ contributes to the activation of the FGF19 signaling pathway, we evaluated the effects of FGF19 on FGFR substrate 2 (FRS2) and extracellular-signal regulated kinase-1 and -2 (ERK1/2) phosphorylation in KLβ- and/or FGFR4-transfected HEK 293 cells, as well as controls. FGF19 did not promote FRS2 or ERK1/2 phosphorylation in cells transfected with an empty expression vector (Fig. 2). HEK 293 cells transfected with KLβ or FGFR4 only showed a weak, dose-dependent increase in ERK1/2 phosphorylation but no detectable FRS2 phosphorylation following exposure to FGF19. The co-transfection of FGFR4 with KLB promoted FGF19 signaling in HEK 293 cells, indicated by the robust, dose-dependent increase of both FRS2 and ERK1/2 phosphorylation. One possible explanation for this effect is that local, high concentrations of FGF19 and FGFR4 allow for weak signaling in the absence of KLβ. However, because FGF19 has an endocrine function and its average circulating concentration is 193 ± 36 pg/mL (range of 49-590 pg/mL), this is unlikely (Lundasen, T et al (J Intern Med 260:530-6). Therefore KLβ's robust induction of FGF19 signaling is likely to occur at physiological concentrations of FGF19.

### Example 11: KLβ active site mutation inhibits FGF19 pathway activation.

The requirement of KLβ for FGF19-stimulated activity was assessed by detection of downstream signaling (i.e. phospho-FRS2 and -ERK1/2) in HEK 293 cells transfected with wild-type (wt) KLβ or KLβ mutants. Wt KLβ-transfected cells showed detectable phospho-FRS2 and phospho-ERK1/2 upon treatment with 100 ng/ml FGF19 (Fig. 7). When treated with the same FGF19 dose, the KLβ E416A mutant (containing a glutamate to alanine mutation in one of the putative active sites; see Ito et al. (2000) Mech. Dev. 98 (1-2):115-119 for a description of this residue) did not show detectable phosphorylation of either FRS2 or ERK1/2. Thus, a mutation in the E416 putative active site of KLβ eliminated FGFR4 downstream signaling, suggesting that KLβ enzymatic activity is required for FGFR4 signaling.

FGF19 treatment of cells transfected with the KLβ E693A mutant ((containing a glutamate to alanine mutation in one of the putative active sites ; see Ito et al., supra, for a description of this residue) showed similar levels of phosphorylation of phospho-FRS2 or phospo-ERK1/2 to FGF19-treated cells expressing wt KLβ (Fig. 7). Thus, a mutation in the E693 putative active site of KLβ did not affect KLβ activity. Therefore only the E416 putative active site of KLβ is required for the FGF19 dependent stimulation of FRS2 and ERK1/2 phosphorylation. KLβ protein expression was detected to demonstrate that cells transfected with vectors expressing wt or mutant KLβ expressed equivalent amounts of protein.

These findings corroborate the finding that FGF19 signaling through FGFR4 is enhanced by the presence of KLβ and further suggest that KLβ enzymatic activity is required for FGFR4 signaling.

### Example 12: Distribution of KLβ expression in mouse tissues in vivo.

To test the hypothesis that FGF19 acts only on tissues that express both FGFR4 and KLβ, we first surveyed KLβ and FGFR4 distribution in various mouse organs using semi-quantitative RT-PCR. The relative mRNA levels represent the relative fold expression, compared with brain (organ with the lowest expression surveyed). KLβ was predominantly expressed in liver (Fig. 4C). Lower levels of KLβ expression were also found in adipose and colon. Additional organs tested showed marginal expression levels of KLβ. FGFR4 was highly expressed in liver, lung, adrenals, kidney and colon (Fig. 4D). Lower levels of FGFR4 expression were also observed in intestine, ovaries, muscle and pancreas. The overall KLβ and FGFR4 distribution in mouse tissues was similar that of human tissues. However, contrary to the findings in human tissues, no consistent KLβ or FGFR4 expression could be detected in mouse pancreas. In addition, a low level of KLβ expression was detected in mouse colon, whereas no expression was found in the corresponding human tissues. These differences might be attributable to species- and/or strain-specific tissue distribution. These data indicate that liver is the only mouse organ in which KLβ and FGFR4 are highly co-expressed.

### Example 13: FGF19 acts specifically on mouse liver.

To determine the FGF19 specific site of action, we compared the levels of c-Fos expression in organs of mice injected with FGF19 with those of mice injected with PBS (controls). We chose to monitor the c-Fos response to FGF19 because c-Fos expression is ubiquitous and its induction is sensitive to FGF19 stimulation. C-Fos expression was 1300-fold higher in the livers of mice injected with FGF19 compared with the livers of mice injected with PBS (Fig. 4E). The FGF19-dependent c-Fos induction was at least 150-fold lower in all other organs tested. The activity of FGF19 in liver was confirmed by a 98% inhibition of CYP7A1 expression (Fig. 4F). These data demonstrate that FGF19 acts specifically in liver, the only mouse organ that expresses high levels of both KLβ and FGFR4.

Together, the data shown in Examples 12 and 13 demonstrate that FGF19 requires KLβ for binding to FGFR4, intracellular signaling, and downstream gene modulation. Most importantly, the requirement for KLβ restricts the endocrine activity of FGF19 to tissues that express both FGFR4 and KLβ. The liver-specific activity of FGF19 is supported by this molecular mechanism. These data demonstrate that the liver is a major site of action of FGF19 in the mouse.

### Example 14: Treatment with anti-KLβ antibody in vivo inhibits FGF19-dependent c-Fos induction in mouse liver.

To evaluate the KLβ requirement for FGF19 activity in vivo, FGF19-dependent c-Fos induction was determined in liver of mouse treated with a KLβ antibody for different lengths of time. Treatment of mice with 2.5 mg/kg of KLβ antibody 3, 9 or 24 hours before a FGF19 injection reduced the liver specific FGF19-mediated c-Fos induction by 58%, 68% and 91% respectively (Fig. 8).

These data indicate that KLβ is required for FGF19 signaling through FGFR4 in vivo. In addition, the data further demonstrate that KLβ-specific antibodies can be used to inhibit FGF19 activity in vivo.

### Example 15: Analysis of KLβ and FGFR4 expression in normal and cancer tissues.

KLβ and FGFR4 expression were evaluated in a variety of human tissues by analyzing the BioExpress database (Gene Logic, Inc., Gaithersburg, MD, USA). In decreasing order of signal intensity, KLβ was expressed in adipose, liver, pancreas, and breast tissues (Fig. 4A). In decreasing order of signal intensity, FGFR4 was expressed in liver, lung, gall bladder, small intestine, pancreas, colon, lymphoid, ovary and breast tissues (Fig. 4B). These data show that KLβ expression is restricted to only a few tissues, whereas FGFR4 expression is more widely distributed. A high level of co-expression of KLβ and FGFR4 was observed only in liver and pancreas. Because the expression of KLβ and FGFR4 are required for FGF19 activity, this finding suggests that liver and pancreas are the major organs in which they are active. Marginal levels of KLβ and FGFR4 expression were also observed in breast tissues. KLβ was highly expressed in adipose tissues but the absence of FGFR4 precludes the function of FGF19 in this tissue. It is possible that KLβ promotes the activity of other endocrine FGF family members with different FGFR binding specificity in adipose tissues. Notably, FGF21 regulates glucose uptake by acting specifically on adipose tissue by an endocrine mechanism (Kharitonenkov, A, et al (2005) J Clin Invest 115:1627-35). Because of its low heparin-binding affinity, FGF21 might require KLβ to signal through FGFR1 and FGFR2 (Goetz, R et al (2007) Mol Cell Biol 27:3417-28; Kharitonenkov, supra).

KLβ expression and FGFR4 were also evaluated in a variety of cancer tissues. KLβ expression is generally reduced in other cancer tissues compared to the relevant normal tissue (Fig. 9). In decreasing order of signal intensity, FGFR4 is expressed in the following cancer tissues: liver, colon, stomach, esophagus, kidney, testis, small intestine, pancreas, ovary and breast (Fig. 10). These data show that FGFR4 expression is widely distributed and that its normal expression is altered in cancer.

### Example 16: Discussion

In this study we have provided evidence that FGF 19 requires KLB for binding to FGFR4, intracellular signaling, and down-stream gene modulation. However, the reason for such a requirement is still unclear. Compared with the paracrine FGF family members, FGF19 has a low heparin-binding affinity that allows it to act in an endocrine fashion without being tethered to the pericellular proteoglycan of the secreting cells (3, 6, 8, 15). The topology of the FGF19 heparin-binding site prevents FGF19 from forming hydrogen bonds with heparin when FGF 19 is bound to its receptor (5). Therefore KLB may act as an FGFR4 co-receptor that stabilizes the weak FGF 19-FGFR4-heparin interaction.

In addition, we have shown that FGFR4 and KLβ readily interacted in a heparin- and ligand-independent manner at the cell surface. This result contrasts with the heparin- and ligand-dependent complex formation observed with the secreted chimeric FGFR4 and KLβ proteins. This discrepancy may indicate a role for the KLβ and FGFR4 transmembrane domains in the complex formation. The KLβ and FGFR4 transmembrane domains could directly interact with each other or promote the interaction of the proteins by tethering them to the cell surface. The KLβ -FGFR4 cell surface complex might alter the heparin- and ligand-induced receptor dimerization that was described previously for paracrine FGFs (18, 20).

We found high expression of KLβ in adipose tissue. However, the absence of FGFR4 expression precludes FGF 19 activity in this tissue. Therefore, it is possible that KLβ promotes the activity of other endocrine FGF family members with different FGFR binding specificity in adipose tissues. Notably, KLβ was recently shown to be required for FGF21 adipose-specific activity (17). Because of its low heparin-binding affinity, FGF21 might require KLβ to signal through FGFR1 and FGFR2 (6, 12).

Together, these data demonstrate that FGF19 requires KLβ for binding to FGFR4, intracellular signaling, and downstream gene modulation. Most importantly, the requirement for KLβ restricts the endocrine activity of FGF19 to tissues that express both FGFR4 and KLβ. The liver-specific activity of FGF19 is supported by this molecular mechanism.

### Partial reference list

1. Ashida, R., et al. 2005. Inflammopharmacology 13:113-25.
2. Chiang, J. Y. 2004. J Hepatol 40:539-51.
3. Choi, M., A.et al. 2006. Nat Med 12:1253-5.
4. Fu, L., et al. 2004. Endocrinology 145:2594-603.
5. Goetz, R., A. et al. 2007. Mol Cell Biol 27:3417-28.
6. Harmer, N. J., et al. 2004. Biochemistry 43:629-40.
7. Hess, J., P. Angel, and M. Schorpp-Kistner. 2004. J Cell Sci 117:5965-73.
8. Inagaki, T., M. et al. 2005. Cell Metab 2:217-25.
9. Ito, S., T. et al. 2005. J Clin Invest 115:2202-8.
10. Ito, S. et al. 2000. Mech Dev 98:115-9.
11. Jelinek, D. F.et al. 1990. J Biol Chem 265:8190-7.
12. Kharitonenkov, A. et al. 2005. J Clin Invest 115:1627-35.
13. Kurosu, H. et al. 2006. J Biol Chem 281:6120-3.
14. Li, Y. C. et al. 1990. J Biol Chem 265:12012-9.
15. Lundasen, T.et al. 2006. J Intern Med 260:530-6.
16. Nicholcs, K. et al 2002. Am J Pathol 160:2295-307.
17. Ogawa, Y. ct al. (2007) Proc. Natl. Acad. Sci. U. S. A. 104, 7432-7437
18. Plotnikov, A. N. et al 1999. Cell 98:641-50.
19. Russell, D. W. 2003. The enzymes, regulation, and genetics of bile acid synthesis. Annu Rev Biochem 72:137-74.
20. Schlessinger, J. et al2000. Mol Cell 6:743-50.
21. Shaulian, E., and M. Karin. 2002. Nat Cell Biol 4:E131-6.
22. Tomlinson, E. et al. 2002. Endocrinology 143:1741-7.
23. Trauner, M., and J. L. Boyer. 2004. Curr Opin Gastroenterol 20:220-30.
24. Urakawa, I. et al. 2006. Nature 444:770-4.
25. Winer, J. et al. 1999. Anal Biochem 270:41-9.
26. Xie, M. H. et al. 1999. Cytokine 11:729-35.

The following numbered clauses represent aspects and are part of the description
1. A method for modulating a disorder associated with expression or activity of KLβ, the method comprising administering an effective amount of a KLβ modulator to an individual in need of such treatment.
2. A method for treating a disorder associated with expression or activity of KLβ, the method comprising administering an effective amount of a KLβ antagonist to an individual in need of such treatment.
3. The method of 2, wherein the disorder is a tumor, a cancer, or a cell proliferative disorder.
4. The method of 3, wherein the tumor, cancer, or cell proliferative disorder is hepatocellular carcinoma, pancreatic cancer, non-small cell lung cancer, breast cancer, or colorectal cancer.
5. The method of 2, wherein the disorder is a liver disorder.
6. The method of 5, wherein the liver disorder is cirrhosis.
7. The method of 2, wherein the disorder is hypoglycemia, cholestasis or dysregulation of bile acid metabolism.
8. The method of 2, wherein the disorder is wasting.
9. The method of 8, wherein the individual further has a tumor, a cancer, and/or a cell proliferative disorder.
10. The method of any of 1-9, further comprising administering to the subject an effective amount of a second medicament, wherein the KLβ antagonist is a first medicament
11. The method of 10, wherein the second medicament is an antibody, a chemotherapeutic agent, a cytotoxic agent, an anti-angiogenic agent, an immunosuppressive agent, a prodrug, a cytokine, a cytokine antagonist, cytotoxic radiotherapy, a corticosteroid, an anti-emetic, a cancer vaccine, an analgesic, or a growth-inhibitory agent.
12. The method of 11, wherein the second medicament is tamoxifen, letrozole, exemestane, anastrozole, irinotecan, cetuximab, fulvestrant, vinorelbine, erlotinib, bevacizumab, vincristine, imatinib, sorafenib, lapatinib, or trastuzumab.
13. The method of 10, wherein the second medicament is administered prior to or subsequent to the administration of the KLβ antagonist.
14. The method of 10, wherein the second medicament is administered concurrently with the KLβ antagonist.
15. The method of any of 2-14, wherein the KLβ antagonist is an antibody
16. The method of 15, wherein the antibody is a monoclonal antibody.
17. The antibody of 15, wherein the antibody is selected from the group consisting of a chimeric antibody, a humanized antibody, an affinity matured antibody, a human antibody, and a bispecific antibody.
18. The antibody of 15, wherein the antibody is an antibody fragment.
19. The antibody of 15, wherein the antibody is an immunoconjugate.
20. The method of any of 2-19, further wherein a biological sample of the individual expresses (i) KLβ, (ii) KLβ and FGF, (iii) KLβ and FGFR, or (iv) KLβ, FGF and FGFR.
21. The method of 20, wherein the biological sample expresses KLβ.
22. The method of 20, wherein the biological sample expresses KLβ and FGFR4.
23. The method of 20, wherein the biological sample expresses KLβ, FGF19 and FGFR4.
24. The method of 1, wherein the KLβ modulator is a KLβ agonist.
25. The method of 24, wherein the disorder is obesity or an obesity-related condition.
26. The method of 25, wherein the obesity-related condition is diabetes mellitus, cardiovascular disease, insulin resistance, hypertension, hypercholesterolemia, thromboembolic disease, atherosclerosis, dyslipidemia, osteoarthritis, gallbladder disease, osteoarthritis, and sleep apnea and other respiratory disorders.
27. The method of 25, wherein the disorder is hyperglycemia.
28. A method for inducing an increase in insulin sensitivity, the method comprising administration of an effective dose of a KLβ agonist to an individual in need of such treatment.
29. A method for reducing total body mass, the method comprising administration of an effective dose of a KLβ agonist to an individual in need of such treatment.
30. A method for reducing at least one of triglyceride and free fatty acid levels, the method comprising administration of an effective dose of a KLβ agonist to an individual in need of such treatment.
31. A method for detection of KLβ, the methods comprising detecting KLβ in a sample.
32. The method of 31, wherein the sample is a biological sample from a cancer, a tumor or a cell proliferative disorder.
33. The method of 32, wherein the biological sample expresses FGF or FGFR.
34. A method of identifying a candidate inhibitor substance that inhibits KLβ binding to FGFR, said method comprising: (a) contacting a candidate substance with a first sample comprising FGFR, FGF and KLβ, and (b) comparing amount of FGFR biological activity in the sample with amount of FGFR biological activity in a reference sample comprising similar amounts of KLβ, FGF and FGFR as the first sample but that has not been contacted with said candidate substance, whereby a decrease in amount of FGFR biological activity in the first sample compared to the reference sample indicates that the candidate substance is capable of inhibiting KLβ binding to FGFR.
35. A method of determining whether a candidate substance promotes KLβ biological activity, said method comprising: (a) contacting a candidate substance with a first sample comprising FGFR and KLβ, and (b) comparing amount of FGFR biological activity in the sample with amount of FGFR biological activity in a reference sample comprising similar amounts of KLβ and FGFR as the first sample but that has not been contacted with said candidate substance, whereby an increase in amount of FGFR biological activity in the first sample compared to the reference sample indicates that the candidate substance is capable of promoting KLβ binding to FGFR.

## Claims

1. A KLβ agonist for use in a method of treating an obesity-related condition, wherein the obesity-related condition is diabetes mellitus or insulin resistance, the method comprising administering the KLβ agonist to an individual in need of such treatment, wherein the KLβ agonist is an anti-KLβ antibody.

2. The KLβ agonist for use according to claim 1 in a method of treating diabetes mellitus.

3. The KLβ agonist for use according to claim 1 or claim 2, wherein the anti-KLβ antibody is a monoclonal antibody.

4. The KLβ agonist for use according to any one of claims 1 to 3, wherein the anti-KLβ antibody is a chimeric antibody, a humanized antibody, an affinity matured antibody or a human antibody.

5. The KLβ agonist for use according to any one of the preceding claims, wherein the anti-KLβ antibody is a bispecific antibody.

6. The KLβ agonist for use according to any one of the preceding claims, wherein the anti-KLβ antibody is an antibody fragment.

7. Use of a KLβ agonist in the preparation of a medicament for use in a method of treating an obesity-related condition, wherein the obesity-related condition is diabetes mellitus or insulin resistance, the method comprising administering the KLβ agonist to an individual in need of such treatment, wherein the KLβ agonist is an anti-KLβ antibody.

8. The use according to claim 7, wherein the medicament is for use in a method of treating diabetes mellitus.

9. The use according to claim 7 or claim 8, wherein the anti-KLβ antibody is a monoclonal antibody.

10. The use according to any one of claims 7 to 9, wherein the anti-KLβ antibody is a chimeric antibody, a humanized antibody, an affinity matured antibody or a human antibody.

11. The use according to any one of claims 7 to 10, wherein the KLβ agonist is a bispecific antibody.

12. The use according to any one of claims 7 to 11, wherein the anti-KLβ antibody is an antibody fragment.

## Patentansprüche

1. KLβ-Agonist zur Verwendung in einem Verfahren zur Behandlung eines Adipositas-bezogenen Leidens, wobei das Adipositas-bezogene Leiden Diabetes mellitus oder Insulinresistenz ist, wobei das Verfahren das Verabreichen des KLβ-Agonisten an ein Individuum, das eine solche Behandlung benötigt, umfasst, wobei der KLβ-Agonist ein Anti-KLβ-Antikörper ist.

2. KLβ-Agonist zur Verwendung nach Anspruch 1 in einem Verfahren zur Behandlung von Diabetes mellitus.

3. KLβ-Agonist zur Verwendung nach Anspruch 1 oder 2, wobei der Anti-KLβ-Antikörper ein monoklonaler Antikörper ist.

4. KLβ-Agonist zur Verwendung nach einem der Ansprüche 1 bis 3, wobei der Anti-KLβ-Antikörper ein chimärer Antikörper, ein humanisierter Antikörper, ein affinitätsgereifter Antikörper oder ein menschlicher Antikörper ist.

5. KLβ-Agonist zur Verwendung nach einem der vorangegangenen Ansprüche, wobei der Anti-KLβ-Antikörper ein bispezifischer Antikörper ist.

6. KLβ-Agonist zur Verwendung nach einem der vorangegangenen Ansprüche, wobei der Anti-KLβ-Antikörper ein Antikörperfragment ist.

7. Verwendung eines KLβ-Agonisten zur Herstellung eines Medikaments zur Verwendung in einem Verfahren zur Behandlung eines Adipositas-bezogenen Leidens, wobei das Adipositas-bezogene Leiden Diabetes mellitus oder Insulinresistenz ist, wobei das Verfahren das Verabreichen des KLβ-Agonisten an ein Individuum, das eine solche Behandlung benötigt, umfasst, wobei der KLβ-Agonist ein Anti-KLβ-Antikörper ist.

8. Verwendung nach Anspruch 7, wobei das Medikament der Verwendung in einem Verfahren zur Behandlung von Diabetes mellitus dient.

9. Verwendung nach Anspruch 7 oder 8, wobei der Anti-KLβ-Antikörper ein monoklonaler Antikörper ist.

10. Verwendung nach einem der Ansprüche 7 bis 9, wobei der Anti-KLβ-Antikörper ein chimärer Antikörper, ein humanisierter Antikörper, ein affinitätsgereifter Antikörper oder ein menschlicher Antikörper ist.

11. Verwendung nach einem der Ansprüche 7 bis 10, wobei der KLβ-Agonist ein bispezifischer Antikörper ist.

12. Verwendung nach einem der Ansprüche 7 bis 11, wobei der Anti-KLβ-Antikörper ein Antikörperfragment ist.

## Revendications

1. Agoniste de KLβ destiné à être utilisé dans un procédé de traitement d'un état associé à l'obésité, dans lequel l'état associé à l'obésité est le diabète sucré ou la résistance à l'insuline, le procédé comprenant l'administration de l'agoniste de KLβ à un individu ayant besoin d'un tel traitement, dans lequel l'agoniste de KLβ est un anticorps anti-KEβ.

2. Agoniste de KLβ destiné à être utilisé selon la revendication 1 dans un procédé de traitement du diabète sucré.

3. Agoniste de KLβ destiné à être utilisé selon la revendication 1 ou la revendication 2, dans lequel l'anticorps anti-KLβ est un anticorps monoclonal.

4. Agoniste de KLβ destiné à être utilisé selon l'une quelconque des revendications 1 à 3, dans lequel l'anticorps anti-KLβ est un anticorps chimérique, un anticorps humanisé, un anticorps mature par affinité ou un anticorps humain.

5. Agoniste de KLβ destiné à être utilisé selon l'une quelconque des revendications précédentes, dans lequel l'anticorps anti-KLβ est un anticorps bispécifique.

6. Agoniste de KLβ destiné à être utilisé selon l'une quelconque des revendications précédentes, dans lequel l'anticorps anti-KLβ est un fragment d'anticorps.

7. Utilisation d'un agoniste de KLβ dans la préparation d'un médicament destiné à être utilisé dans un procédé de traitement d'un état associé à l'obésité, dans lequel l'état associé à l'obésité est le diabète sucré ou la résistance à l'insuline, le procédé comprenant l'administration de l'agoniste de KLβ à un individu ayant besoin d'un tel traitement, dans lequel l'agoniste de KLβ est un anticorps anti-KLβ.

8. Utilisation selon la revendication 7, dans laquelle le médicament est destiné à être utilisé dans un procédé de traitement du diabète sucré.

9. Utilisation selon la revendication 7 ou la revendication 8, dans laquelle l'anticorps anti-KLβ est un anticorps monoclonal.

10. Utilisation selon l'une quelconque des revendications 7 à 9, dans laquelle l'anticorps anti-KLβ est un anticorps chimérique, un anticorps humanisé, un anticorps mature par affinité ou un anticorps humain.

11. Utilisation selon l'une quelconque des revendications 7 à 10, dans laquelle l'agoniste de KLβ est un anticorps bispécifique.

12. Utilisation selon l'une quelconque des revendications 7 à 11, dans laquelle l'anticorps anti-KLβ est un fragment d'anticorps.
